# EUROPEAN PATENT SPECIFICATION

(11) **EP 1 079 832 B1**
(45) Date of publication and mention of the grant of the patent: **30.11.2005**
(21) Application number: 99922119.5
(22) Date of filing: 21.04.1999
(51) Int. Cl.: A61K 31/455, A61K 31/465, A61K 31/44

(54) **USE OF VITAMIN PP COMPOUNDS**
VERWENDUNG VON VITAMIN PP VERBINDUNGEN
UTILISATION DE COMPOSES DE VITAMINE PP

(30) Priority: 22.04.1998 DE 19818044
(43) Date of publication of application: 07.03.2001
(73) Proprietor: Klinge Pharma GmbH, D-81673 München (DE)
(72) Inventor: BIEDERMANN, Elfi, D-85591 Vaterstetten (DE); HASMANN, Max, D-82061 Neuried (DE); LÖSER, Roland, D-82340 Feldafing (DE); RATTEL, Benno, D-81249 Munich (DE); REITER, Friedemann, D-85640 Putzbrunn (DE); SCHEIN, Barbara, D-85375 Neufahrn (DE); SCHEMAINDA, Isabel, D-80804 Munich (DE); SEIBEL, Klaus, D-82166 Gräfelfing (DE); VOGT, Klaus, D-81669 Munich (DE); WOSIKOWSKI, Katja, D-85586 Poing (DE)
(74) Representative: HOFFMANN EITLE
(86) International application number: PCT/EP1999/002686
(87) International publication number: WO 1999/053920

(56) References cited:
- SHNEIDER A B: "[Anti-ischemic heart protection using thiamine and nicotinamide ]. Protivoishemischeskaia zashchita serdtsa s pomoshch'iu tiamina i nikotinamida." PATOLOGICHESKAIA FIZIOLOGIIA I EKSPERIMENTALNAIA TERAPIIA, (1991 JAN-FEB) (1) 9-10. , XP002114703
- KLAIDMAN, LORI K. ET AL: "Nicotinamide as a precursor for NAD+ prevents apoptosis in the mouse brain induced by tertiary-butylhydroperoxide" NEUROSCI. LETT. (1996), 206(1), 5-8 , XP002114704
- POZZILLI P. ET AL: "Vitamin E and nicotinamide have similar effects in maintaining residual beta cell function in recent onset insulin-dependent diabetes. [Erratum to document cited in CA128:18654]" EUR. J. ENDOCRINOL. (1997), 137(5), 558 , XP002114705

## Description

The invention relates to the use of vitamin PP compounds ("PP" = pellagra preventing activity) and/or compounds with anti-pellagra activity such as, for example, nicotinic acid (niacin), and nicotinamide (niacinamide, vitamin PP, vitamin B3) for the reduction, elimination or prevention of side-effects of different degrees of severity as well as for the neutralization of acute side-effects in immunosuppressive or cancerostatic chemotherapy or diagnostics, especially with substituted pyridine carboxamides as tumor inhibitors, optionally in combination with radiation therapy.

Nicotinamide is the most important building block for synthesis and maintenance of cellular stores of nicotinamide adenine dinucleotide (NAD). In gram amounts, it has a relatively low toxicity (*Zackheim, H.S., Vasily, D.B. and Hastings C.W.: Reactions to niacinamide. J. Am. Acad. Dermatol.* *4**: 736-737, 1981*), whereby its therapeutic priority in clinical use is to be seen in treatment of pellagra; see *Green, R.G.: Subclinical pellagra: its diagnosis and treatment* in *Schizophrenia* *2**: 70-79, 1970.* Nicotinamide was further used in order to cure the symptoms of schizophrenia *Greenbaum, G.H.C.: An evaluation of niacinamide in the treatment of childhood schizophrenia* or *Am. J. Psychiatry 127: 89-92, 1970*). Clinical tests for preventing type I diabetes mellitus have been undertaken; see *Elliot, R.B. and Chase, H.P.: Prevention of delay of type I (insulin-dependent) diabetes mellitus in children using nicotinamide* in *Diabetologica* *35**: 362-365, (1991)*.

The NAD synthesized from nicotinic acid or nicotinamide is important for the cellular production of adenosine triphosphate (ATP), the maintenance of the redox potential of cells and as a substrate for poly (ADP-ribose)-polymerase (PARP, EC 2.4.2.30) and the other reactions carried out by ADP-Ribose (*Althaus, F.R. and Richter, C.: ADP-ribosylation of Proteins: Enzymology and Biological Significance, Springer Verlag, Berlin 1987*)*.* PARP is an enzyme which binds to DNA whose activity plays a role in DNA-repair, cell proliferation, apoptosis, cell differentiation and attending to the genome.

For some time, the administration of nicotinamide for increasing the sensitivity of tumors to chemotherapy and/or radiation therapy has been tested in pre-clinical and clinical studies (*Kaanders, J.H.A.M., Pop, L.A.M., Marres, H.A.M., van der Maazen, R.W.M., van der Kogel, A. J. and van Daal, W.A. J. : Radiotherapy with carbogen and nicotinamide in head and neck cancer: feasibility and toxicity; Radiother. Oncol. 37: 190-198, 1995*).

Two effects are discussed in the literature as a mechanism for the sensitizing properties of nicotinamide:
First, nicotinamide could lead to greater blood circulation through tumor tissue (*Horsman, M.R., Chaplin, D.J. and Brown, J.M.: Tumor radiosensitization by nicotinamide: a result of improved blood perfusion and oxygenation. Rad. Res.* *1181**: 139 -150, 1989; Chaplin, D.J., Horsman, M.R. and Aoki, D.S.: Nicotinamide, fluosol DA and carbogen: a strategy to reoxygenate acutely and chronically hypoxic cells in vivo; Br. J. Cancer* *63**: 109 - 113, 1991; Powell, M.E.B., Hill, S.A., Saunders, M.I., Hoskin, P.J. and Chaplin, D.J.: Human* *Tumor Blood Flow Is Enhanced by Nicotinamide and Carbogen Breathing. Cancer Research* *57**: 5261-5264, 1997*). Hypoxic, under-supplied tumor cells could be stimulated to grow through better supply with oxygen (especially by the inhalation of high-percent oxygen/CO₂ gas mixed carbogene) and nutrients which, in turn, would make them more susceptible to the actions of cytostatic agents and radiation.
Second, nicotinamide could cause an inhibition of the enzyme PARP, the latter playing a role in the repair of DNA damage as mentioned above. (*Ben Hur, E., Utsumi, H. and Elkind, M.M.: Inhibitors of poly(ADP-ribose) synthesis enhance X-ray killing of log-phase chinese hamster cell in Radiat. Res.* *97**: 546-555, 1984; George, A.M., Lunec, J., Cramp, W.A., Brennan, S., Lewis, P. D. and Wish, W. J. D. : The effects of benzamide ADP-ribosyl transferase inhibitors on cell survival and DNA strand-break repair in irradiated mammalian cells* in *Int. J. Radiat. Biol. 49: 783 - 798, 1986*)*.* This enzyme uses the cellular pyridine nucleotide NAD as a substrate and transfers its ADP-ribose portion to other proteins whose function is regulated in this manner. Therewith, the DNA damage produced by cytostatic agents or radiation should lead more quickly to cell death by inhibition of PARP.

Previously, a series of medicaments have already been proposed for combating the considerable side-effects arising from cancerostatic chemotherapy such as, for example, dopamine antagonists, for example, chlorpromazine, droperidol, fluphenazine and other compounds, such as antihistamines such as, for example, buclizine, cyclizine, dimenhydrinate or promethazine. Benzquinamide and tronabinol or diphenidol or trimethobenzamide have also been used for this purpose for the therapy of side-effects of cancerostatic agents. However, massive side-effects can be ascribed to these medicinals themselves as is well-known by experts. These agents for the alleviation of side-effects such as, for example, haloperidol, methoclopramide, ondansetrone or domperidone are mainly used against severe nausea and vomiting caused by chemotherapy. Folic acid represents a very specific remedy which can only be administered in connection with methotrexate for reduction of its side-effects in the case of chemotherapeutic treatment.

Therefore, it is an object to find a method which helps to suppress neutralize or at least reduce, to a degree which is easy to bear for patients, the side-effects of different degrees of severity associated with chemotherapy, especially of tumors, or with the chemotherapeutic immunosuppression or with prevention of metastasis formation or anti-proliferative treatment with pharmaceuticals.

A further object is also to entirely eliminate the side-effects which are already reduced in comparison to customary tumor inhibitors of the compounds according to the general formula (I), and, in individual cases, to still further diminish particular individual patient sensitivity.

Surprisingly, it has now been found that, in contrast to the effects and/or effectiveness-increasing activities of nicotinic acid or nicotinic acid amide in connection with radiation therapy discussed at the beginning, these compounds can be used for preventing side-effects or neutralizing the cell growth-inhibiting activity of cancerostatic and/or tumor-inhibiting chemotherapeutic agents based on an opposing effect which has not yet been explained scientifically.

In an unexpected manner, the inventors have now established that by using compounds with vitamin PP activity, side-effects of different degrees of severity which are associated with cancerostatic chemotherapy, for example, with the tumorstatic agents according to the general formula (I) can be reduced, eliminated or prevented. The use according to the invention and/or the medicaments according to the invention have the advantage that the compounds with vitamin PP activity used for the elimination of side-effects, especially according to the general formula (II) to (Vb), themselves do not cause new side-effects. This finding is surprising in view of the previous art in the field of cancerostatic chemotherapy. The unexpected effect could be traced back to a new biochemical mechanism of which no decisive clarity exists at the present time point.

Hence, the invention relates to the use of compounds with vitamin PP activity as a cyto-protective agents for the prevention, reduction or elimination of side-effects and/or neutralization of the effects of cancerostatic agents or immunosuppressive agents, especially of compounds from the series of substituted pyridylalkane, pyridylalkene and pyridylalkine acid amides of the general formula (I), in diagnostics or cytostatic or immunosuppressive chemotherapy, optionally in combination with radiation therapy.

Furthermore, it relates to the use of vitamins of the group PP as cyto-protective agents production of medicaments for prevention, reduction or elimination of side-effects and/or neutralization of the effects of cancerostatic agents or immunosuppressive agents, especially compounds from the series of substituted pyridylalkane, pyridylalkene and pyridylalkine acid amides in diagnostics or cytostatic, anti-proliferative or immunosuppressive chemotherapy, optionally in combination with radiation therapy. The use according to the invention in the application of compounds of the general formula (I) can also be in connection with their administration as an abortive agent.

According to a very preferred embodiment of the invention there is provided a pharmaceutical composition comprising:
(a) at least one cancerostatic or immunosuppressive agent selected from the group consisting of compounds of formula (I) : 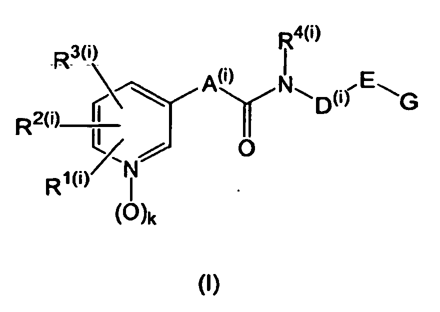 wherein:
   - **R**^{**1(i)**}: is selected from
   hydrogen, halogen, cyano, C₁-C₆-alkyl, C₃-C₆-alkenyl, C₂-C₆-alkinyl, trifluoromethyl, hydroxy, C₃-C₈-cycloalkyl, C₁-C₆-hydroxyalkyl, C₁-C₆-alkoxy, C₃-C₆-alkenyloxy, C₃-C₆-alkinyloxy, benzyloxy, C₁-C₇-alkanoyloxy, C₁-C₇-alkoxycarbonyloxy, C₁-C₆-alkylthio, C₃-C₆-alkenylthio, C₃-C₆-alkinylthio, C₃-C₈-cycloalkyloxy, C₃-C₈-cycloalkylthio, C₂-C₇-alkoxycarbonyl, aminocarbonyl, C₂-C₇-alkylaminocarbonyl, C₃-C₁₃-dialkylaminocarbonyl, carboxy, phenyl, phenoxy, phenylthio, pyridyloxy, pyridylthio, and **NR**^{**5(i)**}**R**^{**6(i)**}**,** wherein
   **R**^{**5(i)**} and **R**^{**6(i)**} are selected independently from each other from hydrogen, C₁-C₆-alkyl, C₃-C₆-alkenyl, C₃-C₆-alkinyl, benzyl and phenyl;
   - **R**^{**2(i)**}: is selected from
   hydrogen, halogen, cyano, C₁-C₆-alkyl, trifluoromethyl, hydroxy, C₁-C₆-alkoxy, benzyl and C₁-C₆-alkanoyloxy; or
   - **R**^{**1(i)**}: and **R**^{**2(i)**} when they are adjacent optionally form a bridge selected from
   -(CH₂)₄-, -(CH=CH)₂- and -CH₂O-**CR**^{**7(i)**}**R**^{**8(i)**}-O-, wherein
   **R**^{**7(i)**} and **R**^{**8(i)**} are selected independently from each other from hydrogen and C₁-C₆-alkyl;
   - **R**^{**3(i)**}: selected is from
   hydrogen, halogen, C₁-C₆-alkyl, trifluoromethyl and C₁-C₆-hydroxyalkyl;
   - **R**^{**4(i)**}: is selected from
   hydrogen, hydroxy, C₁-C₆-alkyl, C₃-C₆-alkenyl, C₃-C₆-alkinyl, C₃-C₆-cycloalkyl, C₁-C₆-alkoxy and benzyloxy;
   - **k**: is 0 or 1,
   - **A**^{**(i)**}: is selected from
   C₁-C₆-alkylene, optionally substituted one- to three-fold by C₁-C₆-alkyl, C₁-C₃-alkoxy, hydroxy, fluorine, or phenyl,
   C₂-C₆-alkylene, wherein a methylene unit is isosterically replaced by O, S, **NR**^{**9(i)**}**,** CO, SO or SO₂, wherein, with the exception of CO, the isosteric substitution is not adjacent to the amide group, and **R**^{**9(i)**} is selected from hydrogen, C₁-C₆-alkyl, C₃-C₆-alkenyl, C₃-C₆-alkinyl, C₃-C₆-acyl and C₁-C₆-alkanesulfonyl,
   1,2-cyclopropylene,
   C₂-C₆-alkenylene, optionally substituted one to three-fold by C₁-C₆-alkyl, hydroxy, C₁-C₃-alkoxy, fluorine, cyano or phenyl,
   C₄-C₆-alkadienylene, optionally substituted once or twice by C₁-C₃-alkyl, fluorine, cyano or phenyl,
   1,3,5-hexatrienylene, optionally substituted by C₁-C₆-alkyl, fluorine, cyano or phenyl, and
   ethinylene;
   - **D**^{**(i)**}: is selected from
   C₁-C₁₂-alkylene, optionally substituted once or twice by C₁-C₆-alkyl, hydroxy, C₁-C₆-alkoxy, or phenyl,
   C₂-C₁₂-alkenylene or C₄-C₁₂-alkadienylene, optionally substituted once or twice by C₁-C₆-alkyl, hydroxy, C₁-C₆-alkoxy, or phenyl, wherein one double-bond can optionally occur to ring **E** in the case that ring **E** is linked over a C-atom,
   C₃-C₁₂-alkinylene or C₄-C₁₂-alkeninylene, optionally substituted once or twice by C₁-C₆-alkyl, hydroxy, C₁-C₆-alkoxy or phenyl, and
   C₁-C₁₂-alkylene, C₂-C₁₂-alkenylene or C₃-C₁₂-alkinylene, wherein, one to three methylene units, with the exception of the (G)-terminal methylene group in the case that E represents a bond, are isosterically replaced by O, S, **NR**^{**10(i)**}**,** CO, SO or SO₂, wherein
   **R**^{**10(i)**} has the same meaning as **R**^{**9(i)**}**,** but is selected independently thereof;
   - **E**: is selected from **E**^{**1(i)**}**, E**^{**2(i)**}**, E**^{**3**}**, E**^{**4**}**, E**^{**5**} and **E**^{**6**}**,** wherein
   - **E**^{**1(i)**}: is 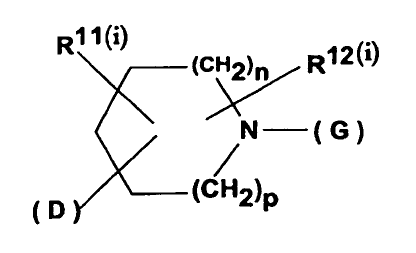
   - **E**^{**2(i)**}: is 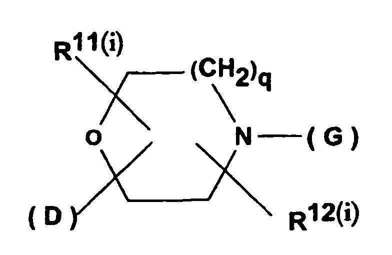
   - **E**^{**3**}: is 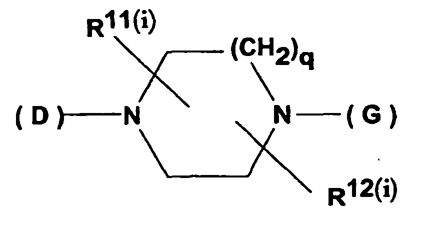
   - **E**^{**4**}: is 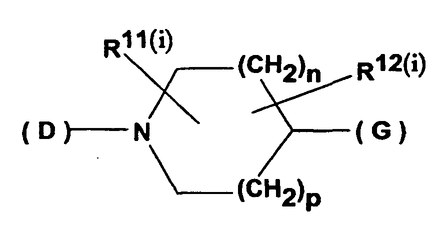
   - **E**^{**5**}: is 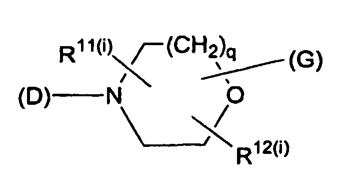 and
   - **E**^{**6**}: represents a single or double bond,
   wherein the heterocyclic rings **E**^{**1(i)**} to **E**^{**5**} optionally have a double bond,
   - **n** and **p**: are, independently from each other, 0, 1, 2, or 3 with the proviso that, **n + p ≤** 4,
   - **q**: is 1, 2 or 3;
   - **R**^{**11(i)**}: is selected from
   hydrogen, C₁-C₆-alkyl, hydroxy, hydroxymethyl, carboxy and C₂-C₇-alkoxycarbonyl,
   - **R**^{**12(i)**}: is selected from
   hydrogen, C₁-C₆-alkyl and an oxo group adjacent to a nitrogen atom, or
   - **R**^{**11(i)**} and **R**^{**12(i)**}**,**: optionally together, form a C₁-C₃-alkylene bridge under formation of a bicyclic ring system, and
   - **(a)**: in the case that E represents **E**^{**1(i)**}**, E**^{**2(i)**}**,** or **E**^{**3**} the substituent G optionally is selected from **G**^{**1(i)**}**, G**^{**2(i)**}**, G**^{**3(i)**}**, G**^{**4(i)**} and **G**^{**5(i)**}**,** wherein
   - **G**^{**1(i)**}: is 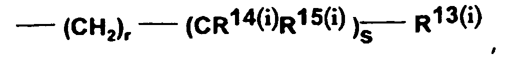 wherein
   - **r**: is 0 to 3 and
   - **s**: is 0 or 1,
   - **R**^{**13(i)**}: is selected from
   hydrogen, C₁-C₆-alkyl, C₃-C₆-alkenyl, C₃-C₆-alkinyl and C₃-C₈-cycloalkyl;
   saturated or unsaturated, four to seven-membered heterocycles which contain one or two hetero-atoms selected from N, S and O;
   benzyl, phenyl;
   monocyclic aromatic five or six-membered heterocycles which contain one to three hetero-atoms selected from N, S and O, and are either bound directly or over a methylene group;
   anellated bi- and tricyclic aromatic or partially hydrogenated carbocyclic ring systems with 8 to 16, 17 or 18 ring atoms and at least one aromatic ring, wherein the linkage occurs either over an aromatic or a hydrogenated ring and either directly or over a methylene group;
   anellated bi- and tricyclic aromatic or partially hydrogenated heterocyclic ring systems with 8 to 16, 17 or 18 ring atoms and at least one aromatic ring, wherein one to three ring atoms are selected from N, S and O and the linkage occurs either over an aromatic or a hydrogenated ring and either directly or over a methylene group;
   - **R**^{**14(i)**}: has the same meaning as **R**^{**13(i)**}**,** but is independently selected therefrom;
   - **R**^{**15(i)**}: is selected from
   hydrogen, hydroxy, methyl, benzyl, phenyl,
   monocyclic aromatic five or six-membered heterocycles which contain one to three hetero-atoms selected from N, S and O and are either bound directly or over a methylene group;
   anellated bi- and tricyclic aromatic or partially hydrogenated carbocyclic ring systems with 8 to 16, 17 or 18 ring atoms and at least one aromatic ring, wherein the linkage occurs either over an aromatic or a hydrogenated ring and either directly or over a methylene group;
   anellated bi- and tricyclic aromatic or partially hydrated heterocyclic ring systems with 8 to 16, 17 or 18 ring atoms and at least one aromatic ring, wherein one to three ring atoms are selected from N, S and O and the linkage occurs either over an aromatic or a hydrogenated ring and either directly or over a methylene group;
   - **G**^{**2(i)**}: is selected from 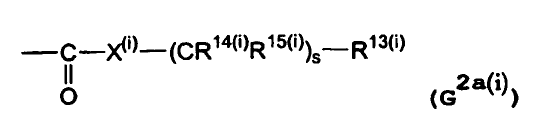 and 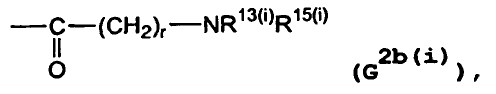 wherein **r, s** and the substituents **R**^{**13(i)**}**, R**^{**14(i)**} and **R**^{**15(i)**} have the above meanings,
   or the group

   -**NR**^{**13(i)**}**R**^{**15(i)**}

   is a nitrogen-containing heterocycle bound over the nitrogen atom, which nitrogen-containing heterocycle is selected from
   saturated and unsaturated monocyclic, four to eight-membered heterocycles, which aside from the essential nitrogen atom, optionally contain one or two further hetero-atoms selected from N, S and O,
   and
   saturated and unsaturated bi- or tricyclic, anellated or bridged heterocycles with 8 to 16, 17 or 18 ring atoms, which aside from the essential nitrogen atom, optionally contain one or two further hetero-atoms selected from N, S and O,
   - **X**^{**(i)**}: is selected from
   methylene, ethylene, ethenylene, propylene, and C₃-C₇-cycloalkylene, or represents a bond;
   - **G**^{**3(i)**}: is

   -**SO**_{**2**}-**(CH**_{**2**}**)**_{**r**}-**R**^{**13(i)**}**,**

   wherein **r** and **R**^{**13(i)**} have the above meanings,
   - **G**^{**4(i)**}: is 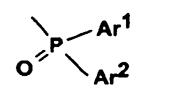 wherein
   **Ar**^{**1**} and **Ar**^{**2**} are selected independently from each other from phenyl, pyridyl and naphthyl;
   - **G**^{**5(i)**}: is

   **-COR**^{**16(i)**}

   wherein
   - **R**^{**16(i)**}: is selected from
   trifluoromethyl, C₁-C₆-alkoxy, C₃-C₆-alkenyloxy, and benzyloxy,
   - **(b)**: in the case that **E** is **E**^{**4**} or **E**^{**5**}**,**
   then **G** optionally is **G**^{**1(i)**}**, G**^{**2(i)**}**, G**^{**6(i)**}**, G**^{**7**} or **G**^{**8**}**,**
   wherein **G**^{**1(i)**} and **G**^{**2(i)**} have the above meanings and
   - **G**^{**6(i)**}: is

   **=(C)**_{**u**}**R**^{**13(i)**}**R**^{**15(i)**}**,**

   wherein **R**^{**13(i)**} and **R**^{**15(i)**} have the above meanings and
   - **u**: is 0 or 1,
   or when **u** = 1, then **R**^{**13(i)**} and **R**^{**15(i)**} together with the carbon atom to which they are attached form a ring system selected from
   C₃-C₈-cycloalkyl,
   saturated, four to seven-membered heterocycles which optionally contain one or two hetero-atoms, selected from N, S and O;
   anellated bi- and tricyclic partially hydrogenated carbocyclic ring systems with 8 to 16, 17 or 18 ring atoms and at least one aromatic ring;
   anellated bi- and tricyclic partially hydrogenated heterocyclic ring systems with 8 to 16, 17 or 18 ring atoms and at least one aromatic ring, wherein one to three ring atoms are selected from N, S and O;
   or when u = 0 then **R**^{**13(i)**} and **R**^{**15(i)**} together with the carbon atom of ring **E** to which they are attached form a ring system selected from
   C₃-C₈-cycloalkyl,
   saturated, four to seven-membered heterocycles which contain one or two hetero-atoms, selected from N, S and O;
   anellated bi- and tricyclic partially hydrogenated carbocyclic ring systems with 8 to 16, 17 or 18 ring atoms and at least one aromatic ring; and
   anellated bi- and tricyclic partially hydrogenated heterocyclic ring systems with 8 to 16, 17 or 18 ring atoms and at least one aromatic ring, wherein one to three ring atoms are selected from N, S and O;
   - **G**^{**7**}: is selected from

   **-NR**^{**17(i)**}**-(CH**_{**2**}**)**_{**r**}**-(CR**^{**14(i)**}**R**^{**15(i)**}**)**_{**s**}**-R**^{**13(i)**} **(G**^{**7a**}**),**

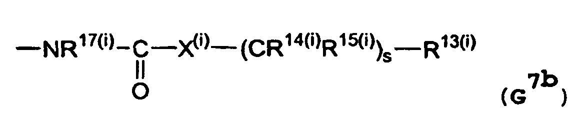

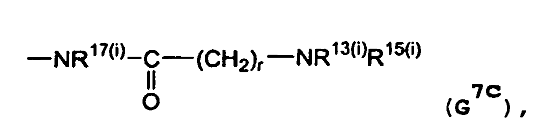

   **-NR**^{**17(i)**}**-SO**_{**2**}**-(CH**_{**2**}**)**_{**r**}**-R**^{**13(i)**} **(G**^{**7d**}**),**

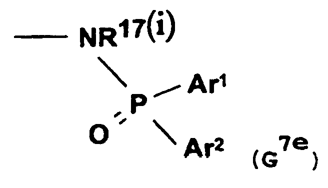

   and

   **-NR**^{**17(i)**}**-COR**^{**16(i)**} **(G**^{**7f**}**),**

   wherein **r, s, X**^{**(i)**}**,** the substituents **R**^{**13(i)**}**, R**^{**14(i)**}**, R**^{**15(i)**} and **R**^{**16(i)**} and the group

   **-NR**^{**13(i)**}**R**^{**15(i)**}

   have the above meanings, and
   - **R**^{**17(i)**}: has the same meanings as **R**^{**5(i)**}**,** but is selected independently thereof,
   - **Ar**^{**1**}: and **Ar**^{**2**} are selected independently from each other from phenyl, pyridyl and naphthyl;
   - **G**^{**8**}: is selected from 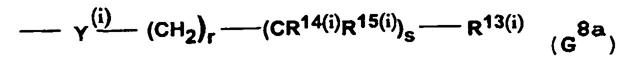 and 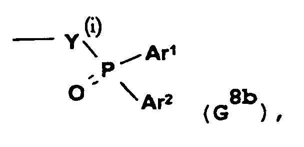 wherein
   **r, s** and the substituents **R**^{**13(i)**}**, R**^{**14(i)**}**, R**^{**15(i)**}**, Ar**^{**1**} and **Ar**^{**2**} have the above meanings, and
   - **Y**^{**(i)**}: is O or S;
   - **(c)**: in the case that the substituent **E** is **E**^{**6**}**,**
   then the substituent **G** optionally is selected from **G**^{**7d**}**, G**^{**7e**}**, G**^{**8b**}**, G**^{**9**}**, G**^{**10**}**, G**^{**11**}**, G**^{**12**}**,** and **G**^{**13**}**,** wherein **G**^{**7d**}**, G**^{**7e**} and **G**^{**8b**} have the above meanings and
   - **G**^{**9**}: is selected from

   -**(CR**^{**13(i)**}**R**^{**19**}**)**_{**s**}**-R**^{**18**} (**G**^{**9a**})

   and

   **-NR**^{**13(i)**}**R**^{**18**} (**G**^{**9b**}),

   wherein **s** and **R**^{**13(i)**} are defined as above, and
   - **R**^{**18**}: is selected from
   benzyl, diphenylmethyl, phenyl;
   monocyclic aromatic five and six-membered heterocycles which can contain one to three hetero-atoms selected from N, S and O and are either bound directly or over a methylene group;
   anellated bi- and tricyclic aromatic or partially hydrogenated carbocyclic ring systems with 8 to 16, 17 or 18 ring atoms and at least one aromatic ring, wherein the linkage occurs either over an aromatic or a hydrogenated ring and either directly or over a methylene group; and
   anellated bi- and tricyclic aromatic or partially hydrogenated heterocyclic ring systems with 8 to 16, 17 or 18 ring atoms and at least one aromatic ring, wherein one to three ring atoms are selected from N, S and O and the linkage occurs either over an aromatic or a hydrogenated ring and either directly or over a methylene group;
   - **R**^{**19**}: has the same meanings as **R**^{**13(i)**} but is selected independently thereof, and in addition can be hydroxy;
   or the group

   -**NR**^{**13(i)**}**R**^{**18**}

   optionally is a nitrogen-containing heterocycle bound over the nitrogen atom, which nitrogen-containing heterocycle is selected from
   anellated bi- and tricyclic aromatic or partially hydrogenated heterocyclic ring systems with 8 to 16, 17 or 18 ring atoms and at least one aromatic ring, which aside from the essential nitrogen atom, optionally contain one or two further hetero-atoms selected from N, S and O;
   - **G**^{**10**}: is

   **=CR**^{**13(i)**}**R**^{**18**} **(G**^{**10**}**)**

   bound to D by means of a double bond, wherein **R**^{**13(i)**} and **R**^{**18**} have the above meanings, or wherein **G**^{**10**}
   optionally is a ring system bound over the carbon atom, selected from
   anellated bi- and tricyclic partially hydrogenated carbocyclic ring systems with 8 to 16, 17 or 18 ring atoms and at least one aromatic ring; and
   anellated bi- and tricyclic partially hydrogenated heterocyclic ring systems with 8 to 16, 17 or 18 ring atoms and at least one aromatic ring, wherein one to three ring atoms optionally are selected from N, S and O;
   - **G**^{**11**}: is selected from

   -**NR**^{**17(i)**}**-(CH**_{**2**}**)**_{**r-**}**(CR**^{**13(i)**}**R**^{**19**}**)**_{**s**}**-R**^{**18**} **(G**^{**11a**}**),**

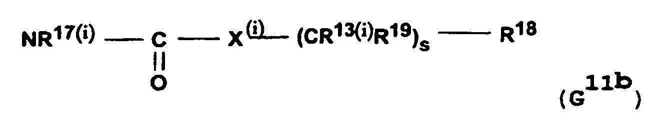

   and 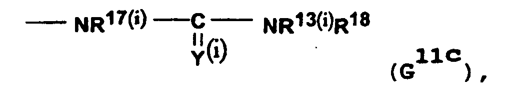 wherein **r, s, X**^{**(i)**}**, Y**^{**(i)**}**,** the substituents **R**^{**13(i)**}**, R**^{**17(i)**}**, R**^{**18**} and **R**^{**19**} and the group **-NR**^{**13(i)**}**R**^{**18**} have the above meanings;
   - **G**^{**12**}: is 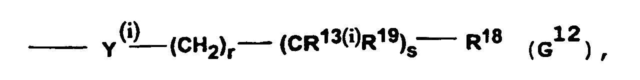 wherein **r, s, Y**^{**(i)**} and the substituents **R**^{**13(i)**}**, R**^{**18**} and **R**^{**19**} have the above meanings;
   - **G**^{**13**}: is selected from 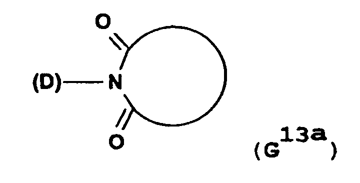 and 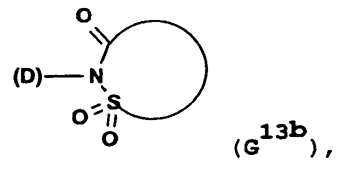 bound to D over the imide nitrogen atom, selected from
   saturated and unsaturated monocyclic imides with 5 to 7 ring atoms, which, aside from the essential imide nitrogen atom, optionally contains one or two further hetero-atoms selected from N, S and O;
   saturated, unsaturated and aromatic anellated, bi-, tri- or tetracyclic imides with 8 to 18 ring atoms, which, aside from the essential imide nitrogen atom, optionally contain one or two further hetero-atoms selected from N, S and O;
   saturated and unsaturated, bridged bi-, tri-, tetra- or pentacyclic imides with 8 to 22 ring-atoms, which, aside from the essential imide nitrogen atom, optionally contain one or two further hetero-atoms selected from N, S and O; and
   saturated-and unsaturated spirocyclic imides, optionally anellated one or two-fold, and with a total of 9 to 23 ring atoms, which, aside from the essential imide nitrogen atom, optionally contain one or two further hetero-atoms selected from N, S and O,
   wherein these cyclic imides optionally are substituted by one to five of the same or different groups selected independently from each other from
   halogen, cyano, C₁-C₆-alkyl, C₁-C₆-alkylidene, trifluoromethyl, C₃-C₈-cycloalkyl, C₃-C₈-cycloalkylidene, phenyl-C₁-C₃-alkyl, phenyl-C₁-C₃-alkylidene, diphenyl-C₁-C₃-alkyl, diphenyl-C₁-C₃-alkylidene, triphenylmethyl, phenyl, hydroxy, C₁-C₆-hydroxyalkyl, C₁-C₆-alkoxy, C₁-C₆-alkoxy entirely or partially substituted by fluorine, benzyloxy, phenoxy, naphthyloxy, mercapto, C₁-C₆-alkylthio, phenylthio, naphthylthio, pyridylthio, C₁-C₆-alkanesulfonyl, phenylsulfonyl, naphthylsulfonyl, pyridylsulfonyl, sulfo, carboxy, C₂-C₇-carboxyalkyl, C₃-C₇-carboxyalkenyl, C₂-C₇-alkoxycarbonyl, benzyloxycarbonyl, nitro, amino, C₁-C₆-aminoalkyl, mono-C₁-C₆-alkylamino di(C₁-C₆-alkyl)amino, phenylamino, phenyl-C₁-C₃-alkylamino, pyridylamino,
   saturated and unsaturated, four to seven-membered heterocycles which contain one or two hetero-atoms selected from N, S and O and are either bound directly or over a methylene group or a methine group,
   monocyclic aromatic five and six-membered heterocycles which contain one to three hetero-atoms, selected from N, S and O and are either bound directly or over methylene group or a methine group,
   anellated bicyclic, aromatic and partially hydrogenated carbocyclic ring systems with 8 to 12 ring atoms which are either bound directly over a methylene group or a methine group, and
   anellated bicyclic aromatic and partially hydrogenated heterocyclic ring systems with 8 to 12 ring atoms, wherein one to three ring atoms are selected from N, S and O and are either bound directly or over a methylene group or a methine group,
   wherein aromatic ring systems in the substituents **R**^{**1(i)**}**, R**^{**2(i)**}**, R**^{**4(i)**}**, R**^{**5(i)**}**, R**^{**6(i)**}**, R**^{**13(i)**}**, R**^{**14(i)**}**, R**^{**15(i)**}**, R**^{**16(i)**}**, R**^{**17(i)**}**, R**^{**18**}**, R**^{**19**}**, Ar**^{**1**} and **Ar**^{**2**}**,** in the groups **A**^{**(i)**} and **D**^{**(i)**}**,** in the ring Systems **=CR**^{**13(i)**}**R**^{**15(i)**}**, =CR**^{**13(i)**}**R**^{**18**}**, -NR**^{**13(i)**}**R**^{**15(i)**} and -**NR**^{**13(i)**}**R**^{**18(i)**} as well as substituents and/or substituents in the cyclic imides **G**^{**13**} optionally are independently substituted by one to three of the same or different groups, selected from
   halogen, cyano, C₁-C₆-alkyl, trifluoromethyl, C₃-C₈-cycloalkyl, benzyl, phenyl, hydroxy, C₁-C₆-hydroxyalkyl, C₁-C₆-alkoxy, C₁-C₆-alkoxy entirely or partially substituted by fluorine, benzyloxy, phenoxy, mercapto, C₁-C₆-alkylthio, phenylthio, sulfo, carboxy, C₂-C₇-carboxyalkyl, C₃-C₇-carboxyalkenyl, C₂-C₇-alkoxycarbonyl, benzyloxycarbonyl, nitro, amino, C₁-C₆-aminoalkyl, mono-C₁-C₆-alkylamino and di(C₁-C₆-alkyl)amino, and in the case of two adjacent residues on the aromatic ring, methylenedioxy,
   wherein alkyl and cycloalkyl residues in the groups **G** optionally are substituted by one or two of the same or different groups, selected from
   hydroxy, carboxy, C₂-C₇-alkoxycarbonyl, benzyloxycarbonyl, amino, mono-C₁-C₆-alkylamino and di(C₁-C₆-alkyl)amino;
   and the stereoisomers or racemic or non-racemic mixtures of stereoisomers thereof,
   and the tautomers thereof when **G** is a heterocyclic aromatic ring or an aromatic ring substituted by a hydroxy, mercapto or amino group,
   and the pharmacologically acceptable acid addition salts thereof;
(b) at least one compound having vitamin PP activity or a prodrug thereof which is selected from the group consisting of compounds of formulae II, IIa, IIb, III, IIIa, IIIb, IIIc, IV, IVa, IVb, V, Va, and Vb: 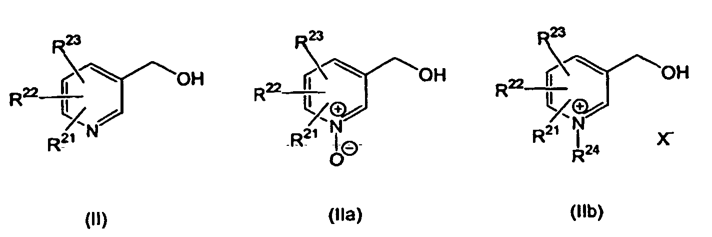 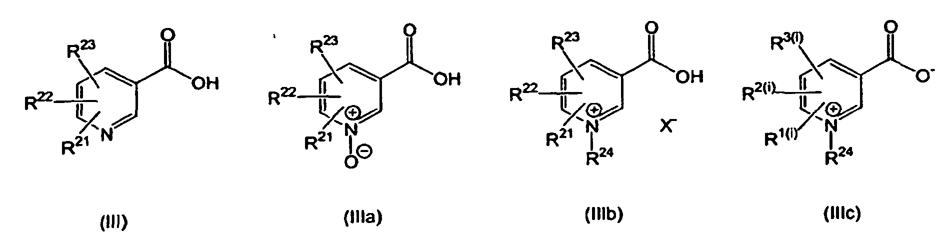 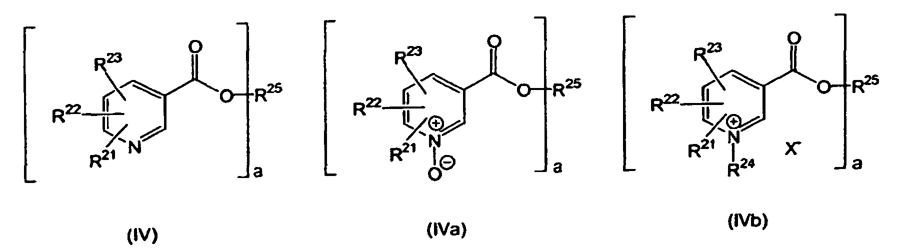 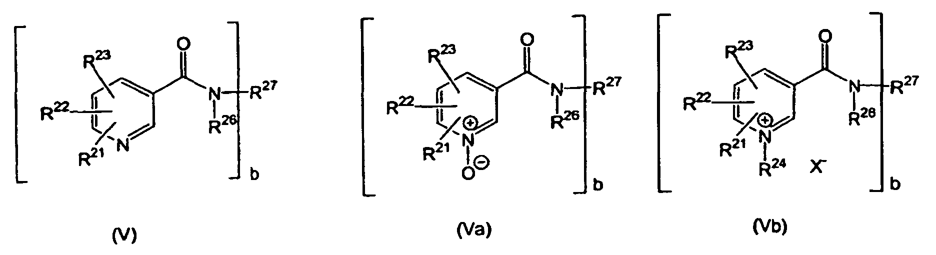 wherein:
   - **a**: is an integer of 1 through 6;
   - **b**: is an integer of 1 through 2;
   - **X**^{**-**}: is selected from the group consisting of fluoride, chloride, bromide, iodide, hydrogensulfate, methanesulfonate, trifluoromethanesulfonate, tosylate, tetrafluoroborate, dihydrogenphosphate, and acetate;
   - **R**^{**21**}: is selected from the group consisting of hydrogen, halogen, cyano, C₁-C₆-alkyl, trifluoromethyl, C₁-C₆-hydroxyalkyl, hydroxy, C₁-C₆-alkoxy, C₁-C₇-alkanoyloxy, C₁-C₆-alkylthio, C₁-C₆-aminoalkyl, amino, C₁-C₆-alkylamino, C₂-C₁₂-dialkylamino, formyl, C₂-C₇-alkoxycarbonyl, aminocarbonyl, C₂-C₇-alkylaminocarbonyl, C₃-C₁₃-dialkylamino-carbonyl and carboxy;
   - **R**^{**22**}: is selected from the group consisting of hydrogen, halogen, C₁-C₆-alkyl, trifluoromethyl, C₁-C₆-hydroxyalkyl, hydroxy, C₁-C₆-alkoxy, C₁-C₇-alkanoyloxy, C₁-C₆-aminoalkyl, amino, C₂-C₇-alkoxycarbonyl, aminocarbonyl, and carboxy;
   - **R**^{**23**}: is selected from the group consisting of hydrogen, C₁-C₆-alkyl, and C₁-C₆-hydroxyalkyl;
   - **R**^{**24**}: is selected from the group consisting of C₁-C₆-alkyl, C₃-C₆-alkenyl, C₂-C₆-hydroxyalkyl, C₂-C₆-alkoxyalkyl and benzyl;
   - **R**^{**25**}: is such that the alcohol **R**^{**25**}**(OH)**_{**a**} is selected from monovalent, linear and branched, C₁₋₁₀-alkanols and ω-dialkylaminoalkanols, benzyl alcohol, divalent linear and branched C₂₋₁₀-diols, mono- or divalent C₅₋₇-cycloalkanols, C₅₋₇-cycloalkanediols, C₅₋₇-cycloalkanemethanols, saturated C₅₋₇-heterocyclomethanols, glycerin, 2,2-bis(hydroxymethyl)-1-octanol, erythritol, pentaerythritol, arabitol, xylitol, sorbitol, mannitol, isosorbitol, tetra(hydroxymethyl)cyclohexanol, and inositol;
   - **R**^{**26**}: is selected from the group consisting of hydrogen, C₁-C₆-alkyl, C₁-C₆-hydroxyalkyl, C₃-C₆-alkoxyalkyl, C₁-C₆-aminoalkyl, C₄-C₁₂-dialkylaminoalkyl and carboxymethyl;
   when b is 1,
   **R**^{**27**} is selected from the group consisting of hydrogen, C₁-C₆-alkyl, C₁-C₆-hydroxyalkyl, C₃-C₆-alkoxyalkyl, C₁-C₆-aminoalkyl, C₄-C₁₂-dialkylaminoalkyl and carboxymethyl;
   when b is 2,
   **R**^{**27**} is C₂-C₁₀-alkylene, or C₅-C₁₀-alkylene wherein a methylene group is isosterically replaced by O, NH or N-alkyl;
   and their thioxo analogs,
   and the acid addition salts or anionic salts thereof; and
(c) at least one physiologically acceptable carrier and at least one toxicologically safe adjuvant.

According to the invention it is further preferred to use of a compound having vitamin PP activity or a prodrug thereof for the manufacture of a pharmaceutical composition for preventing, reducing, or eliminating side effects or neutralizing the side effects of a cancerostatic or immunosuppressive agent administered prophylactically or therapeutically to a patient.

In a more preferred embodiment the compound having vitamin PP activity or a prodrug thereof used according to the invention is selected from the group consisting of compounds of formulae II, IIa, IIb, III, IIIa, IIIb, IIIc, IV, IVa, IVb, V, Va, and Vb as defined above.

In a very preferred embodiment according to the use of the present invention the cancerostatic or immunosuppressive agent is selected from the group consisting of compounds of formula (I) as defined above.

Pursuant to a preferred embodiment according to the invention, the following compound group encompassed by the above defined general formula (I) can be deployed according to the narrower general formula, herein defined with formula (Ia), wherein the substituents of the compounds according to this formula (Ia) 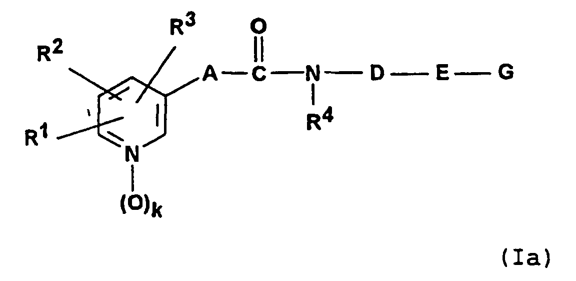 have the following meanings:
- **R**^{**1**}: is hydrogen, fluorine, methyl, trifluoromethyl or hydroxy,
- **R**^{**2**}: and
- **R**^{**3**}: are hydrogen,
- **R**^{**4**}: is hydrogen or hydroxy,
- **k**: is 0,
- **A**: is ethylene, propylene or butylene, which can each be optionally substituted by hydroxy or once or twice by fluorine, or
OCH₂, SCH₂, or
ethenylene and/or vinylene or
1,3-butadienylene,
- **D**: is selected from C₂-C₆-alkylene or C₂-C₆-alkenylene, whereby the double bond can also occur to the ring E,
- **E**: is selected from pyrrolidine, piperidine, hexahydroazepine or morpholine,
- **G**: is selected from benzyl, phenethyl, fluoroenylmethyl, anthrylmethyl, diphenylmethyl, fluoroenyl or dihydrodibenzocycloheptenyl, furylmethyl, thienylmethyl, thiazolylmethyl, pyridylmethyl, benzothienylmethyl, quinolylmethyl, phenylthienylmethyl, phenylpyridylmethyl, dihydrodibenzoxepinyl, dihydrodibenzothiepinyl; acetyl, pivaloyl, phenylacetyl, diphenylacetyl, diphenylpropionyl, naphthylacetyl, benzoyl, naphthoyl, anthrylcarbonyl, oxofluoroenylcarbonyl, oxodihydroanthrylcarbonyl or dioxodihydroanthrylcarbonyl; furoyl, pyridylcarbonyl, chromonylcarbonyl, quinolylcarbonyl;
naphthylaminocarbonyl, dibenzylaminocarbonyl, benzylphenylaminocarbonyl, diphenylaminocarbonyl, indolinyl-1-carbonyl, dihydrodibenzazepin-N-carbonyl, tetrahydroquinolinyl-N-carbonyl, tetrahydrobenzoazepinyl-N-carbonyl;
methanesulfonyl, phenylsulfonyl, p-tosyl, naphthylsulfonyl, quinolinsulfonyl, and diphenylphosphinoyl,
whereby aromatic ring systems can be substituted independently from each other by one to three of the same or different substituents from the series halogen, cyano, C₁-C₆-alkyl, trifluoromethyl, C₃-C₈-cycloalkyl, phenyl, benzyl, hydroxy, C₁-C₆-alkoxy, C₁-C₆-alkoxy which can be entirely or partially substituted by fluorine, benzyloxy, phenoxy, mercapto, C₁-C₆-alkylthio, carboxy, C₁-C₆-alkoxycarbonyl, benzyloxycarbonyl, nitro, amino, mono-C₁-C₆-alkylamino or di-(C₁-C₆-alkyl)-amino, whereby two adjacent groups in the ring or ring systems can form an additional ring over a methylenedioxy bridge.

In a particularly preferred manner, the above defined cancerostatic agents or tumor inhibitors used according to the invention concern substituted pyridylcarboxamides of the general formula (Ia) in the form of the following compounds:
N-[2-(1-benzylpiperidin-4-yl)-ethyl]-3-tpyridin-3-yl)-propionamide;
N-{2-[1-(2-phenylethyl)-piperidin-4-yl]-ethyl}-3-(pyridin-3-yl)-propionamide;
N-{2-[1-(4-phenylbutyl)-piperidin-4-yl]-ethyl}-3-(pyridin-3-yl)-propionamide;
N-{2-[1-(4-hydroxy-4-phenylbutyl)-piperidin-4-yl]-ethyl}-3-(pyridin-3-yl)-propionamide;
N-[2-(1-diphenylmethylpiperidin-4-yl)-ethyl]-3-(pyridin-3-yl)-propionamide,
N-[3-(1-diphenylmethylpiperidin-4-yl)-propyl]-3-(pyridin-3-yl)-propionamide;
N-[4-(1-diphenylmethylpiperidin-4-yl)-butyl]-3-(pyridin-3-yl)-propionamide;
N-[4-(1-benzylpiperidin-4-yl)-butyl]-3-(pyridin-3-yl)-acrylamide;
N-{4-[1-(2-phenylethyl)-piperidin-4-yl]-butyl}-3-(pyridin-3-yl)-acrylamide;
N-{4-[1-(4-biphenylylmethyl)-piperidin-4-yl]-butyl}-3-(pyridin-3-yl)-acrylamide;
N-{4-[1-(1-naphthylmethyl)-piperidin-4-yl]-butyl}-3-(pyridin-3-yl)-acrylamide;
N-{4-[1-(9-anthrylmethyl)-piperidin-4-yl]-butyl}-3-(pyridin-3-yl)-acrylamide;
N-{4-[1-(cyclohexylphenylmethyl)-piperidin-4-yl]-butyl}-3-(pyridin-3-yl)-acrylamide;
N-{4-[1-(10,11-dihydro-5H-dibenzo[a,d]cyclohepten-5-yl)-piperidin-4-yl]-butyl}-3-(pyridin-3-yl)-acrylamide;
N-[2-(1-diphenylmethylpiperidin-4-yl)-ethyl]-3-(pyridin-3-yl)-acrylamide;
N-[3-(1-diphenylmethylpiperidin-4-yl)-propyl]-3-(pyridin-3-yl)-acrylamide;
N-[5-(1-diphenylmethylpiperidin-4-yl)-pentyl]-3-(pyridin-3-yl)-acrylamide;
N-[6-(1-diphenylmethylpiperidin-4-yl)-hexyl]-3-(pyridin-3-yl)-acrylamide;
N-[4-(1-diphenylmethylpiperidin-4-yl)-butyl]-5-(pyridin-3-yl)-2,4-pentadienoic acid amide;
N-(4-{1-[to-(4-fluorophenyl)-methyl]-piperidin-4-yl}-butyl)-3-(pyridin-3-yl)-acrylamide;
N-(4-{1-[to-(2-chlorophenyl)-methyl]-piperidin-4-yl}-butyl)-3-(pyridin-3-yl)-acrylamide;
N-[4-(1-diphenylmethylpiperidin-4-yl)-butyl]-3-(2-fluoropyridin-3-yl)-acrylamide;
N-[4 (1-diphenylmethylpiperidin-4-yl)-butyl]-3-(6-fluoropyridin-3-yl)-acrylamide;
N-[4-(1-diphenylmethylpiperidin-4-yl)-butyl]-3-(pyridin-3-yl)-acrylamide;
N-[4-(1-diphenylmethylpiperidin-4-yl)-butyl]-3-(pyridin-3-yl)-acrylamide•dihydrochloride or
N-[4-(1-diphenylmethylpiperidin-4-yl)-butyl]-3-(pyridin-3-yl)-acrylamide•methanesulfonate;

A further preferred group in this series are presented in the following compounds
N-[4-(1-acetyl-piperidin-4-yl)-butyl]-3-(pyridin-3-yl)-propionamide;
N-[4-(1-benzoyl-piperidin-4-yl)-butyl]-3-(pyridin-3-yl)-propionamide;
N-[4-(1-diphenylacetyl-piperidin-4-yl)-butyl]-3-(pyridin-3-yl)-propionamide;
N-{4-[1-(9-oxo-9H-fluoroen-4-carbonyl)-piperidin-4-yl]-butyl}-3-(pyridin-3-yl)-propionamide;
N-[4-(1-methylsulfonyl-piperidin-4-yl)-butyl]-3-(pyridin-3-yl)-propionamide;
N-{4-[1-(2-naphthyl-sulfonyl)-piperidin-4-yl]-butyl}-3-(pyridin-3-yl)-propionamide;
N-[4-(1-benzyl-piperidin-4-yl)-butyl]-3-(pyridin-3-yl)-propionamide;
N-(4-{1-[to-(2-chlorophenyl)-methyl]-piperidin-4-yl}-butyl)-3-(pyridin-3-yl)-propionamide;
N-{4-[1-(phenylpyridin-3-yl-methyl)-piperidin-4-yl]-butyl}-3-(pyridin-3-yl)-propionamide;
N-{4-[1-(9H-fluoroen-9-yl)-piperidin-4-yl]-butyl}-3-(pyridin-3-yl)-propionamide;
N-{4-[1-(6,11-dihydrodibenzo[b,e]oxepin-11-yl)-piperidin-4-yl]-butyl}-3-(pyridin-3-yl)-propionamide;
N-{4-[1-(1-naphthylamino-carbonyl)-piperidin-4-yl]-butyl}-3-(pyridin-3-yl)-propionamide;
N-[4-(1-diphenylamino-carbonyl-piperidin-4-yl)-butyl]-3-(pyridin-3-yl)-propionamide;
N-{4-[1-(10,11-dihydro-dibenzo[b,f]azepin-5-yl-carbonyl)-piperidin-4-yl]-butyl}-3-(pyridin-3-yl)-propionamide;
N-[4-(1-diphenylphosphinoyl-piperidin-4-yl)-butyl]-3-(pyridin-3-yl)-propionamide;
N-[4-(1-diphenylmethylpiperidin-4-yl)-butyl]-3-(2-fluoro-pyridin-3-yl)-propionamide;
N-[4-(1-diphenylmethylpiperidin-4-yl)-butyl]-3-(5-fluoro-pyridin-3-yl)-propionamide;
N-[4-(1-diphenylmethylpiperidin-4-yl)-butyl]-2-fluoro-3-(pyridin-3-yl)-propionamide;
N-[4-(1-diphenylmethylpiperidin-4-yl)-butyl]-2,2-difluoro-3-(pyridin-3-yl)-propionamide;
N-[5-(1-diphenylmethylpiperidin-4-yl)-pentyl]-3-(pyridin-3-yl)-propionamide;
N-[6-(1-diphenylmethylpiperidin-4-yl)-hexyl]-3-(pyridin-3-yl)-propionamide;
N-[2-(1-diphehylmethylpiperidin-4-yl)-ethyl]-5-(pyridin-3-yl)-pentanoic acid amide;
N-[4-(1-diphenylmethylpiperidin-4-yl)-butyl]-5-(pyridin-3-yl)-pentanoic acid amide;
N-[4-(1-diphenylmethylpiperidin-4-yl)-butyl]-N-hydroxy-3-(pyridin-3-yl)-propionamide;
N-[4-(1-diphenylmethylpiperidin-4-yl)-butyl]-2-hydroxy-3-(pyridin-3-yl)-propionamide;
N-[4-(1-diphenylmethylpiperidin-4-yl)-butyl]-3-hydroxy-3-(pyridin-3-yl)-propionamide and
N-[4-(1-diphenylmethylpiperidin-4-yl)-butyl]-3-(pyridin-3-yl)-propionamide;

Furthermore, the following group of compounds are also preferred in this series:
N-[4-(1-methylsulfonylpiperidin-4-yl)-butyl]-3-(pyridin-3-yl)-acrylamide;
N-{4-[1-(2-naphthylsulfonyl)-piperidin-4-yl]-butyl}-3-(pyridin-3-yl)-acrylamide;
N-{4-[1-(2-naphthylsulfonyl)-piperidin-4-yl]-butyl}-5-(pyridin-3-yl)-2,4-pentadienoic acid amide;
N-{4-[1-(1-naghthylaminocarbonyl)-piperidin-4-yl]-butyl}-3-(pyridin-3-yl)-acrylamide;
N-[4-(1-diphenylaminocarbonylpiperidin-4-yl)-butyl]-3-(pyridin-3-yl)-acrylamide;
N-[4-(1-diphenylaminocarbonyl-piperidin-4-yl)-butyl]-5-(pyridin-3-yl)-2,4-pentadienoic acid amide;
N-{4-[1-(10,11-dihydrodibenzo[b,f]azepin-5-yl-carbonyl)-piperidin-4-yl]-butyl}-3-(pyridin-3-yl)-acrylamide;
N-[4-(1-Diphenylphosphinoyl-piperidin-4-yl)-butyl]-3-(pyridin-3-yl)-acrylamide;
N-[4-(1-acetylpiperidin-4-yl)-butyl]-3-(pyridin-3-yl)-acrylamide;
N-[4-(1-diphenylacetylpiperidin-4-yl)-butyl]-3-(pyridin-3-yl)-acrylamide;
N-{4-[1-(3,3-diphenylpropionyl)-pigeridin-4-yl]-butyl}-3-(pyridin-3-yl)-acrylamide;
N-[4-(1-benzoylpiperidin-4-yl)-butyl]-3-(pyridin-3-yl)-acrylamide;
N-[4-(1-benzoylpiperidin-4-yl)-butyl]-5-(pyridin-3-yl)-2,4-pentadienoic acid amide;
N-{4-[1-(9-oxo-9H-fluoren-4-yl-carbonyl)-piperidin-4-yl]-butyl}-3-(pyridin-3-yl)-acrylamide;
N-{4-[1-(phenylpyridin-3-yl-methyl)-piperidin-4-yl]-butyl}-3-(pyridin-3-yl)-acrylamide;
N-{4-[1-iphenylpyridin-4-yl-methyl)-piperidin-4-yl]-butyl}-3-(pyridin-3-yl)-acrylamide;
N-{4-[1-(6,11-dihydrodibenzo[b,e]oxepin-11-yl)-piperidin-4-yl]-butyl}-3-(pyridin-3-yl)-acrylamide;
N-{4-[1-(6,11-dihydrodibenzo[b,e]thiepin-11-yl)-piperidin-4-yl]-butyl}-3-(pyridin-3-yl)-acrylamide;
N-[7-(1-diphenylmethylpiperidin-4-yl)-heptyl]-3-(pyridin-3-yl)-acrylamide;
N-[8-(1-diphenylmethylpiperidin-4-yl)-octyl]-3-(pyridin-3-yl)-acrylamide;
N-[3-(1-diphenylmethylpiperidin-4-yloxy)-propyl]-3-(pyridin-3-yl)-acrylamide;
N-[3-(1-benzylpiperidin-4-yloxy)-propyl]-3-(pyridin-3-yl)-acrylamide;
N-[2-(1-diphenylmethylpiperidin-4-yl)-ethyl]-5-(pyridin-3-yl)-2,4-pentadienoic acid amide;
N-[4-(1-diphenylmethylpiperidin-4-yl)-butyl]-5-(pyridin-3-yl)-2,4-pentadienoic acid amide;
N-[5-(1-diphenylmethylpiperidin-4-yl)-pentyl]-5-(pyridin-3-yl)-2,4-pentadienoic acid amide; or
N-[6-(1-diphenylmethylpiperidin-4-yl)-hexyl]-5-(pyridin-3-yl)-2,4-pentadienoic acid amide;

A further preferred embodiment according to the invention is in the use of the following compound group, also falling under the above general formula (I), according to the narrower general formula, herein defined with formula (Ia) and which is described above. This compound group relates to compounds of the general formula (Ia), wherein
- **R**^{**1**}: is selected from
hydrogen, fluorine, methyl, trifluoromethyl, ethylthio;
- **R**^{**2**}**, R**^{**3**} and **R**^{**4**}: are each hydrogen;
- **k**: is 0,
- **A**: is selected from
ethylene, propylene or butylene, which are each optionally substituted by hydroxy or one or two fluorine atoms; or
OCH₂, SCH₂;
ethenylene or 1,3-butadienylene;
- **D**: is selected from
C₂-c₆-alkylene, is optionally substituted by hydroxy;
C₄-C₆-alkenylene;
C₄-C₆-alkinylene; or
C₂-C₆-alkylene, C₄-C₆-alkenylene or C₄-C₆-alkinylene, wherein one or two methylene units are isosterically replaced by O, NH, CO or SO_{2;}
- **E**: is selected from
piperazine or hexahydro-1,4-diazepine;
- **G**: is selected from
phenyl, benzyl, phenethyl, diphenylmethyl, naphthyl, tetrahydronaphthyl, naphthylmethyl, fluoroenyl, fluoroenylmethyl, anthrylmethyl, dihydrodibenzocycloheptenyl;
furylmethyl, thienylmethyl, thiazolylmethyl, pyridylmethyl, benzothienylmethyl, quinolylmethyl, phenylthienylmethyl, phenylpyridylmethyl, benzocycloheptapyridinyl, dihydrobenzocycloheptapyridinyl, dihydrodibenzooxepinyl, dihydrodibenzothiepinyl, dihydrodibenzoazepinyl, dihydrobenzopyridodiazepinyl;
formyl, acetyl, pivaloyl, phenylacetyl, diphenylacetyl, diphenylpropionyl, naphthylacetyl, benzoyl, naphthoyl, oxofluoroenylcarbonyl, oxodihydroanthrylcarbonyl, dioxodihydroanthrylcarbonyl, furoyl, pyridylacetyl, pyridylcarbonyl, chromonylcarbonyl, quinolylcarbonyl;
phenylylaminocarbonyl, naphthylaminocarbonyl, tetrahydronaphthylaminocarbonyl, dibenzylaminocarbonyl, benzylphenylaminocarbonyl, diphenylaminocarbonyl, indolinyl-N-carbonyl, isoindolin-N-carbonyl, tetrahydroquinolinyl-N-carbonyl, carbazolyl-N-carbonyl, tetrahydrobenzoazepinyl-N-carbonyl, dihydrodibenzoazepin-N-carbonyl, dihydrobenzopyridoazepinyl-N-carbonyl, oxodihydrobenzopyridoazepinyl-N-carbonyl; methanesulfonyl, tolylsulfonyl, naphthylsulfonyl, quinolinsulfonyl and diphenylphosphinoyl,
whereby aromatic ring systems can be substituted independently from each other by one to three of the same or different groups that are selected from
halogen, cyano, C₁-C₆-Alkyl, trifluoromethyl, C₃-C₈-cycloalkyl, phenyl, benzyl, hydroxy, C₁-C₆-hydroxyalkyl, C₁-C₆-alkoxy, C₁-C₆-Alkoxy entirely or partially substituted by fluorine, benzyloxy, phenoxy, mercapto, C₁-C₆-alkylthio, carboxy, C₂-C₇-carboxyalkyl, C₂-C₇-carboxyalkenyl, C₂-C₇-alkoxycarbonyl, benzyloxycarbonyl, nitro, amino, mono-C₁-C₆-alkylamino, Di-(C₁-C₆-alkyl)-amino and, in the case of two adjacent residues on an aromatic ring, methylenedioxy, and whereby
alkyl, alkenyl and cycloalkyl residues in the group G can be substituted by one or two of the same or different groups that are selected from
hydroxy, carboxy, C₂-C₇-alkoxycarbonyl, benzyloxycarbonyl, amino, mono-C₁-C₆-alkylamino or di-(C₁-C₆-alkyl)-amino.

From the above defined group, the following piperazinyl substituted compounds according to the general formula (I) are preferably used according to the invention:
N-[4-(4-diphenylmethylpiperazin-1-yl)-3-hydroxy-butyl]-3-pyridin-3-yl-acrylamide; N-[3-(4-diphenylmethylpiperazin-1-yl)-propoxy]-3-pyridin-3-yl-acrylamide; N-[4-(4-diphenylmethylpiperazin-1-yl)-4-oxo-butyl]-3-pyridin-3-yl-acrylamide; N-[3-(4-diphenylmethylpiperazin-1-sulfonyl)-propyl]-3-pyridin-3-yl-acrylamide; N-{2-[2-(4-diphenylmethylpiperazin-1-yl)-ethoxy]-ethyl}-3-pyridin-3-yl-acrylamide; N-(4-{4-[to-(4-fluorophenyl)-methyl]-piperazin-1-yl}-but-2-inyl)-3-pyridin-3-yl-acrylamide; N-{4-[4-(4-carboxyphenyl-phenylmethyl)-piperazin-1-yl]-butyl}-3-pyridin-3-yl-acrylamide; N-(4-{4-[(4-aminophenyl)-phenylmethyl]-piperazin-1-yl}-butyl)-3-pyridin-3-yl-acrylamide; N-{4-[4-(9H-fluoroen-9-yl)-piperazin-1-yl]-butyl}-2-(pyridin-3-yloxy)-acetamide; N-{5-[4-(9H-fluoroen-9-yl)-piperazin-1-yl]-pentyl}-3-pyridin-3-yl-acrylamide; N-{6-[4-(9H-fluoroen-9-yl)-piperazin-1-yl]-hexyl}-3-pyridin-3-yl-acrylamide; 3-pyridin-3-yl-N-{4-[4-(1,2,3,4-tetrahydronaphthalin-1-yl)-piperazin-1-yl]-butyl}-acrylamide; 3- pyridin-3-yl-N-{4-[4-(5,6,7,8-tetrahydronaphthalin-1-yl)-piperazin-1-yl]-butyl}-acrylamide; N-{4-[4-(naphthalin-1-yl)-piperazin-1-yl]-butyl}-3-pyridin-3-yl-acrylamide; N-[4-(4-biphenyl-2-yl-piperazin-1-yl)-butyl]-3-pyridin-3-yl-propionamide; N-[5-(4-biphenyl-2-yl-piperazin-1-yl)-pentyl]-3-pyridin-3-yl-acrylamide; N-[6-(4-biphenyl-2-yl-piperazin-1-yl)-hexyl]-3-pyridin-3-yl-acrylamide; N-[4-(4-biphenyl-2-yl-piperazin-1-ly)-butyl]-2-(pyridin-3-yloxy)-acetamide; N-[4-(4-biphenyl-2-yl-piperazin-1-yl)-butyl]-5-(pyridin-3-yl)-penta-2,4-dienoic acid amide; N-{4-[4-(10,11-dihydro-5H-dibenzo[a,d]cyclohepten-5-yl)-piperazin-1-yl]-butyl}-3-pyridin-3-yl-propionamide; N-{5-[4-(10,11-dihydro-5H-dibenzo[a,d]cyclohepten-5-yl)-piperazin-1-yl]-pentyl}-3-pyridin-3-yl-acrylamide; N-{6-[4-(10,11-dihydro-5H-dibenzo[a,d]cyclohepten-5-yl)-piperazin-1-yl]-hexyl}-3-pyridin-3-yl-acrylamide; N-{4-[4-(10,11-dihydro-5H-dibenzo[a,d]cyclohepten-5-yl)-piperazin-1-yl]-butyl}-5-(pyridin-3-yl)-penta-2,4-dienoic acid amide; N-{4-[4-(6,11-dihydro-dibenzo[b,e]oxepin-11-yl)-piperazin-1-yl]-butyl-3-pyridin-3-yl-propionamide; N-{2-[4-(6,11-dihydro-dibenzo[b,e]thiepin-11-yl)-piperazin-1-yl]-ethyl}-3-pyridin-3-yl-acrylamide; N-[4-(4-diphenylacetylpiperazin-1-yl)-butyl]-3-pyridin-3-yl-acrylamide; N-[4-(4-benzoylpiperazin-1-yl)-butyl]-3-pyridin-3-yl-acrylamide; N-{4-[4-(2-aminobenzoyl)-piperazin-1-yl]-butyl}-3-pyridin-3-yl-acrylamide; N-{4-[4-(4-carboxybenzoyl)-piperazin-1-yl]-butyl}-3-pyridin-3-yl-acrylamide; N-{4-[4-(biphenyl-2-carbonyl)-piperazin-1-yl]-butyl}-3-pyridin-3-yl-acrylamide; N-{4-[4-(9-oxo-9H-fluoroen-4-carbonyl)-piperazin-1-yl]-butyl}-3-pyridin-3-yl-acrylamide; N-{4-[4-(furan-2-carbonyl)-piperazin-1-yl]-butyl}-3-pyridin-3-yl-acrylamide; N-{4-[4-(naphthalin-1-yl-aminocarbonyl)-piperazin-1-yl]-butyl}-3-pyridin-3-yl-propionamide; N-{4-[4-(diphenylaminocarbonyl)-piperazin-1-yl]-butyl}-3-pyridin-3-yl-acrylamide; N-{4-[4-(naphthalin-2-sulfonyl)-piperazin-1-yl]-butyl}-3-pyridin-3-yl-acrylamide; N-[4-(4-diphenylphosphinonyl-piperazin-1-yl)-butyl]-3-pyridin-3-yl-acrylamide; N-[4-(4-biphenyl-2-yl-piperazin-1-yl)-butyl]-3-pyridin-3-yl-acrylamide; N-{4-[4-(9H-fluoroen-9-yl)-piperazin-1-yl]-butyl}-3-pyridin-3-yl-acrylamide as well as N-{4-[4-(10,11-dihydro-5H-dibenzo[a,d]cyclohepten-5-yl)-piperazin-1-yl]-butyl}-3-pyridin-3-yl-acrylamide;

Furthermore, according to the invention, the use of the following group of compounds according to the general formula (I) described above represent a further preferred embodiment. In these compounds, the substituents have the following meanings:
- **R**^{**1**}: is selected from
hydrogen, fluorine, methyl, trifluoromethyl, ethylthio;
- **R**^{**2**}**, R**^{**3**} and **R**^{**4**}: are each hydrogen;
- **k**: is 0,
- **A**: is selected from
ethylene, propylene or butylene, optionally substituted by hydroxy or one or two fluorine atoms, or OCH₂ or SCH₂;
ethenylene or 1,3-butadienylene;
- **D**: is selected from
C₄-C₆-alkylene, optionally substituted by hydroxy, C₄-C₆-alkenylene, C₄-C₆-alkinylene or
C₄-C₆-alkylene, C₄-C₆-alkenylene or C₄-C₆-alkinylene, wherein one or two methylene units are isosterically replaced by O, NH, CO or SO₂;
- **E**: is piperidine;
- **G**: is selected from
diphenylmethyl, diphenylhydroxymethyl, diphenylmethylene, naphthyl, tetrahydronaphthyl, tetrahydronaphthylidene, fluorenyl, fluoroenylidene,
tetrahydrobenzocycloheptenyl or tetrahydrobenzocycloheptenylidene, dihydrodibenzocycloheptenyl or dihydrodibenzocycloheptenylidene; mixed diphenyl;
phenylthienylmethyl, phenylthienylmethylene, phenylpyridylmethyl, phenylpyridylmethylene, tetrahydroquinolinyl, tetrahydroisoquinolinyl, benzocycloheptapyridinyl, benzocycloheptapyridinylidene, dihydrobenzocycloheptapyridinyl, dihydrobenzocycloheptapyridinylidene, dihydrodibenzooxepinyl, dihydrodibenzooxepinylidene, dihydrodibenzothiepinyl, dihydrodibenzothiepinylidene, dihydrobenzothienothiepinyl or dihydrobenzothienothiepinylidene;
indolyl, oxobenzoimidazolyl, oxobenzothiazolyl, benzoisothiazolyl or benzotriazolyl;
dibenzylaminocarbonyl, diphenylaminocarbonyl, indolinyl-N-carbonyl, isoindolinyl-N-carbonyl, tetrahydroquinolinyl-N-carbonyl, tetrahydrobenzoazepinyl-N-carbonyl, carbazolyl-N-carbonyl, dihydrodibenzoazepinyl-N-carbonyl or oxodihydrobenzopyridodiazepinyl-N-carbonyl; diphenylmethylamino, diphenylmethyl-methylamino, dibenzylamino, benzylphenylamino or triphenylmethylamino;
acetylamino, pivaloylamino, phenylacetylamino, diphenylacetylamino, diphenylpropionylamino, naphthylacetylamino, benzoylamino, benzoylmethylamino, naphthoylamino or oxofluoroenylcarbonylamino;
furoylamino, pyridylacetylamino or pyridylcarbonylamino;
benzylaminocarbonylamino, naphthylmethylaminocarbonylamino, indanylaminocarbonylamino, tetrahydronaphthylaminocarbonyl, dibenzylaminocarbonylamino, phenylylaminocarbonylamino, naphthylaminocarbonylamino, benzylphenylaminocarbonylamino or diphenylaminocarbonyl-amino;
indolinyl-N-carbonylamino, isoindolinyl-N-carbonylamino, tetrahydroquinolinyl-N-carbonylamino, tetrahydrobenzoazepinyl-N-carbonylamino, carbazolyl-N-carbonylamino, dihydrophenanthridinyl-N-carbonylamino, dihydrodibenzoazepin-N-carbonylamino, dihydrobenzopyridoazepinyl-N-carbonylamino or oxodihydrobenzopyridodiazepinyl-N-carbonylamino;
methanesulfonylamino, tolylsulfonylamino, naphthylsulfonylamino or diphenylphosphinoylamino;
diphenylmethyloxy or diphenylphosphinoyloxy,
whereby aromatic ring systems can be substituted independently from each other by one to three of the same or different groups, selected from
halogen, cyano, C₁-C₆-alkyl, trifluoromethyl, C₃-C₈-cycloalkyl, phenyl, benzyl, hydroxy, C₁-C₆-hydroxyalkyl, C₁-C₆-alkoxy, C₁-C₆-alkoxy entirely or partially substituted by fluorine, benzyloxy, phenoxy, mercapto, C₁-C₆-alkylthio, carboxy, C₂-C₇-carboxyalkyl, C₂-C₇-carboxyalkenyl, C₂-C₇-alkoxycarbonyl, benzyloxycarbonyl, nitro, amino, mono-C₁-C₆-alkyl-amino, di-(C₁-C₆-alkyl)-amino and, in the case of two adjacent residues in an aromatic ring, methylenedioxy, and whereby
alkyl, alkenyl and cycloalkyl residues in the group G can be substituted once or twice of the same or different group, selected from
hydroxy, carboxy, C₂-C₇-alkoxycarbonyl, benzyloxycarbonyl, amino, mono-C₁-C₆-alkylamino and di-(C₁-C₆-alkyl)amino; as well as the salts, isomers and tautomers of the above defined compounds as well as, optionally, their mixtures.

Among this group, the following piperidyl-substituted derivatives according to the general formula (I) are preferably used according to the invention:
N-[4-(4-phenylpiperidin-1-yl)-butyl]-3-pyridin-3-yl-acrylamide; N-{4-[4-(1H-indol-3-yl)-piperidin-1-yl]-butyl}-3-pyridin-3-yl-acrylamide; N-{4-[4-(2-oxo-2,3-dihydrobenzimidazol-1-yl)-piperidin-1-yl]-butyl}-3-pyridin-3-yl-acrylamide; N-[4-(4-benzotriazol-1-yl-piperidin-1-yl)-butyl]-3-pyridin-3-yl-acrylamide; N-{4-[4-(hydroxy-diphenylmethyl)-piperidin-1-yl]-butyl}-2-(pyridin-3-yloxy)-acetamide; N-[4-(4,4-diphenylpiperidin-1-yl)-butyl]-3-pyridin-3-yl-acrylamide; N-{4-[4-(6,11-dihydrodibenzo[b,e]thiepin-11-yliden)-piperidin-1-yl]-butyl}-3-pyridin-3-ylpropionamide•dihydrochloride/ semi-isopropanol; N-{4-[4-(6,11-dihydrodibenzo[b,e]thiepin-11-yliden)-piperidin-1-yl]-butyl}-5-pyridin-3-yl-pentanamide; N-{4-[4-(4,9-dihydro-thieno[2,3-b]-benzo[e]thiepin-4-yliden)-piperidin-1-yl]-butyl}-3-pyridin-3-yl-propionamide; N-{4-[4-(4,9-dihydro-thieno[2,3-b]-benzo[e]thiepin-4-yliden)-piperidin-1-yl]-butyl}-3-pyridin-3-yl-acrylamide; N-[4-(4-diphenylphosphinoyloxypiperidin-1-yl)-butyl]-3-pyridin-3-yl-acrylamide as well as N-[4-(1,4-dioxa-8-azaspiro[4.5]dec-8-yl)-butyl]-3-pyridin-3-yl-acrylamide;

A further preferred embodiment according to the invention relates to a group of compounds according to the general formula, herein defined as formula (Ib) 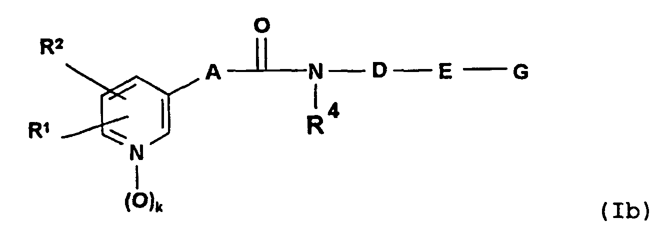 which also falls under the scope of the above given formula (I), wherein the defined substituents have the following meanings:
- **R**^{**1**}: is selected from
hydrogen, fluorine, methyl, trifluoromethyl, ethylthio;
- **R**^{**2**}: and **R**^{**4**} are hydrogen;
- **k**: is 0,
- **A**: is selected from
ethylene or butylene, optionally substituted by hydroxy or one or two fluorine atoms, or
OCH₂, SCH₂,
ethenylene or 1,3-butadienylene;
- **D**: is selected from
C₄-C₆-alkylene, optionally substituted by hydroxy; C₄-C₆-alkenylene;
C₄-C₆-alkinylene; or
C₄-C₆-alkylene, C₄-C₆-alkenylene or C₄-C₆-alkinylene wherein one or two methylene units are isosterically replaced by O, NH or CO;
- **E**: represent a single bond,
- **G**: is selected from
monocyclic imides such as succinimide, maleinimide, glutarimide, imidazolidindione, imidazolidintrione, thiazolidindione, oxazolidindione, piperazin-2,6-dione, hexahydrodiazepin-2,7-dione;
anellated bicyclic imides such as phthalimide, homophthalimide, pyridin-2,3-dicarboximide, pyridin-3,4-dicarboximide, isatoic acid imide;
anellated tricyclic imides such as naphthalin-1,2-dicarboximide, naphthalin-2,3-dicarboximide, 1,8-naphthalimide, diphenic acid imide;
anellated tetracyclic imides such as 7,8-dihydroacenaphthen-2(6H)-on-1,8a-dicarboximide, anthracen-2,3-dicarboximide, anthracen-1,9-dicarboximide, phenanthren-9,10-dicarboximide,
bridged polycyclic imides such as bicyclo[2.2.1]-hept-5-en-2,3-dicarboximide, 7-oxa-bicyclo[2.2.1]-hept-5-en-2,3-dicarboximide, benzobicyclo[2.2.2]-octan-2,3-dicarboximide, dibenzobicyclo[2.2.2]-octan-2,3-dicarboximide; and
spirocyclic imides such as spiro[dioxoimidazolidin-indane], spiro[dioxoimidazolidin-piperidine], spiro[dioxoimidazolidin-oxoindoline] and spiro[dioxoimidazolidin-tetrahydronaphthaline],
whereby these cyclic imides can be substituted by one to four of the same or different groups, selected independently from each other from
halogen, C₁-C₄-alkyl, trifluoromethyl, hydroxy, hydroxymethyl, methoxy, ethoxy, tert-butoxy, trifluormethoxy, benzyloxy, phenoxy, phenylthio, pyridylthio, phenylsulfonyl, sulfo, carboxy, C₂-C₇-carboxyalkyl, C₂-C₇-carboxyalkenyl, C₂-C₇-alkoxycarbonyl, benzyloxycarbonyl, nitro, amino, aminomethyl, dimethylamino, diethylamino, phenylamino, pyridylamino;
benzyl, benzylidene, phenylethyl, naphthylmethyl, diphenylmethyl, diphenylmethylene, triphenylmethyl, phenyl, naphthyl;
pyrrolidinyl, piperidinyl, morpholinyl, thiomorpholinyl, piperazinyl, hexahydroazepinyl, hexahydrodiazepinyl;
furyl, furylmethyl, thienyl, thienylmethyl, thiazolyl, thiazolylmethyl, pyridyl, pyridylmethyl;
benzofuryl, benzothienyl, indolyl, indolylmethyl, oxodihydroindolyl, benzoimidazolyl, benzoimidazolylmethyl, oxodihydrobenzoimidazolyl, benzooxazolyl, oxodihydrobenzooxazolyl, benzothiazolyl, oxodihydrobenzothiazolyl, quinolinyl, quinolinylmethyl, oxodihydroquinolinyl, isoquinolinyl, oxodihydroisoquinolinyl,
and whereby aryl and heteroaryl residues as substituents of the cyclic imides can be substituted themselves by one to three of the same or different groups selected from
halogen, cyano, C₁-C₆-alkyl, trifluoromethyl, C₃-C₈-cycloalkyl, benzyl, phenyl, hydroxy, C₁-C₆-hydroxyalkyl, C₁-C₆-alkoxy, C₁-C₆-alkoxy entirely or partially substituted by fluorine, benzyloxy, phenoxy, mercapto, C₁-C₆-alkylthio, phenylthio, carboxy, C₂-C₇-carboxyalkyl, C₂-C₇-carboxyalkenyl, C₂-C₇-alkoxycarbonyl, benzyloxycarbonyl, nitro, amino, C₁-C₆-aminoalkyl, mono-C₁-C₆-alkylamino, di-(C₁-C₆-alkyl)amino and, in the case of two adjacent residues, methylenedioxy.

Among this compound group the following imide substituted compounds of the general formula (I) as preferred in the use according to the invention:
N-[4-(2,5-dioxo-3,4-diphenyl-2,5-dihydropyrrol-1-yl)-butyl]-3-pyridin-3-yl-acrylamide; N-[4-(2,6-dioxo-4-phenylpiperidin-1-yl)-butyl]-3-pyridin-3-yl-acrylamide; N-[4-(1,3-dioxo-4,5,6,7-tetraphenyl-1,3-dihydro-isoindol-2-yl)-butyl]-3-pyridin-3-yl-acrylamide; N-[4-(3-benzyl-2,4,5-trioxoimidazolidin-1-yl)-butyl]-3-pyridin-3-yl-acrylamide; N-[4-(1,3,10-trioxo-1,4,5,6,10,10a-hexahydro-acenaphtho- [1,8a-c]pyrrol-2-yl)-butyl]-3-pyridin-3-yl-acrylamide; N-[4-(2,5-dioxo-4,4-diphenylimidazolidin-1-yl)-butyl-3-pyridin-3-yl-acrylamide; N-[4-(2,5-dioxo-3-phenyl-2,5-dihydropyrrol-1-yl)-butyl]-3-pyridin-3-yl-acrylamide; N-[3-(2,5-dioxo-3,4-diphenyl-2,5-dihydro-pyrrol-1-yl)-propyl]-3-pyridin-3-yl-acrylamide; N-[4-(3-pyridin-3-yl-acroylamino)-butyl]-2,3:5,6-dibenzobicyclo[2.2.2]octan-7,8-dicarboximide; N-[4-(5-benzyliden-2,4-dioxothiazolidin-3-yl)-butyl]-3-pyridin-3-yl-acrylamide; N-[4-(4-benzyl-2,6-dioxopiperazin-1-yl)-butyl]-3-pyridin-3-yl-acrylamide; N-[6-(2,5-dioxo-3,4-diphenyl-2,5-dihydropyrrol-1-yl)-hexyl]-3-pyridin-3-yl-acrylamide; N-[4-(2,5-dioxo-3,4-diphenyl-2,5-dihydropyrrol-1-yl)-butyl]-3-pyridin-3-yl-propionamide; N-[4-(1,3-dioxo-1,3-dihydroisoindol-2-yl)-butyl]-3-pyridin-3-yl-acrylamide; N-[4-(1,3-dioxo-1H,3H-benzo[de]isoquinolin-2-yl)-butyl]-3-(1-oxidogyridin-3-yl)-acrylamide; N-[6-(1,3-dioxo-1H,3H-benzo[de]isoquinolin-2-yl)-hexyl]-3-pyridin-3-yl-acrylamide; N-[2-(1,3-dioxo-1H,3H-benzo[de]isoquinolin-2-yl)-ethyl]-3-pyridin-3-yl-acrylamide as well as N-[4-(1,3-dioxo-1H,3H-benzo[de]isoquinolin-2-yl)-butyl]-3-pyridin-3-yl-acrylamide;

Furthermore, a group of compounds according to general formula, herein defined as general formual (Ic), which also falls under the above broader general formula (I), used according to the invention is: 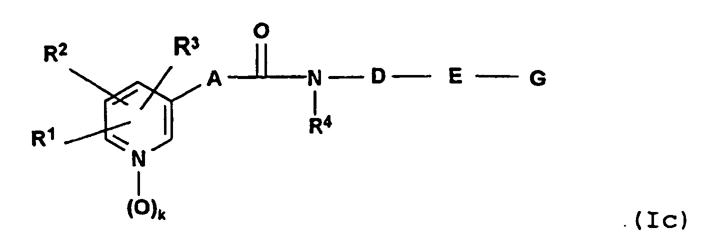 with the following substituent meanings being preferred:
- **R**^{**1**}: is selected from
hydrogen, fluorine, methyl, trifluoromethyl, ethylthio;
- **R**^{**2**}**, R**^{**3**} and **R**^{**4**}: are hydrogen;
- **k**: is 0,
- **A**: is selected from ethylene or butylene, optionally substituted by hydroxy or one or two fluorine atoms, or OCH₂, SCH₂;
ethenylene or 1,3-butadienylene;
- **D**: is selected from
C₃-C₈-alkylene, optionally substituted by hydroxy or phenyl; C₃-C₈-alkenylene, optionally substituted by phenyl; C₃-C₈-alkinylene; or
C₃-C₈-alkylene, C₃-C₈-alkenylene or C₃-C₈-alkinylene in which one or two methylene units are isosterically replaced by O, NH or CO;
- **E**: is a single or double bond;
- **G**: is selected from
cyclopentylphenylmethylene, cyclohexylphenylmethyl, cyclohexylhydroxyphenylmethyl, diphenylmethyl, diphenylhydroxymethyl, diphenylmethylene, diphenylethyl, diphenylhydroxyethyl, diphenylethylene, triphenylmethyl, triphenylethyl, triphenylhydroxyethyl, triphenylethylene, naphthylmethylene, naphthyl, tetrahydronaphthyl, hydroxytetrahydronaphthyl,
tetrahydronaphthylidene, fluoroenyl, hydroxyfluoroenyl, fluoroenylidene, tetrahydrobenzocycloheptenyl, hydroxytetrahydrobenzocycloheptenyl, tetrahydrobenzocycloheptanyl, dihydrodibenzocycloheptenyl, hydroxydihydrodibenzocycloheptenyl, dihydrodibenzocycloheptenylidene;
phenylthienylmethyl, phenylthienylhydroxymethyl, phenylthienylmethylene, dithienylmethyl, dithienylhydroxymethyl, dithienylmethylene, phenylfurylmethyl, phenylfurylhydroxymethyl, phenylfurylmethylene, phenylpyridylmethyl, phenylpyridylhydroxymethyl, phenylpyridylmethylene;
tetrahydroquinolinyl, tetrahydroisoquinolinyl, benzocycloheptapyridinyl, benzocycloheptapyridinylidene, dihydrobenzocycloheptapyridinyl, dihydrobenzocycloheptapyridinylidene, dihydrodibenzooxepinyl, dihydrodibenzooxepinylidene, dihydrodibenzothiepinyl, dihydrodibenzothiepinylidene;
phenylpyrrolyl, diphenylpyrrolyl, phenylthienyl, diphenylthienyl, phenylpyrazolyl, diphenylpyrazolyl, phenylimidazolyl, diphenylimidazolyl, phenylpyridyl, diphenylpyridyl, indolyl, oxoindolinyl, benzoimidazolyl, oxobenzoimidazolyl, benzothiazolyl, oxobenzothiazolyl, benzoisothiazolyl, benzooxazolyl, oxobenzooxazolyl, benzotriazolyl;
diphenylmethylamino, diphenylmethyl-methylamino, dibenzylamino, benzylphenylamino, cyclohexylphenylamino, triphenylmethylamino, biphenylylamino, diphenylamino; N-indolinyl, N-isoindolinyl, N-tetrahydroquinolinyl, N-tetrahydrobenzazepinyl, N-phenyltetrahydrobenzoazepinyl, N-1,1-dioxo-1-thia-2-aza-acenaphthenyl, N-1H,3H-benzo[de]-isoquinolinyl, N-dihydrodibenzoazepinyl;
diphenylmethyloxy, diphenylmethylthio;
diphenylacetylamino, diphenylacetylphenylamino, diphenylpropionylamino, diphenylacryloylamino, naphthylacetylamino, furoylacrylamino, benzoylamino, naphthoylamino, oxofluoroenylcarbonylamino, furoylamino;
diphenylmethylaminocarbonylamino, dibenzylaminocarbonylamino, naphthylmethylaminocarbonylamino, dibenzylaminocarbonylamino, biphenylylaminocarbonylamino, naphthylaminocarbonylamino, benzylphenylaminocarbonylamino, diphenylaminocarbonylamino; diphenylaminocarbonylphenylamino; diphenylfurylaminocarbonylamino, indolinyl-N-carbonylamino, isoindolinyl-N-carbonylamino, 1H,3H-benzo[de]isoquinolinyl-N-carbonylamino, tetrahydrobenzoazepinyl-N-carbonylamino, phenyltetrahydrobenzoazepinyl-N-carbonylamino, dihydrodibenzoazepin-N-carbonylamino, dihydrobenzopyridoazepinyl-N-carbonylamino;
tolylsulfonylamino, naphthylsulfonylamino, diphenylphosphinoylamino and diphenylphosphinoyloxy,
and whereby aromatic ring systems in **G** can be substituted independently from each other by one to three of the same or different groups selected from
halogen, cyano, C₁-C₆-Alkyl, trifluoromethyl, C₃-C₈-cycloalkyl, benzyl, phenyl, hydroxy, C₁-C₆-hydroxyalkyl, C₁-C₆-alkoxy, C₁-C₆-alkoxy entirely or partially substituted by fluorine, benzyloxy, phenoxy, mercapto, C₁-C₆-alkylthio, phenylthio, sulfo, carboxy, C₂-C₇-carboxyalkyl, C₃-C₇-carboxy-alkenyl, C₂-C₇-alkoxycarbonyl, benzyloxycarbonyl, nitro, amino, C₁-C₆-aminoalkyl, mono-C₁-C₆-alkylamino, di-(C₁-C₆-alkyl)amino and, for two adjacent residues in an aromatic ring, methylenedioxy, and
whereby alkyl and cycloalkyl residues in the group G can be substituted by one or two of the same or different groups, selected from
hydroxy, carboxy, C₂-C₇-alkoxycarbonyl, benzyloxycarbonyl, amino, mono-C₁-C₆-alkylamino and di-(C₁-C₆-alkyl)amino.

Among the above defined compound group, the following derivatives represent particularly preferred compounds used according to the invention:
N-[8,8-to-(4-fluorophenyl)-octyl]-3-pyridin-3-yl-acrylamide•hydrochloride; N-[6-(3,3-diphenylureido)-hexyl]-3-pyridin-3-yl-acrylamide; N-[4-(1-phenyl-1,2,4,5-tetrahydrobenzo[d]azepin-3-yl)-butyl]-3-pyridin-3-yl-acrylamide; N-(8,8-diphenyloctyl)-3-pyridin-3-yl-acrylamide; N-(8-hydroxy-8,8-diphenyloctyl)-3-pyridin-3-yl-acrylamide; N-[4-(3,3-diphenylureido)-butyl]-3-pyridin-3-yl-acrylamide; N-[4-(1H,3H-benzo[de]isoquinolin-2-yl)-butyl]-3-pyridin-3-yl-acrylamide; N-[6-(10,11-dihydro-dibenzo[b,f]azepin-5-yl-carbonyl-amino)-hexyl]-3-pyridin-3-yl-acrylamide; 3-pyridin-3-yl-N-[6-(tosylamino)-hexyl]-acrylamide; N-[4-(1,1-dioxo-1-thia-2-aza-acenaphthylen-2-yl)-butyl]-3-pyridin-3-yl-acrylamide; N-(6-hydroxy-6,6-diphenylhexyl)-3-pyridin-3-yl-acrylamide; N-(6,6-diphenyl-hex-5-enyl)-3-pyridin-3-yl-acrylamide; N-[4-(4,5-diphenylimidazol-1-yl)-butyl]-3-pyridin-3-yl-acrylamide; N-[4-(trans-2-phenylcyclopropylcarbonylamino)-butyl]-3-pyridin-3-yl-acrylamide; N-(5-hydroxy-5,5-diphenyl-pentyl)-3-pyridin-3-yl-acrylamide; N-(7-phenylheptyl)-3-pyridin-3-yl-acrylamide; N-(4-diphenylacetylamino-butyl)-3-pyridin-3-yl-acrylamide; N-[4-(benzhydrylamino)-butyl]-3-pyridin-3-yl-acrylamide as well as N-(4-{[2-(benzhydrylmethylamino)-ethyl]-methylamino}-butyl)-3-pyridin-3-yl-acrylamide.

The compounds in the form of their pharmaceutically acceptable acid addition salts with inorganic or organic acids can also be present with respect to the various groups of compounds presented above whereby preferred examples for addition salts with suitable inorganic acids are represented by hydrochlorides, hydrobromides, hydroiodides, sulfates and phosphates and addition salts of organic acids are preferably represented by acetates, benzoates, citrates, fumarates, gluconates, malates, maleates, methanesulfonates, lactates, oxalates, succinates, tartrates and tosylates. These compounds can also optionally be present as hydrates or other solvates.

This is also valid in the same manner for the stereoiosomers, which include the cis- and trans-isomers as well as E- and Z-isomers of the above defined compounds, especially in the case that D contains one or more double bonds, including the enantiomers, diastereomers and other isomers of the above defined compounds, in optionally pure form or as their racemic and/or non-racemic mixtures.

A multitude of compounds according to the general formula (I) are previously described in the publications PCT/EP97/03243=WO97/48695 with the priority DE 196 24 704.7, PCT/EP97/03245=WO97/48696 with the priority DE 19624 659.8 and PCT/EP97/03244=WO97/48397 with the priority DE 196 24 668.7 with respect to their known uses discussed there as well as their production. The remaining compound groups of the compounds according to the general formula (I) are subject matter of the older priority patent applications in comparison to the present application subject matter (i.e. not yet published at the present filing date). DE-19756212.4 dated 17.12.1997, DE-19756235.3 dated 17.12.1997, DE-19756236.1 dated 17.12.1997 and DE-19756261.2, also dated 17.12.1997, whereby the compounds described therein are distinguished namely by pharmacological, preferably cancerostatic activities.

The production of the compounds falling under the general formula (I) as well as their sub-groups according to the above general formulas (Ia) to (Ic) is well-known to experts. Greater details on the production process in the form of so-called analogy methods, the starting materials (educts) and intermediate products (intermediates) necessary for this are disclosed in the above-mentioned PCT and/or DE patent applications.

Their possible stereoisomers depending on the chemical structures, the cis- and trans-isomers as well as E- and Z-isomers including the enantiomers, diastereomers and other isomers in optionally pure.form or as their racemic and/or non-racemic mixtures are also included, as well as the tautomers of the above defined compounds as well as the corresponding pharmacologically acceptable acid addition salts of the above defined compounds. Furthermore, for example, acid addition salts of the above compounds with organic acids such as acetates, ascorbates, benzoates, citrates, fumarates, gluconates, malates, maleates, methansulfonates, lactates, oxalates, succinates, tartrates and tosylates or inorganic acids such as hydrochlorides, hydrobromides, hydroiodides, sulfates and phosphates including their hydrates and solvates as well as the anionic salts, such as alkali and alkaline earth salts such as the sodium, potassium or magnesium and calcium salts and other salts such as aluminium salts are also included.

In a particularly preferred manner, nicotinic acid and/or the nicotinamide are used as vitamin PP active compounds.

Preferably, compounds with vitamin PP activity and/or corresponding prodrugs according to the invention are used for example in the form of the following compounds, which fall, in part, under the general formulas (II) - (Vb) N¹-alkylnicotinamide, such as, for example, N¹-methylnicotinamide or N¹-ethylnicotinamide, nicotinic acid diethylamide, diethylaminoethylnicotinate, fructofuranose-1,3,4,6-tetranicotinate, butoxyethylnicotinate, 2-diethylaminoethyl-p-nicotinamidobenzoate, nicotinoylprocaine, N-{3-[4-(p-fluoro-phenyl)-1-piperazinyl]-1-methylpropyl}-nicotinamide, nicotinylalcohol (3-pyridylcarbino), tetrahydrofurfurylnicotinate, 2-hexyl-2-(hydroxymethyl)-1,3-propandioltrinicotinate, 3-[α-(p-chlorphenoxy)-isobutyryloxy]-propylnicotinate, N-(α-methylphenethyl)-nicotinamide, N-(1,2-diphenylethyl)-nicotinamide, morphindinicotinate, 4-nicotinamido-1,5-dimethyl-2-phenyl-3-pyrazolone, N-(2-hydroxyethyl)-nicotinamide, optionally as a nitrate, 3-pyridylmethyl ester, 2-(4-chlorophenoxy)-2-methylpropionic acid, 1-(p-chlorophenyl)-isobutylnicotinate, 5-methoxy-2-methyl-1-nicotinoylindol-3-acetic acid, N-(2-hydroxymethyl)-nicotinamido-2-(p-chlorophenoxy)-2-methylpropionate, N-(hydroxymethyl)-nicotinamide, nicotindiethylamid-N¹-methylhydroxide, 4-nicotinoylmorpholine, 17β-hydroxy-4-andro-sten-3-on-17-nicotinate, estradiol-17-nicotinat-3-propionate, 4-(6,7-dimethoxy-1-isoquinolylmethyl)-o-phenylendinicotinate, trans-3,3,5-trimethylcyclohexylnicotinate, 1,10-bis-(2-nicotinoyloxyethylthio)-decane,
4,5-epoxy-3-methoxy-N-methyl-7-morphinen-6-ylnicotinate, 4,5-epoxy-3-methoxy-17-methylmorphinan-6-ylnicotinate, 4,5-epoxy-17-methylmorphin-7-en-3,6-diyldinicotinate, 17-allyl-4,5-epoxymorphin-7-en-3,6-diyldinicotinate, 2,3-dihydroxypropyl-2-(3-chloro-o-toluidino)-nicotinate, 2-morpholinoethyl-2-(α,α,α-trifluoro-m-toluidino)-nicotinate,
2-(1,3-dichloroanilino)-nicotinic acid, 3-pyridylmethanolnicotinate, 3-hydroxymethylpyridine, optionally in form of the nicotinyltartrate or nicotinylmaleate, acetamidophenylnicotinate, N-(α-methylphenethyl)-thionicotinamide,
N-(2,3-dimethyl-5-oxo-1-phenyl-3-pyrazolin-4-yl)-nicotinamide,
N-methyl-N-(2,3-dimethyl-5-oxo-1-phenyl-3-pyrazolin-4-yl)-nicotinamide, 4-(3-trifluoromethylanilino)-nicotinic acid, 2-(3-chloro-o-toluidino)-nicotinic acid, 2-(2-methyl-3-trifluoromethylanilino)-nicotinic acid, 2-(2,3-xylidino)-nicotinic acid, 2-(2,6-xylidino)-nicotinic acid, 2-(p-butylanilino)-nicotinic acid, 2-hydroxy-2',6'-nicotinoxylidide, N-(2,3-dimethyl-5-oxo-1-phenyl-3-pyrazolin-4-yl)-α-nicotinamido-acetamide, N-(1,2-diphenylethyl)-nicotinamide, N-(1-phehyl-heptyl)-nicotinamide, nicotinamide adenine dinucleotide, or N-[N-iso-propyl-N-(2,3-dimethyl-5-oxo-1-phenyl-3-pyrazolin-4-yl)-aminomethyl]-nicotinamide.

As further named compounds already partially mentioned under the above exemplified compounds or corresponding prodrugs as particularly preferred substances with vitamin PP activity which fall under the general formulas (II) - (Vb), the following are named:
the ethanolamine salt, sodium salt, aluminium salt or magnesium salt of the nicotinic acid, the (RS)-7-{2-hydroxy-3-[2-hydroxyethyl)-methylamino]-propyl}-theophylline salt of the nicotinic acid, the N-oxide of the nicotinic acid, methyl-, ethyl-, 2-diethylaminoethyl-, 2-hydroxyethyl-, 2-aminoethyl-, propyl-, isopropyl-, 2-butoxyethyl-, tetrahydrofurfuryl-, 3-pyridinylmethyl-, hexyl-, 2-diethylaminoethyl-, benzyl-, oxyethyl-theophyllin-, 1-(4-chlorophenyl)-2-methylpropyl-, 2-[2-(4-chlorophenoxy)-2-methyl-1-oxopropoxy]-ethyl-, 2,5,7,8-tetramethyl-2-(4,8,12-trimethyltridecyl)-chroman-6-yl- or 3,3,5-trimethylcyclohexyl ester of nicotinic acid, dinicotinate of 7-(2,3-dihydroxypropyl)-theophylline, trinicotinate of 2-hexyl-2-hydroxymethyl-1,3-propantriol, tetranicotinate of pentaerythritol, 1,3,4,6-tetranicotinate of d-fructofuranose, 1,1,3,3-tetranicotinate of 2-hydroxy-1,1,3,3-cyclohexantetramethanol, the 2',3',4',5'-tetra-nicotinate of riboflavin, the hexanicotinate of mesoinositol or the hexanicotinate of sorbitol, 2-methyl-3-carbomethoxy-5-(1,2,3,4-tetranicotinoyloxybutyl)-furan, 8β-[(5-bromonicotinoyloxy)-methyl]-1,6-dimethyl-10α-methoxy-ergoline, 3-pyridincarboxylic acid methylester methylhydroxide and optionally its iodide, 4-[(3-pyridinylcarbonyl)-amino]-benzoic acid-2-(di-ethylamino)-ethyl ester, 7-(2-)-hydroxyethyltheophylline nicotinate, 2-[2-(4-chlorophenoxy)-2-methyl-1-oxopropoxy]-ethylnicotinate, nicotinic acid methylbetaine, N¹-methylnicotinamide chloride or nicotinic acid diethylamide.

Depending on the particular pharmacokinetics, for example, pH dependent pharmacokinetics, and particular metabolism, the named derivatives can have a prolonged effect optionally as a prodrug, or can be used topically, locally or regionally as a pharmaceutical with partially additional pharmacological effects such as increasing circulation, as analgesic agents or as morphine antagonists, especially in connection with the cancerostatic chemotherapy deployed according to the invention with the aim of neutralizing or decreasing side-effects.

A further particularly preferred embodiment of the invention is in the use of tryptophan as a vitamin PP precursor compound which is metabolized in vivo to a compound with vitamin PP activity (prodrug).

According to the a further particularly preferred embodiment according to the invention, one or more cancerostatic or immunosuppressive compounds different from the tumor inhibitors according to the general formula (I) is used in addition to the compound mixture comprising a cancerostatic agent, especially a compound according to the general formula (I), plus a compound with vitamin PP activity. This means that in the use according to the invention of vitamin PP compounds and/or the combinations according to the invention with an amount of these compounds, further cancerostatic agents, optionally alone, are considered in addition to the anti-tumor compounds especially those of the general formula (I), in cytostatic agents or immunosuppressive agents applied in chemotherapy in the form of the following compounds or compound groups:
Alkylizing, cytostatic agents (alkylanizers), for example derivatives without nitrogen such as cyclophosphamide, trofosfamide, ifosfamide, melphalane, mechloroethamine, chlorambucil as well as ethylenimine derivatives (aziridines) such as thiotepa. A further alkylizing compound would be for example busulfane. Additionally, among these exemplified alkylating cytostatic agents are carmustine, lomustine or nimustine, which can be considered in a wider sense as belonging to this compound group of alkylanizers as N-nitroso-urea derivatives. For example, procarbazine and dacarbazine also belong to these. Diaziquone, hexamethylmelamine, trimelamole or temozolamide are also referred to with respect to the series of alkylanizers.

Examples for cancerostatic anti-metabolites are folic acid antagonists, such as methotrexate, edatrexate, trimetrexate or its ethyl derivative 10-ethyl-10-desaza-aminopterine as well as purine and pyrimidine base antagonists or antagonists with other points of attack, for example, 6-mercaptopurine and 6-thioguanine, 5-fluorouracil, floxuridine, ara-c, hydroxyurea, desoxycoformycine, triazofurine, acivicine, PALA, pyrazofurine as well as cytarabine, vidarabine, fludarabine, desoxycoformycine, 2-chloro-2'-desoxy-adenosine or tiazofurine.

In this connection, various IFN compounds, IL2 or leucovorine are particularly considered as synergists.

A further important group of cancerostatic agents are represented by the platinum complexes such as cisplatin and carboplatin . Podophyllotoxins or epipodophyllotoxins as antimitotic agents such as etoposide or teniposide have achieved certain importance as cancerostatic agents. This is true in a similar manner for vincaalkaloids, for example vinblastin, vincristin, vindesin, navelbin or 17-desacetylvinblastin.
Cytostatically effective antibiotics capable of being combined are also considered in connection with the invention such as, for example, actinomycins such as dactinomycin or actinomycin D, anthracyclines such as daunorubicin, doxorubicin, rubidazone, detorubicin, epirubicin, carminomycin, aclacinomycin A, marcellomycin, AD-32, THP-doxorubicin, esorubicin, idarubicin, menogaril, or mitoxantrone or bleomycin as well as mitomycin C with comparable antibiotic effects. Morpholinyl derivatives with the name MRA, MRA-MO, MRA-CN or MRD-nr-CN or the morpholinyl anthracycline derivative MX2 also belong to these.

The series of the cancerostatic agents from different compound groups can be extended with newer compounds such as L-alanosine, amonafide, amsacrine, mafosfamide, bisantrene, carbethimer, desoxyspergualine, α-difluoromethylornithine, mitolactol, didemnine B, echinomycin, ellipticiniumacetate, gallium nitrate, optionally, cimetidine or retinoids, homoharringtonine, razoxane, indicin-N-oxide, lonidamine, menogaril, mitiguazone, N-methylformamide, mitozolamide, piritrexime, spirogermanium, spiromustin, suramin, taxol, taxoter, teroxirone, hydroxyurea, 1,2,4-triglyci-dylurazole, 1-asparaginase, lentinane, vinzolidine or radioactive isotopes such as ³²P, ⁹⁰Y, ²¹¹At or ¹³¹I, which can each be coupled to an antibody for targeting purposes.

An important group of cancerostatic agents are represented by hormonal compounds, i.e. hormones themselves such as androgens/anti-androgens, glucocorticoids or estrogens/anti-estrogens, depot estrogens, gestagens as well as hormone antagonists/hypothalamus hormones, estrogen receptor antagonists or aromatase inhibitors. Among others, examples of compounds of this type are mentioned in the following: busereline, gosereline, leuproreline, triptoreline, such as for example, stilbestrol, diethylstilbestrol, megestrol(acetate), diethyl-stilbestrol diphosphate, estradiol(undecylate), gonadoliberine, fosfestrol, estramustine, tamoxifene, drostanolone(propionate), fluoxymesterone, testolactone, aminoglutethimide, mitotane, medroxiprogesterone(acetate) flutamide, megestrol(acetate), aminoglutethimide, 4-hydroxyandrostendione, plumestane, exemestane, pyridoglutethimide, fadrazole, arimidex, vorazole, flutamide, cyproterone, mifepristone, hydroxyprogesterone, progesterone or ethinylestradiol,

For immunosuppressive therapies cyclosporine can be used for example, whereby corticosteroids, such as for example, prednisone or methylprednisolol as well as methotrexate and azathioprine or cyclophosphamide can also be applied. A known combination of immunosuppressive agents of this type is the combined use of cyclosporine or prednisone together with azathioprine.

Included in the series of the above customary, exemplified cancerostatic agents as well as cancerostatic agents not named here are pharmacolgically acceptable salts, esters, ethers, optic isomers, stereoisomers and other analogously effective derivatives depending on their structure.

The preferred single doses of the cancerostatic agents and/or immunosuppressive agents are oriented in accordance with customary therapeutic recommendations given in the literature and company information. Depending on effectiveness and the therapeutic spectrum of the cytcostatic agents, they range for example from 0,1 mg up to 10 000 mg. Expressed in absolute dosage units, the unit doses can be 0.1, 0.2, 0.5, 1, 2, 5, 10, 20, 50, 75, 100, 150, 200, 300, 400, 500, 600, 750, 800, 900, 1000, in individual cases up to 1 200, 1 500, 2 000, 5 000 or 10 000 mg. In the case of the use of interferon compounds, the administered dosage amounts expressed in units are for example 1, 2, 3, 4, 5, 10, 15, 20, 25, 30, 40 or 50 million units. This is also true in a similar manner for cancerostatic enzymes, such as for example, asparaginases, which can be administered in an amount of 1000, 6000 or 10 000 units per m² body surface.
The given dosage units also extend to the application of pyridylcarboxamide compounds according to the general formula (I), which can lie in the range between 0.1 mg, 1, 10 mg up to 5000 or 10 000 mg individual doses as discussed above. Depending on the existence of a synergistic, super-additive effect combination of two or more cancerostatic agents, the customary dosages can also be reduced. Therewith, the side-effects associated with the administration of cancerostatic agents can be further reduced. Conversely, if need be, considerably higher dose of cancerostatic agents can be administered without danger for patients in the case that the vitamin PP compounds reduce the side-effects in accordance with the invention.

The above discussion of the respective suitable cancerostatic agents merely has an exemplifying character and no limiting character in any way because it cannot be complete as a result of the incalculable abundance of known medicaments; further customary cancerostatic agents and carrier systems for this are to be taken from relevant handbooks such as for example M.Peckham, H.Pinedo and U. Veronesi: Oxford Textbook of Oncology Vol. 1 & Vol. 2, Oxford University Press, Oxford, New York, Tokyo (1995) or W.Forth, D.Henschler, W. Rummel, , K.Starke: General and specific pharmacology, SPEKTRUM, Heidelberg, Berlin Oxford, 7. Edition (1996) or E. Mutschler: Medicament activities, textbook of pharmacology & toxicology, WVG, Stuttgart, 6. Edition (1991) or other compendia and the remaining literature or the art on anti-tumor agents.

In the use according to the invention, the dosage units of compounds of the general formula (I) respectively applied for the reduction, elimination or prevention of less severe side-effects lie in the range 5, 10, 20, 25, 30, 50, 75, 100, 200, 250, 300, 400, 500, 750 mg to 1000 mg and for eliminating a acute side-effects and/or neutralizing the cancerostatic agent effects lie in the range of 100, 250, 300, 500, 750 or 1000 up to 10 000 mg.

Furthermore, medicaments are created according to the invention which are characterized in that they contain one or more compounds of the general formula (I) as well as one or more compounds of the above defined general formulas (II) to (Vb) aside from physiologically acceptable carriers and toxicologically safe adjuvants.

Aside from cancerostatic chemotherapy, the medicaments and modes of use made available according to the invention are also connected with anti-proliferative therapy and metastases formation inhibiting, i.e. prophylactic, therapy or the control of immune reactions such as autoimmune diseases or tumor diagnostics.

According to the invention, the above named medicaments can also be distinguished in that they contain one or more further cancerostatic or immunosuppressive compounds which are different from the compounds according to general formula (I) in addition to the cancerostatic agents, especially the tumor inhibitors according to the general formula (I).

In the above named indications, for example for reduction, elimination or prevention of less severe side-effects, the respective dosage units of compounds of the general formula (I) in the medicaments can be in the range of 5, 10, 20, 25, 30, 50, 100, 200, 250, 300, 500, 750, 1000 mg to 1000 mg and 100, 200, 250, 300, 500, 750 or 1000 to 10 000 mg for elimination of acute side-effects and/or for neutralization of cancerostatic agent effects.

In addition to the cancerostatic agents in combination with one or several compounds of the general formulas (II) to (Vb), the medicaments can contain one or more active ingredients customary in the indications given in the above claims. Preferably, the medicaments are present in a solid, peroral administrable form as a table, capsule, coated tablet, optionally as a sustained action and/or gastric fluid-resistant preparation, or as a liquid medicinal form, for example as a respective peroral administrable solution, suspension effervescent tablet, in the form of tabs or sachets optionally, in sustained action form and/or with protracted and/or accelerated or controlled release of the active ingredients. Preferably, the above named medicinal forms are used in connection with the application according to the invention.

Pursuant to a further preferred embodiment according to the invention, the medicaments are characterized in that the active ingredients combined with each other are contained separate from each other and/or in separate dosage units in the pharmaceutical package (kit-of-parts).

A further embodiment according to the invention relates to a medicament in the form of a suitable injection or infusion preparation together with suitable pharmaceutically acceptable carriers and adjuvants, optionally in sustained action form and/or as a parenteral depot form or implant or in the form of a concentrate, powder or lyophilisate, whereby the parenteral dilution agent can be optionally manufactured in the package separately therefrom such that the mixing of the compounds contained therein with the common parenterally applicable dilution agent is possible immediately before use.

Furthermore, the medicament can preferably be in the form of an inhalation therapeutic agent, for example, in the form of a spray together with suitable pharmaceutically acceptable propellants, carriers and adjuvants, whereby the pharmaceutically acceptable carrier and/or diluent can be an aerosol propellant.

Preferably, the medicament can also be present in the form of transdermal therapeutic system for systematic treatment.

Moreover, it can also preferably be present in the form of a gastro-intestinal therapeutic system (GITS) for systematic treatment.

As a topical administrable medicament, it can preferably be formulated as a salve, suspension, emulsion, balm, or plaster or as an externally applicable solution.

Further preferred embodiments of the medicament used or produced according to the invention are in the application forms of dose aerosols, dry powder dosage formulations, rectal genital or transurethral administrable emulsions, solutions, liposomal solutions, implants, suppositories or capsules.

Equally, the medicaments used or compounded according to the invention are preferably in the form of compositions capable of being applied nasally, otologically or ophthalmologically in buccally applicable form.

According to the invention, the dosage units applied or contained in the medicaments for single administration can be 0.001 up to 1000, 2000, 3000, 4000 or 5000 mg, preferably 0.01 - 100 mg, in a preferred manner 1 - 10 mg, especially 1, 2, 5, 10, 20, 25, 30, 50, 75, 100, 200, 300, 400, 500, 600 or 800 mg cancerostatic agent, especially a tumor inhibitor of the general formula (I).

In the case that the medicament is present as a propellant aerosol, one or more propellants can preferably be tetrafluoroethane and/or heptafluoropropane and/or propane, butane or dimethylether or their mixtures, whereby the propellant aerosol can contain surface adjuvants.

In the case that the medicament is present as a dry powder dosage formulation, glucose and/or lactose can be contained as carrier agents.

In a preferred manner, the medicament with an amount of a prodrug can have an amount of tryptophan, whereby, in this case, it preferably can contain 30-, 40-, 50- or 60-fold amount of this prodrug tryptophan in comparison to m nicotinamide as a vitamin PP reference substance for example, 500, 1000, 2000, 3000, 5000, 10 000 or 50 000 mg. The respective suitable or preferred dosage units for other prodrugs such as analogous esters and the like can easily be determined in individual cases based on the molecular portions and based on easily determined pharmacokinetic data for the patients and/or individual tumor type or other indication.

### Therapeutic Administration Forms - Medicaments

The production of medicaments with an amount of one or more compounds according to the invention and/or their use in the application according to the invention occurs in the customary manner by means of common pharmaceutical technology methods. For this, the active ingredients as such or in the form of their salts are processed together with suitable, pharmaceutically acceptable adjuvants and carriers to medicinal forms suitable for the various indications and types of application. Thereby, the medicaments can be produced in such a manner that the respective desired release rate is obtained, for example a quick flooding and/or a sustained or depot effect.

Preparations for parenteral use, to which injections and infusions belong, are among the most important systematically employed medicaments for tumor treatment as well as for other indications.

Preferably, injections are administered for the treatment of tumors. These are prepared either in the form of vials or also as so-called ready-to-use injection preparations, for example as ready-to-use syringes or single use syringes in addition to perforation bottles for multiple withdrawals. Administration of the injection preparations can occur in the form of subcutaneous (s.c.), intramuscular (i.m.), intravenous (i.v.) or intracutaneous (i.c.) application. The respective suitable injection forms can especially be produced as solutions, crystal suspensions, nanoparticular or colloid-disperse systems, such as for example, hydrosols.

The injectable formulations can also be produced as concentrates which can be adjusted with aqueous isotonic dilution agents to the desired active ingredient dosage. Furthermore, they can also be produced as powders, such as for example lyophilisates, which are then preferably dissolved or dispersed immediately before application with suitable diluents. The infusions can also be formulated in the form of isotonic solutions, fat emulsions, liposome formulations, microemulsions and liquids based on mixed micells, for example, based on phospholipids. As with injection preparations, infusion formulations can also be prepared in the form of concentrates to dilute. The injectable formulations can also be applied in the form of continuous infusions as in stationary as well as in out-patient therapy, for example in the form of mini-pumps.

Albumin, plasma expanders, surface active compounds, organic solvents, pH influencing compounds, complex forming compounds or polymeric compounds can be added to the parenteral medicinal forms, especially as substances for influencing the adsorption of the active ingredients to protein or polymers or also with the aim of decreasing the adsorption of the active ingredient to materials such as injection instruments or packaging materials, for example plastic or glass.

Depending on the chemical structure and binding capability, the cancerostatic agents can be used in the form of conjugates with albumin or compounds none convalently bound to albumin for example; see for this among others the publications US-A-5 308 604, DE-A-195 14 087, DE-A-195 48 114, DE-A-196 02 295, DE-A-4 122 210, DE-A-4 433 890, DE-A-4 435 087, WO96/09071, WO96/25956, WO96/32134, PCT/DE/96/02487 or PCT/DE97/00166. Corresponding experiments were also undertaken with conjugates based on transferrin, cross-linked dextran, fibrinogen, casein or hemoglobin. Additionally, experiments to couple cancerostatics and/or their prodrugs with suitable antibodies used in tumor therapy seem to be promising. Combinations or monotherapies of this type with antibodies are therefore included within the present scope of the invention. Moreover, targeting experiments with albumin magnetically marked with magnetite (Fe³O⁴) were undertaken in order to enrich the tumor inhibitor in the form of so-called microballs (microspheres) in higher concentration at the place of action.

Carrier systems of this type are suitable as targeting transport media to enable a targeted delivery of the pharmaceutical to the target organ. In this manner, protection of healthy tissue can be better ensured, a greater enrichment of the active ingredient can be obtained, and, finally, better protection of cancerostatic agents sensitive against biological degradation can be obtained during the transport to the place of action. For these purposes, active ingredient liposomes can be produced in a known manner by means of coating and/or embedding methods by coating suitable carriers, whereby the active ingredients are encapsulated within membranes having a thickness down to the nanometer range. With respect to the art on liposomal compositions, reference is made here to US-A-4 895 719 (=EP-A-0 223 831) and the publication WO-A-9 116 882 merely as representatives for many preparation methods.

There are numerous types of microballs (microspheres) or microparticles based on different adjuvants as carrier systems such as lipoproteins of low density (low density lipoproteins), activated charcoal particles, nanocapsules, nanoparticles, unilaminare or multilaminare vesicles (liposomes), polystyrol latex particles, microcapsules, whereby experiments with erythrocytes as a carrier medium were even carried out. For example polycyanoacrylates, polyacrylamide, gelatin based on cancerostatic agent/dextran conjugates, agarose, "ferromagnetic" ethylcellulose, poly(HEMA-BGA), carnauba wax, starch, vesicles of non-ionic tensides (niosomes), albumin/polyaspartic acid, phospholipids, viral sub-units (which lead to the formation of so-called virosomes) or polylactide (polylactic acid ester) are considered as carriers for microparticle systems of this type.

The active ingredients can be bound to nanoparticles in the preparations for parenteral use, for example on finely dispersed particles based on poly(meth)acrylates, polyacetates, polyglycolates, polyamino acids or polyether urethanes. The parenteral formulations can also be constructively modified as depot preparations, for example on the multiple unit principle, where the active ingredients are incorporated in a most finely distributed and/or dispersed, suspended form or as crystal suspensions, or on the single unit principle, where the active ingredient is enclosed in a medicinal form, for example, a tablet or a seed which is subsequently implanted. Often, these implantations or depot medicaments in single unit and multiple unit medicinal forms consist of so-called biodegradable polymers, such as for example, polyether urethanes of lactic and glycolic acid, polyether urethanes, polyamino acids, poly(meth)acrylates or polysaccharides.

Sterilized water, pH value influencing substances, such as for example organic and inorganic acids or bases as well as their salts, buffer substances for setting the pH value, agents for isotonicity, such as for example sodium chloride, monosodium carbonate, glucose and fructose, tensides and/or surface active substances and emulsifiers, such as for example, partial fatty acid esters of polyoxyethylene sorbitan (Tween®) or for example fatty acid esters of polyoxethylene (Cremophor®), fatty oils such as for example peanut oil, soybean oil and castor oil, synthetic fatty acid esters, such as for example ethyl oleate, isopropyl myristate and neutral oil (Miglyol®) as well as polymer adjuvants such as for example gelatine, dextran, polyvinylpyrrolidone, organic solvent additives which increase solubility, such as for example propylene glycol, ethanol, N,N-dimethylacetamide, propylene glycol or complex forming compounds such as for example citrates and urea, preservatives, such as for example hydroxypropyl benzoate and hydroxymethyl benzoate, benzyl alcohol, anti-oxidants, such as for example sodium sulphite and stabilizers, such as for example EDTA, are suitable as adjuvants and carriers in the production of preparations for parenteral use.

In suspensions, addition of thickening agents to prevent the settling of the active ingredients from tensides and peptizers, to secure the ability of the sediment to be shaken, or complex formers, such as EDTA, ensues. This can also be achieved with the various polymeric agent complexes, for example with polyethylene glycols, polystyrol, carboxymethylcellulose, Pluronics® or polyethylene glycol sorbitan fatty acid esters. The active ingredient can also be incorporated in liquid formulations in the form of inclusion compounds, for example with cyclodextrins. As further adjuvants, dispersion agents are also suitable. For production of lyophilisates, builders are also used, such as for example mannite, dextran, saccharose, human albumin, lactose, PVP or gelatine varieties.

As long as the active ingredients are not incorporated in the liquid medicinal formulations in the form of a base, they are used in the form of their acid addition salts, hydrates or solvates in the preparations for parenteral use.

A further systemic application form of importance is peroral administration as tablets, hard or soft gelatine capsules, coated tablets, powders, pellets, microcapsules, oblong compressives, granules, chewable tablets, lozenges, gums or sachets. These solid peroral administration forms can also be prepared as sustained action and/or depot systems. Among these are medicaments with an amount of one or more micronized active ingredients, diffusions and erosion forms based on matrices, for example by using fats, wax-like and/or polymeric compounds, or so-called reservoir systems. As a retarding agent and/or agent for controlled release, film or matrix forming substances, such as for example ethylcellulose, hydroxypropylmethylcellulose, poly(meth)acrylate derivatives (for example Eudragit®), hydroxypropylmethylcellulose phthalate are suitable in organic solutions as well as in the form of aqueous dispersions. In this connection, so-called bio-adhesive preparations are also to be named in which the increased retention time in the body is achieved by intensive contact with the mucus membranes of the body. An example of a bio-adhesive polymer is the group of Carbomers®.

For sublingual application, compressives, such as for example non-disintegrating tablets in oblong form of a suitable size with a slow release of active ingredient, are especially suitable. For purposes of a targeted release of active ingredients in the various sections of the gastrointestinal tract, mixtures of pellets which release at the various places are employable, for example mixtures of gastric fluid soluble and small intestine soluble and/or gastric fluid resistant and large intestine soluble pellets. The same goal of releasing at various sections of the gastrointestinal tract can also be conceived by suitably produced laminated tablets with a core, whereby the coating of the agent is quickly released in gastric fluid and the core of the agent is slowly released in the small intestine milieu. The goal of controlled release at various sections of the gastrointestinal tract can also be attained by multilayer tablets. The pellet mixtures with differentially released agent can be filled into hard gelatine capsules.

Anti-stick and lubricant and separating agents, dispersion agents such as flame dispersed silicone dioxide, disintegrates, such as various starch types, PVC, cellulose esters as granulating or retarding agents, such as for example wax-like and/or polymeric compounds on the basis of Eudragit®, cellulose or Cremophor® are used as a further adjuvants for the production of compressives, such as for example tablets or hard and soft gelatine capsules as well as coated tablets and granulates.

Anti-oxidants, sweetening agents, such as for example saccharose, xylite or mannite, masking flavors, aromatics, preservatives, colorants, buffer substances, direct tableting agents, such as for example microcrystalline cellulose, starch and starch hydrolysates (for example Celutab®), lactose, polyethylene glycols, polyvinylpyrrolidone and dicalcium phosphate, lubricants, fillers, such as lactose or starch, binding agents in the form of lactose, starch varieties, such as for example wheat or corn and/or rice starch, cellulose derivatives, for example methylcellulose, hydroxypropylcellulose or silica, talcum powder, stearates, such as for example magnesium stearate, aluminium stearate, calcium stearate, talc, siliconized talc, stearic acid, acetyl alcohol or hydrated fats, etc. are also used.

In this connection, oral therapeutic systems constructed especially on osmotic principles, such as for example GIT (gastrointestinal therapeutic system) or OROS (oral osmotic system), are also to be mentioned.

Effervescent tablets or tabsolute both of which represent immediately drinkable instant medicinal forms which are quickly dissolved or suspended in water are among the perorally administrable compressives.

Among the perorally administrable forms are also solutions, for example drops, juices and suspensions, which can be produced according to the above given method, and can still contain preservatives for increasing stability and optionally aromatics for reasons of easier intake, and colorants for better differentiation as well as antioxidants and/or vitamins and sweeteners such as sugar or artificial sweetening agents. This is also true for inspissated juices which are formulated with water before ingestion. Ion exchange resins in combination with one or more active ingredients are also to be mentioned for the production of liquid injectable forms.

A special release form consists in the preparation of so-called floating medicinal forms, for example based on tablets or pellets which develop gas after contact with body fluids and therefore float on the surface of the gastric fluid. Furthermore, so-called electronically controlled release systems can also be formulated by which active ingredient release can be selectively adjusted to individual needs.

A further group of systemic administration and also optionally topically effective medicinal forms are represented by rectally applicable medicaments. Among these are suppositories and enema formulations. The enema formulations can be prepared based on tablets with aqueous solvents for producing this administration form. Rectal capsules can also be made available based on gelatine or other carriers.

Hardened fat, such as for example Witepsol®, Massa Estarinum®, Novata®, coconut fat, glycerol-gelatine masses, glycerol-soap-gels and polyethylene glycols are suitable as suppository bases.

For long-term application with a systematic active ingredient release up to several weeks, pressed implants are suitable which are preferably formulated on the basis of so-called biodegradable polymers.

As a further important group of systematically active medicaments, transdermal systems are also to be emphasized which distinguish themselves, as with the above-mentioned rectal forms, by circumventing the liver circulation system and/or liver metabolism. These plasters can be especially prepared as transdermal systems which are capable of releasing the active ingredient in a controlled manner over longer or shorter time periods based on different layers and/or mixtures of suitable adjuvants and carriers. Aside from suitable adjuvants and carriers such as solvents and polymeric components, for example based on Eudragit®, membrane infiltration increasing substances and/or permeation promoters, such as for example oleic acid, Azone®, adipinic acid derivatives, ethanol, urea, propylglycol are suitable in the production of transdermal systems of this type for the purpose of improved and/or accelerated penetration.

As topically, locally or regionally administration medicaments, the following are suitable as special formulations: vaginally or genitally applicable emulsions, creams, foam tablets, depot implants, ovular or transurethral administration instillation solutions. For opthalmological application, highly sterile eye ointments, solutions and/or drops or creams and emulsions are suitable.

In the same manner, corresponding otological drops, ointments or creams can be designated for application to the ear. For both of the above-mentioned applications, the administration of semi-solid formulations, such as for example gels based on Carbopols® or other polymer compounds such as for example polyvinylpyrolidone and cellulose derivatives is also possible.

For customary application to the skin or also to the mucus membrane, normal emulsions, gels, ointments, creams or mixed phase and/or amphiphilic emulsion systems (oil/water-water/oil mixed phase) as well as liposomes and transfersomes can be named. Sodium algenate as a gel builder for production of a suitable foundation or cellulose derivatives, such as for example guar or xanthene gum, inorganic gel builders, such as for example aluminium hydroxides or bentonites (so-called thixotropic gel builder), polyacrylic acid derivatives, such as for example Carbopol®, polyvinylpyrolidone, microcrystalline cellulose or carboxymethylcellulose are suitable as adjuvants and/or carriers. Furthermore, amphiphilic low and high molecular weight compounds as well as phospholipids are suitable. The gels can be present either as hydrogels based on water or as hydrophobic organogels, for example based on mixtures of low and high molecular paraffin hydrocarbons and Vaseline.

Anionic, cationic or neutral tensides can be employed as emulsifiers, for example alkalized soaps, methyl soaps, amine soaps, sulfonated compounds, cationic soaps, high fatty alcohols, partial fatty acid esters of sorbitan and polyoxyethylene sorbitan, for example lanette types, wool wax, lanolin, or other synthetic products for the production of oil/water and/or water/oil emulsions.

Hydrophilic organogels can be formulated, for example, on the basis of high molecular polyethylene glycols. These gel-like forms are washable. Vaseline, natural or synthetic waxes, fatty acids, fatty alcohols, fatty acid esters, for example as mono-, di-, or triglycerides, paraffin oil or vegetable oils, hardened castor oil or coconut oil, pig fat, synthetic fats, for example based on acrylic, caprinic, lauric and stearic acid, such as for example Softisan® or triglyceride mixtures such as Miglyol® are employed as lipids in the form of fat and/or oil and/or wax-like components for the production of ointments, creams or emulsions.

Osmotically effective acids and bases, such as for example hydrochloric acid, citric acid, sodium hydroxide solution, potassium hydroxide solution, monosodium carbonate, further buffer systems, such as for example citrate, phosphate, tries-buffer or triethanolamine are used for adjusting the pH value.

Preservatives, for example such as methyl- or propyl benzoate (parabenes) or sorbic acid can be added for increasing stability.

Pastes, powders or solutions are to be mentioned as further topically applicable forms. Pastes often contain lipophilic and hydrophilic auxiliary agents with very high amounts of fatty matter as a consistency-giving base.

Powders or topically applicable powders can contain for example starch varieties such as wheat or rice starch, flame dispersed silicon dioxide or silica, which also serve as diluents, for increasing flowability as well as lubricity as well as for preventing agglomerates.

Nose drops or nose sprays serve as nasal application forms. In this connection, nebulizers or nose creams or ointments can come to use.

Furthermore, nose spray or dry powder formulations as well as controlled dosage aerosols are also suitable for systemic administration of the active ingredients.

These pressure and/or controlled dosage aerosols and dry powder formulations can be inhaled and/or insufflated. Administration forms of this type also certainly have importance for direct, regional application in the lung or bronchi and larynx. Thereby, the dry powder compositions can be formulated for example as active ingredient-soft pellets, as an active ingredient-pellet mixture with suitable carriers, such as for example lactose and/or glucose. For inhalation or insufflation, common applicators are suitable which are suitable for the treatment of the nose, mouth and/or pharynx. The active ingredients can also be applied by means of an ultrasonic nebulizing device. As a propellant gas for aerosol spray formulations and/or controlled dosage aerosols, tetrafluoroethane or HFC 134a and /or heptafluoropropane or HFC 227 are suitable, wherein non-fluorinated hydrocarbons or other propellants which are gaseous at normal pressure and room temperature, such as for example propane, butane or dimethyl ether can be preferred. Instead of controlled dosage aerosols, propellant-free, manual pump systems can also be used.

The propellant gas aerosols can also suitably contain surface active adjuvants, such as for example isopropyl myristate, polyoxyethylene sorbitan fatty acid ester, sorbitan trioleate, lecithins or soya lecithin.

For regional application in situ, solutions for instillation, for example for transurethral administration in bladder tumors or genital tumors, or for profusion in liver tumors or other organ carcinomas are suitable.

The respective suitable medicinal forms can be produced in accordance with the prescription and procedures based on pharmaceutical-physical fundamentals as they are described for example in the following handbooks and are included in the present inventive subject-matter with respect to the production of the respective suitable medicaments:
Physical Pharmacy (A.N. Martin, J. Swarbrick, A. Cammarata), 2nd Ed., Philadelphia Pennsylvania, (1970), German version: Physikalische Pharmazie, (1987), 3rd edition, Stuttgart;
R. Voigt, M. Bornschein, Lehrbuch der pharmazeutischen Technologie, Verlag Chemie, Weinheim, (1984), 5th edition;
P.H. List, Arzneimformenlehre, Wissenschaftliche Verlagsgesellschaft mbH, Stuttgart, (1985), 4th edition;
H. Sucker, P. Fuchs, P. Speiser, Pharmazeutische Technologie, Georg Thieme Verlag, Stuttgart - New York, (1991), 2nd edition;
A.T. Florence, D. Attwood, Physicochemical Principles of Pharmacy, The Maximillan Press Ltd., Hong Kong, (1981);
L.A. Trissel, Handbook on Injectable Drugs, American Society of Hospital Pharmacists, (1994), 8th edition;
Y.W. Chien, Transdermal Controlled Systemic Medications, Marcel Dekker Inc., New York - Basel, (1987);
K.E. Avis, L. Lachmann, H.A. Liebermann, Pharmaceutical Dosage Forms: Parenteral Medications, volume 2, Marcel Dekker Inc., New York - Basel, (1986);
B.W. Müller, Controlled Drug Delivery, Paperback APV, volume 17, Wissenschaftliche Verlagsgesellschaft mbH, Stuttgart, (1987);
H. Asch, D. acetic , P.C. Schmidt, Technologie von Salben, Suspensionen and Emulsionen, Wissenschaftliche Verlagsgesellschaft mbH, Stuttgart, (1984);
H.A. Liebermann, L. Lachman, J.B. Schwartz, Pharmaceutical Desage forms: Tablets, Volume 1, Marcel Dekker Inc., New York, 2nd Edition (1989);
D. Chulin, M. Deleuil, Y. Pourcelot, Powder Technology and Pharmaceutical Processes, in J.C. Williams, T. Allen, Handbook of Powder Technology, Elsevier Amsterdam - London - New York - Tokyo, (1994);
J.T. Carstensen, Pharmaceutical Principles of Solid Dosage Forms, Technomic Publishing Co., Inc., Lancaster - Basel, (1993).

### PRODUCTION EXAMPLES FOR MEDICAMENTS

### A. Separately administrable or separately packaged medicinal forms with an amount of cancerostatic agent

### 1.1 Injection therapeutics

### a) Parenteral Solution

| | |
|---|---|
| cancerostatic agent used according to the invention | 5.000 g |
| acid sodium phosphate | 5.000 g |
| sodium tartrate | 12.000 g |
| benzyl alcohol | 7.500 g |
| water for injection purposes | to 1000.000 ml |

The solution is produced according to the customary method, sterilized and filled into 10 ml vials. One vial contains 50 mg of the cancerostatic agent according to the invention.

### b) Parenteral Solution

| | |
|---|---|
| cancerostatic agent used according to the invention | 1.000 g |
| hydrochloric acid, dilute | 5.000 g |
| sodium chloride | 6.000 g |
| water for injection purposes | to 1000.000 ml |

The solution is produced according to a customary method by stirring; the medicinal form is adjusted to a suitable pH value by acid addition and subsequently filled into 100 ml vials and sterilized. A vial contains 100 mg of the compound according to the invention.

### c) Parenteral Dispersion

| | |
|---|---|
| cancerostatic agent used according to the invention | 10.000 g |
| soya lecithin | 20.000 g |
| saturated triglycerides | 100.000 g |
| sodium hydroxide | 7.650 g |
| water for injection purposes | to 1000.000 ml |

The cancerostatic agent used according to the invention is dispersed in the saturated triglycerides. Then the soya lecithin is added under stirring, and subsequent to this, the aqueous solution of sodium hydroxide is added with subsequent homogenization. The dispersion is sterilized and filled into 10 ml vials. A vial contains 50 mg of the compound according to the invention.

### d) Biodegradable Parenteral Depot Medicinal Form

| | |
|---|---|
| cancerostatic agent used according to the invention | 10.000 g |
| polylactic acid /polygylcolic acid polymer | 70.000 g |
| polyvinylpyrrolidone | 0.200 g |
| gelatine | 2.000 g |
| soya lecithin | 2.000 g |
| isotonic sodium chloride solution | to 1000.000 ml |

First, the active ingredient is incorporated into the biodegradable polymer comprising polylactic acid and polyglycolic acid by a suitable method (spray drying, solvent-evaporation or phase separation) and subsequently subjected to a sterilization process. The particles are introduced into a 2-chamber ready-made syringe in which the adjuvant solution, which is also produced in a sterile manner, is filled. The biodegradable microparticles are mixed with the dispersion agent shortly before application and dispersed. A ready-made syringe contains 200 mg of the cancerostatic agent according to the invention.

### e) Parenteral Dispersion for Subcutaneous Instillation

| | |
|---|---|
| cancerostatic agent used according to the invention | 25,000 g |
| soya lecithin | 25,000 g |
| arachis oil | 400,000 g |
| benzyl alcohol | 50,000 g |
| Miglyole® | to 1000,000 g |

The active ingredient is dispersed together with soya lecithin and arachis oil. The benzyl alcohol is dissolved in Miglyole® and added to the dispersion. The entire dispersion is sterilized and subsequently filled into vials with 2 ml content. A vial contains 50 mg cancerostatic agent.

### f) Parenteral Perfusions Solution

The solution named under example 1.1.b) can also be used for perfusion of the liver for example.

According to need, instead of ampules with injection solution, so-called perforation bottles (vials), which can also be optionally preserved, and infusion solutions with an amount of one or more active ingredients according to the invention can also be made available in the customary manner under addition of buffer substances for adjustment of physiological pH value and/or the isotonicity and/or a best possible suitable pH value for the medicinal form (euhydria) and optional further required nutrients, vitamins, amino acids, stablizers and other necessary adjuvants, possibly in combination with further medicinal agents suitable for the mentioned indications.

### 1.2 Solid, Peroral Administrable Medicaments

### a) Tablets

| | |
|---|---|
| cancerostatic agent used according to the invention | 10.000 g |
| lactose | 5.200 g |
| starch, soluble | 1.800 g |
| hydroxypropylmethylcellulose | 900 g |
| magnesium stearate | 100 g |

The above components are mixed with each other and compacted in a conventional manner, wherein a tablet weight of 180 mg is set. Each tablet contains 100 mg cancerostatic agent. If desired, the tablets obtained in this manner are coated, provided with a film coat and/or enterically coated.

### b) Coated Tablet Core

| | |
|---|---|
| cancerostatic agent used according to the invention | 10.000 g |
| high (X-ray) dispersed silicon dioxide | 500 g |
| corn starch | 2.250 g |
| stearic acid | 350 g |
| ethanol | 3.0 l |
| gelatine | 900 g |
| purified water | 10.0 l |
| talcum | 300 g |
| magnesium stearate | 180 g |

From these components, a granulate is produced which is pressed to the desired coated tablet cores. Each core contains 50 mg of tumor inhibitor. The core can be further processed in a customary manner to coated tablets. If desired, a gastric fluid resistant or retarding film coat can be applied in a known manner.

### c) Vials for drinking

| | |
|---|---|
| cancerostatic agent used according to the invention | 0.050 g |
| glycerine | 0.500 g |
| sorbite, 70% solution | 0.500 g |
| sodium saccharinate | 0.010 g |
| methyl-p-hydroxybenzoate | 0.040 g |
| aromatic agent q.s. | |
| sterile water q.s. | to 5 ml |

The above-mentioned components are mixed in a customary manner to a suspension and filled in a suitable drink vial having 5 ml content.

### d) Poorly Soluble Sublingual Tablets

| | |
|---|---|
| cancerostatic agent used according to the invention | 0.030 g |
| lactose | 0.100 g |
| stearic acid | 0.004 g |
| talcum purum | 0.015 g |
| sweetener q.s. | |
| aromatic agent q.s. | |
| rice starch q.s. | to 0.500 g |

The active ingredient is compacted together with the adjuvants under high pressure to sublingual tablets, favourably in oblong form.

### e) Soft Gel Capsule

| | |
|---|---|
| cancerostatic agent used according to the invention | 0.050 g |
| fatty acid glyceride mixture (Miglyole®) q.s. | to 0.500 g |

The active ingredient is impasted together with the fluid carrier mixture and mixed together with further adjuvants suitable for the encapsulation and filled into elastic soft gelatine capsules which are sealed.

### f) Hard Gelatine Capsules

| | |
|---|---|
| cancerostatic agent used according to the invention | 0.150 g |
| microcrystalline cellulose | 0.100 g |
| hydroxypropylmethylcellulose | 0.030 g |
| mannite | 0.100 g |
| ethylcellulose | 0.050 g |
| triethyl citrate | 0.010 g |

The active ingredient is mixed together with the adjuvants, microcrystalline cellulose, hydroxypropylmethylcellulose and mannite, wet with granulation liquid and formed into pellets. These are subsequently coated with a solution of ethylcellulose and triethyl citrate in organic solvents in a fluidized-bed apparatus. A hard gelatine capsule contains 150 mg of active ingredient.

### 1.3 Topically Administrable Medicinal Forms

### a) Hydrophilic Ointment

| | |
|---|---|
| cancerostatic agent used according to the invention | 0.500 g |
| Eucerinum® anhydricum | 60.000 g |
| microcrystalline wax | 15.000 g |
| Vaseline oil q.s. | to 100.000 g |

The above-mentioned adjuvants are melted and further processed together with the tumor inhibitor to an ointment in a customary manner.

### b) Lipophilic Ointment

| | |
|---|---|
| cancerostatic agent used according to the invention | 10.000 g |
| propylene glycol | 50.000 g |
| paraffin, liquid | 100.000 g |
| paraffin wax | 100.000 g |
| Vaseline | to 1000.000 ml |

The cancerostatic agent used according to the invention is dissolved in propylene glycol at ca. 60°C. At the same time, the lipophilic components are melted at 60-70°C and subsequently combined with the active ingredient solution. The ointment is emulsified at first at 60-70°C and subsequently cooled to 35-40°C under constant emulsification and then filled in 10 g tubes. A tube contains 100 mg of the cancerostatic agent.

### 1.4 Inhalation Therapeutic Agent

Further subject-matter of the use according to the invention are pharmaceutical formulations which are characterized in that they contain one or more cancerostatic agents used according to the invention as a base or a physiologically acceptable salt thereof together with carriers and/or diluents customary for this and suitable for administration by means of inhalation.

In this connection, particularly suitable physiologically acceptable salts of the active ingredients are, as already illustrated in the description section, acid addition salts derived from inorganic or organic acids such as for example especially hydrochloride, hydrobromide, sulfate, phosphate, maleate, tartrate, citrate, benzoate, 4-methoxybenzoate, 2- or 4-hydroxybenzoate, 4-chlorobenzoate, p-tosylate, methane sulfonate, ascorbate, salicylate, acetate, formate, succinate, lactate, glutarate, gluconate or tricarballylate.

The administration of the cancerostatic agents used according to the invention by means of inhalation occurs in conventional ways customary for administrations of this type, for example in the form of a commercial controlled dosage aerosol or in combination with a spacer. In controlled dosage aerosols, a metering valve is delivered with whose help, a dosed amount of the composition is administered. For spraying, the present compositions can be formulated for example as aqueous solutions or suspensions and be administered by means of an atomizer. Aerosol spray formulations in which the active ingredient is either suspended with one or two stabilizers in a propellant as a carrier and/or diluent, for example tetrafluoroethane or HFC 134a and/or heptafluoropropane or HFC 227 can equally be used, whereby however, non-fluorinated hydrocarbons or other propellants which are gaseous at normal pressure and room temperature, such as propane, butane or dimethyl ether, can be preferred. Thereby, propellant-free manual pump systems or dry powder systems as described below can also be used.

Suitably, the propellant aerosols can also contain surface active adjuvants, such as for example isopropyl myristate, polyoxyethylene sorbitan fatty acid ester, sorbitan trioleate, lecithins, oleic acid.

For administration by means of inhalation and/or insufflation, the medicaments with an amount of compounds according to the invention can also be formulated in the form of dry powder compositions, for example as active ingredient-soft pellets or as an active ingredient-powder mixture with a suitable carrier, such as for example lactose and/or glucose. The powder compositions can be formulated and administered as single doses or as multiple doses.

The cancerostatic agent and/or tumor inhibitors according to the invention are preferably administered by means of a controlled dosage aerosol or in the form of a dry powder dosage formulation, wherein the latter preferably contains glucose and/or lactose as a carrier substance.

As applicators for inhalation of the pharmaceutical formulations containing one or more of the cancerostatic agents used according to the invention, all applicators are generally suitable which are suitable for controlled dosage aerosols and/or a dry powder dosage formulation, such as for example usual applicators for the nose, mouth and or pharynx, or also devices standing under propellant gas for the delivery of a spray (as controlled dosage aerosol or dry powder dosage formulation) as they are also used for inhalations in the nose, mouth and/or pharynx.

A further embodiment can also consist of an aqueous solution of the cancerostatic agents considered which also optionally contains further active ingredients and/or additives that are applied by means of an ultrasound atomizer.

### Controlled Dosage Aerosol a)

| | Intended dose per stroke | per aerosol % by weight |
|---|---|---|
| cancerostatic agent | 0.500 mg | 0.66 |
| stabilizer | 0.075 mg | 0.10 |
| HFC 134a | 75.500 mg | 99.24 |

### Controlled Dosage Aerosol b)

| | Intended dose per stroke | per aerosol % by weight |
|---|---|---|
| cancerostatic agent | 0.250 mg | 0.32 |
| Stabilizer | 0.038 mg | 0.05 |
| HFC 227 | 79.180 mg | 99.63 |

In the examples a) and b) the micronized cancerostatic agent is, after previous dispersion in a small amount of the stabilizer, placed in a suspension vessel in which the bulk amount of propellant gas solution is found. The corresponding suspension is dispersed by means of a suitable stirring system (for example high performance mixer or ultrasound mixer) until an ultra-fine dispersion results. The suspension is then continuously held in flux in a filling apparatus suitable for cold propellants or pressure fillings. Alternatively, the suspension can also be produced in a suitable cooled stabilizer solution in HFC 134a/227.

The examples c) to d) describe the composition and production of dosage dry powder formulations.

### c) Dosage-Dry Powder Formulation

| | mg/dose |
|---|---|
| cancerostatic agent used according to the invention | 0.500 mg |

### d) Dosage-Dry Powder Formulation

| | mg/dose |
|---|---|
| cancerostatic agent used according to the invention | 0.500 mg |
| lactose Ph.Eur. | up to 2.5 mg or |
| | up to 5.0 mg |

### e) Dosage-Dry Powder Formulation

| | mg/dose |
|---|---|
| cancerostatic agent used according to the invention | 0.250 mg |
| lactose Ph.Eur. | up to 2.5 mg or |
| | up to 5.0 mg |

In example c) the cancerostatic agent is formulated after micronization under addition of steam in the form of pellets with an MMAD between 0,1 and 0,3 mm diameter and brought to use in a multi-dose powder applicator.

In the examples d) and e) the cancerostatic agent is micronized, thereafter, bulk material is mixed with the lactose in the given amounts, and subsequently, filled in a multi-dose powder inhalator.

In all of the above described examples, active ingredients or the medicinals can be present in the form of the respective suitable pharmaceutically acceptable salts and/or acid addition salts as long as the base is not preferred in the individual case.

Preferably, the tumor inhibitors of the general formula (I), but also suitably effective cancerostatic agents of the remaining art can be used as cancerostatic agents in the above medicaments provided as examples, above all as they are described in Section E below for the combined use according to the invention for illustration of the invention.

### B. Medicinal forms with an amount of vitamin PP compounds for separate administration or packaging in a combination medicament (kit-of-parts)

### 2.1 Injection therapeutics

### a) Parenteral Solution

| | |
|---|---|
| nicotinamide | 25.000 g |
| sodium chloride | 4.500 g |
| water for injection purposes | to 1000.000 ml |

The solution is produced according to the customary method, sterilized and filtered into 2 ml vials. A vial contains 50 mg nicotinamide (niacinamide). This medicinal substance can be replaced by another given vitamin PP compound or a compound effective as vitamin PP.

### b) Parenteral Solution

| | |
|---|---|
| nicotinamide | 10.000 g |
| sodium chloride | 6.750 g |
| water for injection purposes | to 1000.000 ml |

The solution is produced according to the customary method, filled into 100 ml vials and sterilized. A vial contains 1000 mg nicotinamide (niacinamide). The medicinal substance can be replaced by another given vitamin PP compound or a compound effect as Vitamin PP.

### 2.2 Peroral administrable medicament

### a) Tablets

| | |
|---|---|
| nicotinamide | 2,000 g |
| microcristalline cellulose | 750 g |
| corn starch | 2,000 g |
| talcum | 250 g |

The above components are mixed with each other and compacted in a conventional manner. The tablet weight is 500 mg. Each tablet contains 200 mg nicotinamide. If desired, a gastric fluid-resistant or sustained action film coating can be applied in an known manner.

### b) Tablets

| | |
|---|---|
| tryptophan | 5,000 g |
| microcristalline cellulose | 1,070 g |
| corn starch | 2,000 g |
| hydroxypropylmethylcellulose | 250 g |
| silicon dioxide | 120 g |
| magnesium stearate | 60 g |

From the above components, a granulate is produced which is pressed to tablets. Each tablet contains 500 mg tryptophan with a tablet weight of 850 mg.

### c) Coated Tablet Core

| | |
|---|---|
| nicotinic acid | 10,000 g |
| lactose | 3,500 g |
| microcristalline cellulose | 2,200 g |
| corn starch | 3,500 g |
| hydroxypropylcellulose | 600 g |
| magnesium stearate | 200 g |

From the above ingredients, a granulate is produced which is pressed to coated tablet cores. Each core contains 100 mg nicotinic acid with a core weight of 200 mg. The core can be further processed in a customary manner to coated tablets.

### d) Vials for drinking

| | |
|---|---|
| nicotinamide | 0.050 g |
| sorbite, 70 % solution | 1.000 g |
| sodium saccharinate | 0.010 g |
| aromatic agent | q.s. |
| water | to 5.000 ml |

The above mentioned components are mixed in a customary manner and filled into suitable drink vials having 5 ml content.

### e) Hard Gelatin Capsules

| | |
|---|---|
| nicotinamide | 0.100 g |
| microcristalline cellulose | 0.150 g |
| hydroxypropylcellulose | 0.030 g |
| mannite | 0.100 g |

All components are mixed, moistened with granulation liquid and formed to pellets. The pellets can be filled in hard gelatin capsules either directly or after they after been provided with a functional coating. A capsule contains 100 mg nicotinamide. As a result of the multi-particulate medicinal pellets, the possibility also exists to fill differently coated pellets in a hard gelatin capsule. Two possibilities are to be particularly named. On the one hand, gastric fluid resistant pellets can be filled together with sustained action pellets. On the other hand, gastric fluid-resistant pellets can also be combined with one or more other sorts of pellets or other working forms which release the active ingredient at different pH values of the surroundings.

In the above described medicinal forms, nicotinamide (niacinamide) can also be replaced by another given vitamin PP compound or a compound effective as vitamin PP, for example in the form of nicotinic acid or its derivatives.

### 2.3 Topical administrable medicinal forms

### a) Eye drops

| | |
|---|---|
| nicotinamide | 100.0 mg |
| hydroxyethylcellulose | 2.0 mg |
| benzalkonium chloride | 0.1 mg |
| sterile water | to 10.0 ml |

The hydroxyethylcellulose is given to the source in a portion of water. The other components are dissolved in the residual water and subsequently combined with the hydroxyethylcellulose solution and filled into eye drop bottles.

### b) Eye gel

| | |
|---|---|
| nicotinamide | 0.500 g |
| carbomer | 0.020 g |
| polyvinylpyrrolidone | 0.600 g |
| sodium hydroxide solution | q.s. |
| sterile water | to 10.000 g |

The carbomer is dispersed in a portion of water. The nicotinamide and the polyvinylpyrrolidone are dissolved in the other portion and subsequently both partial amounts are combined. The addition of the sodium hydroxide solution occurs thereafter.

In the above topical and/or local or other regional adminstrable medicinal forms, nicotinamide (niacinamide) can also be replaced by another given vitamin PP compound or a compound effective as vitamin PP, for example in the form of nicotinic acid or its derivatives.

### 2.4 Inhalation therapeutic agent

### a) End contents

| | |
|---|---|
| nicotinamide | 100.0 mg |
| sodium chloride | 67.5 mg |
| sterile water | to 10.0 ml |

After dissolving the components in water, the solution is sterilely filled in a suitable container (for example: bottle pack method). The application can occur with commercial atomizers, for example ultra sound atomizers.

### C. Vitamin PP compounds in fixed combination with one or more tumor inhibitors.

The following Examples for combination medicinal forms listed under Item 3 can be used according to the invention, especially in the reduction of side-effects, preferably in the dosage region of 10 - 1000 mg nicotinamide or suitably adjusted dosage of other vitamin PP compounds.

### 3.1 Injection therapeutic agent

### a) Parenteral solution

| | |
|---|---|
| tumor inhibitor | 5.000 g |
| nicotinamide | 5.000 g |
| sodium hydrogen phosphate | 4.500 g |
| sodium tartrate | 11.000 g |
| benzylalkohol | 7.500 g |
| water for injection purposes | to 1000.000 ml |

The solution is produced according to the customary method, sterilized and filled in 10 ml vials. A vial contains 50 mg tumor inhibitor and 50 mg nicotinamide.

### b) Parenteral solution

| | |
|---|---|
| tumor inhibitor | 1.000 g |
| nicotinic acid | 2.000 g |
| hydrochloric acid, diluted | 4.000 g |
| sodium chloride | 5.900 g |
| water for injection purposes | to 1000.000 ml |

The solution is produced according to a customary method; the solution is adjusted to a suitable pH value by addition of acid and subsequently filled in 100 ml vials and sterilized. A vial contains 100 mg tumor inhibitor and 200 mg nicotinic acid.

### c) Biodegradable parenteral depot medicinal form

| | |
|---|---|
| tumor inhibitor | 5.000 g |
| nicotinamide | 5.000 g |
| polylactic acid/polyglycolic acid polymer | 70.000 g |
| polyvinylpyrrolidone | 0.200 g |
| gelatin | 2.000 g |
| soya lecithin | 2.000 g |
| isotonic sodium chloride solution | to 1000.000 ml |

First, the active ingredient is incorporated into the biodegradable polymer comprising polylactic acid and polyglycolic acid by a suitable method (spray drying, solvent-evaporation or phase separation) and subsequently subjected to a sterilization process. The particles are introduced into a 2-chamber ready-made syringe in which the adjuvant solution, which is also produced in a sterile manner, is filled. The biodegradable microparticles are mixed with the dispersion agent shortly before application and dispersed. The content of a ready-made syringe is measured such that this contains 100 mg of the tumor inhibitor and 100 mg nicotinamide.

### d) Parenteral perfusions solution

The solutions named under Example 3.1.b) can also be used for perfusion of the liver for example.

According to need, instead of ampules with injection solution, so-called perforation bottles (vials), which can also be optionally preserved, and infusion solutions with an amount of one or more active ingredients according to the invention can also be made available in the customary manner under addition of buffer substances for adjustment of physiological pH value and/or the isotonicity and/or a best possible suitable pH value for the medicinal form (euhydria) and optional further required nutrients, vitamins, amino acids, stablizers and other necessary adjuvants, possibly in combination with further medicinal agents suitable for the mentioned indications.

In the above described parenteral agents, nicotinamide (niacinamide) can also be replaced by one or more other given vitamin PP compounds or other compounds effective as vitamin PP, for example in the form of nicotinic acid or its derivatives.

### 3.2 Peroral administrable medicaments

### a) Tablets

| | |
|---|---|
| tumor inhibitor | 500 g |
| nicotinamide | 1,500 g |
| microcristalline cellulose | 750 g |
| corn starch | 2,000 g |
| talcum | 250 g |

The above components are mixed with each other and compacted in a conventional manner. The tablet weight amounts to 500 mg. Each tablet contains 50 mg tumor inhibitor and 150 mg nicotinamide. If desired, a gastric fluid-resistant or sustained action film-coating can also be applied in a known manner.

### b) Coated tablet cores

| | |
|---|---|
| tumor inhibitor | 3,000 g |
| nicotinic acid | 5,000 g |
| lactose | 4,500 g |
| microcristalline cellulose | 2,700 g |
| corn starch | 4,000 g |
| hydroxypropylcellulose | 600 g |
| magnesium stearate | 200 g |

From the above components, a granulate is produced which is pressed to coated tablet cores. Each core contains 30 mg tumor inhibitor and 50 mg nicotinic acid with a core weight of 200 mg. The core can be further processed to coated tablets in a customary manner.

### c) Vials for drinking

| | |
|---|---|
| tumor inhibitor | 0.020 g |
| nicotinamide | 0.050 g |
| Sorbite, 70% solution | 1.000 g |
| sodium saccharinate | 0.010 g |
| aromatic agent | q.s. |
| water | to 5.000 ml |

The above named components are mixed in a customary manner and filled in suitable drink vials having 5 ml content.

### d) Hard gelatin capsule

| | |
|---|---|
| tumor inhibitor | 0.050 g |
| nicotinamide | 0.100 g |
| microcristalline cellulose | 0.150 g |
| hydroxypropylcellulose | 0.030 g |
| mannite | 0.100 g |

All components are mixed, moistened with granulation liquid and formed to pellets. The pellets can be filled in hard gelatin capsules either directly or after they after been provided with a functional coating. A capsule contains 50 mg tumor inhibitor and 100 mg nicotinamide. Through the multi-particulate medicinal pellets, the possibility also exists to fill differently coated pellets in a hard gelatin capsule. Two possibilities are to be particularly named. On the one hand, gastric fluid resistant pellets can be filled together with sustained action pellets. On the other hand, gastric fluid resistant pellets can also be combined with one or more other sorts of pellets or other working forms which release the active ingredient at different pH values of the surroundings.

Fundamentally, the possibility also exists for the combination of the tumor inhibitors in form of pellet plus nicotinamide in pellet form, if necessary, with different functional coatings, each combined in a hard gelatin capsule.

### 3.3 Topic administrable medicinal forms

### a) Gel

| | |
|---|---|
| tumor inhibitor | 0.500 g |
| nicotinic acid benzylester | 1.500 g |
| carbomer | 1.000 g |
| glycerin | 10.000 g |
| isopropanol | 15.000 g |
| hydrochloric acid, diluted | q.s. |
| sodium hydroxide solution | q.s. |
| water | to 100.000 g |

The tumor inhibitor is dissolved in diluted hydrochloric acid and the nicotinic acid benzylester in isopropanol and water. Carbomer is dispersed in a portion of the water.
Subsequently, all partial amounts are combined and the sodium hydroxide solution is added.

### b) Lipophilic salve

| | |
|---|---|
| tumor inhibitor | 1.000 g |
| nicotinamide | 2.000 g |
| propylene glycol | 5.000 g |
| paraffin, liquid | 10.000 g |
| paraffin wax | 8.000 g |
| vaseline | to 100.000 g |

The active ingredients are dissolved in propylene glycol at ca 60°C. Simultaneously the lipophilic components are melted at 60-70°C and subsequently combined with the active ingredient solution. The salve is at first emulsified at 60-70°C, then cooled to 35-40°C under continuous emulsification and filled.

The nicotinamide (niacinamide) can also be replaced by another given vitamin PP compound or a compound effective as vitamin PP, for example in the form of nicotinic acid or its derivatives in these topically and/or locally or regionally appliable medicaments, optionally in the form of liquid compositions. The suitable dosage of the active ingredients can be adjusted depending on the particular pharmacokinetics, the responsiveness of patients as well as according to the appraisal of a doctor.

### D. Examples for medical forms with a sole amount of vitamin PP compounds for separate administration

### (next to the application of one or more cancerostatic agents)

Preferably, these examples find their use according to the invention in the neutralization of sporadically existing, acute side-effects as a result of the chemotherapeutic application of cancerostatic agents, whereby dosages and/or dosage units of the vitamin PP compounds here can preferably be considered in the range of 1 000 mg to 10 000 mg nicotinamide. In the case of the use of other vitamin PP compounds, the dosaging among others of nicotinic acid can be correspondingly adjusted according to the appraisal of a doctor.

### 4.1 Injection therapeutic agents

### a) Parenteral solution

| | |
|---|---|
| nicotinamide | 50.000 g |
| water for injection purposes | to 1000.000 ml |

The solution is produced according to customary method, sterlized and filled in 10 ml vials. A vial contains 500 mg nicotinamide.

### b) Parenteral solution

| | |
|---|---|
| nicotinamide | 200.000 g |
| waster for injection purposes | to 1000.000 ml |

The solution is produced according to customary method, filled in 100 ml vials and sterilized. A vial contains 20 g nicotinamide. Since the solution is hypertonic, it must be suitably diluted before the application.

### 4.2 Peroral administrable medicament

### a) Tablets

| | |
|---|---|
| nicotinamide | 10,000 g |
| microcristalline cellulose | 2,600 g |
| crospovidone | 300 g |
| talcum | 600 g |

The above components are mixed with each other and compacted in a convention manner. The tablic weight amounts to 1350 mg. Each tablet contains 1000 mg nicotinamide. If desired, a gastric fluid-resistant or sustained action film-coating can also be applied in a known manner.

### b) Vail for drinking

| | |
|---|---|
| nicotinamide | 5.000 g |
| Sorbite, 70 % solution | 4.000 g |
| sodium saccharinate | 0.040 g |
| aromatic agent | q.s. |
| water | to 20.000 ml |

The above named components are mixed in a customary manner and filled in suitable drinking vials having 20 ml content.

### c) Hard gelatin capsule

| | |
|---|---|
| nicotinamide | 0.500 g |
| microcristalline cellulose | 0.150 g |
| hydroxypropylcellulose | 0.030 g |
| mannite | 0.050 g |

All components are mixed, moistened with granulation liquid and formed to pellets. The pellets can be filled in hard gelatin capsules either directly or after they after been provided with a functional coating. A capsule contains 500 mg nicotinamide. Through the multi-particulate medicinal pellets, the possibility also exists to fill differently coated pellets in a hard gelatin capsule. Two possibilities are to be particularly named. On the one hand, gastric fluid resistant pellets can be filled together with sustained action pellets. On the other hand, gastric fluid resistant pellets can also be combined with one or more other sorts of pellets or other working forms which release the active ingredient at different pH values of the surroundings.

### 4.3 Inhalation therapeutics

### a) Dry powder dosage formulation

| | mg/Doses |
|---|---|
| nicotinamide | 10.0 mg |
| lactose | 5.0 mg |

The active ingredients are formulated to small pellets with lactose after micronization, subsequently filled in hard gelatin capsules and placed in a single-dosage-powder applicator for use.

### E. Combination of different tumor inhibitors and/or cytostatic agents or cancerostatic agents with the vitamin PP compounds

As already mentioned above, further cancerostatic agents, optionally as sole cytostatic agents or immunosuppressive agents for example in the form of the following compounds or compound groups can be formulated, in addition to the anti-tumor compounds especially those of the general formula (I) into the above given examples for the use of the vitamin PP compounds according to the invention and/or the combinations according to the invention with an amount of these compounds:

Alkylizing, cytostatic agents (alkylanizers), for example, derivatives without nitrogen such as cyclophosphamide, trofosfamide, ifosfamide, melphalane, mechloroethamine, chlorambucil as well as ethylenimine derivatives (aziridines) such as thiotepa; further alkylizing compound would be for example busulfane; additionally, among these exemplified alkylating cytostatic agents are carmustine, lomustine or nimustine, which can be considered in a wider sense as belonging to this compound group of alkylanizers as N-nitroso-urea derivatives; procarbazine and dacarbazine are also mentioned as belonging to this series; diaziquone, hexamethylmelamine, trimelamole or temozolamide are also referred to with respect to the group of alkanizers.

Examples for cancerostatic anti-metabolites are folic acid antagonists, such as methotrexate, edatrexate, trimetrexate or its ethyl derivative 10-ethyl-10-desaza-aminopterine as well as purine and pyrimidine base antagonists or antagonists with other points of attack, for example, 6-mercaptopurine and 6-thioguanine, 5-fluorouracil, floxuridine, ara-c, hydroxyurea, desoxycoformycine, triazofurine, acivicine, PALA, pyrazofurine as well as cytarabine, vidarabine, fludarabine, desoxycoformycine, 2-chloro-2'-desoxy-adenosine or tiazofurine.

In this connection, various IFN compounds, IL2 or leucovorine are particularly considered as synergists.

A further important group of cancerostatic agents are represented by the platinum complexes such as cisplatin and carboplatin. Podophyllotoxins or epipodophyllotoxins as antimitotic agents such as etoposide or teniposide have achieved certain importance as cancerostatic agents. This is true in a similar manner for vinca alkaloids, for example vinblastin, vincristin, vindesin, navelbin or 17-desacetylvinblastin.

Cytostatically effective antibiotics capable of being combined are also considered in connection with the invention such as, for example, actinomycins, such as dactinomycin or actinomycin D, anthracyclines such as daunorubicin, doxorubicin, rubidazone, detorubicin, epirubicin, carminomycin, aclacinomycin A, marcellomycin, AD-32, THP-doxorubicin, esorubicin, idarubicin, menogaril, or mitoxantrone or bleomycin as well as mitomycin C with comparable antibiotic effects. Morpholinyl derivatives with the name MRA, MRA-MO, MRA-CN or MRD-nr-CN or the morpholinyl anthracycline derivative MX2 also belong to these.

The series of the cancerostatic agents from different compound groups can be extended with newer compounds such as L-alanosine, amonafide, amsacrine, mafosfamide, bisantrene, carbethimer, desoxyspergualine, α-difluoromethylornithine, mitolactol, didemnine B, echinomycin, ellipticiniumacetate, gallium nitrate, optionally, cimetidine or retinoids, homoharringtonine, razoxane, indicin-N-oxide, lonidamine, menogaril, mitiguazone, N-methylformamide, mitozolamide, piritrexime, spirogermanium, spiromustin, suramin, taxol, taxoter, teroxirone, hydroxyurea, 1,2,4-triglycidylurazole, 1-asparaginase, lentinane, vinzolidine or radioactive isotopes such as ³²P, ⁹⁰Y, ²¹¹At or ¹³¹I, whcih can each be coupled to an antibody for targeting purposes.

An important group of cancerostatic agents are represented by hormonal compounds, i.e. hormones themselves such as androgens/anti-androgens, glucocorticoids or estrogens/anti-estrogens, depot estrogens, gestagens as well as hormone antagonists/hypothalamus hormones, estrogen receptor antagonists or aromatase inhibitors. Among others, examples of compounds of this type are mentioned in the following: busereline, gosereline, leuproreline, triptoreline, such as for example, stilbestrol, diethylstilbestrol, megestrol(acetate), diethyl-stilbestrol diphosphate, estradiol(undecylate), gonadoliberine, fosfestrol, estramustine, tamoxifene, drostanolone(propionate), fluoxymesterone, testolactone, aminoglutethimide, mitotane, medroxiprogesterone(acetate) flutamide, megestrol(acetate), aminoglutethimide, 4-hydroxyandrostendione, plumestane, exemestane, pyridoglutethimide, fadrazole, arimidex, vorazole, flutamide, cyproterone, mifepristone, hydroxyprogesterone, progesterone or ethinylestradiol,

For immunosuppressive therapies cyclosporin can be used for example, whereby corticosteroids, such as for example, prednisone or methylprednisolol as well as methotrexate and azathioprine or cyclophosphamide can also be applied. A known combination of immunsuppressive agents of this type is the combined use of cyclosporine or prednisone together with azathioprine.

Included in the series of the above customary, exemplified cancerostatic agents as well as cancerostatic agents not named here are pharmacolgically acceptable salts, esters, ethers, optic isomers, stereoisomers and other analogously effective derivatives depending on their structure.

The preferred single doses of the cancerostatic agents and/or immunosupressive agents are oriented in accordance with customary therapeutic recommendations given in the literature and company information. Depending on effectiveness and the therapeutic spectrum of the cytcostatic agents, they range for example from 0,1 mg up to 10 000 mg. Expressed in absolute dosage units, the unit doses can be 0.1, 0.2, 0.5, 1, 2, 5, 10, 20, 50, 75, 100, 150, 200, 300, 400, 500, 600, 750, 800, 900, 1000, in individual cases up to 1 200, 1 500, 2 000, 5 000 or 10 000 mg. In the case of the use of interferon compounds, the administered dosage amounts expressed in units are for example 1, 2, 3, 4, 5, 10, 15, 20, 25, 30 40 or 50 million units. This is also true in a similar manner for cancerostatic enzymes, such as for example, asparaginases, which can be administered in an amount of 1000, 6000 or 10 000 units per m² body surface.
The given dosage units also extend to the application of pyridylcarboxamide compounds according to the general formula (I), which can lie in the range between 0.1 mg, 1, 10 mg up to 5000 or 10 000 mg individual doses as discussed above.

Depending on the existence of a synergistic, super-additive effect combination of two or more cancerostatic agents, the customary dosages can also be reduced. Therewith, the side-effects associated with the administration of cancerostatic agents can be further reduced. Conversely, if need be, considerably higher dose of cancerostatic agents can be administered without danger for patients in the case that the vitamin PP compounds reduce the side-effects in accordance with the invention.

The above discussion of the respective suitable cancerostatic agents merely has an exemplifying character and no limiting character in any way because it cannot be complete as a result of the incalculable abundance of known medicaments; further customary cancerostatic agents and carrier systems for this are to be taken from relevant handbooks such as for example M.Peckham, H.Pinedo and U. Veronesi: Oxford Textbook of Oncology Vol. 1 & Vol. 2, Oxford University Press, Oxford, New York, Tokyo (1995) or W.Forth, D.Henschler, W. Rummel, , K.Starke: General and specific pharmacology, SPEKTRUM, Heidelberg, Berlin Oxford, 7. Edition (1996) or E. Mutschler: Medicament activities, textbook of pharmacology & toxicology, WVG, Stuttgart, 6. Edition (1991) or other compendia and the remaining literature or the art on anti-tumor agents.

### PHARMACOLOGICALL - EXPERIMENTAL PORTION

In the following examples, an anti-tumor substance was used which was synthesized by Klinge Pharma GmbH under the label K22.097 and is described in the publication
PCT/EP97/03293=WO97/48695 as substance Nr.54 (production example 4 in the form of N-[4-(1-diphenylmethylpiperidin-4-yl)-butyl]-3-(pyridin-3-yl)-propionamide.

### 1. Neutralization of the growth-inhibiting effect of anti-tumor substances by nicotinic acid and nicotinamide in human leukaemia cells.

THP-1 cells derived from a human monocytic leukaemia plated at a density of 200,000 cells/ml in 96-well plastic dishes. Cultivation occurred in RPMI 1640 nutrient medium with 10% foetal calf serum (FCS) in a tissue culture incubator with a gas mixture of 5% CO₂ and 95% air at a temperature of 37°C. For the individual concentrations and the controls without test substances as well as for the background with nutrient medium but without cells, three-fold batches were done for each. After four days of substance incubation 20 µl WST-1 reagent (Boehringer Mannheim) was respectfully pipetted in each individual well. After 30 to 60 minute incubation in the tissue culture incubator at 37°C and 5% CO₂, the light extinction was measured in an ELISA reader at 450 nm wave length. The backgrounds were each subtracted from the typical measured valves. (The IC₅₀-values (defined as that concentration in which the cell growth was inhibited by 50%) was taken from the dose-response curves and given as a comparative measurement for the activity of the test compounds.

The measured values obtained in this manner are given in the following Table 1:

**Table 1**

| **Test substances** | **IC**_{**50**}**-Value for K22.097** |
|---|---|
| K22.097 alone | 0,01 µM |
| K22.097 + 0,1 mM nicotinic acid | > 10 µM |
| K22.097 + 1 mM nicotinamide | > 10 µM |

As can be seen from Table 1, the growth-inhibiting effect of K22.097 is abrogated by nicotinic acid as well as by nicotinamide.

### 2. Neutralization of the growth-inhibiting effect of anti-tumor substances by nicotinic acid and nicotinamide in normal lymphocytes

Many cytostatic agents and radiation therapies as well have a cytcotoxic effect not only on tumor cells but also on the blood cell system. This leads to a weakening of the immune defence which can lead to life threatening infections. In addition, this can result in a so-called tumor lyse syndrome which can lead to the death of the patient by the relatively quick death of large cell masses. In order to test the possibility for a therapeutical use of nicotinic acid and/or nicotinamide for preventing such life threatening conditions by massive cell death, the effect of the substance was tested on freshly isolated lymphocytes as follows:

The spleen of a Swiss mouse served as a lymphocyte source. The lymphocyte population was isolated from the spleen cell suspension over a ficoll gradient and taken up in IMEM-ZO culture medium with 0,1% dextran 70,000 and 2% foetal calf serum. The cells were plated at a density of ca. 500,000 cells/well/ml in a 12-well plate, 1 ml doubly concentrated test substance solution was pipetted per well and this was subsequently incubated in a tissue culture incubator at 37°C and 5% CO₂. After 2 days, a 1 ml-aliquot with 5 µl of the fluorescent dye solutions propidium iodide (8 mg/ml) and 3,3'-dihexyloxacarbocyanin iodide (40 µg/ml) each was added per well, and incubated for 3 minutes at room temperature. Subsequently, 10,000 cells per each sample were measured on a flow-through cytometer and the percentage amount of vital cells in the population was determined. By means of the dose-response curves, IC₅₀-values were calculated which were also employed in the following Tables for the characterization of the individual substances:

**Table 2**

| **Test substances** | **IC**_{**50**}**-Value for K22.097** |
|---|---|
| K22.097 alone | 0,001 µM |
| | |
| K22.097 + 10 mM nicotinamide | > 1 µM |

### 3. Neutralization of growth-inhibiting effect of anti-tumor substances by nicotinic acid in nicotinamide in primary intestine cells.

Cryptic cells of the large intestine were isolated according to the method of Booth et al. (1995) with slight modifications. The removed large intestine was cut into pieces, the parts were finally cut after several-fold washing and digested with collagenase and dispase. Subsequently, the larger tissue pieces remaining in the suspension were sedimented, the supernatant was decanted and stored. The pellet was digested again under vigorous shaking with medium in order to release additional cryptic cells. After sedimentation, the supernatant was poured off again, combined with the first fraction and the cryptic cells were centrifuged at 50 × g. After a further washing step, the centrifuge cryptic cells were suspended in culture medium and seeded at a density of 800 cells per well in 24-well-cultured dishes which were previously coated with collagen. The cells were incubated at 37°C in a saturated water vapour atmosphere with 92.5% air and 7.5% CO₂. 50% of the nutrient medium was renewed every two days. The test substances were added 48 hours after the plating of the cryptic cells; the treatment with them lasted a total of 4 days.

The growth of the culture was photometrically quantified after staining with crystal violet as a measure for the cell number and the vitality of the cells was determined with the MTT-test whose reaction depends on living cells with active mitochondria. Based on dose response curves, the IC₅₀-values were calculated which are also used in the following Table 3 for characteization of the individual substance activity:

**Table 3**

| **Test substances** | **IC**_{**50**}**-Value for K22.097** |
|---|---|
| K22.097 alone | 0,1 µM |
| K22.097 + 1 mM nicotinamide | > 10 µM |

### 4. Neutralization of the toxic effect of anti-tumor substances by nicotinamide in NMRI Mice

Groups of 6 mice each were treated over 4 days with different doses of the anti-tumor substance K22.097 orally administered twice daily. Parallel to this, 500 mg/kg nicotinamide was simultaneously intraperitoneally administered each to the individual dosage groups. 7 days after the beginning of treatment, the blood picture of the individual animals was examined. The results are summarized in following Table 4:

**Table 4**

| **Test substances** | **Mortality** | **Leukocyte number** |
|---|---|---|
| 0,5% carboxymethylcellulose (control) | 0 / 6 | 5,100 per µl |
| 2×100 mg/kg K22.097 | 1 / 6 | 1,100 per µl |
| 2×120 mg/kg K22.097 | 3 / 6 | 500 per µl |
| 2×500 mg/kg nicotinamide | 0 / 6 | 4,800 per µl |
| 2×100 mg/kg K22.097 + 2×500 mg/kg nicotinamide | 0 / 6 | 6,900 per µl |
| 2×120 mg/kg K22.097 + 2×500 mg/kg nicotinamide | 0 / 6 | 5,900 per µl |

The results of Table 4 show that the cases of death caused by the anti-tumor substance as well as the strong reduction of leucocyte cells could be completely prevented.

It is evident from the experimental results represented in Tables 1 - 4 that the use of compounds of the vitamin PP group according to the invention is capable of suppressing the unavoidable side-effects of anti-tumor substances or is at least capable of elevating these and/or neutralizing the cancerostatic activity in a surprising manner in unexpected threatening incidents. This result was not to be expected in view of the known, sensitivity-increasing and effect-increasing activities of nicotinic acid and/or its amide discussed at the beginning.

As already discussed in the above mentioned publications and also the older patent applications of the applicant not yet published at the present application date, the tumorstatic agents according to the general formula (I) are especially suitable also in connection with the invention subject matter described herein, preferably in the chemotherapy of malignant diseases of humans and animals. The anti-neoplastic effect of the described substances of formula (I) can be used for prophylactic, adjuvant, palliative and curative treatment of solid tumors, leukeamic diseases and lymphomas, as well as for reducing or preventing metastases formation in humans and animals. The therapeutic use is possible in the following illnesses for example: gynaecological tumors, ovarian carcinomas, testicle tumors, prostate carcinomas, skin cancer, kidney cancer, bladder tumors, oesophagus carcinomas, stomach cancer, rectal carcinomas, pancreas carcinomas, thyroid cancer, adrenal tumors, leukaemia and lymphomas, Hodgkin's disease, tumor illnesses of the CNS, soft-tissue sarcomas, bone sarcomas, benign and malignant mesotheliomas, but especially intestine cancer, liver cancer, breast cancer, bronchial and lung carcinomas, melanomas, acute and chronic leukaemias. Benign papillomatosis tumors can also be limited in their growth with the named substances.

The use according to the invention and the medicaments according to the invention for neutralization of acute side-effects or reduction of side-effects can also be performed with these substances according to general formula (I) in tumors which are resistant against customary cytostatic agents. In addition, as already mentioned above, combinations of these tumor inhibitors with other known pharmaceutical agents which are used in chemotherapy are promising due to their independent characteristics as long as their properties are complemented in a suitable manner.

The integration of these pyridylcarboxamides in a therapy scheme can be particularly successful for example with one or more substances from the following classes:
anti-metabolites (for example cytarabine, 5-fluorouracil, 6-mercaptopurine, methotrexate), alkylating agents (for example busulfane, carmustine, cisplatin, carboplatin, cyclophosphamide, dacarbazine, melphalane, thiotepa), DNA-intercalating substances and topoisomerase inhibitors (for example actinomycin D, daunorubicin, doxorubicin, mitomycin C, mitoxantrone, etoposide, teniposide, topotecane, irinotecane), spindle poisons (for example vincristine, navelbine, taxol, taxoter), hormonally active agents (for example tamoxifene, flutamide, formestane, gosereline) or other cytostatic agents with complex modes of action (for example L-asparaginase, bleomycin, hydroxyurea). Resistant tumor cells can be made sensitive again for example by interaction of the new compounds with a mechanism of resistance for common cytostatic agents (for example P-glycoprotein, MRP, glutathione-S-transferase, metallothionein).

Many anti-tumor agents have not only a cytotoxic effect on tumor cells, but also on the blood cell system. This leads to a weakening of the immune defence, which can, in turn, be specifically employed to suppress the rejection reaction after an organ transplantation for example. The use in immunological disease such as for example psoriasis or autoimmune diseases is therefore also possible. Hence, the independent structural class of the preferred compounds from the group of substituted 3-pyridylcarboxyl amides also makes possible a combination with other known immunosuppressive agents such as for example, cyclosporine A, tacrolimus, rapamycin, azathioprine and glucocorticoids.

Furthermore, the anti-tumor substance Nr.159 with the internal label K22.175 according to the publication PCT/EP97/03245=WO97/48696 in the form of N-[4-(1-benzoylpiperidin-4-yl)-butyl]-3-(pyridin-3-yl)-acrylamide synthesized by the applicant was included in the experimental examinations. The results obtained therefrom confirm the above described finding on the useful effect according to the invention of the vitamin PP compounds. The results corresponding to the method used in Example 1, Table 1, are given in Table 5 as follows:

**Table 5**

| **Test substances** | **IC**_{**50**}**-Value for K22.175** |
|---|---|
| K22.175 alone | 0.5 nM |
| K22.175 + 0.1 mM nicotinic acid | > 30 nM |
| K22.175 + 1 mM nicotinamide | > 30 nM |

As already mentioned at the beginning, the use according to the invention and the medicaments produced and applied according to the invention also relate to the combination of cancerostatic chemotherapy with radiation and/or radiotherapy, whereby hypothermia as a heat irradiation treatment or other physical tumor therapies are included within the invention.

## Claims

1. A pharmaceutical composition comprising:
(a) at least one cancerostatic or immunosuppressive agent selected from the group consisting of compounds of formula (I) : 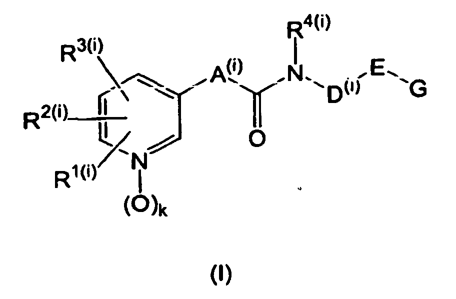 wherein:
**R**^{**1(i)**} is selected from
hydrogen, halogen, cyano, C₁-C₆-alkyl, C₃-C₆-alkenyl, C₂-C₆-alkinyl, trifluoromethyl, hydroxy, C₃-C₈-cycloalkyl, C₁-C₆-hydroxyalkyl, C₁-C₆-alkoxy, C₃-C₆-alkenyloxy, C₃-C₆-alkinyloxy, benzyloxy, C₁-C₇-alkanoyloxy, C₁-C₇-alkoxycarbonyloxy, C₁-C₆-alkylthio, C₃-C₆-alkenylthio, C₃-C₆-alkinylthio, C₃-C₈-cycloalkyloxy, C₃-C₈-cycloalkylthio, C₂-C₇-alkoxycarbonyl, aminocarbonyl, C₂-C₇-alkylaminocarbonyl, C₃-C₁₃-dialkylaminocarbonyl, carboxy, phenyl, phenoxy, phenylthio, pyridyloxy, pyridylthio, and **NR**^{**5(i)**}**R**^{**6(i)**}**,** wherein
**R**^{**5(i)**} and **R**^{**6(i)**} are selected independently from each other from hydrogen, C₁-C₆-alkyl, C₃-C₆-alkenyl, C₃-C₆-alkinyl, benzyl and phenyl;
**R**^{**2(i)**} is selected from
hydrogen, halogen, cyano, C₁-C₆-alkyl, trifluoromethyl, hydroxy, C₁-C₆-alkoxy, benzyl and C₁-C₆-alkanoyloxy; or
**R**^{**1(i)**} and **R**^{**2(i)**} when they are adjacent optionally form a bridge selected from
-(CH₂)₄-, -(CH=CH)₂- and -CH₂O-**CR**^{**7(i)**}**R**^{**8(i)**}-O-, wherein
**R**^{**7(i)**} and **R**^{**8(i)**} are selected independently from each other from hydrogen and C₁-C₆-alkyl;
**R**^{**3(i)**} selected is from
hydrogen, halogen, C₁-C₆-alkyl, trifluoromethyl and C₁-C₆-hydroxyalkyl;
**R**^{**4(i)**} is selected from
hydrogen, hydroxy, C₁-C₆-alkyl, C₃-C₆-alkenyl, C₃-C₆-alkinyl, C₃-C₆-cycloalkyl, C₁-C₆-alkoxy and benzyloxy;
**k** is 0 or 1,
**A**^{**(i)**} is selected from
C₁-C₆-alkylene, optionally substituted one- to three-fold by C₁-C₆-alkyl, C₁-C₃-alkoxy, hydroxy, fluorine, or phenyl,
C₂-C₆-alkylene, wherein a methylene unit is isosterically replaced by O, S, **NR**^{**9(i)**}**,** CO, SO or SO₂, wherein, with the exception of CO, the isosteric substitution is not adjacent to the amide group, and **R**^{**9(i)**} is selected from hydrogen, C₁-C₆-alkyl, C₃-C₆-alkenyl, C₃-C₆-alkinyl, C₃-C₆-acyl and C₁-C₆-alkanesulfonyl,
1,2-cyclopropylene,
C₂-C₆-alkenylene, optionally substituted one to three-fold by C₁-C₆-alkyl, hydroxy, C₁-C₃-alkoxy, fluorine, cyano or phenyl,
C₄-C₆-alkadienylene, optionally substituted once or twice by C₁-C₃-alkyl, fluorine, cyano or phenyl,
1,3,5-hexatrienylene, optionally substituted by C₁-C₆-alkyl, fluorine, cyano or phenyl, and
ethinylene;
**D**^{**(i)**} is selected from
C₁-C₁₂-alkylene, optionally substituted once or twice by C₁-C₆-alkyl, hydroxy, C₁-C₆-alkoxy, or phenyl,
C₂-C₁₂-alkenylene or C₄-C₁₂-alkadienylene, optionally substituted once or twice by C₁-C₆-alkyl, hydroxy, C₁-C₆-alkoxy, or phenyl, wherein one double-bond can
optionally occur to ring **E** in the case that ring **E** is linked over a C-atom,
C₃-C₁₂-alkinylene or C₄-C₁₂-alkeninylene, optionally substituted once or twice by C₁-C₆-alkyl, hydroxy, C₁-C₆-alkoxy or phenyl, and
C₁-C₁₂-alkylene, C₂-C₁₂-alkenylene or C₃-C₁₂-alkinylene, wherein, one to three methylene units, with the exception of the (G)-terminal methylene group in the case that E represents a bond, are isosterically replaced by O, S, **NR**^{**10(i)**}**,** CO, SO or SO₂, wherein **R**^{**10(i)**} has the same meaning as **R**^{**9(i)**}**,** but is selected independently thereof;
**E** is selected from **E**^{**1(i)**}**, E**^{**2(i)**}**, E**^{**3**}**, E**^{**4**}**, E**^{**5**} and **E**^{**6**}**,** wherein
**E**^{**1(i)**} is 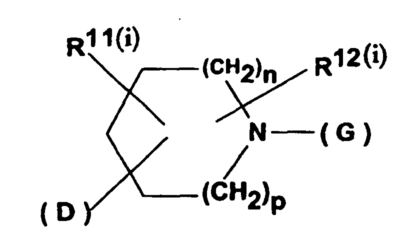
**E**^{**2(i)**} is 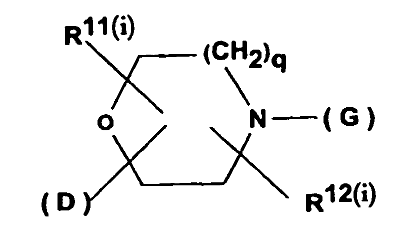
**E**^{**3**} is 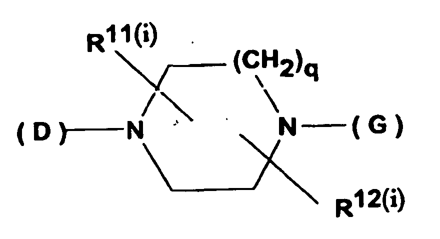
**E**^{**4**} is 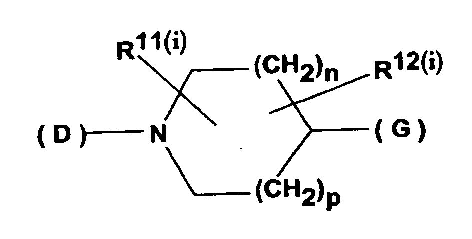
**E**^{**5**} is 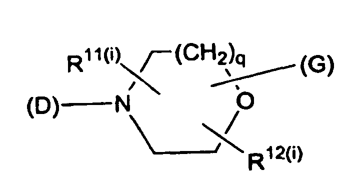 and
**E**^{**6**} represents a single or double bond,
wherein the heterocyclic rings **E**^{**1(i)**} to **E**^{**5**} optionally have a double bond,
**n** and **p** are, independently from each other, 0, 1, 2, or 3 with the proviso that, **n + p** ≤ 4,
**q** is 1, 2 or 3;
**R**^{**11(i)**} is selected from
hydrogen, C₁-C₆-alkyl, hydroxy, hydroxymethyl, carboxy and C₂-C₇-alkoxycarbonyl,
**R**^{**12(i)**} is selected from
hydrogen, C₁-C₆-alkyl and an oxo group adjacent to a nitrogen atom, or
**R**^{**11(i)**} and **R**^{**12(i)**}**,** optionally together, form a C₁-C₃-alkylene bridge under formation of a bicyclic ring system, and
**(a)** in the case that **E** represents **E**^{**1(i)**}**, E**^{**2(i)**}**,** or **E**^{**3**} the substituent G optionally is selected from **G**^{**1(i)**}**, G**^{**2(i)**}**, G**^{**3(i)**}**, G**^{**4(i)**} and **G**^{**5(i)**}**,** wherein
**G**^{**1(i)**} is 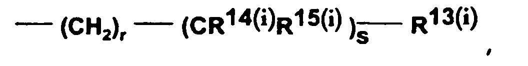 wherein
**r** is 0 to 3 and
**s** is 0 or 1,
**R**^{**13(i)**} is selected from
hydrogen, C₁-C₆-alkyl, C₃-C₆-alkenyl, C₃-C₆-alkinyl and C₃-C₈-cycloalkyl;
saturated or unsaturated, four to seven-membered heterocycles which contain one or two hetero-atoms selected from N, S and O;
benzyl, phenyl;
monocyclic aromatic five or six-membered heterocycles which contain one to three hetero-atoms selected from N, S and O, and are either bound directly or over a methylene group;
anellated bi- and tricyclic aromatic or partially hydrogenated carbocyclic ring systems with 8 to 16, 17 or 18 ring atoms and at least one aromatic ring, wherein the linkage occurs either over an aromatic or a hydrogenated ring and either directly or over a methylene group;
anellated bi- and tricyclic aromatic or partially hydrogenated heterocyclic ring systems with 8 to 16, 17 or 18 ring atoms and at least one aromatic ring, wherein one to three ring atoms are selected from N, S and O and the linkage occurs either over an aromatic or a hydrogenated ring and either directly or over a methylene group;
**R**^{**14(i)**} has the same meaning as **R**^{**13(i)**}**,** but is independently selected therefrom;
**R**^{**15(i)**} is selected from
hydrogen, hydroxy, methyl, benzyl, phenyl,
monocyclic aromatic five or six-membered heterocycles which contain one to three hetero-atoms selected from N, S and O and are either bound directly or over a methylene group;
anellated bi- and tricyclic aromatic or partially hydrogenated carbocyclic ring systems with 8 to 16, 17 or 18 ring atoms and at least one aromatic ring, wherein the linkage occurs either over an aromatic or a hydrogenated ring and either directly or over a methylene group;
anellated bi- and tricyclic aromatic or partially hydrated heterocyclic ring systems with 8 to 16, 17 or 18 ring atoms and at least one aromatic ring, wherein one to three ring atoms are selected from N, S and O and the linkage occurs either over an aromatic or a hydrogenated ring and either directly or over a methylene group;
**G**^{**2(i)**} is selected from 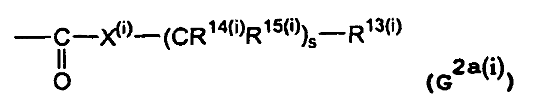 and 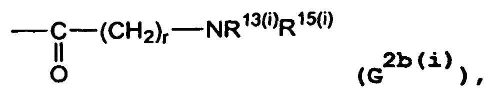 wherein **r, s** and the substituents **R**^{**13(i)**}**, R**^{**14(i)**} and **R**^{**15(i)**} have the above meanings,
or the group
-**NR**^{**13(i)**}**R**^{**15(i)**}
is a nitrogen-containing heterocycle bound over the nitrogen atom, which nitrogen-containing heterocycle is selected from
saturated and unsaturated monocyclic, four to eight-membered heterocycles, which aside from the essential nitrogen atom, optionally contain one or two further hetero-atoms selected from N, S and O,
and
saturated and unsaturated bi- or tricyclic, anellated or bridged heterocycles with 8 to 16, 17 or 18 ring atoms, which aside from the essential nitrogen atom, optionally contain one or two further hetero-atoms selected from N, S and O,
**X**^{**(i)**} is selected from
methylene, ethylene, ethenylene, propylene, and C₃-C₇-cycloalkylene, or represents a bond;
**G**^{**3(i)**} is
**-SO**_{**2-**}**(CH**_{**2**}**)**_{**r-**}**R**^{**13(i)**}**,**
wherein **r** and **R**^{**13(i)**} have the above meanings,
**G**^{**4(i)**} is 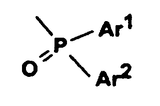 wherein
**Ar**^{**1**} and **Ar**^{**2**} are selected independently from each other from phenyl, pyridyl and naphthyl;
**G**^{**5(i)**} is
**-COR**^{**16(i)**}
wherein
**R**^{**16(i)**} is selected from
trifluoromethyl, C₁-C₆-alkoxy, C₃-C₆-alkenyloxy, and benzyloxy,
**(b)** in the case that **E** is **E**^{**4**} or **E**^{**5**}**,**
then **G** optionally is **G**^{**1(i)**}**, G**^{**2(i)**}**, G**^{**6(i)**}**, G**^{**7**} or **G**^{**8**}**,**
wherein **G**^{**1(i)**} and **G**^{**2(i)**} have the above meanings and
**G**^{**6(i)**} is
**=(C)**_{**u**}**R**^{**13(i)**}**R**^{**15(i)**}**,**
wherein **R**^{**13(i)**} and **R**^{**15(i)**} have the above meanings and
**u** is 0 or 1,
or when **u** = 1, then **R**^{**13(i)**} and **R**^{**15(i)**} together with the carbon atom to which they are attached form a ring system selected from
C₃-C₈-cycloalkyl,
saturated, four to seven-membered heterocycles which optionally contain one or two hetero-atoms, selected from N, S and O;
anellated bi- and tricyclic partially hydrogenated carbocyclic ring systems with 8 to 16, 17 or 18 ring atoms and at least one aromatic ring;
anellated bi- and tricyclic partially hydrogenated heterocyclic ring systems with 8 to 16, 17 or 18 ring atoms and at least one aromatic ring, wherein one to three ring atoms are selected from N, S and O;
or when **u** = 0 then **R**^{**13(i)**} and **R**^{**15(i)**} together with the carbon atom of ring **E** to which they are attached form a ring system selected from
C₃-C₈-cycloalkyl,
saturated, four to seven-membered heterocycles which contain one or two hetero-atoms, selected from N, S and O;
anellated bi- and tricyclic partially hydrogenated carbocyclic ring systems with 8 to 16, 17 or 18 ring atoms and at least one aromatic ring; and
anellated bi- and tricyclic partially hydrogenated heterocyclic ring systems with 8 to 16, 17 or 18 ring atoms and at least one aromatic ring, wherein one to three ring atoms are selected from N, S and O;
**G**^{**7**} is selected from
**-NR**^{**17(i)**}**-(CH**_{**2**}**)**_{**r**}**-(CR**^{**14(i)**}**R**^{**15(i)**}**)**_{**s**}**-R**^{**13(i)**} **(G**^{**7a**}**)**, 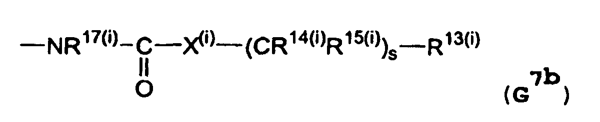 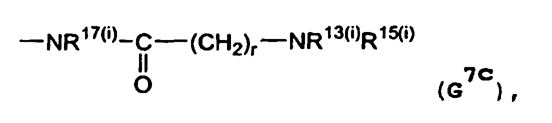
**-NR**^{**17(i)**}**-SO**_{**2**}**-(CH**_{**2**}**)**_{**r**}**-R**^{**13(i)**} **(G**^{**7d**}**),** 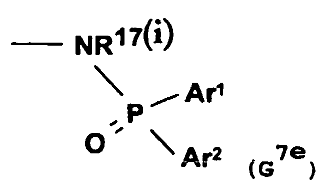
and
**-NR**^{**17(i)**}**-COR**^{**16(i)**} **(G**^{**7f**}**),**
wherein **r, s, X**^{**(i)**}**,** the substituents **R**^{**13(i)**}**, R**^{**14(i)**}**, R**^{**15(i)**} and **R**^{**16(i)**} and the group
**-NR**^{**13(i)**}**R**^{**15(i)**}
have the above meanings, and
**R**^{**17(i)**} has the same meanings as **R**^{**5(i)**}**,** but is selected independently thereof,
**Ar**^{**1**} and **Ar**^{**2**} are selected independently from each other from phenyl, pyridyl and naphthyl;
**G**^{**8**} is selected from 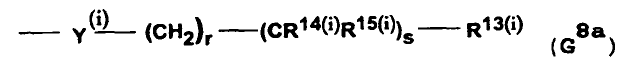 and 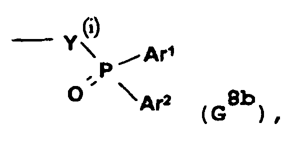 wherein
**r, s** and the substituents **R**^{**13(i)**}**, R**^{**14(i)**}**, R**^{**15(i)**}**, Ar**^{**1**} and
**Ar**^{**2**} have the above meanings, and
**Y**^{**(i)**} is O or S;
**(c)** in the case that the substituent **E** is **E**^{**6**}**,**
then the substituent **G** optionally is selected from **G**^{**7d**}**, G**^{**7e**}**, G**^{**8b**}**, G**^{**9**}**, G**^{**10**}**, G**^{**11**}**, G**^{**12**}**,** and **G**^{**13**}**,** wherein **G**^{**7d**}**, G**^{**7e**} and **G**^{**8b**} have the above meanings and
**G**^{**9**} is selected from
**-(CR**^{**13(i)**}**R**^{**19**}**)**_{**S**}**-R**^{**18**} **(G**^{**9a**}**)**
and
-**NR**^{**13(i)**}**R**^{**18**} **(G**^{**9b**}**),**
wherein **s** and **R**^{**13(i)**} are defined as above, and
**R**^{**18**} is selected from
benzyl, diphenylmethyl, phenyl;
monocyclic aromatic five and six-membered heterocycles which can contain one to three hetero-atoms selected from N, S and O and are either bound directly or over a methylene group;
anellated bi- and tricyclic aromatic or partially hydrogenated carbocyclic ring systems with 8 to 16, 17 or 18 ring atoms and at least one aromatic ring, wherein the linkage occurs either over an aromatic or a hydrogenated ring and either directly or over a methylene group; and
anellated bi- and tricyclic aromatic or partially hydrogenated heterocyclic ring systems with 8 to 16, 17 or 18 ring atoms and at least one aromatic ring, wherein one to three ring atoms are selected from N, S and O and the linkage occurs either over an aromatic or a hydrogenated ring and either directly or over a methylene group;
**R**^{**19**} has the same meanings as **R**^{**13(i)**} but is selected independently thereof, and in addition can be hydroxy;
or the group
-**NR**^{**13(i)**}**R**^{**18**}
optionally is a nitrogen-containing heterocycle bound over the nitrogen atom, which nitrogen-containing heterocycle is selected from
anellated bi- and tricyclic aromatic or partially hydrogenated heterocyclic ring systems with 8 to 16, 17 or 18 ring atoms and at least one aromatic ring, which aside from the essential nitrogen atom, optionally contain one or two further hetero-atoms selected from N, S and O;
**G**^{**10**} is
**=CR**^{**13(i)**}**R**^{**18**} **(G**^{**10**}**)**
bound to D by means of a double bond, wherein **R**^{**13(i)**} and **R**^{**18**} have the above meanings, or wherein **G**^{**10**}
optionally is a ring system bound over the carbon atom, selected from
anellated bi- and tricyclic partially hydrogenated carbocyclic ring systems with 8 to 16, 17 or 18 ring atoms and at least one aromatic ring; and
anellated bi- and tricyclic partially hydrogenated heterocyclic ring systems with 8 to 16, 17 or 18 ring atoms and at least one aromatic ring, wherein one to three ring atoms optionally are selected from N, S and O;
**G**^{**11**} is selected from
-**NR**^{**17(i)**}**-(CH**_{**2**}**)**_{**r**}**-(CR**^{**13(i)**}**R**^{**19**}**)**_{**s**} **-R**^{**18**} **(G**^{**11a**}**),** 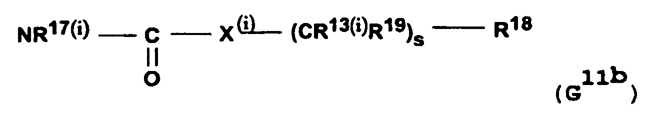
and 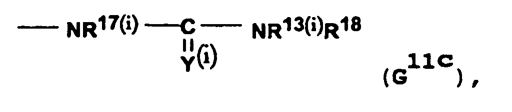 wherein **r, s, X**^{**(i)**}**, Y**^{**(i)**}**,** the substituents **R**^{**13(i)**}**, R**^{**17(i)**}**, R**^{**18**} and **R**^{**19**} and the group **-NR**^{**13(i)**}**R**^{**18**} have the above meanings;
**G**^{**12**} is
-**Y**^{**(i)**}**-(CH**_{**2**}**)**_{**r**}**-(CR**^{**13(i)**}**R**^{**19**}**)**_{**s**}**-R**^{**18**} **(G**^{**12**}**),**
wherein **r, s, Y**^{**(i)**} and the substituents **R**^{**13(i)**}**, R**^{**18**} and **R**^{**19**} have the above meanings;
**G**^{**13**} is selected from 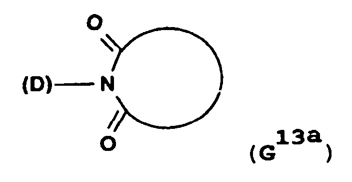 and 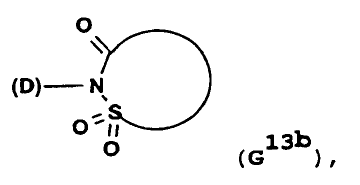 bound to D over the imide nitrogen atom, selected from
saturated and unsaturated monocyclic imides with 5 to 7 ring atoms, which, aside from the essential imide nitrogen atom, optionally contains one or two further hetero-atoms selected from N, S and O;
saturated, unsaturated and aromatic anellated, bi-, tri- or tetracyclic imides with 8 to 18 ring atoms, which, aside from the essential imide nitrogen atom, optionally contain one or two further hetero-atoms selected from N, S and O;
saturated and unsaturated, bridged bi-, tri-, tetra- or pentacyclic imides with 8 to 22 ring-atoms, which, aside from the essential imide nitrogen atom, optionally contain one or two further hetero-atoms selected from N, S and O; and
saturated and unsaturated spirocyclic imides, optionally anellated one or two-fold, and with a total of 9 to 23 ring atoms, which, aside from the essential imide nitrogen atom, optionally contain one or two further hetero-atoms selected from N, S and O,
wherein these cyclic imides optionally are substituted by one to five of the same or different groups selected independently from each other from
halogen, cyano, C₁-C₆-alkyl, C₁-C₆-alkylidene, trifluoromethyl, C₃-C₈-cycloalkyl, C₃-C₈-cycloalkylidene, phenyl-C₁-C₃-alkyl, phenyl-C₁-C₃-alkylidene, diphenyl-C₁-C₃-alkyl, diphenyl-C₁-C₃-alkylidene, triphenylmethyl, phenyl, hydroxy, C₁-C₆-hydroxyalkyl, C₁-C₆-alkoxy, C₁-C₆-alkoxy entirely or partially substituted by fluorine, benzyloxy, phenoxy, naphthyloxy, mercapto, C₁-C₆-alkylthio, phenylthio, naphthylthio, pyridylthio, C₁-C₆-alkanesulfonyl, phenylsulfonyl, naphthylsulfonyl, pyridylsulfonyl, sulfo, carboxy, C₂-C₇-carboxyalkyl, C₃-C₇-carboxyalkenyl, C₂-C₇-alkoxycarbonyl, benzyloxycarbonyl, nitro, amino, C₁-C₆-aminoalkyl, mono-C₁-C₆-alkylamino di(C₁-C₆-alkyl)amino, phenylamino, phenyl-C₁-C₃-alkylamino, pyridylamino,
saturated and unsaturated, four to seven-membered heterocycles which contain one or two hetero-atoms selected from N, S and O and are either bound directly or over a methylene group or a methine group,
monocyclic aromatic five and six-membered heterocycles which contain one to three hetero-atoms, selected from N, S and O and are either bound directly or over methylene group or a methine group,
anellated bicyclic, aromatic and partially hydrogenated carbocyclic ring systems with 8 to 12 ring atoms which are either bound directly over a methylene group or a methine group, and
anellated bicyclic aromatic and partially hydrogenated heterocyclic ring systems with 8 to 12 ring atoms, wherein one to three ring atoms are selected from N, S and O and are either bound directly or over a methylene group or a methine group,
wherein aromatic ring systems in the substituents **R**^{**1(i)**}**, R**^{**2(i)**}**, R**^{**4(i)**}**, R**^{**5(i)**}**, R**^{**6(i)**}**, R**^{**13(i)**}**, R**^{**14(i)**}**, R**^{**15(i)**}**, R**^{**16(i)**}**, R**^{**17(i)**}**, R**^{**18**}**, R**^{**19**}**, Ar**^{**1**} and **Ar**^{**2**}**,** in the groups **A**^{**(i)**} and **D**^{**(i)**}**,** in the ring systems =C**R**^{**13(i)**}**R**^{**15(i)**}**, =CR**^{**13(i)**}**R**^{**18**}**, -NR**^{**13(i)**}**R**^{**15(i)**} and -**NR**^{**13(i)**}**R**^{**18(i)**} as well as substituents and/or substituents in the cyclic imides **G**^{**13**} optionally are independently substituted by one to three of the same or different groups, selected from
halogen, cyano, C₁-C₆-alkyl, trifluoromethyl, C₃-C₈-cycloalkyl, benzyl, phenyl, hydroxy, C₁-C₆-hydroxyalkyl, C₁-C₆-alkoxy, C₁-C₆-alkoxy entirely or partially substituted by fluorine, benzyloxy, phenoxy, mercapto, C₁-C₆-alkylthio, phenylthio, sulfo, carboxy, C₂-C₇-carboxyalkyl, C₃-C₇-carboxyalkenyl, C₂-C₇-alkoxycarbonyl, benzyloxycarbonyl, nitro, amino, C₁-C₆-aminoalkyl, mono-C₁-C₆-alkylamino and di(C₁-C₆-alkyl)amino, and in the case of two adjacent residues on the aromatic ring, methylenedioxy,
wherein alkyl and cycloalkyl residues in the groups G optionally are substituted by one or two of the same or different groups, selected from
hydroxy, carboxy, C₂-C₇-alkoxycarbonyl, benzyloxycarbonyl, amino, mono-C₁-C₆-alkylamino and di(C₁-C₆-alkyl)amino;
and the stereoisomers or racemic or non-racemic mixtures of stereoisomers thereof,
and the tautomers thereof when **G** is a heterocyclic aromatic ring or an aromatic ring substituted by a hydroxy, mercapto or amino group,
and the pharmacologically acceptable acid addition salts thereof;
(b) at least one compound having vitamin PP activity or a prodrug thereof which is selected from the group consisting of compounds of formulae II, IIa, IIb, III, IIIa, IIIb, IIIc, IV, IVa, IVb, V, Va, and Vb: 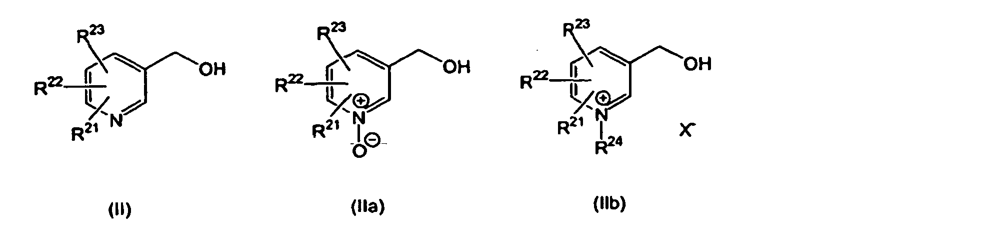 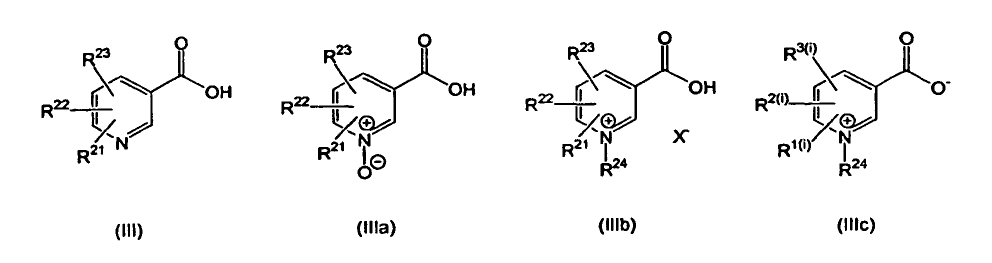 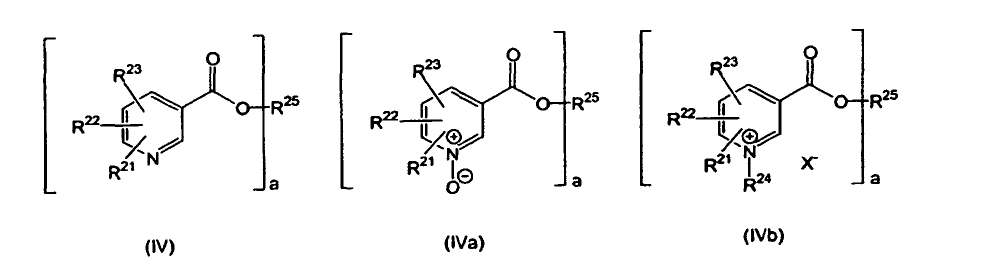 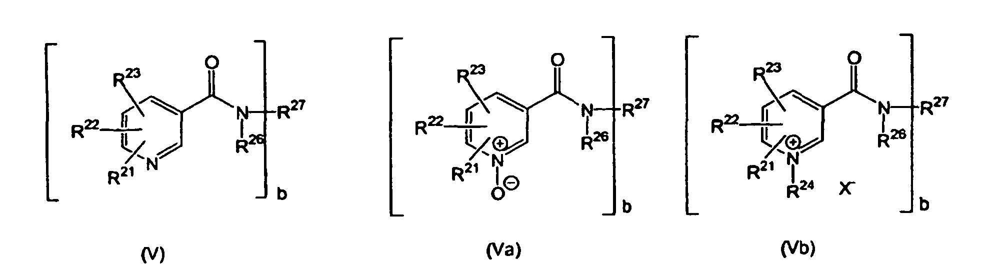 wherein:
**a** is an integer of 1 through 6;
**b** is an integer of 1 through 2;
**X**^{**-**} is selected from the group consisting of fluoride, chloride, bromide, iodide, hydrogensulfate, methanesulfonate, trifluoromethanesulfonate, tosylate, tetrafluoroborate, dihydrogenphosphate, and acetate;
**R**^{**21**} is selected from the group consisting of hydrogen, halogen, cyano, C₁-C₆-alkyl, trifluoromethyl, C₁-C₆-hydroxyalkyl, hydroxy, C₁-C₆-alkoxy, C₁-C₇-alkanoyloxy, C₁-C₆-alkylthio, C₁-C₆-aminoalkyl, amino, C₁-C₆-alkylamino, C₂-C₁₂-dialkylamino, formyl, C₂-C₇-alkoxycarbonyl, aminocarbonyl, C₂-C₇-alkylaminocarbonyl, C₃-C₁₃-dialkylamino-carbonyl and carboxy;
**R**^{**22**} is selected from the group consisting of hydrogen, halogen, C₁-C₆-alkyl, trifluoromethyl, C₁-C₆-hydroxyalkyl, hydroxy, C₁-C₆-alkoxy, C₁-C₇-alkanoyloxy, C₁-C₆-aminoalkyl, amino, C₂-C₇-alkoxycarbonyl, aminocarbonyl, and carboxy;
**R**^{**23**} is selected from the group consisting of hydrogen, C₁-C₆-alkyl, and C₁-C₆-hydroxyalkyl;
**R**^{**24**} is selected from the group consisting of C₁-C₆-alkyl, C₃-C₆-alkenyl, C₂-C₆-hydroxyalkyl, C₂-C₆-alkoxyalkyl and benzyl;
**R**^{**25**} is such that the alcohol **R**^{**25**}**(OH)**_{**a**} is selected from monovalent, linear and branched, C₁₋₁₀-alkanols and ω-dialkylaminoalkanols, benzyl alcohol, divalent linear and branched C₂₋₁₀-diols, mono- or divalent C₅₋₇-cycloalkanols, C₅₋₇-cycloalkanediols, C₅₋₇-cycloalkanemethanols, saturated C₅₋₇-heterocyclomethanols, glycerin, 2,2-bis(hydroxymethyl)-1-octanol, erythritol, pentaerythritol, arabitol, xylitol, sorbitol, mannitol, isosorbitol, tetra(hydroxymethyl)cyclohexanol, and inositol;
**R**^{**26**} is selected from the group consisting of hydrogen, C₁-C₆-alkyl, C₁-C₆-hydroxyalkyl, C₃-C₆-alkoxyalkyl, C₁-C₆-aminoalkyl, C₄-C₁₂-dialkylaminoalkyl and carboxymethyl;
when b is 1,
**R**^{**27**} is selected from the group consisting of hydrogen, C₁-C₆-alkyl, C₁-C₆-hydroxyalkyl, C₃-C₆-alkoxyalkyl, C₁-C₆-aminoalkyl, C₄-C₁₂-dialkylaminoalkyl and carboxymethyl;
when b is 2,
**R**^{**27**} is C₂-C₁₀-alkylene, or C₅-C₁₀-alkylene wherein a methylene group is isosterically replaced by O, NH or N-alkyl;
and their thioxo analogs,
and the acid addition salts or anionic salts thereof; and
(c) at least one physiologically acceptable carrier and at least one toxicologically safe adjuvant.

2. The composition according to claim 1, wherein the compound(s) of formula (I) is/are selected from:
N-[2-(1-benzylpiperidin-4-yl)-ethyl]-3-(pyridin-3-yl)-propionamide;
N-{2-[1-(2-phenylethyl)-piperidin-4-yl]-ethyl}-3-(pyridin-3-yl)-propionamide;
N-{2-[1-(4-phenylbutyl)-piperidin-4-yl]-ethyl}-3-(pyridin-3-yl)-propionamide;
N-{2-[1-(4-hydroxy-4-phenylbutyl)-piperidin-4-yl]-ethyl}-3-(pyridin-3-yl)-propionamide;
N-[2-(1-diphenylmethylpiperidin-4-yl)-ethyl]-3-(pyridin-3-yl)-propionamide;
N-[3-(1-diphenylmethylpiperidin-4-yl)-propyl]-3-(pyridin-3-yl)-propionamide;
N-[4-(1-diphenylmethylpiperidin-4-yl)-butyl]-3-(pyridin-3-yl)-propionamide;
N-[4-(1-benzylpiperidin-4-yl)-butyl]-3-(pyridin-3-yl)-acrylamide;
N-{4-[1-(2-phenylethyl)-piperidin-4-yl]-butyl}-3-(pyridin-3-yl)-acrylamide;
N-{4-[1-(4-biphenylylmethyl)-piperidin-4-yl]-butyl}-3-(pyridin-3-yl)-acrylamide;
N-{4-[1-(1-naphthylmethyl)-piperidin-4-yl]-butyl}-3-(pyridin-3-yl)-acrylamide;
N-{4-[1-(9-anthrylmethyl)-piperidin-4-yl]-butyl}-3-(pyridin-3-yl)-acrylamide:
N-{4-[1-(cyclohexylphenylmethyl)-piperidin-4-yl]-butyl}-3-(pyridin-3-yl)-acrylamide;
N-{4-[1-(10,11-dihydro-5H-dibenzo[a,d]cyclohepten-5-yl)-piperidin-4-yl]-butyl}-3-(pyridin-3-yl)-acrylamide;
N-[2-(1-diphenylmethylpiperidin-4-yl)-ethyl]-3-(pyridin-3-yl)-acrylamide;
N-[3-(1-diphenylmethylpiperidin-4-yl)-propyl]-3-(pyridin-3-yl)-acrylamide;
N-[5-(1-diphenylmethylpiperidin-4-yl)-pentyl]-3-(pyridin-3-yl)-acrylamide;
N-[6-(1-diphenylmethylpiperidin-4-yl)-hexyl]-3-(pyridin-3-yl)-acrylamide;
N-[4-(1-diphenylmethylpiperidin-4-yl)-butyl]-5-(pyridin-3-yl)-2,4-pentadienic acid amide;
N-(4-{1-[bis-(4-fluorophenyl)-methyl]-piperidin-4-yl}-butyl)-3-(pyridin-3-yl)-acrylamide;
N-(4-{1-[bis-(2-chlorophenyl)-methyl]-piperidin-4-yl}-butyl)-3-(pyridin-3-yl)-acrylamide:
N-[4-(1-diphenylmethylpiperidin-4-yl)-butyl]-3-(2-fluoropyridin-3-yl)-acrylamide;
N-[4-(1-diphenylmethylpiperidin-4-yl)-butyl]-3-(6-fluoropyridin-3-yl)-acrylamide;
N-[4-(1-diphenylmethylpiperidin-4-yl)-butyl]-3-(pyridin-3-yl)-acrylamide;
N-[4-(1-diphenylmethylpiperidin-4-yl)-butyl]-3-(pyridin-3-yl)-acrylamide dihydrochloride;
N-[4-(1-diphenylmethylpiperidin-4-yl)-butyl]-3-(pyridin-3-yl)-acrylamide methanesulfonate;
N-[4-(1-acetyl-piperidin-4-yl)-butyl]-3-(pyridin-3-yl)-propionamide;
N-[4-(1-benzoyl-piperidin-4-yl)-butyl]-3-(pyridin-3-yl)-propionamide;
N-[4-(1-diphenylacetyl-piperidin-4-yl)-butyl]-3-(pyridin-3-yl)-propionamide;
N-{4-[1-(9-oxo-9H-fluoren-4-carbonyl)-piperidin-4-yl]-butyl}-3-(pyridin-3-yl)-propionamide;
N-[4-(1-methylsulfonyl-piperidin-4-yl)-butyl]-3-(pyridin-3-yl)-propionamide;
N-{4-[1-(2-naphthyl-sulfonyl)-piperidin-4-yl]-butyl}-3-(pyridin-3-yl)-propionamide;
N-[4-(1-benzyl-piperidin-4-yl)-butyl]-3-(pyridin-3-yl)-propionamide;
N-(4-{1-[bis-(2-chlorophenyl)-methyl]-piperidin-4-yl}-butyl)-3-(pyridin-3-yl)-propionamide;
N-{4-[1-(phenylpyridin-3-yl-methyl)-piperidin-4-yl]-butyl}-3-(pyridin-3-yl)-propionamide;
N-{4-[1-(9H-fluoren-9-yl)-piperidin-4-yl]-butyl}-3-(pyridin-3-yl)-propionamide;
N-{4-[1-(6,11-dihydrodibenzo[b,e]oxepin-11-yl)-piperidin-4-yl]-butyl}-3-(pyridin-3-yl)-propionamide;
N-{4-[1-(1-naphthylamino-carbonyl)-piperidin-4-yl]-butyl}-3-(pyridin-3-yl)-propionamide;
N-[4-(1-diphenylamino-carbonyl-piperidin-4-yl)-butyl]-3-(pyridin-3-yl)-propionamide;
N-{4-[1-(10,11-dihydro-dibenzo[b,f]azepin-5-yl-carbonyl)-piperidin-4-yl]-butyl}-3-(pyridin-3-yl)-propionamide;
N-[4-(1-diphenylphosphinoyl-piperidin-4-yl)-butyl]-3-(pyridin-3-yl)-propionamide;
N-[4-(1-diphenylmethylpiperidin-4-yl)-butyl]-3-(2-fluoropyridin-3-yl)-propionamide;
N-[4-(1-diphenylmethylpiperidin-4-yl)-butyl]-3-(5-fluoropyridin-3-yl)-propionamide;
N-[4-(1-diphenylmethylpiperidin-4-yl)-butyl]-2-fluoro-3-(pyridin-3-yl)-propionamide;
N-[4-(1-diphenylmethylpiperidin-4-yl)-butyl]-2,2-difluoro-3-(pyridin-3-yl)-propionamide;
N-[5-(1-diphenylmethylpiperidin-4-yl)-pentyl]-3-(pyridin-3-yl)-propionamide;
N-[6-(1-diphenylmethylpiperidin-4-yl)-hexyl]-3-(pyridin-3-yl)-propionamide;
N-[2-(1-diphenylmethylpiperidin-4-yl)-ethyl]-5-(pyridin-3-yl)-pentanoic acid amide;
N-[4-(1-diphenylmethylpiperidin-4-yl)-butyl]-5-(pyridin-3-yl)-pentanoic acid amide;
N-[4-(1-diphenylmethylpiperidin-4-yl)-butyl]-N-hydroxy-3-(pyridin-3-yl)-propionamide;
N-[4-(1-diphenylmethylpiperidin-4-yl)-butyl]-2-hydroxy-3-(pyridin-3-yl)-propionamide;
N-[4-(1-diphenylmethylpiperidin-4-yl)-butyl]-3-hydroxy-3-(pyridin-3-yl)-propionamide;
N-[4-(1-diphenylmethylpiperidin-4-yl)-butyl]-3-(pyridin-3-yl)-propionamide;
N-[4-(1-methylsulfonylpiperidin-4-yl)-butyl]-3-(pyridin-3-yl)-acrylamide;
N-{4-[1-(2-naphthylsulfonyl)-piperidin-4-yl]-butyl}-3-(pyridin-3-yl)-acrylamide;
N-{4-[1-(2-naphthylsulfonyl)-piperidin-4-yl]-butyl}-5-(pyridin-3-yl)-2,4-pentadienic acid amide;
N-{4-[1-(1-naphthylaminocarbonyl)-piperidin-4-yl]-butyl}-3-(pyridin-3-yl)-acrylamide;
N-[4-(1-diphenylaminocarbonylpiperidin-4-yl)-butyl]-3-(pyridin-3-yl)-acrylamide;
N-[4-(1-diphenylaminocarbonyl-piperidin-4-yl)-butyl]-5-(pyridin-3-yl)-2,4-pentadienic acid amide;
N-{4-[1-(10,11-dihydrodibenzo[b,f]azepin-5-yl-carbonyl)-piperidin-4-yl]-butyl}-3-(pyridin-3-yl)-acrylamide;
N-[4-(1-diphenylphosphinoyl-piperidin-4-yl)-butyl]-3-(pyridin-3-yl)-acrylamide;
N-[4-(1-acetylpiperidin-4-yl)-butyl]-3-(pyridin-3-yl)-acrylamide;
N-[4-(1-diphenylacetylpiperidin-4-yl)-butyl]-3-(pyridin-3-yl)-acrylamide;
N-{4-[1-(3,3-diphenylpropionyl)-piperidin-4-yl]-butyl}-3-(pyridin-3-yl)-acrylamide;
N-[4-(1-benzoylpiperidin-4-yl)-butyl]-3-(pyridin-3-yl)-acrylamide;
N-[4-(1-benzoylpiperidin-4-yl)-butyl]-5-(pyridin-3-yl)-2,4-pentadienic acid amide;
N-{4-[1-(9-oxo-9H-fluoren-4-yl-carbonyl)-piperidin-4-yl]-butyl}-3-(pyridin-3-yl)-acrylamide;
N-{4-[1-(phenylpyridin-3-yl-methyl)-piperidin-4-yl]-butyl}-3-(pyridin-3-yl)-acrylamide;
N-{4-[1-(phenylpyridin-4-yl-methyl)-piperidin-4-yl]-butyl}-3-(pyridin-3-yl)-acrylamide;
N-{4-[1-(6,11-dihydrodibenzo[b,e]oxepin-11-yl)-piperidin-4-yl]-butyl}-3-(pyridin-3-yl)-acrylamide;
N-{4-[1-(6,11-dihydrodibenzo[b,e]thiepin-11-yl)-piperidin-4-yl]-butyl}-3-(pyridin-3-yl)-acrylamide;
N-[7-(1-diphenylmethylpiperidin-4-yl)-heptyl]-3-(pyridin-3-yl)-acrylamide;
N-[8-(1-diphenylmethylpiperidin-4-yl)-octyl]-3-(pyridin-3-yl)-acrylamide;
N-[3-(1-diphenylmethylpiperidin-4-yloxy)-propyl]-3-(pyridin-3-yl)-acrylamide;
N-[3-(1-benzylpiperidin-4-yloxy)-propyl]-3-(pyridin-3-yl)-acrylamide;
N-[2-(1-diphenylmethylpiperidin-4-yl)-ethyl]-5-(pyridin-3-yl)-2,4-pentadienic acid amide;
N-[4-(1-diphenylmethylpiperidin-4-yl)-butyl]-5-(pyridin-3-yl)-2,4-pentadienic acid amide;
N-[5-(1-diphenylmethylpiperidin-4-yl)-pentyl]-5-(pyridin-3-yl)-2,4-pentadienic acid amide;
N-[6-(1-diphenylmethylpiperidin-4-yl)-hexyl]-5-(pyridin-3-yl)-2,4-pentadienic acid amide;
N-[4-(4-diphenylmethylpiperazin-1-yl)-3-hydroxy-butyl]-3-pyridin-3-yl-acrylamide;
N-[3-(4-diphenylmethylpiperazin-1-yl)-propoxy]-3-pyridin-3-yl-acrylamide;
N-[4-(4-diphenylmethylpiperazin-1-yl)-4-oxo-butyl]-3-pyridin-3-yl-acrylamide;
N-[3-(4-diphenylmethylpiperazin-1-sulfonyl)-propyl]-3-pyridin-3-yl-acrylamide;
N-{2-[2-(4-diphenylmethylpiperazin-1-yl)-ethoxy]-ethyl}-3-pyridin-3-yl-acrylamide;
N-(4-{4-[bis-(4-fluorophenyl)-methyl]-piperazin-1-yl}-but-2-inyl)-3-pyridin-3-yl-acrylamide;
N-{4-[4-(4-carboxyphenyl-phenylmethyl)-piperazin-1-yl]-butyl}-3-pyridin-3-yl-acrylamide;
N-(4-{4-[(4-aminophenyl)-phenylmethyl]-piperazin-1-yl}-butyl)-3-pyridin-3-yl-acrylamide;
N-{4-[4-(9H-fluoren-9-yl)-piperazin-1-yl]-butyl}-2-(pyridin-3-yloxy)-acetamide;
N-{5-[4-(9H-fluoren-9-yl)-piperazin-1-yl]-pentyl}-3-pyridin-3-yl-acrylamide;
N-{6-[4-(9H-fluoren-9-yl)-piperazin-1-yl]-hexyl}-3-pyridin-3-yl-acrylamide;
3-pyridin-3-yl-N-{4-[4-(1,2,3,4-tetrahydronaphthalen-1-yl)-piperazin-1-yl]-butyl}-acrylamide;
3-pyridin-3-yl-N-{4-[4-(5,6,7,8-tetrahydronaphthalen-1-yl)-piperazin-1-yl]-butyl}-acrylamide;
N-{4-[4-(naphthalen-1-yl)-piperazin-1-yl]-butyl}-3-pyridin-3-yl-acrylamide;
N-[4-(4-biphenyl-2-yl-piperazin-1-yl)-butyl]-3-pyridin-3-yl-propionamide;
N-[5-(4-biphenyl-2-yl-piperazin-1-yl)-pentyl]-3-pyridin-3-yl-acrylamide;
N-[6-(4-biphenyl-2-yl-piperazin-1-yl)-hexyl]-3-pyridin-3-yl-acrylamide;
N-[4-(4-biphenyl-2-yl-piperazin-1-yl)-butyl]-2-(pyridin-3-yloxy)-acetamide;
N-[4-(4-biphenyl-2-yl-piperazin-1-yl)-butyl]-5-(pyridin-3-yl)-penta-2,4-dienic acid amide;
N-{4-[4-(10,11-dihydro-5H-dibenzo[a,d]cyclohepten-5-yl)-piperazin-1-yl]-butyl}-3-pyridin-3-yl-propionamide;
N-{5-[4-(10,11-dihydro-5H-dibenzo[a,d]cyclohepten-5-yl)-piperazin-1-yl]-pentyl}-3-pyridin-3-yl-acrylamide;
N-{6-[4-(10,11-dihydro-5H-dibenzo[a,d]cyclohepten-5-yl)-piperazin-1-yl]-hexyl}-3-pyridin-3-yl-acrylamide;
N-{4-[4-(10,11-dihydro-5H-dibenzo[a,d]cyclohepten-5-yl)-piperazin-1-yl]-butyl}-5-(pyridin-3-yl)-penta-2,4-dienic acid amide;
N-{4-[4-(6,11-dihydro-dibenzo[b,e]oxepin-11-yl)-piperazin-1-yl]-butyl-3-pyridin-3-yl-propionamide;
N-{2-[4-(6,11-dihydro-dibenzo[b,e]thiepin-11-yl)-piperazin-1-yl]-ethyl}-3-pyridin-3-yl-acrylamide;
N-[4-(4-diphenylacetylpiperazin-1-yl)-butyl]-3-pyridin-3-yl-acrylamide;
N-[4-(4-benzoylpiperazin-1-yl)-butyl]-3-pyridin-3-yl-acrylamide;
N-{4-[4-(2-aminobenzoyl)-piperazin-1-yl]-butyl}-3-pyridin-3-yl-acrylamide;
N-{4-[4-(4-carboxybenzoyl)-piperazin-1-yl]-butyl}-3-pyridin-3-yl-acrylamide;
N-{4-[4-(biphenyl-2-carbonyl)-piperazin-1-yl]-butyl}-3-pyridin-3-yl-acrylamide;
N-{4-[4-(9-oxo-9H-fluoren-4-carbonyl)-piperazin-1-yl]-butyl}-3-pyridin-3-yl-acrylamide;
N-{4-[4-(furan-2-carbonyl)-piperazin-1-yl]-butyl}-3-pyridin-3-yl-acrylamide;
N-{4-[4-(naphthalen-1-yl-aminocarbonyl)-piperazin-1-yl]-butyl}-3-pyridin-3-yl-propionamide;
N-{4-[4-(diphenylaminocarbonyl)-piperazin-1-yl]-butyl}-3-pyridin-3-yl-acrylamide;
N-{4-[4-(naphthalen-2-sulfonyl)-piperazin-1-yl]-butyl}-3-pyridin-3-yl-acrylamide;
N-[4-(4-diphenylphosphinonyl-piperazin-1-yl)-butyl]-3-pyridin-3-yl-acrylamide;
N-[4-(4-biphenyl-2-yl-piperazin-1-yl)-butyl]-3-pyridin-3-yl-acrylamide;
N-{4-[4-(9H-fluoren-9-yl)-piperazin-1-yl]-butyl}-3-pyridin-3-yl-acrylamide;
N-{4-[4-(10,11-dihydro-5H-dibenzo[a,d]cyclohepten-5-yl)-piperazin-1-yl]-butyl}-3-pyridin-3-yl-acrylamide;
N-[4-(4-phenylpiperidin-1-yl)-butyl]-3-pyridin-3-yl-acrylamide;
N-{4-[4-(1H-indol-3-yl)-piperidin-1-yl]-butyl}-3-pyridin-3-yl-acrylamide;
N-{4-[4-(2-oxo-2,3-dihydrobenzimidazol-1-yl)-piperidin-1-yl]-butyl}-3-pyridin-3-yl-acrylamide;
N-[4-(4-benzotriazol-1-yl-piperidin-1-yl)-butyl]-3-pyridin-3-yl-acrylamide;
N-{4-[4-(hydroxy-diphenylmethyl)-piperidin-1-yl]-butyl}-2-(pyridin-3-yloxy)-acetamide;
N-[4-(4,4-diphenylpiperidin-1-yl)-butyl]-3-pyridin-3-yl-acrylamide;
N-{4-[4-(6,11-dihydrodibenzo[b,e]thiepin-11-yliden)-piperidin-1-yl]-butyl}-3-pyridin-3-ylpropionamide dihydrochloride semi-isopropanol;
N-{4-[4-(6,11-dihydrodibenzo[b,e]thiepin-11-yliden)-piperidin-1-yl]-butyl}-5-pyridin-3-yl-pentanamide;
N-{4-[4-(4,9-dihydro-thieno[2,3-b]-benzo[e]thiepin-4-yliden)-piperidin-1-yl]-butyl}-3-pyridin-3-yl-propionamide;
N-{4-[4-(4,9-dihydro-thieno[2,3-b]-benzo[e]thiepin-4-yliden)-piperidin-1-yl]-butyl}-3-pyridin-3-yl-acrylamide;
N-[4-(4-diphenylphosphinoyloxypiperidin-1-yl)-butyl]-3-pyridin-3-yl-acrylamide;
N-[4-(1,4-dioxa-8-azaspiro[4.5]dec-8-yl)-butyl]-3-pyridin-3-yl-acrylamide;
N-[4-(2,5-dioxo-3,4-diphenyl-2,5-dihydropyrrol-1-yl)-butyl]-3-pyridin-3-yl-acrylamide;
N-[4-(2,6-dioxo-4-phenylpiperidin-1-yl)-butyl]-3-pyridin-3-yl-acrylamide;
N-[4-(1,3-dioxo-4,5,6,7-tetraphenyl-1,3-dihydro-isoindol-2-yl)-butyl]-3-pyridin-3-yl-acrylamide;
N-[4-(3-benzyl-2,4,5-trioxo-imidazolidin-1-yl)-butyl]-3-pyridin-3-yl-acrylamide;
N-[4-(1,3,10-trioxo-1,4,5,6,10,10a-hexahydro-acenaphtho-[1,8a-c]pyrrol-2-yl)-butyl]-3-pyridin-3-yl-acrylamide;
N-[4-(2,5-dioxo-4,4-diphenylimidazolidin-1-yl)-butyl-3-pyridin-3-yl-acrylamide;
N-[4-(2,5-dioxo-3-phenyl-2,5-dihydropyrrol-1-yl)-butyl]-3-pyridin-3-yl-acrylamide;
N-[3-(2,5-dioxo-3,4-diphenyl-2,5-dihydro-pyrrol-1-yl)-propyl]-3-pyridin-3-yl-acrylamide;
N-[4-(3-pyridin-3-yl-acroylamino)-butyl]-2,3:5,6-dibenzobicyclo[2.2.2]octan-7,8-dicarboximide;
N-[4-(5-benzyliden-2,4-dioxothiazolidin-3-yl)-butyl]-3-pyridin-3-yl-acrylamide;
N-[4-(4-benzyl-2,6-dioxopiperazin-1-yl)-butyl]-3-pyridin-3-yl-acrylamide;
N-[6-(2,5-dioxo-3,4-diphenyl-2,5-dihydropyrrol-1-yl)-hexyl]-3-pyridin-3-yl-acrylamide;
N-[4-(2,5-dioxo-3,4-diphenyl-2,5-dihydropyrrol-1-yl)-butyl]-3-pyridin-3-yl-propionamide;
N-[4-(1,3-dioxo-1,3-dihydro-isoindol-2-yl)-butyl]-3-pyridin-3-yl-acrylamide;
N-[4-(1,3-dioxo-1H,3H-benzo[de]isoquinolin-2-yl)-butyl]-3-(1-oxidopyridin-3-yl)-acrylamide;
N-[6-(1,3-dioxo-1H,3H-benzo[de]isoquinolin-2-yl)-hexyl]-3-pyridin-3-yl-acrylamide;
N-[2-(1,3-dioxo-1H,3H-benzo[de]isoquinolin-2-yl)-ethyl]-3-pyridin-3-yl-acrylamide;
N-[4-(1,3-dioxo-1H,3H-benzo[de]isoquinolin-2-yl)-butyl]-3-pyridin-3-yl-acrylamide;
N-[8,8-bis-(4-fluorophenyl)-octyl]-3-pyridin-3-yl-acrylamide hydrochloride;
N-[6-(3,3-diphenylureido)-hexyl]-3-pyridin-3-yl-acrylamide;
N-[4-(1-phenyl-1,2,4,5-tetrahydrobenzo[d]azepin-3-yl)-butyl]-3-pyridin-3-yl-acrylamide;
N-(8,8-diphenyloctyl)-3-pyridin-3-yl-acrylamide;
N-(8-hydroxy-8,8-diphenyloctyl)-3-pyridin-3-yl-acrylamide;
N-[4-(3,3-diphenylureido)-butyl]-3-pyridin-3-yl-acrylamide;
N-[4-(1H,3H-benzo[de]isoquinolin-2-yl)-butyl]-3-pyridin-3-yl-acrylamide;
N-[6-(10,11-dihydro-dibenzo[b,f]azepin-5-yl-carbonylamino)-hexyl]-3-pyridin-3-yl-acrylamide;
3-pyridin-3-yl-N-[6-(tosylamino)-hexyl]-acrylamide;
N-[4-(1,1-dioxo-1-thia-2-aza-acenaphthylen-2-yl)-butyl]-3-pyridin-3-yl-acrylamide;
N-(6-hydroxy-6,6-diphenylhexyl)-3-pyridin-3-yl-acrylamide;
N-(6,6-diphenyl-hex-5-enyl)-3-pyridin-3-yl-acrylamide;
N-[4-(4,5-diphenylimidazol-1-yl)-butyl]-3-pyridin-3-yl-acrylamide;
N-[4-(trans-2-phenylcyclopropylcarbonylamino)butyl]-3-pyridin-3-yl-acrylamide;
N-(5-hydroxy-5,5-diphenyl-pentyl)-3-pyridin-3-yl-acrylamide;
N-(7-phenylheptyl)-3-pyridin-3-yl-acrylamide;
N-(4-diphenylacetylamino-butyl)-3-pyridin-3-yl-acrylamide;
N-[4-(benzhydrylamino)-butyl]-3-pyridin-3-yl-acrylamide; and
N-(4-{[2-(benzhydrylmethylamino)-ethyl]-methylamino}-butyl)-3-pyridin-3-yl-acrylamide.

3. The composition according to claim 1 or 2 comprising a further cancerostatic or immunosuppressive agent that is not a compound of formula (I).

4. The composition according to any one of claims 1 to 3, wherein the compounds(s) of formula (I) and the compound(s) of fomulae (II) to (Vb) are contained separately within the composition.

5. The composition according to any one of claims 1 to 4, wherein the compounds(s) of formula (I) and the compound(s) of fomulae (II) to (Vb) are present in separate dosage forms, and the dosage forms are packaged together for co-administration.

6. The composition according to any one of claims 1 to 5, which contains tryptophan as a prodrug.

7. The composition according to any one of claims 1 to 6, which is:
in a solid, peroral administrable form as a tablet, capsule, coated tablet, optionally as sustained action or gastric fluid-resistant preparation, as a liquid medicinal form, peroral administrable solution, suspension, effervescent tablet, in the form of tabs or sachets, optionally in sustained action form,
in the form of a suitable injection or infusion preparation together with suitable pharmaceutically acceptable carriers and adjuvants, optionally in sustained action form or as a parenteral depot medicinal form or implant, in the form of a concentrate, powder or lyophilisate,
in the form of an inhalation therapeutic agent, in the form of a spray together with suitable pharmaceutically acceptable propellants, carriers and adjuvants,
in the form of a transdermal therapeutic system for systemic treatment,
in the form of a gastrointestinal therapeutic system (GITS) for systemic treatment,
in the form of a salve, suspension, emulsion, a balm or plaster or in the form of an externally applicable solution,
in the form of a rectal, genital, or transurethral administrable emulsion, a solution, a liposomal solution, an implant, suppository or a capsule.
in the form of a nasally, otologically or ophthalmologically applicable composition, or
in a buccally applicable form.

8. The composition according to any one of claims 1 to 6 for administration by means of a controlled dosage aerosol or in the form of a dry powder dosage formulation.

9. The composition according to any one of claims 1 to 8, wherein a dosage unit for individual administration contains 0.001 to 1000, 2000, 3000, 4000 or 5000 mg of the compound(s) according to formula (I).

10. Use of a compound having vitamin PP activity or a prodrug thereof for the manufacture of a pharmaceutical composition for preventing, reducing, or eliminating side effects or neutralizing the side effects of a cancerostatic or immunosuppressive agent administered prophylactically or therapeutically to a patient.

11. The use according to claim 10, wherein the compound having vitamin PP activity or a prodrug thereof is selected from the group consisting of compounds of formulae II, IIa, IIb, III, IIIa, IIIb, IIIc, IV, IVa, IVb, V, Va, and Vb: 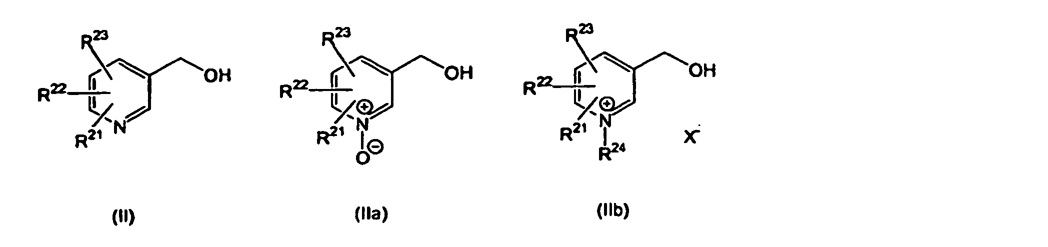 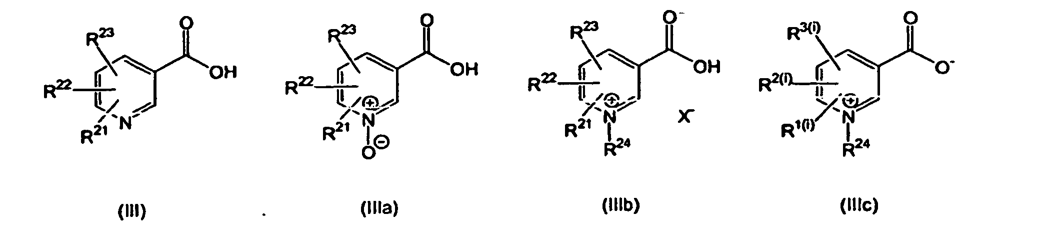 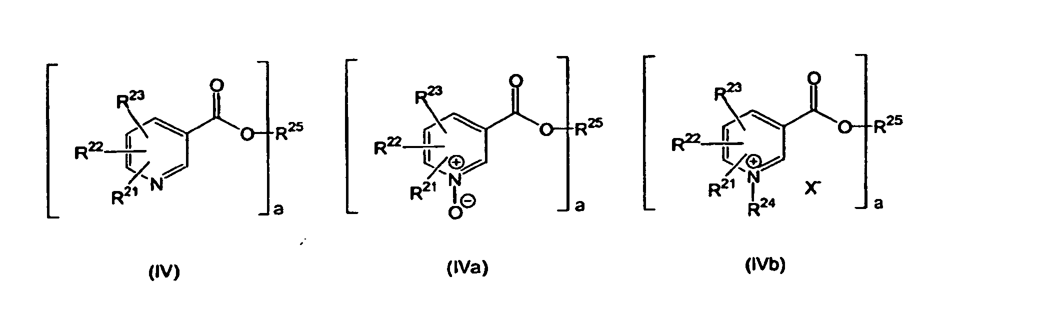 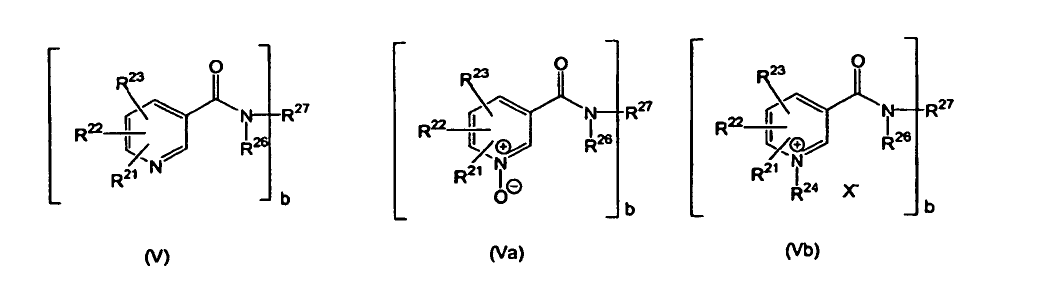 wherein:
**a** is an integer of 1 through 6;
**b** is an integer of 1 through 2;
**X**^{**-**} is selected from the group consisting of fluoride, chloride, bromide, iodide, hydrogensulfate, methanesulfonate, trifluoromethanesulfonate, tosylate, tetrafluoroborate, dihydrogenphosphate, and acetate;
**R**^{**21**} is selected from the group consisting of hydrogen, halogen, cyano, C₁-C₆-alkyl, trifluoromethyl, C₁-C₆-hydroxyalkyl, hydroxy, C₁-C₆-alkoxy, C₁-C₇-alkanoyloxy, C₁-C₆-alkylthio, C₁-C₆-aminoalkyl, amino, C₁-C₆-alkylamino, C₂-C₁₂-dialkylamino, formyl, C₂-C₇-alkoxycarbonyl, aminocarbonyl, C₂-C₇-alkylaminocarbonyl, C₃-C₁₃-dialkylamino-carbonyl and carboxy;
**R**^{**22**} is selected from the group consisting of hydrogen, halogen, C₁-C₆-alkyl, trifluoromethyl, C₁-C₆-hydroxyalkyl, hydroxy, C₁-C₆-alkoxy, C₁-C₇-alkanoyloxy, C₁-C₆-aminoalkyl, amino, C₂-C₇-alkoxycarbonyl, aminocarbonyl, and carboxy;
**R**^{**23**} is selected from the group consisting of hydrogen, C₁-C₆-alkyl, and C₁-C₆-hydroxyalkyl;
**R**^{**24**} is selected from the group consisting of C₁-C₆-alkyl, C₃-C₆-alkenyl, C₂-C₆-hydroxyalkyl, C₂-C₆-alkoxyalkyl and benzyl;
**R**^{**25**} is such that the alcohol **R**^{**25**}**(OH)**_{**a**} is selected from monovalent, linear and branched, C₁₋₁₀-alkanols and ω-dialkylaminoalkanols, benzyl alcohol, divalent linear and branched C₂₋₁₀-diols, mono- or divalent C₅₋₇-cycloalkanols, C₅₋₇-cycloalkanediols, C₅₋₇-cycloalkanemethanols, saturated C₅₋₇-heterocyclomethanols, glycerin, 2,2-bis(hydroxymethyl)-1-octanol, erythritol, pentaerythritol, arabitol, xylitol, sorbitol, mannitol, isosorbitol, tetra(hydroxymethyl)cyclohexanol and inositol;
**R**^{**26**} is selected from the group consisting of hydrogen, C₁-C₆-alkyl, C₁-C₆-hydroxyalkyl, C₃-C₆-alkoxyalkyl, C₁-C₆-aminoalkyl, C₄-C₁₂-dialkylaminoalkyl and carboxymethyl;
when b is 1,
**R**^{**27**} is selected from the group consisting of hydrogen, C₁-C₆-alkyl, C₁-C₆-hydroxyalkyl, C₃-C₆-alkoxyalkyl, C₁-C₆-aminoalkyl, C₄-C₁₂-dialkylaminoalkyl and carboxymethyl;
when b is 2,
**R**^{**27**} is C₂-C₁₀-alkylene, or C₅-C₁₀-alkylene wherein a methylene group is isosterically replaced by O, NH or N-alkyl;
and their thioxo analogs,
and the acid addition salts or anionic salts thereof.

12. The use according to claim 10 or 11, wherein the compound having vitamin PP activity or a prodrug thereof is selected from the group consisting of nicotinic acid, nicotinamide, and their pharmaceutically acceptable ester and amide derivatives, anionic, quaternary, and addition salts, and analogous thioxo derivatives, their isomers, and prodrugs thereof.

13. The use according to any one of claims 10 to 12, wherein the compound having vitamin PP activity or a prodrug thereof is selected from the group consisting of nicotinic acid, nicotinamide, and mixtures thereof.

14. The use according to any one of claims 10 to 12, wherein the compound having vitamin PP activity or a prodrug thereof is tryptophan.

15. The use according to any one of claims 10 to 14, wherein the cancerostatic or immunosuppressive agent is selected from the group consisting of compounds of formula (I): 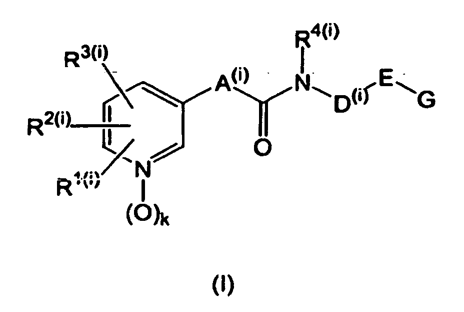 wherein:
**R**^{**1(i)**} is selected from
hydrogen, halogen, cyano, C₁-C₆-alkyl, C₃-C₆-alkenyl, C₂-C₆-alkinyl, trifluoromethyl, hydroxy, C₃-C₈-cycloalkyl, C₁-C₆-hydroxyalkyl, C₁-C₆-alkoxy, C₃-C₆-alkenyloxy, C₃-C₆-alkinyloxy, benzyloxy, C₁-C₇-alkanoyloxy, C₁-C₇-alkoxycarbonyloxy, C₁-C₆-alkylthio, C₃-C₆-alkenylthio, C₃-C₆-alkinylthio, C₃-C₈-cycloalkyloxy, C₃-C₈-cycloalkylthio, C₂-C₇-alkoxycarbonyl, aminocarbonyl, C₂-C₇-alkylaminocarbonyl, C₃-C₁₃-dialkylaminocarbonyl, carboxy, phenyl, phenoxy, phenylthio, pyridyloxy, pyridylthio, and **NR**^{**5(i)**}**R**^{**6(i)**}**,** wherein
**R**^{**5(i)**} and **R**^{**6(i)**} are selected independently from each other from hydrogen, C₁-C₆-alkyl, C₃-C₆-alkenyl, C₃-C₆-alkinyl, benzyl and phenyl;
**R**^{**2(i)**} is selected from
hydrogen, halogen, cyano, C₁-C₆-alkyl, trifluoromethyl, hydroxy, C₁-C₆-alkoxy, benzyl and C₁-C₆-alkanoyloxy; or
**R**^{**1(i)**} and **R**^{**2(i)**} when they are adjacent, optionally form a bridge selected from
-(CH₂)₄-, -(CH=CH)₂- and -CH₂O-**CR**^{**7(i)**}**R**^{**8(i)**}-O-, wherein
**R**^{**7(i)**} and **R**^{**8(i)**} are selected independently from each other from hydrogen and C₁-C₆-alkyl;
**R**^{**3(i)**} selected is from
hydrogen, halogen, C₁-C₆-alkyl, trifluoromethyl and C₁-C₆-hydroxyalkyl;
**R**^{**4(i)**} is selected from
hydrogen, hydroxy, C₁-C₆-alkyl, C₃-C₆-alkenyl, C₃-C₆-alkinyl, C₃-C₆-cycloalkyl, C₁-C₆-alkoxy and benzyloxy;
**k** is 0 or 1,
**A**^{**(i)**} is selected from
C₁-C₆-alkylene, optionally substituted one- to three-fold by C₁-C₆-alkyl, C₁-C₃-alkoxy, hydroxy, fluorine, or phenyl,
C₂-C₆-alkylene, wherein a methylene unit is isosterically replaced by O, S, **NR**^{**9(i)**}**,** CO, SO or SO₂,
wherein, with the exception of CO, the isosteric substitution is not adjacent to the amide group, and **R**^{**9(i)**} is selected from hydrogen, C₁-C₆-alkyl, C₃-C₆-alkenyl, C₃-C₆-alkinyl, C₃-C₆-acyl and C₁-C₆-alkanesulfonyl,
1,2-cyclopropylene,
C₂-C₆-alkenylene, optionally substituted one to three-fold by C₁-C₆-alkyl, hydroxy, C₁-C₃-alkoxy, fluorine, cyano or phenyl,
C₄-C₆-alkadienylene, optionally substituted once or twice by C₁-C₃-alkyl, fluorine, cyano or phenyl,
1,3,5-hexatrienylene, optionally substituted by C₁-C₆-alkyl, fluorine, cyano or phenyl, and
ethinylene;
**D**^{**(i)**} is selected from
C₁-C₁₂-alkylene, optionally substituted once or twice by C₁-C₆-alkyl, hydroxy, C₁-C₆-alkoxy, or phenyl,
C₂-C₁₂-alkenylene or C₄-C₁₂-alkadienylene, optionally substituted once or twice by C₁-C₆-alkyl, hydroxy, C₁-C₆-alkoxy, or phenyl, wherein one double-bond can optionally occur to ring **E** in the case that ring **E** is linked over a C-atom,
C₃-C₁₂-alkinylene or C₄-C₁₂-alkeninylene, optionally substituted once or twice by C₁-C₆-alkyl, hydroxy, C₁-C₆-alkoxy or phenyl, and
C₁-C₁₂-alkylene, C₂-C₁₂-alkenylene or C₃-C₁₂-alkinylene, wherein, one to three methylene units, with the exception of the (G)-terminal methylene group in the case that E represents a bond, are isosterically replaced by O, S, **NR**^{**10(i)**}**,** CO, SO or SO₂, wherein **R**^{**10(i)**} has the same meaning as **R**^{**9(i)**}**,** but is selected independently thereof;
**E** is selected from **E**^{**1(i)**}**, E**^{**2(i)**}**, E**^{**3**}**, E**^{**4**}**, E**^{**5**} and **E**^{**6**}**,** wherein
**E**^{**1(i)**} is 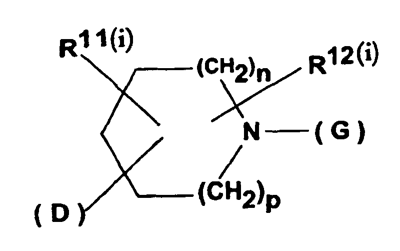
**E**^{**2(i)**} is 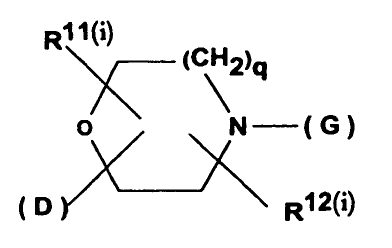
**E**^{**3**} is 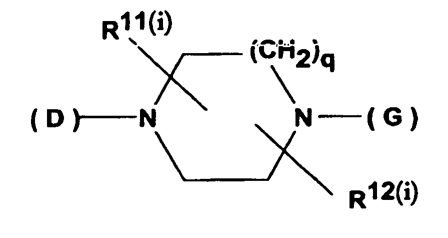
**E**^{**4**} is 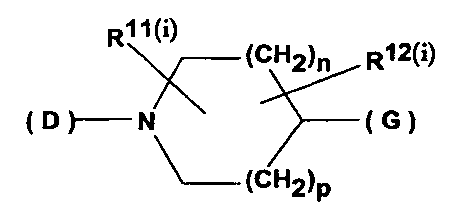
**E**^{**5**} is 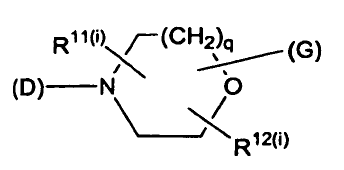 and
**E**^{**6**} represents a single or double bond,
wherein the heterocyclic rings **E**^{**1(i)**} to **E**^{**5**} optionally have a double bond,
**n** and **p** are, independently from each other, 0, 1, 2, or 3 with the proviso that, **n** + **p** ≤ 4,
**q** is 1, 2 or 3;
**R**^{**11(i)**} is selected from
hydrogen, C₁-C₆-alkyl, hydroxy, hydroxymethyl, carboxy and C₂-C₇-alkoxycarbonyl,
**R**^{**12(i)**} is selected from
hydrogen, C₁-C₆-alkyl and an oxo group adjacent to a nitrogen atom, or
**R**^{**11(i)**} and **R**^{**12(i)**}**,** optionally together, form a C₁-C₃-alkylene bridge under formation of a bicyclic ring system, and
**(a)** in the case that E represents **E**^{**1(i)**}**, E**^{**2(i)**}**,** or **E**^{**3**} the substituent G optionally is selected from **G**^{**1(i)**}**, G**^{**2(i)**}**, G**^{**3(i)**}**, G**^{**4(i)**} and **G**^{**5(i)**}**,** wherein
**G**^{**1(i)**} is 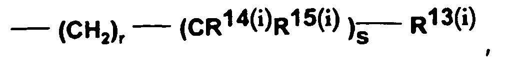 wherein
r is 0 to 3 and
**s** is 0 or 1,
**R**^{**13(i)**} is selected from
hydrogen, C₁-C₆-alkyl, C₃-C₆-alkenyl, C₃-C₆-alkinyl and C₃-C₈-cycloalkyl;
saturated or unsaturated, four to seven-membered heterocycles which contain one or two hetero-atoms selected from N, S and O;
benzyl, phenyl;
monocyclic aromatic five or six-membered heterocycles which contain one to three hetero-atoms selected from N, S and O, and are either bound directly or over a methylene group;
anellated bi- and tricyclic aromatic or partially hydrogenated carbocyclic ring systems with 8 to 16, 17 or 18 ring atoms and at least one aromatic ring, wherein the linkage occurs either over an aromatic or a hydrogenated ring and either directly or over a methylene group;
anellated bi- and tricyclic aromatic or partially hydrogenated heterocyclic ring systems with 8 to 16, 17 or 18 ring atoms and at least one aromatic ring, wherein one to three ring atoms are selected from N, S and O and the linkage occurs either over an aromatic or a
hydrogenated ring and either directly or over a methylene group;
**R**^{**14(i)**} has the same meaning as **R**^{**13(i)**}**,** but is independently selected therefrom;
**R**^{**15(i)**} is selected from
hydrogen, hydroxy, methyl, benzyl, phenyl,
monocyclic aromatic five or six-membered heterocycles which contain one to three hetero-atoms selected from N, S and O and are either bound directly or over a methylene group;
anellated bi- and tricyclic aromatic or partially hydrogenated carbocyclic ring systems with 8 to 16, 17 or 18 ring atoms and at least one aromatic ring, wherein the linkage occurs either over an aromatic or a hydrogenated ring and either directly or over a methylene group;
anellated bi- and tricyclic aromatic or partially hydrated heterocyclic ring systems with 8 to 16, 17 or 18 ring atoms and at least one aromatic ring, wherein one to three ring atoms are selected from N, S and O and the linkage occurs either over an aromatic or a hydrogenated ring and either directly or over a methylene group;
**G**^{**2(i)**} is selected from 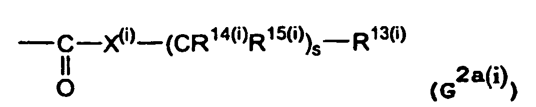 and 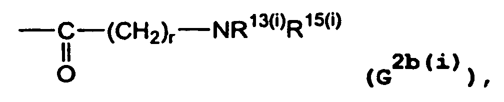 wherein **r, s** and the substituents **R**^{**13(i)**}**, R**^{**14(i)**} and **R**^{**15(i)**} have the above meanings,
or the group
**-NR**^{**13(i)**}**R**^{**15(i)**}
is a nitrogen-containg heterocycle bound over the nitrogen atom, which nitrogen-containing heterocycle is selected from
saturated and unsaturated monocyclic, four to eight-membered heterocycles, which aside from the essential nitrogen atom, optionally contain one or two further hetero-atoms selected from N, S and O,
and
saturated and unsaturated bi- or tricyclic, anellated or bridged heterocycles with 8 to 16, 17 or 18 ring atoms, which aside from the essential nitrogen atom, optionally contain one or two further hetero-atoms selected from N, S and O,
**X**^{**(i)**} is selected from
methylene, ethylene, ethenylene, propylene, C₃-C₇-cycloalkylene, or represents a bond;
**G**^{**3(i)**} is
**-SO**_{**2-**}**(CH**_{**2**}**)**_{**r-**}**R**^{**13(i)**}**,**
wherein **r** and **R**^{**13(i)**} have the above meanings,
**G**^{**4(i)**} is 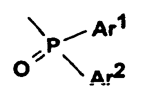 wherein
**Ar**^{**1**} and **Ar**^{**2**} are selected independently from each other from phenyl, pyridyl and naphthyl;
**G**^{**5(i)**} is
-**COR**^{**16(i)**}
wherein
**R**^{**16(i)**} is selected from
trifluoromethyl, C₁-C₆-alkoxy, C₃-C₆-alkenyloxy, and benzyloxy,
**(c)** in the case that **E** is **E**^{**4**} or **E**^{**5**}**,**
then **G** optionally is **G**^{**1(i)**}**, G**^{**2(i)**}**, G**^{**6(i)**}**, G**^{**7**} or **G**^{**8**}**,**
wherein **G**^{**1(i)**} and **G**^{**2(i)**} have the above meanings and
**G**^{**6(i)**} is
**=(C)**_{**u**}**R**^{**13(i)**}**R**^{**15(i)**}**,**
wherein **R**^{**13(i)**} and **R**^{**15(i)**} have the above meanings and
**u** is 0 or 1,
or when **u** = 1, then **R**^{**13(i)**} and **R**^{**15(i)**} together with the carbon atom to which they are attached form a ring system selected from
C₃-C₈-cycloalkyl,
saturated, four to seven-membered heterocycles which optionally contain one or two hetero-atoms, selected from N, S and O;
anellated bi- and tricyclic partially hydrogenated carbocyclic ring systems with 8 to 16, 17 or 18 ring atoms and at least one aromatic ring;
anellated bi- and tricyclic partially hydrogenated heterocyclic ring systems with 8 to 16, 17 or 18 ring atoms and at least one aromatic ring, wherein one to three ring atoms are selected from N, S and O;
or when **u** = 0 then **R**^{**13(i)**} and **R**^{**15(i)**} together with the carbon atom of ring **E** to which they are attached form a ring system selected from
C₃-C₈-cycloalkyl,
saturated, four to seven-membered heterocycles which contain one or two hetero-atoms, selected from N, S and O;
anellated bi- and tricyclic partially hydrogenated carbocyclic ring systems with 8 to 16, 17 or 18 ring atoms and at least one aromatic ring; and
anellated bi- and tricyclic partially hydrogenated heterocyclic ring systems with 8 to 16, 17 or 18 ring atoms and at least one aromatic ring, wherein one to three ring atoms are selected from N, S and O;
**G**^{**7**} is selected from
**-NR**^{**17(i)**}**-(CH**_{**2**}**)**_{**r-**}**(CR**^{**14(i)**}**R**^{**15(i)**}**)**_{**s-**}**R**^{**13(i)**} **(G**^{**7a**}**),** 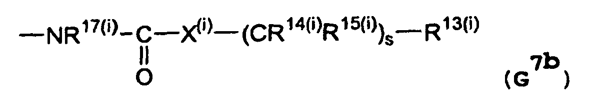 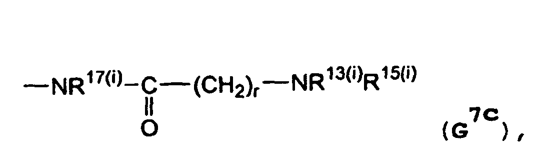
**-NR**^{**17(i)**}**-SO**_{**2**}**-(CH**_{**2**}**)**_{**r**}**-R**^{**13(i)**} **(G**^{**7d**}**),** 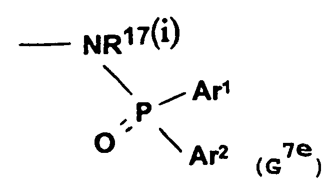
and
**-NR**^{**17(i)**}**-COR**^{**16(i)**} **(G**^{**7f**}**),**
wherein **r, s, X**^{**(i)**}**,** the substituents **R**^{**13(i)**}**, R**^{**14(i)**}**, R**^{**15(i)**} and **R**^{**16(i)**} and the group
**-NR**^{**13(i)**}**R**^{**15(i)**}
have the above meanings, and
**R**^{**17(i)**} has the same meanings as **R**^{**5(i)**}**,** but is selected independently thereof,
**Ar**^{**1**} and **Ar**^{**2**} are selected independently from each other from phenyl, pyridyl and naphthyl;
**G**^{**8**} is selected from 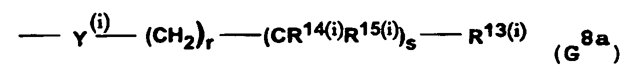 and 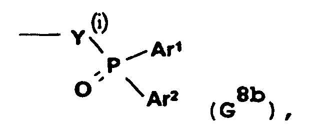 wherein
**r, s** and the substituents **R**^{**13(i)**}**, R**^{**14(i)**}**, R**^{**15(i)**}**, Ar**^{**1**} and **Ar**^{**2**} have the above meanings, and
**Y**^{**(i)**} is O or S;
**(c)** in the case that the substituent **E** is **E**^{**6**}**,**
then the substituent **G** optionally is selected from **G**^{**7d**}**, G**^{**7e**}**, G**^{**8b**}**, G**^{**9**}**, G**^{**10**}**, G**^{**11**}**, G**^{**12**}**,** and **G**^{**13**}**,** wherein **G**^{**7d**}**, G**^{**7e**} and **G**^{**8b**} have the above meanings and
**G**^{**9**} is selected from
-**(CR**^{**13(i)**}**R**^{**19**}**)**_{**s**}**-R**^{**18**} **(G**^{**9a**}**)**
and
-**NR**^{**13(i)**}**R**^{**18**} **(G**^{**9b**}**),**
wherein **s** and **R**^{**13(i)**} are defined as above, and
**R**^{**18**} is selected from
benzyl, diphenylmethyl, phenyl;
monocyclic aromatic five and six-membered heterocycles which can contain one to three hetero-atoms selected from N, S and O and are either bound directly or over a methylene group;
anellated bi- and tricyclic aromatic or partially hydrogenated carbocyclic ring systems with 8 to 16, 17 or 18 ring atoms and at least one aromatic ring, wherein the linkage occurs either over an aromatic or a hydrogenated ring and either directly or over a methylene group; and
anellated bi- and tricyclic aromatic or partially hydrogenated heterocyclic ring systems with 8 to 16, 17 or 18 ring atoms and at least one aromatic ring, wherein one to three ring atoms are selected from N, S and O and the linkage occurs either over an aromatic or a hydrogenated ring and either directly or over a methylene group;
**R**^{**19**} has the same meanings as **R**^{**13(i)**} but is selected independently thereof, and in addition can be hydroxy;
or the group
-**NR**^{**13(i)**}**R**^{**18**}
optionally is a nitrogen-containing heterocycle bound over the nitrogen atom, which nitrogen-containing heterocycle is selected from
anellated bi- and tricyclic aromatic or partially hydrogenated heterocyclic ring systems with 8 to 16, 17 or 18 ring atoms and at least one aromatic ring, which aside from the essential nitrogen atom, optionally contain one or two further hetero-atoms selected from N, S and O;
**G**^{**10**} is
**=CR**^{**13(i)**}**R**^{**18**} **(G**^{**10**}**)**
bound to D by means of a double bond, wherein **R**^{**13(i)**} and **R**^{**18**} have the above meanings, or wherein **G**^{**10**}
optionally is a ring system bound over the carbon atom, selected from
anellated bi- and tricyclic partially hydrogenated carbocyclic ring systems with 8 to 16, 17 or 18 ring atoms and at least one aromatic ring; and
anellated bi- and tricyclic partially hydrogenated heterocyclic ring systems with 8 to 16, 17 or 18 ring atoms and at least one aromatic ring, wherein one to three ring atoms optionally are selected from N, S and O;
**G**^{**11**} is selected from
-**NR**^{**17(i)**}**-(CH**_{**2**}**)**_{**r-**}**(CR**^{**13(i)**}**R**^{**19**}**)**_{**s**}**-R**^{**18**} **(G**^{**11a**}**),** 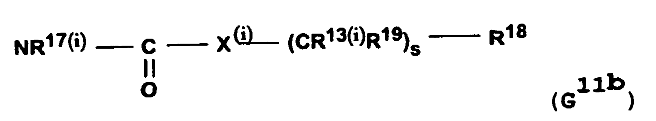
and 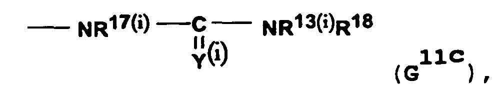 wherein **r, s, X**^{**(i)**}**, Y**^{**(i)**}**,** the substituents **R**^{**13(i)**}**, R**^{**17(i)**}**, R**^{**18**} and **R**^{**19**} and the group -**R**^{**13(i)**}**R**^{**18**} have the above meanings;
**G**^{**12**} is 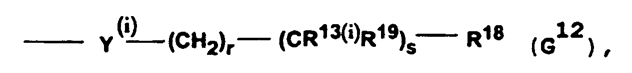 wherein **r, s, Y**^{**(i)**} and the substituents **R**^{**13(i)**}**, R**^{**18**} and **R**^{**19**} have the above meanings;
**G**^{**13**} is selected from 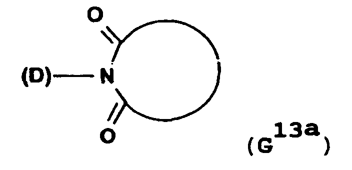 and 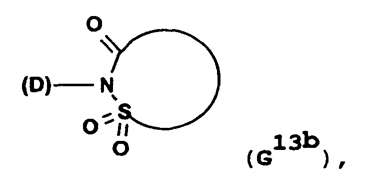 bound to D over the imide nitrogen atom, selected from
saturated and unsaturated monocyclic imides with 5 to 7 ring atoms, which, aside from the essential imide nitrogen atom, optionally contains one or two further hetero-atoms selected from N, S and O;
saturated, unsaturated and aromatic anellated, bi-, tri- or tetracyclic imides with 8 to 18 ring atoms, which, aside from the essential imide nitrogen atom, optionally contain one or two further hetero-atoms selected from N, S and O;
saturated and unsaturated, bridged bi-, tri-, tetra- or pentacyclic imides with 8 to 22 ring-atoms, which, aside from the essential imide nitrogen atom, optionally contain one or two further hetero-atoms selected from N, S and O; and
saturated and unsaturated spirocyclic imides, optionally anellated one or two-fold, and with a total of 9 to 23 ring atoms, which, aside from the essential imide nitrogen atom, optionally contain one or two further hetero-atoms selected from N, S and O,
wherein these cyclic imides optionally are substituted by one to five of the same or different groups selected independently from each other from
halogen, cyano, C₁-C₆-alkyl, C₁-C₆-alkylidene, trifluoromethyl, C₃-C₈-cycloalkyl, C₃-C₈-cycloalkylidene, phenyl-C₁-C₃-alkyl, phenyl-C₁-C₃-alkylidene, diphenyl-C₁-C₃-alkyl, diphenyl-C₁-C₃-alkylidene, triphenylmethyl, phenyl, hydroxy, C₁-C₆-hydroxyalkyl, C₁-C₆-alkoxy, C₁-C₆-alkoxy entirely or partially substituted by fluorine, benzyloxy, phenoxy, naphthyloxy, mercapto, C₁-C₆-alkylthio, phenylthio, naphthylthio, pyridylthio, C₁-C₆-alkanesulfonyl, phenylsulfonyl, naphthylsulfonyl, pyridylsulfonyl, sulfo, carboxy, C₂-C₇-carboxyalkyl, C₃-C₇-carboxyalkenyl, C₂-C₇-alkoxycarbonyl, benzyloxycarbonyl, nitro, amino, C₁-C₆-aminoalkyl, mono-C₁-C₆-alkylamino di(C₁-C₆-alkyl)amino, phenylamino, phenyl-C₁-C₃-alkylamino, pyridylamino,
saturated and unsaturated, four to seven-membered heterocycles which contain one or two hetero-atoms selected from N, S and O and are either bound directly or over a methylene group or a methine group,
monocyclic aromatic five and six-membered heterocycles which contain one to three hetero-atoms, selected from N, S and O and are either bound directly or over methylene group or a methine group,
anellated bicyclic, aromatic and partially hydrogenated carbocyclic ring systems with 8 to 12 ring atoms which are either bound directly over a methylene group or a methine group, and
anellated bicyclic aromatic and partially hydrogenated heterocyclic ring systems with 8 to 12 ring atoms, wherein one to three ring atoms are selected from N, S and O and are either bound directly or over a methylene group or a methine group,
wherein aromatic ring systems in the substituents **R**^{**1(i)**}**, R**^{**2(i)**}**, R**^{**4(i)**}**, R**^{**5(i)**}**, R**^{**6(i)**}**, R**^{**13(i)**}**, R**^{**14(i)**}**, R**^{**15(i)**}**, R**^{**16(i)**}**, R**^{**17(i)**}**, R**^{**18**}**, R**^{**19**}**, Ar**^{**1**} and **Ar**^{**2**}**,** in the groups **A**^{**(i)**} and **D**^{**(i)**}**,** in the ring systems **=CR**^{**13(i)**}**R**^{**15(i)**}**, =CR**^{**13(i)**}**R**^{**18**}**, -NR**^{**13(i)**}**R**^{**15(i)**} and **-NR**^{**13(i)**}**R**^{**18(i)**} as well as substituents and/or substituents in the cyclic imides **G**^{**13**} optionally are independently substituted by one to three of the same or different groups, selected from
halogen, cyano, C₁-C₆-alkyl, trifluoromethyl, C₃-C₈-cycloalkyl, benzyl, phenyl, hydroxy, C₁-C₆-hydroxyalkyl, C₁-C₆-alkoxy, C₁-C₆-alkoxy entirely or partially substituted by fluorine, benzyloxy, phenoxy, mercapto, C₁-C₆-alkylthio, phenylthio, sulfo, carboxy, C₂-C₇-carboxyalkyl, C₃-C₇-carboxyalkenyl, C₂-C₇-alkoxycarbonyl, benzyloxycarbonyl, nitro, amino, C₁-C₆-aminoalkyl, mono-C₁-C₆-alkylamino and di(C₁-C₆-alkyl)amino, and in the case of two adjacent residues on the aromatic ring, methylenedioxy,
wherein alkyl and cycloalkyl residues in the groups G optionally are substituted by one or two of the same or different groups, selected from
hydroxy, carboxy, C₂-C₇-alkoxycarbonyl, benzyloxycarbonyl, amino, mono-C₁-C₆-alkylamino and di(C₁-C₆-alkyl)amino;
and the stereoisomers or racemic or non-racemic mixtures of stereoisomers thereof,
and the tautomers thereof when **G** is a heterocyclic aromatic ring or an aromatic ring substituted by a hydroxy, mercapto or amino group,
and the pharmacologically acceptable acid addition salts thereof.

16. The use according to claim 15, wherein the compound of formula (I) is selected from the group consisting of:
N-[2-(1-benzylpiperidin-4-yl)-ethyl]-3-(pyridin-3-yl)-propionamide;
N-{2-[1-(2-phenylethyl)-piperidin-4-yl]-ethyl}-3-(pyridin-3-yl)-propionamide;
N-{2-[1-(4-phenylbutyl)-piperidin-4-yl]-ethyl}-3-(pyridin-3-yl)-propionamide;
N-{2-[1-(4-hydroxy-4-phenylbutyl)-piperidin-4-yl]-ethyl}-3-(pyridin-3-yl)-propionamide;
N-[2-(1-diphenylmethylpiperidin-4-yl)-ethyl]-3-(pyridin-3-yl)-propionamide;
N-[3-(1-diphenylmethylpiperidin-4-yl)-propyl]-3-(pyridin-3-yl)-propionamide;
N-[4-(1-diphenylmethylpiperidin-4-yl)-butyl]-3-(pyridin-3-yl)-propionamide:
N-[4-(1-benzylpiperidin-4-yl)-butyl]-3-(pyridin-3-yl)-acrylamide;
N-{4-[1-(2-phenylethyl)-piperidin-4-yl]-butyl}-3-(pyridin-3-yl)-acrylamide;
N-{4-[1-(4-biphenylylmethyl)-piperidin-4-yl]-butyl}-3-(pyridin-3-yl)-acrylamide;
N-{4-[1-(1-naphthylmethyl)-piperidin-4-yl]-butyl}-3-(pyridin-3-yl)-acrylamide;
N-{4-[1-(9-anthrylmethyl)-piperidin-4-yl]-butyl}-3-(pyridin-3-yl)-acrylamide;
N-{4-[1-(cyclohexylphenylmethyl)-piperidin-4-yl]-butyl}-3-(pyridin-3-yl)-acrylamide;
N-{4-[1-(10,11-dihydro-5H-dibenzo[a,d]cyclohepten-5-yl)-piperidin-4-yl]-butyl}-3-(pyridin-3-yl)-acrylamide;
N-[2-(1-diphenylmethylpiperidin-4-yl)-ethyl]-3-(pyridin-3-yl)-acrylamide;
N-[3-(1-diphenylmethylpiperidin-4-yl)-propyl]-3-(pyridin-3-yl)-acrylamide;
N-[5-(1-diphenylmethylpiperidin-4-yl)-pentyl]-3-(pyridin-3-yl)-acrylamide;
N-[6-(1-diphenylmethylpiperidin-4-yl)-hexyl]-3-(pyridin-3-yl)-acrylamide;
N-[4-(1-diphenylmethylpiperidin-9-yl)-butyl]-5-(pyridin-3-yl)-2,4-pentadienic acid amide;
N-(4-{1-[bis-(4-fluorophenyl)-methyl]-piperidin-4-yl}-butyl)-3-(pyridin-3-yl)-acrylamide;
N-(4-{1-[bis-(2-chlorophenyl)-methyl]-piperidin-4-yl}-butyl)-3-(pyridin-3-yl)-acrylamide:
N-[4-(1-diphenylmethylpiperidin -4-yl)-butyl]-3-(2-fluoro-pyridin-3-yl)-acrylamide;
N-[4-(1-diphenylmethylpiperidin-4-yl)-butyl]-3-(6-fluoro-pyridin-3-yl)-acrylamide;
N-[4-(1-diphenylmethylpiperidin-4-yl)-butyl]-3-(pyridin-3-yl)-acrylamide;
N-[4-(1-diphenylmethylpiperidin-4-yl)-butyl]-3-(pyridin-3-yl)-acrylamide dihydrochloride;
N-[4-(1-diphenylmethylpiperidin-4-yl)-butyl]-3-(pyridin-3-yl)-acrylamide methanesulfonate;
N-[4-(1-acetyl-piperidin-4-yl)-butyl]-3-(pyridin-3-yl)-propionamide;
N-[4-(1-benzoyl-piperidin-4-yl)-butyl]-3-(pyridin-3-yl)-propionamide;
N-[4-(1-diphenylacetyl-piperidin-4-yl)-butyl]-3-(pyridin-3-yl)-propionamide;
N-{4-[1-(9-oxo-9H-fluoren-4-carbonyl)-piperidin-4-yl]-butyl}-3-(pyridin-3-yl)-propionamide;
N-[4-(1-methylsulfonyl-piperidin-4-yl)-butyl]-3-(pyridin-3-yl)-propionamide;
N-{4-[1-(2-naphthyl-sulfonyl)-piperidin-4-yl]-butyl}-3-(pyridin-3-yl)-propionamide;
N-[4-(1-benzyl-piperidin-4-yl)-butyl]-3-(pyridin-3-yl)-propionamide;
N-(4-{1-[bis-(2-chlorophenyl)-methyl]-piperidin-4-yl}-butyl)-3-(pyridin-3-yl)-propionamide;
N-{4-[1-(phenylpyridin-3-yl-methyl)-piperidin-4-yl]-butyl}-3-(pyridin-3-yl)-propionamide;
N-{4-[1-(9H-fluoren-9-yl)-piperidin-4-yl]-butyl}-3-(pyridin-3-yl)-propionamide;
N-{4-[1-(6,11-dihydrodibenzo[b,e]oxepin-11-yl)-piperidin-4-yl]-butyl}-3-(pyridin-3-yl)-propionamide;
N-{4-[1-(1-naphthylamino-carbonyl)-piperidin-4-yl]-butyl}-3-(pyridin-3-yl)-propionamide;
N-[4-(1-diphenylamino-carbonyl-piperidin-4-yl)-butyl]-3-(pyridin-3-yl)-propionamide;
N-{4-[1-(10,11-dihydro-dibenzo[b,f]azepin-5-yl-carbonyl)-piperidin-4-yl]-butyl}-3-(pyridin-3-yl)-propionamide;
N-[4-(1-diphenylphosphinoyl-piperidin-4-yl)-butyl]-3-(pyridin-3-yl)-propionamide;
N-[4-(1-diphenylmethylpiperidin-4-yl)-butyl]-3-(2-fluoro-pyridin-3-yl)-propionamide;
N-[4-(1-diphenylmethylpiperidin-4-yl)-butyl]-3-(5-fluoro-pyridin-3-yl)-propionamide;
N-[4-(1-diphenylmethylpiperidin-4-yl)-butyl]-2-fluoro-3-(pyridin-3-yl)-propionamide;
N-[4-(1-diphenylmethylpiperidin-4-yl)-butyl]-2,2-difluoro-3-(pyridin-3-yl)-propionamide;
N-[5-(1-diphenylmethylpiperidin-4-yl)-pentyl]-3-(pyridin-3-yl)-propionamide;
N-[6-(1-diphenylmethylpiperidin-4-yl)-hexyl]-3-(pyridin-3-yl)-propionamide;
N-[2-(1-diphenylmethylpiperidin-4-yl)-ethyl]-5-(pyridin-3-yl)-pentanoic acid amide;
N-[4-(1-diphenylmethylpiperidin-4-yl)-butyl]-5-(pyridin-3-yl)-pentanoic acid amide;
N-[4-(1-diphenylmethylpiperidin-4-yl)-butyl]-N-hydroxy-3-(pyridin-3-yl)-propionamide;
N-[4-(1-diphenylmethylpiperidin-4-yl)-butyl]-2-hydroxy-3-(pyridin-3-yl)-propionamide;
N-[4-(1-diphenylmethylpiperidin-4-yl)-butyl]-3-hydroxy-3-(pyridin-3-yl)-propionamide;
N-[4-(1-diphenylmethylpiperidin-4-yl)-butyl]-3-(pyridin-3-yl)-propionamide;
N-[4-(1-methylsulfonylpiperidin-4-yl)-butyl]-3-(pyridin-3-yl)-acrylamide;
N-{4-[1-(2-naphthylsulfonyl)-piperidin-4-yl]-butyl}-3-(pyridin-3-yl)-acrylamide;
N-{4-[1-(2-naphthylsulfonyl)-piperidin-4-yl]-butyl}-5-(pyridin-3-yl)-2,4-pentadienic acid amide;
N-{4-[1-(1-naphthylaminocarbonyl)-piperidin-4-yl]-butyl}-3-(pyridin-3-yl)-acrylamide;
N-[4-(1-diphenylaminocarbonylpiperidin-4-yl)-butyl]-3-(pyridin-3-yl)-acrylamide;
N-[4-(1-diphenylaminocarbonyl-piperidin-4-yl)-butyl]-5-(pyridin-3-yl)-2,4-pentadienic acid amide;
N-{4-[1-(10,11-dihydrodibenzo[b,f]azepin-5-yl-carbonyl)-piperidin-4-yl]-butyl}-3-(pyridin-3-yl)-acrylamide;
N-[4-(1-diphenylphosphinoyl-piperidin-4-yl)-butyl]-3-(pyridin-3-yl)-acrylamide;
N-[4-(1-acetylpiperidin-4-yl)-butyl]-3-(pyridin-3-yl)-acrylamide;
N-[4-(1-diphenylacetylpiperidin-4-yl)-butyl]-3-(pyridin-3-yl)-acrylamide;
N-{4-[1-(3,3-diphenylpropionyl)-piperidin-4-yl]-butyl}-3-(pyridin-3-yl)-acrylamide;
N-[4-(1-benzoylpiperidin-4-yl)-butyl]-3-(pyridin-3-yl)-acrylamide;
N-[4-(1-benzoylpiperidin-4-yl)-butyl]-5-(pyridin-3-yl)-2,4-pentadienic acid amide;
N-{4-[1-(9-oxo-9H-fluoren-4-yl-carbonyl)-piperidin-4-yl]-butyl}-3-(pyridin-3-yl)-acrylamide;
N-{4-[1-(phenylpyridin-3-yl-methyl)-piperidin-4-yl]-butyl}-3-(pyridin-3-yl)-acrylamide;
N-{4-[1-(phenylpyridin-4-yl-methyl)-piperidin-4-yl]-butyl}-3-(pyridin-3-yl)-acrylamide;
N-{4-[1-(6,11-dihydrodibenzo[b,e]oxepin-11-yl)-piperidin-4-yl]-butyl}-3-(pyridin-3-yl)-acrylamide;
N-{4-[1-(6,11-dihydrodibenzo[b,e]thiepin-11-yl)-piperidin-4-yl]-butyl}-3-(pyridin-3-yl)-acrylamide;
N-[7-(1-diphenylmethylpiperidin-4-yl)-heptyl]-3-(pyridin-3-yl)-acrylamide;
N-[8-(1-diphenylmethylpiperidin-4-yl)-octyl]-3-(pyridin-3-yl)-acrylamide;
N-[3-(1-diphenylmethylpiperidin-4-yloxy)-propyl]-3-(pyridin-3-yl)-acrylamide;
N-[3-(1-benzylpiperidin-4-yloxy)-propyl]-3-(pyridin-3-yl)-acrylamide;
N-[2-(1-diphenylmethylpiperidin-4-yl)-ethyl]-5-(pyridin-3-yl)-2,4-pentadienic acid amide;
N-[4-(1-diphenylmethylpiperidin-4-yl)-butyl]-5-(pyridin-3-yl)-2,4-pentadienic acid amide;
N-[5-(1-diphenylmethylpiperidin-4-yl)-pentyl]-5-(pyridin-3-yl)-2,4- pentadienic acid amide;
N-[6-(1-diphenylmethylpiperidin-4-yl)-hexyl]-5-(pyridin-3-yl)-2,4- pentadienic acid amide;
N-[4-(4-diphenylmethylpiperazin-1-yl)-3-hydroxy-butyl]-3-pyridin-3-yl-acrylamide;
N-[3-(4-diphenylmethylpiperazin-1-yl)-propoxy]-3-pyridin-3-yl-acrylamide;
N-[4-(4-diphenylmethylpiperazin-1-yl)-4-oxo-butyl]-3-pyridin-3-yl-acrylamide;
N-[3-(4-diphenylmethylpiperazin-1-sulfonyl)-propyl]-3-pyridin-3-yl-acrylamide;
N-{2-[2-(4-diphenylmethylpiperazin-1-yl)-ethoxy]-ethyl}-3-pyridin-3-yl-acrylamide;
N-(4-{4-[bis-(4-fluorophenyl)-methyl]-piperazin-1-yl}-but-2-inyl)-3-pyridin-3-yl-acrylamide;
N-{4-[4-(4-carboxyphenyl-phenylmethyl)-piperazin-1-yl]-butyl}-3-pyridin-3-yl-acrylamide;
N-(4-{4-[(4-aminophenyl)-phenylmethyl]-piperazin-1-yl}-butyl)-3-pyridin-3-yl-acrylamide;
N-{4-[4-(9H-fluoren-9-yl)-piperazin-1-yl]-butyl}-2-(pyridin-3-yloxy)-acetamide;
N-{5-[4-(9H-fluoren-9-yl)-piperazin-1-yl]-pentyl}-3-pyridin-3-yl-acrylamide;
N-{6-[4-(9H-fluoren-9-yl)-piperazin-1-yl]-hexyl}-3-pyridin-3-yl-acrylamide;
3-pyridin-3-yl-N-{4-[4-(1,2,3,4-tetrahydronaphthalen-1-yl)-piperazin-1-yl]-butyl}-acrylamide;
3-pyridin-3-yl-N-{4-[4-(5,6,7,8-tetrahydronaphthalen-1-yl)-piperazin-1-yl]-butyl}-acrylamide;
N-{4-[4-(naphthalen-1-yl)-piperazin-1-yl]-butyl}-3-pyridin-3-yl-acrylamide;
N-[4-(4-biphenyl-2-yl-piperazin-1-yl)-butyl]-3-pyridin-3-yl-propionamide;
N-[5-(4-biphenyl-2-yl-piperazin-1-yl)-pentyl]-3-pyridin-3-yl-acrylamide;
N-[6-(4-biphenyl-2-yl-piperazin-1-yl)-hexyl]-3-pyridin-3-yl-acrylamide;
N-[4-(4-biphenyl-2-yl-piperazin-1-yl)-butyl]-2-(pyridin-3-yloxy)-acetamide;
N-[4-(4-biphenyl-2-yl-piperazin-1-yl)-butyl]-5-(pyridin-3-yl)-penta-2,4-dienic acid amide;
N-{4-[4-(10,11-dihydro-5H-dibenzo[a,d]cyclohepten-5-yl)-piperazin-1-yl]-butyl}-3-pyridin-3-yl-propionamide;
N-{5-[4-(10,11-dihydro-5H-dibenzo[a,d]cyclohepten-5-yl)-piperazin-1-yl]-pentyl}-3-pyridin-3-yl-acrylamide;
N-{6-[4-(10,11-dihydro-5H-dibenzo[a,d]cyclohepten-5-yl)-piperazin-1-yl]-hexyl}-3-pyridin-3-yl-acrylamide;
N-{4-[4-(10,11-dihydro-5H-dibenzo[a,d]cyclohepten-5-yl)-piperazin-1-yl]-butyl}-5-(pyridin-3-yl)-penta-2,4-dienic acid amide;
N-{4-[4-(6,11-dihydro-dibenzo[b,e]oxepin-11-yl)-piperazin-1-yl]-butyl-3-pyridin-3-yl-propionamide;
N-{2-[4-(6,11-dihydro-dibenzo[b,e]thiepin-11-yl)-piperazin-1-yl]-ethyl}-3-pyridin-3-yl-acrylamide;
N-[4-(4-diphenylacetylpiperazin-1-yl)-butyl]-3-pyridin-3-yl-acrylamide;
N-[4-(4-benzoylpiperazin-1-yl)-butyl]-3-pyridin-3-yl-acrylamide;
N-{4-[4-(2-aminobenzoyl)-piperazin-1-yl]-butyl}-3-pyridin-3-yl-acrylamide;
N-{4-[4-(4-carboxybenzoyl)-piperazin-1-yl]-butyl}-3-pyridin-3-yl-acrylamide;
N-{4-[4-(biphenyl-2-carbonyl)-piperazin-1-yl]-butyl}-3-pyridin-3-yl-acrylamide;
N-{4-[4-(9-oxo-9H-fluoren-4-carbonyl)-piperazin-1-yl]-butyl}-3-pyridin-3-yl-acrylamide;
N-{4-[4-(furan-2-carbonyl)-piperazin-1-yl]-butyl}-3-pyridin-3-yl-acrylamide;
N-{4-[4-(naphthalen-1-yl-aminocarbonyl)-piperazin-1-yl]-butyl}-3-pyridin-3-yl-propionamide;
N-{4-[4-(diphenylaminocarbonyl)-piperazin-1-yl]-butyl}-3-pyridin-3-yl-acrylamide;
N-{4-[4-(naphthalen-2-sulfonyl)-piperazin-1-yl]-butyl}-3-pyridin-3-yl-acrylamide;
N-[4-(4-diphenylphosphinonyl-piperazin-1-yl)-butyl]-3-pyridin-3-yl-acrylamide;
N-[4-(4-biphenyl-2-yl-piperazin-1-yl)-butyl]-3-pyridin-3-yl-acrylamide;
N-{4-[4-(9H-fluoren-9-yl)-piperazin-1-yl]-butyl}-3-pyridin-3-yl-acrylamide;
N-{4-[4-(10,11-dihydro-5H-dibenzo[a,d]cyclohepten-5-yl)-piperazin-1-yl]-butyl}-3-pyridin-3-yl-acrylamide;
N-[4-(4-phenylpiperidin-1-yl)-butyl]-3-pyridin-3-yl-acrylamide;
N-{4-[4-(1H-indol-3-yl)-piperidin-1-yl]-butyl}-3-pyridin-3-yl-acrylamide;
N-{4-[4-(2-oxo-2,3-dihydrobenzimidazol-1-yl)-piperidin-1-yl]-butyl}-3-pyridin-3-yl-acrylamide;
N-[4-(4-benzotriazol-1-yl-piperidin-1-yl)-butyl]-3-pyridin-3-yl-acrylamide;
N-{4-[4-(hydroxy-diphenylmethyl)-piperidin-1-yl]-butyl}-2-(pyridin-3-yloxy)-acetamide;
N-[4-(4,4-diphenylpiperidin-1-yl)-butyl]-3-pyridin-3-yl-acrylamide;
N-{4-[4-(6,11-dihydrodibenzo[b,e]thiepin-11-yliden)-piperidin-1-yl]-butyl}-3-pyridin-3-ylpropionamide dihydrochloride semi-isopropanol;
N-{4-[4-(6,11-dihydrodibenzo[b,e]thiepin-11-yliden)-piperidin-1-yl]-butyl}-5-pyridin-3-yl-pentanamide;
N-{4-[4-(4,9-dihydro-thieno[2,3-b]-benzo[e]thiepin-4-yliden)-piperidin-1-yl]-butyl}-3-pyridin-3-yl-propionamide;
N-{4-[4-(4,9-dihydro-thieno[2,3-b]-benzo[e]thiepin-4-yliden)-piperidin-1-yl]-butyl}-3-pyridin-3-yl-acrylamide;
N-[4-(4-diphenylphosphinoyloxypiperidin-1-yl) butyl]-3-pyridin-3-yl-acrylamide;
N-[4-(1,4-dioxa-8-azaspiro[4.5]dec-8-yl)-butyl]-3-pyridin-3-yl-acrylamide;
N-[4-(2,5-dioxo-3,4-diphenyl-2,5-dihydropyrrol-1-yl)-butyl]-3-pyridin-3-yl-acrylamide;
N-[4-(2,6-diaxo-4-phenylpiperidin-1-yl)-butyl]-3-pyridin-3-yl-acrylamide;
N-[4-(1,3-dioxo-4,5,6,7-tetraphenyl-1,3-dihydro-isoindol-2-yl)-butyl]-3-pyridin-3-yl-acrylamide;
N-[4-(3-benzyl-2,4,5-trioxo-imidazolidin-1-yl)-butyl]-3-pyridin-3-yl-acrylamide;
N-[4-(1,3,10-trioxo-1,4,5,6,10,10a-hexahydro-acenaphtho-[1,8a-c]pyrrol-2-yl)-butyl]-3-pyridin-3-yl-acrylamide;
N-[4-(2,5-dioxo-4,4-diphenylimidazolidin-1-yl)-butyl-3-pyridin-3-yl-acrylamide;
N-[4-(2,5-dioxo-3-phenyl-2,5-dihydropyrrol-1-yl)-butyl]-3-pyridin-3-yl-acrylamide;
N-[3-(2,5-dioxo-3,4-diphenyl-2,5-dihydro-pyrrol-1-yl)-propyl]-3-pyridin-3-yl-acrylamide;
N-[4-(3-pyridin-3-yl-acroylamino)-butyl]-2,3:5,6-dibenzobicyclo[2.2.2]octan-7,8-dicarboximide;
N-[4-(5-benzyliden-2,4-dioxothiazolidin-3-yl)-butyl]-3-pyridin-3-yl-acrylamide;
N-[4-(4-benzyl-2,6-dioxopiperazin-1-yl)-butyl]-3-pyridin-3-yl-acrylamide;
N-[6-(2,5-dioxo-3,4-diphenyl-2,5-dihydropyrrol-1-yl)-hexyl]-3-pyridin-3-yl-acrylamide;
N-[4-(2,5-dioxo-3,4-diphenyl-2,5-dihydropyrrol-1-yl)-butyl]-3-pyridin-3-yl-propionamide;
N-[4-(1,3-dioxo-1,3-dihydro-isoindol-2-yl)-butyl]-3-pyridin-3-yl-acrylamide;
N-[4-(1,3-dioxo-1H,3H-benzo[de]isoquinolin-2-yl)-butyl]-3-(1-oxidopyridin-3-yl)-acrylamide;
N-[6-(1,3-dioxo-1H,3H-benzo[de]isoquinolin-2-yl)-hexyl]-3-pyridin-3-yl-acrylamide;
N-[2-(1,3-dioxo-1H,3H-benzo[de]isoquinolin-2-yl)-ethyl]-3-pyridin-3-yl-acrylamide;
N-[4-(1,3-dioxo-1H,3H-benzo[de]isoquinolin-2-yl)-butyl]-3-pyridin-3-yl-acrylamide;
N-[8,8-bis-(4-fluorophenyl)-octyl]-3-pyridin-3-yl-acrylamide hydrochloride;
N-[6-(3,3-diphenylureido)-hexyl]-3-pyridin-3-yl-acrylamide;
N-[4-(1-phenyl-1,2,4,5-tetrahydrobenzo[d]azepin-3-yl)-butyl]-3-pyridin-3-yl-acrylamide;
N-(8,8-diphenyloctyl)-3-pyridin-3-yl-acrylamide;
N-(8-hydroxy-8,8-diphenyloctyl)-3-pyridin-3-yl-acrylamide;
N-[4-(3,3-diphenylureido)-butyl]-3-pyridin-3-yl-acrylamide;
N-[4-(1H,3H-benzo[de]isoquinolin-2-yl)-butyl]-3-pyridin-3-yl-acrylamide;
N-[6-(10,11-dihydro-dibenzo[b,f]azepin-5-yl-carbonylamino)-hexyl]-3-pyridin-3-yl-acrylamide;
3-pyridin-3-yl-N-[6-(tosylamino)-hexyl]-acrylamide;
N-[4-(1,1-dioxo-1-thia-2-aza-acenaphthylen-2-yl)-butyl]-3-pyridin-3-yl-acrylamide;
N-(6-hydroxy-6,6-diphenylhexyl)-3-pyridin-3-yl-acrylamide;
N-(6,6-diphenyl-hex-5-enyl)-3-pyridin-3-yl-acrylamide;
N-[4-(4,5-diphenylimidazol-1-yl)-butyl]-3-pyridin-3-yl-acrylamide;
N-[4-(trans-2-phenylcyclopropylcarbonylamino)butyl]-3-pyridin-3-yl-acrylamide;
N-(5-hydroxy-5,5-diphenyl-pentyl)-3-pyridin-3-yl-acrylamide;
N-(7-phenylheptyl)-3-pyridin-3-yl-acrylamide;
N-(4-diphenylacetylamino-butyl)-3-pyridin-3-yl-acrylamide;
N-[4-(benzhydrylamino)-butyl]-3-pyridin-3-yl-acrylamide; and
N-(4-{[2-(benzhydrylmethylamino)-ethyl]-methylamino}-butyl)-3-pyridin-3-yl-acrylamide.

17. The use according to claim 15 or 16, wherein additionally to the prophylactically or therapeutically administered cancerostatic or immunosuppressive agent according to formula (I) a further cancerostatic or immunosuppressive agent different therefrom is administered.

## Patentansprüche

1. Pharmazeutische Zusammensetzung, umfassend:
(a) mindestens ein cancerostatisches oder immunsuppressives Mittel, ausgewählt aus der Gruppe, bestehend aus Verbindungen der Formel (I): 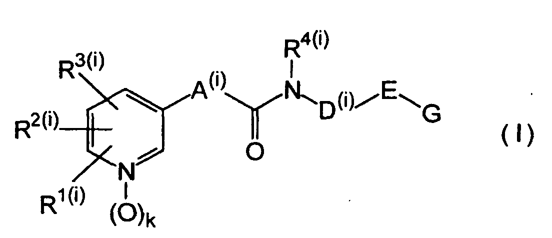 worin:
R¹⁽ⁱ⁾ ausgewählt ist aus Wasserstoff, Halogen, Cyano, C₁₋₆-Alkyl, C₃₋₆-Alkenyl, C₂₋₆-Alkinyl, Trifluormethyl, Hydroxy, C₃₋₈-Cycloalkyl, C₁₋₆-Hydroxyalkyl, C₁₋₆-Alkoxy, C₃₋₆-Alkenyloxy, C₃₋₆-Alkinyloxy, Benzyloxy, C₁₋₇-Alkanoyloxy, C₁₋₇-Alkoxycarbonyloxy, C₁₋₆-Alkylthio, C₃₋₆-Alkenylthio, C₃₋₆-Alkinylthio, C₃₋₈-Cycloalkyloxy, C₃₋₈-Cycloalkylthio, C₂₋₇-Alkoxycarbonyl, Aminocarbonyl, C₂₋₇-Alkylaminocarbonyl, C₃₋₁₃-Dialkylaminocarbonyl, Carboxy, Phenyl, Phenoxy, Phenylthio, Pyridyloxy, Pyridylthio und NR⁵⁽ⁱ⁾R⁶⁽ⁱ⁾, worin
R⁵⁽ⁱ⁾ und R⁶⁽ⁱ⁾ unabhängig voneinander ausgewählt sind aus Wasserstoff, C₁₋₆-Alkyl, C₃₋₆-Alkenyl, C₃₋₆-Alkinyl, Benzyl und Phenyl;
R²⁽ⁱ⁾ ausgewählt ist aus Wasserstoff, Halogen, Cyano, C₁₋₆-Alkyl, Trifluormethyl, Hydroxy, C₁₋₆-Alkoxy, Benzyl und C₁₋₆-Alkanoyloxy; oder
R¹⁽ⁱ⁾ und R²⁽ⁱ⁾, wenn sie benachbart sind, gegebenenfalls eine Brücke bilden, ausgewählt aus -(CH₂)₄-, -(CH=CH)₂- und
-CH₂O-CR⁷⁽ⁱ⁾R⁸⁽ⁱ⁾-O-, worin
R⁷⁽ⁱ⁾ und R⁸⁽ⁱ⁾ unabhängig voneinander ausgewählt sind aus Wasserstoff und C₁₋₆-Alkyl;
R³⁽ⁱ⁾ ausgewählt ist aus Wasserstoff, Halogen, C₁₋₆-Alkyl, Trifluormethyl und C₁₋₆-Hydroxyalkyl;
R⁴⁽ⁱ⁾ ausgewählt ist aus Wasserstoff, Hydroxy, C₁₋₆-Alkyl, C₃₋₆-Alkenyl, C₃₋₆-Alkinyl, C₃₋₆-Cycloalkyl, C₁₋₆-Alkoxy und Benzyloxy;
k 0 oder 1 ist,
A⁽ⁱ⁾ ausgewählt ist aus
C₁₋₆-Alkylen, gegebenenfalls ein- bis dreimal substituiert durch C₁₋₆-Alkyl, C₁₋₃-Alkoxy, Hydroxy, Fluor oder Phenyl,
C₂₋₆-Alkylen, worin eine Methyleneinheit isosterisch ersetzt ist durch O, S, NR⁹⁽ⁱ⁾,
CO, SO oder SO₂, worin, mit Ausnahme von CO, die isosterische Substitution nicht zur Aminogruppe benachbart ist und R⁹⁽ⁱ⁾ ausgewählt ist aus Wasserstoff, C₁₋₆-Alkyl, C₃₋₆-Alkenyl, C₃₋₆-Alkinyl, C₃₋₆-Acyl und C₁₋₆-Alkansulfonyl,
1,2-Cyclopropylen,
C₂₋₆-Alkenylen, gegebenenfalls ein- bis dreimal substituiert durch C₁₋₆-Alkyl, Hydroxy, C₁₋₃-Alkoxy, Fluor, Cyano oder Phenyl,
C₄₋₆-Alkadienylen, gegebenenfalls ein- oder zweimal substituiert durch C₁₋₃-Alkyl, Fluor, Cyano oder Phenyl,
1,3,5-Hexatrienylen, gegebenenfalls substituiert durch C₁₋₆-Alkyl, Fluor, Cyano oder Phenyl, und
Ethinylen;
D⁽ⁱ⁾ ausgewählt ist aus
C₁₋₁₂-Alkylen, gegebenenfalls ein- oder zweimal substituiert durch C₁₋₆-Alkyl, Hydroxy, C₁₋₆-Alkoxy oder Phenyl,
C₂₋₁₂-Alkenylen oder C₄₋₁₂-Alkadienylen, gegebenenfalls ein- oder zweimal substituiert durch C₁₋₆-Alkyl, Hydroxy, C₁₋₆-Alkoxy oder Phenyl, worin eine Doppelbindung gegebenenfalls benachbart zu Ring E sein kann,
für den Fall, dass Ring E über ein C-Atom verknüpft ist,
C₃₋₁₂-Alkinylen oder C₄₋₁₂-Alkeninylen, gegebenenfalls ein- oder zweimal substituiert durch C₁₋₆-Alkyl, Hydroxy, C₁₋₆-Alkoxy oder Phenyl, und
C₁₋₁₂-Alkylen, C₂₋₁₂-Alkenylen oder C₃₋₁₂-Alkinylen, worin ein bis drei Methyleneinheiten, mit Ausnahme der (G)-terminalen Methylengruppe, im Fall, dass E eine Bindung darstellt, isosterisch ersetzt sind durch O, S, NR¹⁰⁽ⁱ⁾, CO, SO oder SO₂, worin R¹⁰⁽ⁱ⁾ die gleiche Bedeutung wie R⁹⁽ⁱ⁾ hat, aber unabhängig davon ausgewählt ist;
E ausgewählt ist aus E¹⁽ⁱ⁾, E²⁽ⁱ⁾, E³, E⁴, E⁵ und E⁶, worin
E¹⁽ⁱ⁾ 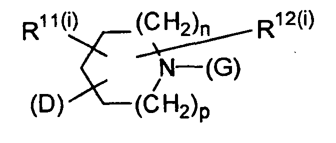 ist,
E²⁽ⁱ⁾ 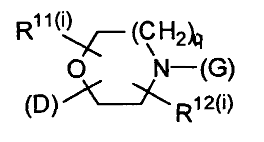 ist,
E³ 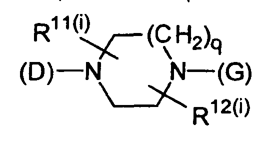 ist,
E⁴ 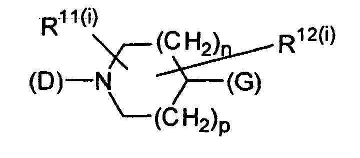 ist,
E⁵ 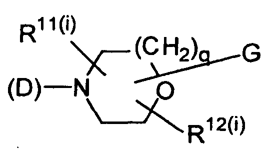 ist, und
E⁶ eine Einfach- oder Doppelbindung darstellt,
worin die heterocyclischen Ringe E¹⁽ⁱ⁾ bis E⁵ gegebenenfalls eine Doppelbindung aufweisen,
n und p unabhängig voneinander 0, 1, 2 oder 3 sind, mit dem Proviso, dass n + p ≤ 4,
q 1, 2 oder 3 ist;
R¹¹⁽ⁱ⁾ ausgewählt ist aus Wasserstoff, C₁₋₆-Alkyl, Hydroxy, Hydroxymethyl, Carboxy und C₂₋₇-Alkoxycarbonyl,
R¹²⁽ⁱ⁾ ausgewählt ist aus Wasserstoff, C₁₋₆-Alkyl und einer zu einem Stickstoffatom benachbarten Oxogruppe, oder
R¹¹⁽ⁱ⁾ und R¹²⁽ⁱ⁾ gegebenenfalls zusammen eine C₁₋₃-Alkylenbrücke unter Bildung eines bicyclischen Ringsystems bilden, und
(a) im Fall, dass E E¹⁽ⁱ⁾, E²⁽ⁱ⁾ oder E³ darstellt, der Substituent G gegebenenfalls ausgewählt ist aus G¹⁽ⁱ⁾, G²⁽ⁱ⁾, G³⁽ⁱ⁾, G⁴⁽ⁱ⁾ und G⁵⁽ⁱ⁾, worin
G¹⁽ⁱ⁾ 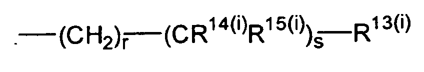 ist, worin
r 0 bis 3 ist und
s 0 oder 1 ist,
R¹³⁽ⁱ⁾ ausgewählt ist aus
Wasserstoff, C₁₋₆-Alkyl, C₃₋₆-Alkenyl, C₃₋₆-Alkinyl und C₃₋₈-Cycloalkyl;
gesättigten oder ungesättigten, 4- bis 7-gliedrigen Heterocyclen, die ein oder zwei Heteroatome, ausgewählt aus N, S und O, enthalten;
Benzyl, Phenyl;
monocyclischen, aromatischen, 5- oder 6-gliedrigen Heterocyclen, die ein bis drei Heteroatome, ausgewählt aus N, S und O, enthalten, und entweder direkt oder über eine Methylengruppe gebunden sind;
kondensierten, bi- und tricyclischen, aromatischen oder teilweise hydrierten, carbocyclischen Ringsystemen mit 8 bis 16, 17 oder 18 Ringatomen und mindestens einem aromatischen Ring, worin die Verknüpfung entweder über einen aromatischen oder einen hydrierten Ring und entweder direkt oder über eine Methylengruppe geschieht;
kondensierten, bi- und tricyclischen, aromatischen oder teilweise hydrierten, heterocyclischen Ringsystem mit 8 bis 16, 17 oder 18 Ringatomen, und mindestens einem aromatischen Ring, worin ein bis drei Ringatome ausgewählt sind aus N, S und O, und die Verknüpfung entweder über einen aromatischen oder einen hydrierten Ring und entweder direkt oder über eine Methylengruppe geschieht;
R¹⁴⁽ⁱ⁾ die gleiche Bedeutung wie R¹³⁽ⁱ⁾ aufweist, aber unabhängig davon ausgewählt ist;
R¹⁵⁽ⁱ⁾ ausgewählt ist aus
Wasserstoff, Hydroxy, Methyl, Benzyl, Phenyl,
monocyclischen, aromatischen, 5- oder 6-gliedrigen Heterocyclen, die ein bis drei Heteroatome, ausgewählt aus N, S und O, enthalten und entweder direkt oder über eine Methylengruppe gebunden sind;
kondensierten, bi- und tricyclischen, aromatischen oder teilweise hydrierten, carbocyclischen Ringsystemen mit 8 bis 16, 17 oder 18 Ringatomen und mindestens einem aromatischen Ring, worin die Verknüpfung entweder über einen aromatischen oder einen hydrierten Ring und entweder direkt oder über eine Methylengruppe geschieht;
kondensierten, bi- und tricyclischen, aromatischen oder teilweise hydrierten, heterocyclischen Ringsystemen mit 8 bis 16, 17 oder 18 Ringatomen und mindestens einem aromatischen Ring, worin ein bis drei Ringatome ausgewählt sind aus N, S und O, und die Verknüpfung entweder über einen aromatischen oder einen hydrierten Ring, und entweder direkt oder über eine Methylengruppe geschieht;
G²⁽ⁱ⁾ ausgewählt ist aus 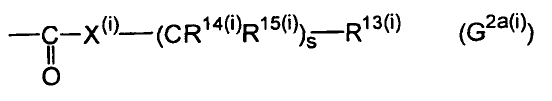 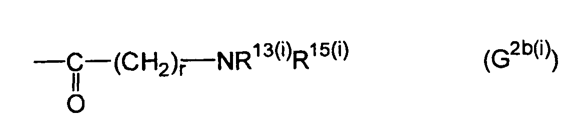 worin r, s und die Substituenten R¹³⁽ⁱ⁾, R¹⁴⁽ⁱ⁾ und R¹⁵⁽ⁱ⁾ die obigen Bedeutungen aufweisen,
oder die Gruppe
-NR¹³⁽ⁱ⁾R¹⁵⁽ⁱ⁾
ein über das Stickstoffatom gebundener stickstoffhaltiger Heterocyclus ist, wobei der stickstoffhaltige Heterocyclus ausgewählt ist aus
gesättigten und ungesättigten, monocyclischen, 4- bis 8-gliedrigen Heterocyclen, die neben dem essentiellen Stickstoffatom gegebenenfalls ein oder zwei weitere Heteroatome, ausgewählt aus N, S und O, enthalten,
und
gesättigten und ungesättigten, bi- oder tricyclischen, kondensierten oder überbrückten Heterocyclen mit 8 bis 16, 17 oder 18 Ringatomen, die neben dem essentiellen Stickstoffatom gegebenenfalls ein oder zwei weitere Heteroatome, ausgewählt aus N, S und O, enthalten,
X⁽ⁱ⁾ ausgewählt ist aus Methylen, Ethylen, Ethenylen, Propylen und C₃₋₇-Cycloalkylen oder eine Bindung darstellt;
G³⁽ⁱ⁾ 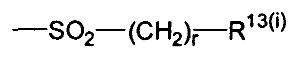 ist, worin r und R¹³⁽ⁱ⁾ die obigen Bedeutungen aufweisen,
G⁴⁽ⁱ⁾ 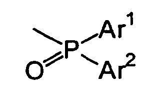 ist, worin Ar¹ und Ar² unabhängig voneinander ausgewählt sind aus Phenyl, Pyridyl und Naphthyl;
G⁵⁽ⁱ⁾
-COR¹⁶⁽ⁱ⁾
ist, worin
R¹⁶⁽ⁱ⁾ ausgewählt ist aus Trifluormethyl, C₁₋₆-Alkoxy, C₃₋₆-Alkenyloxy und Benzyloxy,
(b) im Fall, dass E E⁴ oder E⁵ ist, dann G gegebenenfalls G¹⁽ⁱ⁾, G²⁽ⁱ⁾, G⁶⁽ⁱ⁾, G⁷ oder G⁸ ist,
worin G¹⁽ⁱ⁾ und G²⁽ⁱ⁾ die obigen Bedeutungen aufweisen und
G⁶⁽ⁱ⁾
=(C)ᵤR¹³⁽ⁱ⁾R¹⁵⁽ⁱ⁾
ist, worin R¹³⁽ⁱ⁾ und R¹⁵⁽ⁱ⁾ die obigen Bedeutungen aufweisen, und
u 0 oder 1 ist,
oder, wenn u = 1, dann bilden R¹³⁽ⁱ⁾ und R¹⁵⁽ⁱ⁾ zusammen mit dem Kohlenstoffatom, an
das sie gebunden sind, ein Ringsystem, ausgewählt aus
C₃₋₈-Cycloalkyl,
gesättigten, 4- bis 7-gliedrigen Heterocyclen, die gegebenenfalls ein oder zwei Heteroatome, ausgewählt aus N, S und O, enthalten;
kondensierten, bi- und tricyclischen, teilweise hydrierten, carbocyclischen Ringsystemen mit 8 bis 16, 17 oder 18 Ringatomen und mindestens einem aromatischen Ring;
kondensierten, bi- und tricyclischen, teilweise hydrierten, heterocyclischen Ringsystemen mit 8 bis 16, 17 oder 18 Ringatomen und mindestens einem aromatischen Ring, worin ein bis drei Ringatome ausgewählt sind aus N, S und O;
oder, wenn u = 0, dann bilden R¹³⁽ⁱ⁾ und R¹⁵⁽ⁱ⁾ zusammen mit dem Kohlenstoffatom des Rings E, an das sie gebunden sind, ein Ringsystem, ausgewählt aus
C₃₋₈-Cycloalkyl,
gesättigten, 4- bis 7-gliedrigen Heterocyclen, die ein oder zwei Heteroatome, ausgewählt aus N, S und O, enthalten;
kondensierten, bi- und tricyclischen, teilweise hydrierten, carbocyclischen Ringsystemen mit 8 bis 16, 17 oder 18 Ringatomen und mindestens einem aromatischen Ring; und
kondensierten, bi- und tricyclischen, teilweise hydrierten, heterocyclischen Ringsystemen mit 8 bis 16, 17 oder 18 Ringatomen und mindestens einem aromatischen Ring, worin ein bis drei Ringatome ausgewählt sind aus N, S und O;
G⁷ ausgewählt ist aus 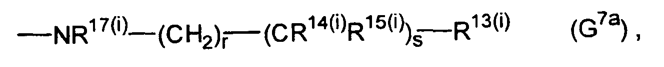 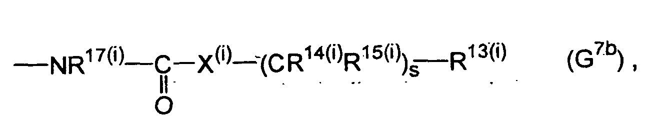 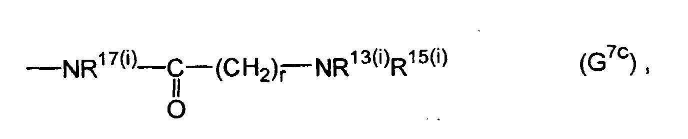 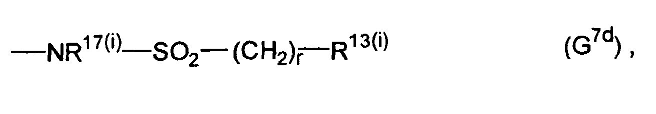 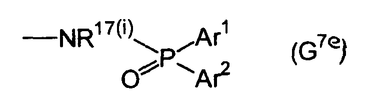 und
-**NR**^{**17(i)**}**-COR**^{**16(i)**} **(G**^{**7f**}**),**
worin r, s, X⁽ⁱ⁾, die Substituenten R¹³⁽ⁱ⁾, R¹⁴⁽ⁱ⁾, R¹⁵⁽ⁱ⁾ und R¹⁶⁽ⁱ⁾ und die Gruppe
**-NR**^{**13(i)**}**R**^{**15(i)**}
die obigen Bedeutungen aufweisen, und
R¹⁷⁽ⁱ⁾ die gleichen Bedeutungen wie R⁵⁽ⁱ⁾ aufweist, aber unabhängig davon ausgewählt ist,
Ar¹ und Ar² unabhängig voneinander ausgewählt sind aus Phenyl, Pyridyl und Naphthyl;
G⁸ ausgewählt ist aus 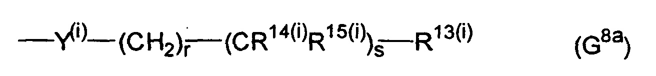 und 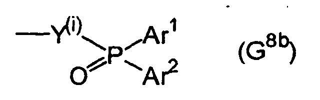 worin r, s und die Substituenten R¹³⁽ⁱ⁾, R¹⁴⁽ⁱ⁾, R¹⁵⁽ⁱ⁾, Ar¹ und Ar² die obigen Bedeutungen aufweisen, und
Y⁽ⁱ⁾ O oder S ist;
(c) im Fall, dass der Substituent E E⁶ ist, dann ist der Substituent G gegebenenfalls ausgewählt aus G^{7d}, G^{7e}, G^{8b}, G⁹, G¹⁰, G¹¹, G¹² und G¹³, worin G^{7d}, G^{7e} und G^{8b} die obigen Bedeutungen aufweisen, und
G⁹ ausgewählt ist aus 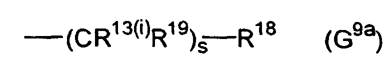 und
**-NR**^{**13(i)**}**R**^{**18**} **(G**^{**9b**}**)**
worin s und R¹³⁽ⁱ⁾ wie oben definiert sind, und
R¹⁸ ausgewählt ist aus
Benzyl, Diphenylmethyl, Phenyl;
monocyclischen, aromatischen, 5- und 6-gliedrigen Heterocyclen, die ein bis drei Heteroatome, ausgewählt aus N, S und O, enthalten können und entweder direkt oder über eine Methylengruppe gebunden sind;
kondensierten, bi- und tricyclischen, aromatischen oder teilweise hydrierten, carbocyclischen Ringsystemen mit 8 bis 16, 17 oder 18 Ringatomen und mindestens einem aromatischen Ring, worin die Verknüpfung entweder über einen aromatischen oder einen hydrierten Ring und entweder direkt oder über eine Methylengruppe geschieht; und
kondensierten, bi- und tricyclischen, aromatischen oder teilweise hydrierten, heterocyclischen Ringsystemen mit 8 bis 16, 17 oder 18 Ringatomen und mindestens einem aromatischen Ring, worin ein bis drei Ringatome ausgewählt sind aus N, S und O, und die Verknüpfung entweder über einen aromatischen oder einen hydrierten Ring und entweder direkt oder über eine Methylengruppe geschieht;
R¹⁹ die gleichen Bedeutungen wie R¹³⁽ⁱ⁾ aufweist, aber unabhängig davon ausgewählt ist, und zusätzlich Hydroxy sein kann;
oder die Gruppe
**-NR**^{**13(i)**}**R**^{**18**}
ist gegebenenfalls ein über das Stickstoffatom gebundener, stickstoffhaltiger Heterocyclus, wobei der stickstoffhaltige Heterocyclus ausgewählt ist aus
kondensierten, bi- und tricyclischen, aromatischen oder teilweise hydrierten, heterocyclischen Ringsystemen mit 8 bis 16, 17 oder 18 Ringatomen und mindestens einem aromatischen Ring, der neben dem essentiellen Stickstoffatom gegebenenfalls ein oder zwei weitere Heteroatome, ausgewählt aus N, S und O, enthält;
G¹⁰
**=CR**^{**13(i)**}**R**^{**18**} **(G**^{**10**}**)**
ist, das an D über eine Doppelbindung gebunden ist, worin R¹³⁽ⁱ⁾ und R¹⁸ die obigen Bedeutungen aufweisen oder worin G¹⁰ gegebenenfalls ein über das Kohlenstoffatom gebundenes Ringsystem ist, ausgewählt aus
kondensierten, bi- und tricyclischen, teilweise hydrierten, carbocyclischen Ringsystemen mit 8 bis 16, 17 oder 18
Ringatomen und mindestens einem aromatischen Ring; und
kondensierten, bi- und tricyclischen, teilweise hydrierten, heterocyclischen Ringsystemen mit 8 bis 16, 17 oder 18 Ringatomen und mindestens einem aromatischen Ring, worin ein bis drei Ringatome gegebenenfalls ausgewählt sind aus N, S und O;
G¹¹ ausgewählt ist aus 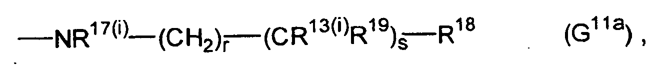 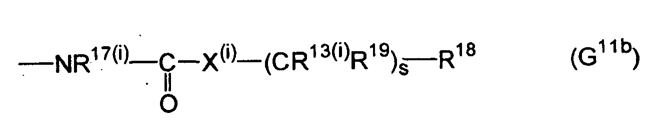 und 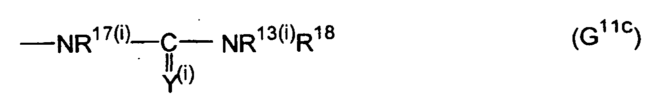 worin r, s, X⁽ⁱ⁾, Y⁽ⁱ⁾, die Substituenten R¹³⁽ⁱ⁾, R¹⁷⁽ⁱ⁾, R¹⁸ und R¹⁹ und die Gruppe -NR¹³⁽ⁱ⁾R¹⁸⁽ⁱ⁾ die obigen Bedeutungen aufweisen;
G¹² 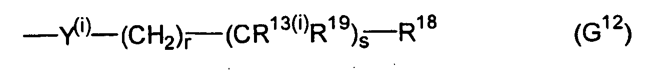 ist, worin r, s, Y⁽ⁱ⁾ und die Substituenten R¹³⁽ⁱ⁾, R¹⁸ und R¹⁹ die obigen Bedeutungen aufweisen;
G¹³ ausgewählt ist aus 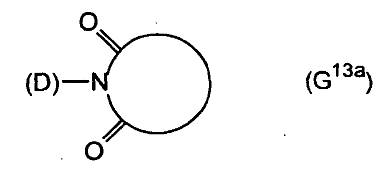 und 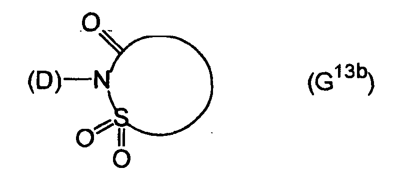 die an D über das Imidstickstoffatom gebunden sind, ausgewählt aus
gesättigten und ungesättigten monocyclischen Imiden mit 5 bis 7 Ringatomen, die, neben dem essentiellen Imidstickstoffatom, gegebenenfalls ein oder zwei weitere Heteroatome, ausgewählt aus N, S und O, enthalten;
gesättigten, ungesättigten und aromatischen, kondensierten, bi-, tri- oder tetracyclischen Imiden mit 8 bis 18 Ringatomen, die, neben dem essentiellen Imidstickstoffatom, gegebenenfalls ein oder zwei weitere Heteroatome, ausgewählt aus N, S und O, enthalten;
gesättigten und ungesättigten, überbrückten bi-, tri-, tetra- oder pentacyclischen Imiden mit 8 bis 22 Ringatomen, die, neben dem essentiellen Imidstickstoffatom, gegebenenfalls ein oder zwei weitere Heteroatome, ausgewählt aus N, S und O, enthalten; und
gesättigten und ungesättigten, spirocyclischen Imiden, gegebenenfalls ein- oder zweifach kondensiert und mit einer Gesamtzahl von 9 bis 23 Ringatomen, die, neben dem essentiellen Imidstickstoffatom, gegebenenfalls ein oder zwei weitere Heteroatome, ausgewählt aus N, S und O, enthalten,
worin diese cyclischen Imide gegebenenfalls durch ein bis fünf der gleichen oder unterschiedlichen Gruppen substituiert sind, unabhängig ausgewählt voneinander aus
Halogen, Cyano, C₁₋₆-Alkyl, C₁₋₆-Alkyliden, Trifluormethyl, C₃₋₈-Cycloalkyl, C₃₋₈-Cycloalkyliden, Phenyl-C₁₋₃-alkyl, Phenyl-C₁₋₃-alkyliden, Diphenyl-C₁₋₃-alkyl, Diphenyl-C₁₋₃-alkyliden, Triphenylmethyl, Phenyl, Hydroxy, C₁₋₆-Hydroxyalkyl, C₁₋₆-Alkoxy, C₁₋₆-Alkoxy, das vollständig oder teilweise durch Fluor substituiert ist, Benzyloxy, Phenoxy, Naphthyloxy, Mercapto, C₁₋₆-Alkylthio, Phenylthio, Naphthylthio, Pyridylthio, C₁₋₆-Alkansulfonyl, Phenylsulfonyl, Naphthylsulfonyl, Pyridylsulfonyl, Sulfo, Carboxy, C₂₋₇-Carboxyalkyl, C₃₋₇-Carboxyalkenyl, C₂₋₇-Alkoxycarbonyl, Benzyloxycarbonyl, Nitro, Amino, C₁₋₆-Aminoalkyl, Mono-C₁₋₆-alkylamino, Di(C₁₋₆-alkyl)amino, Phenylamino, Phenyl-C₁₋₃-alkylamino, Pyridylamino, gesättigten und ungesättigten, 4- bis 7-gliedrigen Heterocyclen, die ein oder zwei Heteroatome, ausgewählt aus N, S und O, enthalten und entweder direkt oder über eine Methylengruppe oder eine Methingruppe gebunden sind,
monocyclischen, aromatischen, 5- und 6-gliedrigen Heterocyclen, die ein bis drei Heteroatome enthalten, ausgewählt aus N, S und O, und die entweder direkt oder über eine Methylengruppe oder eine Methingruppe gebunden sind,
kondensierten, bicyclischen, aromatischen und teilweise hydrierten, carbocyclischen Ringsystemen mit 8 bis 12 Ringatomen, die entweder direkt oder über eine Methylengruppe oder eine Methingruppe gebunden sind, und
kondensierten, bicyclischen, aromatischen und teilweise hydrierten, heterocyclischen Ringsystemen mit 8 bis 12 Ringatomen, worin ein bis drei Ringatome ausgewählt sind aus N, S und O, und die entweder direkt oder über eine Methylengruppe oder eine Methingruppe gebunden sind,
worin aromatische Ringsysteme in den Substituenten R¹⁽ⁱ⁾, R²⁽ⁱ⁾, R⁴⁽ⁱ⁾, R⁵⁽ⁱ⁾, R⁶⁽ⁱ⁾, R¹³⁽ⁱ⁾, R¹⁴⁽ⁱ⁾, R¹⁵⁽ⁱ⁾, R¹⁶⁽ⁱ⁾, R¹⁷⁽ⁱ⁾, R¹⁸, R¹⁹, Ar¹ und Ar², in den Gruppen A⁽ⁱ⁾ und D⁽ⁱ⁾, in den Ringsystemen =CR¹³⁽ⁱ⁾R¹⁵⁽ⁱ⁾, =CR¹³⁽ⁱ⁾R18, -NR¹³⁽ⁱ⁾R¹⁵⁽ⁱ⁾ und -NR¹³⁽ⁱ⁾R¹⁸⁽ⁱ⁾ sowie Substituenten und/oder Substituenten in den cyclischen Imiden G¹³ gegebenenfalls unabhängig voneinander substituiert sind durch ein bis drei der gleichen oder unterschiedlichen Gruppen, ausgewählt aus
Halogen, Cyano, C₁₋₆-Alkyl, Trifluormethyl, C₃₋₈-Cycloalkyl, Benzyl, Phenyl, Hydroxy, C₁₋₆-Hydroxyalkyl, C₁₋₆-Alkoxy, C₁₋₆-Alkoxy, das vollständig oder teilweise durch Fluor substituiert ist, Benzyloxy, Phenoxy, Mercapto, C₁₋₆-Alkylthio, Phenylthio, Sulfo, Carboxy, C₂₋₇-Carboxyalkyl, C₃₋₇-Carboxyalkenyl, C₂₋₇-Alkoxycarbonyl, Benzyloxycarbonyl, Nitro, Amino, C₁₋₆-Aminoalkyl, Mono-C₁₋₆-alkylamino und Di(C₁₋₆-alkyl)amino, und im Fall von zwei benachbarten Resten am aromatischen Ring, Methylendioxy,
worin Alkyl- und Cycloalkylreste in den Gruppen G gegebenenfalls durch ein oder zwei der gleichen oder verschiedenen Gruppen substituiert sind, ausgewählt aus
Hydroxy, Carboxy, C₂₋₇-Alkoxycarbonyl, Benzyloxycarbonyl, Amino, Mono-C₁₋₆-alkylamino und Di(C₁₋₆-alkyl)amino;
und die Stereoisomere oder racemische oder nichtracemische Mischungen von Stereoisomeren davon,
und die Tautomere davon, wenn G ein heterocyclischer aromatischer Ring oder ein aromatischer Ring, substituiert durch eine Hydroxy-, Mercapto- oder Aminogruppe, ist,
und die pharmakologisch annehmbaren Säureadditionssalze davon;
(b) mindestens eine Verbindung mit Vitamin PP-Aktivität oder ein Prodrug davon, ausgewählt aus der Gruppe bestehend aus Verbindungen der Formeln (II), (IIa), (IIb), (III), (IIIa), (IIIb), (IIIc), (IV), (IVa), (IVb), (V), (Va) und (Vb) : 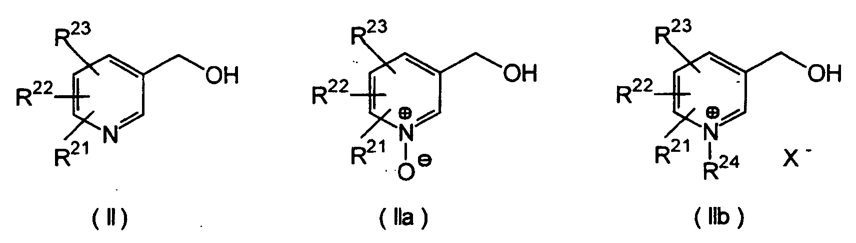 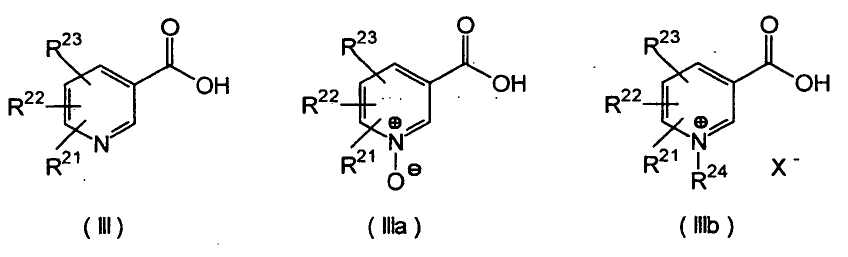 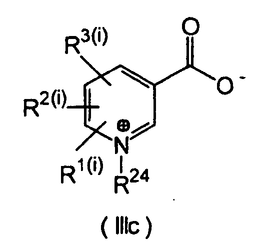 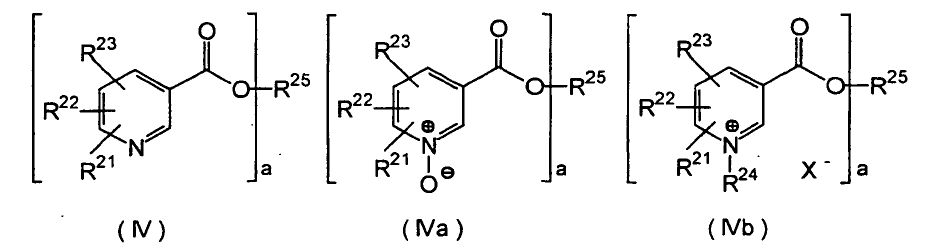 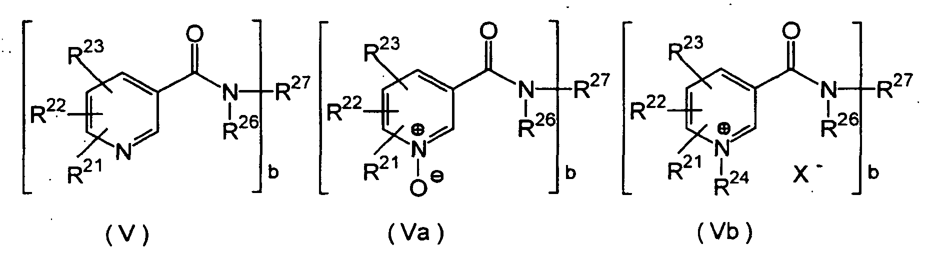 worin:
a eine ganze Zahl von 1 bis 6 ist;
b eine ganze Zahl von 1 bis 2 ist;
X⁻ ausgewählt ist aus der Gruppe bestehend aus Fluorid, Chlorid, Bromid, Iodid, Hydrogensulfat, Methansulfonat, Trifluormethansulfonat, Tosylat, Tetrafluorborat, Dihydrogenphosphat und Acetat;
R²¹ ausgewählt ist aus der Gruppe bestehend aus Wasserstoff, Halogen, Cyano, C₁₋₆-Alkyl, Trifluormethyl, C₁₋₆-Hydroxyalkyl, Hydroxy, C₁₋₆-Alkoxy, C₁₋₇-Alkanoyloxy, C₁₋₆-Alkylthio, C₁₋₆-Aminoalkyl, Amino, C₁₋₆-Alkylamino, C₂₋₁₂-Dialkylamino, Formyl, C₂₋₇-Alkoxycarbonyl, Aminocarbonyl, C₂₋₇-Alkylaminocarbonyl, C₃₋₁₃-Dialkylamino-carbonyl und Carboxy;
R²² ausgewählt ist aus der Gruppe bestehend aus Wasserstoff, Halogen, C₁₋₆-Alkyl, Trifluormethyl, C₁₋₆-Hydroxyalkyl, Hydroxy, C₁₋₆-Alkoxy, C₁₋₇-Alkanoyloxy, C₁₋₆-Aminoalkyl, Amino, C₂₋₇-Alkoxycarbonyl, Aminocarbonyl und Carboxy;
R²³ ausgewählt ist aus der Gruppe bestehend aus Wasserstoff, C₁₋₆-Alkyl und C₁₋₆-Hydroxyalkyl;
R²⁴ ausgewählt ist aus der Gruppe bestehend aus C₁₋₆-Alkyl, C₃₋₆-Alkenyl, C₂₋₆-Hydroxyalkyl, C₂₋₆-Alkoxyalkyl und Benzyl;
R²⁵ derart ist, dass der Alkohol R²⁵(OH)ₐ ausgewählt ist aus monovalenten, linearen und verzweigten C₁₋₁₀-Alkanolen und ω-Dialkylaminoalkanolen, Benzylalkohol, divalenten, linearen und verzweigten C₂₋₁₀-Diolen, mono- oder divalenten C₅₋₇-Cycloalkanolen, C₅₋₇-Cycloalkandiolen, C₅₋₇-Cycloalkanmethanolen, gesättigten C₅₋₇-Heterocyclomethanolen, Glycerin, 2,2-Bis(hydroxymethyl)-1-octanol, Erythritol, Pentaerythritol, Arabitol, Xylitol, Sorbitol, Mannitol, Isobarbitol, Tetra(hydroxymethyl)cyclohexanol und Inositol;
R²⁶ ausgewählt ist aus der Gruppe bestehend aus Wasserstoff, C₁₋₆-Alkyl, C₁₋₆-Hydroxyalkyl, C₃₋₆-Alkoxyalkyl, C₁₋₆-Aminoalkyl, C₄₋₁₂-Dialkylaminoalkyl und Carboxymethyl;
wenn b 1 ist,
dann ist R²⁷ ausgewählt aus der Gruppe bestehend aus Wasserstoff, C₁₋₆-Alkyl, C₁₋₆-Hydroxyalkyl, C₃₋₆-Alkoxyalkyl, C₁₋₆-Aminoalkyl, C₄₋₁₂-Dialkylaminoalkyl und Carboxymethyl;
wenn b 2 ist,
dann ist R²⁷ C₂₋₁₀-Alkylen oder C₅₋₁₀-Alkylen, worin eine Methylengruppe isosterisch durch O, NH oder N-Alkyl ersetzt ist;
und ihre Thioxoanaloge,
und die Säureadditionssalze oder anionischen Salze davon; und
(c) mindestens einen physiologisch annehmbaren Träger und mindestens ein toxikologisch sicheres Adjuvans.

2. Zusammensetzung gemäss Anspruch 1, worin die Verbindung(en) der Formel (I) ausgewählt ist/sind aus:
N-[2-(1-Benzylpiperidin-4-yl)-ethyl]-3-(pyridin-3-yl)-propionamid;
N-{2-[1-(2-Phenylethyl)-piperidin-4-yl]-ethyl}-3-(pyridin-3-yl)-propionamid;
N-{2-[1-(4-Phenylbutyl)-piperidin-4-yl]-ethyl}-3-(pyridin-3-yl)-propionamid;
N-{2-[1-(4-Hydroxy-4-phenylbutyl)-piperidin-4-yl]-ethyl}-3-(pyridin-3-yl)-propionamid;
N-[2-(1-Diphenylmethylpiperidin-4-yl)-ethyl]-3-(pyridin-3-yl)-propionamid;
N-[3-(1-Diphenylmethylpiperidin-4-yl)-propyl]-3-(pyridin-3-yl)-propionamid;
N-[4-(1-Diphenylmethylpiperidin-4-yl)-butyl]-3-(pyridin-3-yl)-propionamid;
N-[4-(1-Benzylpiperidin-4-yl)-butyl]-3-(pyridin-3-yl)-acrylamid;
N-{4-[1-(2-Phenylethyl)-piperidin-4-yl]-butyl}-3-(pyridin-3-yl)-acrylamid;
N-{4-[1-(4-Biphenylylmethyl)-piperidin-4-yl]-butyl}-3-(pyridin-3-yl)-acrylamid;
N-{4-[1-(1-Naphthylmethyl)-piperidin-4-yl]-butyl}-3-(pyridin-3-yl)-acrylamid;
N-{4-[1-(9-Anthrylmethyl)-piperidin-4-yl]-butyl}-3-(pyridin-3-yl)-acrylamid;
N-{4-[1-(Cyclohexylphenylmethyl)-piperidin-4-yl]-butyl}-3-(pyridin-3-yl)-acrylamid;
N-{4-[1-(10,11-Dihydro-5H-dibenzo[a,d]cyclohepten-5-yl)-piperidin-4-yl]-butyl}-3-(pyridin-3-yl)-acrylamid;
N-[2-(1-Diphenylmethylpiperidin-4-yl)-ethyl]-3-(pyridin-3-yl)-acrylamid;
N-[3-(1-Diphenylmethylpiperidin-4-yl)-propyl]-3-(pyridin-3-yl)-acrylamid;
N-[5-(1-Diphenylmethylpiperidin-4-yl)-pentyl]-3-(pyridin-3-yl)-acrylamid;
N-[6-(1-Diphenylmethylpiperidin-4-yl)-hexyl]-3-(pyridin-3-yl)-acrylamid;
N-[4-(1-Diphenylmethylpiperidin-4-yl)-butyl]-5-(pyridin-3-yl)-2,4-pentadiensäureamid;
N-(4-{1-[Bis-(4-fluorphenyl)-methyl]-piperidin-4-yl}-butyl)-3-(pyridin-3-yl)-acrylamid;
N-(4-{1-[Bis-(2-chlorphenyl)-methyl]-piperidin-4-yl}-butyl)-3-(pyridin-3-yl)-acrylamid;
N-[4-(1-Diphenylmethylpiperidin-4-yl)-butyl]-3-(2-fluorpyridin-3-yl)-acrylamid;
N-[4-(1-Diphenylmethylpiperidin-4-yl)-butyl]-3-(6-fluorpyridin-3-yl)-acrylamid;
N-[4-(1-Diphenylmethylpiperidin-4-yl)-butyl]-3-(pyridin-3-yl)-acrylamid;
N-[4-(1-Diphenylmethylpiperidin-4-yl)-butyl]-3-(pyridin-3-yl)-acrylamid-Dihydrochlorid;
N-[4-(1-Diphenylmethylpiperidin-4-yl)-butyl]-3-(pyridin-3-yl)-acrylamid-Methansulfonat;
N-[4-(1-Acetyl-piperidin-4-yl)-butyl]-3-(pyridin-3-yl)-propionamid;
N-[4-(1-Benzoyl-piperidin-4-yl)-butyl]-3-(pyridin-3-yl)-propionamid;
N-[4-(1-Diphenylacetyl-piperidin-4-yl)-butyl]-3-(pyridin-3-yl)-propionamid;
N-{4-[1-(9-Oxo-9H-fluoren-4-carbonyl)-piperidin-4-yl]-butyl}-3-(pyridin-3-yl)-propionamid;
N-[4-(1-Methylsulfonyl-piperidin-4-yl)-butyl]-3-(pyridin-3-yl)-propionamid;
N-{4-[1-(2-Naphthyl-sulfonyl)-piperidin-4-yl]-butyl}-3-(pyridin-3-yl)-propionamid;
N-[4-(1-Benzyl-piperidin-4-yl)-butyl]-3-(pyridin-3-yl)-propionamid;
N-(4-{1-[Bis-(2-chlorphenyl)-methyl]-piperidin-4-yl}-butyl)-3-(pyridin-3-yl)-propionamid;
N-{4-[1-(Phenylpyridin-3-yl-methyl)-piperidin-4-yl]-butyl}-3-(pyridin-3-yl)-propionamid;
N-{4-[1-(9H-Fluoren-9-yl)-piperidin-4-yl]-butyl}-3-(pyridin-3-yl)-propionamid;
N-{4-[1-(6,11-Dihydrodibenzo[b,e]oxepin-11-yl)-piperidin-4-yl]-butyl}-3-(pyridin-3-yl)-propionamid;
N-{4-[1-(1-Naphthylamino-carbonyl)-piperidin-4-yl]-butyl}-3-(pyridin-3-yl)-propionamid;
N-[4-(1-Diphenylamino-carbonyl-piperidin-4-yl)-butyl]-3-(pyridin-3-yl)-propionamid;
N-{4-[1-(10,11-Dihydro-dibenzo[b,f]azepin-5-yl-carbonyl)-piperidin-4-yl]-butyl}-3-(pyridin-3-yl)-propionamid;
N-[4-(1-Diphenylphosphinoyl-piperidin-4-yl)-butyl]-3-(pyridin-3-yl)-propionamid;
N-[4-(1-Diphenylmethylpiperidin-4-yl)-butyl]-3-(2-fluorpyridin-3-yl)-propionamid;
N-[4-(1-Diphenylmethylpiperidin-4-yl)-butyl]-3-(5-fluorpyridin-3-yl)-propionamid;
N-[4-(1-Diphenylmethylpiperidin-4-yl)-butyl]-2-fluor-3-(pyridin-3-yl)-propionamid;
N-[4-(1-Diphenylmethylpiperidin-4-yl)-butyl]-2,2-difluor-3-(pyridin-3-yl)-propionamid;
N-[5-(1-Diphenylmethylpiperidin-4-yl)-pentyl]-3-(pyridin-3-yl)-propionamid;
N-[6-(1-Diphenylmethylpiperidin-4-yl)-hexyl]-3-(pyridin-3-yl)-propionamid;
N-[2-(1-Diphenylmethylpiperidin-4-yl)-ethyl]-5-(pyridin-3-yl)-pentansäureamid;
N-[4-(1-Diphenylmethylpiperidin-4-yl)-butyl]-5-(pyridin-3-yl)-pentansäureamid;
N-[4-(1-Diphenylmethylpiperidin-4-yl)-butyl]-N-hydroxy-3-(pyridin-3-yl)-propionamid;
N-[4-(1-Diphenylmethylpiperidin-4-yl)-butyl]-2-hydroxy-3-(pyridin-3-yl)-propionamid;
N-[4-(1-Diphenylmethylpiperidin-4-yl)-butyl]-3-hydroxy-3-(pyridin-3-yl)-propionamid;
N-[4-(1-Diphenylmethylpiperidin-4-yl)-butyl]-3-(pyridin-3-yl)-propionamid;
N-[4-(1-Methylsulfonylpiperidin-4-yl)-butyl]-3-(pyridin-3-yl)-acrylamid;
N-{4-[1-(2-Naphthylsulfonyl)-piperidin-4-yl]-butyl}-3-(pyridin-3-yl)-acrylamid;
N-{4-[1-(2-Naphthylsulfonyl)-piperidin-4-yl]-butyl}-5-(pyridin-3-yl)-2,4-pentadiensäureamid;
N-{4-[1-(1-Naphthylaminocarbonyl)-piperidin-4-yl]-butyl}-3-(pyridin-3-yl)-acrylamid;
N-[4-(1-Diphenylaminocarbonylpiperidin-4-yl)-butyl]-3-(pyridin-3-yl)-acrylamid;
N-[4-(1-Diphenylaminocarbonyl-piperidin-4-yl)-butyl]-5-(pyridin-3-yl)-2,4-pentadiensäureamid;
N-{4-[1-(10,11-Dihydrodibenzo[b,f]azepin-5-yl-carbonyl)-piperidin-4-yl]-butyl}-3-(pyridin-3-yl)-acrylamid;
N-[4-(1-Diphenylphosphinoyl-piperidin-4-yl)-butyl]-3-(pyridin-3-yl)-acrylamid;
N-[4-(1-Acetylpiperidin-4-yl)-butyl]-3-(pyridin-3-yl)-acrylamid;
N-[4-(1-Diphenylacetylpiperidin-4-yl)-butyl]-3-(pyridin-3-yl)-acrylamid;
N-{4-[1-(3,3-Diphenylpropionyl)-piperidin-4-yl]-butyl}-3-(pyridin-3-yl)-acrylamid;
N-[4-(1-Benzoylpiperidin-4-yl)-butyl]-3-(pyridin-3-yl)-acrylamid;
N-[4-(1-Benzoylpiperidin-4-yl)-butyl]-5-(pyridin-3-yl)-2,4-pentadiensäureamid;
N-{4-[1-(9-Oxo-9H-fluoren-4-yl-carbonyl)-piperidin-4-yl]-butyl}-3-(pyridin-3-yl)-acrylamid;
N-{4-[1-(Phenylpyridin-3-yl-methyl)-piperidin-4-yl]-butyl}-3-(pyridin-3-yl)-acrylamid;
N-{4-[1-(Phenylpyridin-4-yl-methyl)-piperidin-4-yl]-butyl}-3-(pyridin-3-yl)-acrylamid;
N-{4-[1-(6,11-Dihydrodibenzo[b,e]oxepin-11-yl)-piperidin-4-yl]-butyl}-3-(pyridin-3-yl)-acrylamid;
N-{4-[1-(6,11-Dihydrodibenzo[b,e]thiepin-11-yl)-piperidin-4-yl]-butyl}-3-(pyridin-3-yl)-acrylamid;
N-[7-(1-Diphenylmethylpiperidin-4-yl)-heptyl]-3-(pyridin-3-yl)-acrylamid;
N-[8-(1-Diphenylmethylpiperidin-4-yl)-octyl]-3-(pyridin-3-yl)-acrylamid;
N-[3-(1-Diphenylmethylpiperidin-4-yloxy)-propyl]-3-(pyridin-3-yl)-acrylamid;
N-[3-(1-Benzylpiperidin-4-yloxy)-propyl]-3-(pyridin-3-yl)-acrylamid;
N-[2-(1-Diphenylmethylpiperidin-4-yl)-ethyl]-5-(pyridin-3-yl)-2,4-pentadiensäureamid;
N-[4-(1-Diphenylmethylpiperidin-4-yl)-butyl]-5-(pyridin-3-yl)-2,4-pentadiensäureamid;
N-[5-(1-Diphenylmethylpiperidin-4-yl)-pentyl]-5-(pyridin-3-yl)-2,4-pentadiensäureamid;
N-[6-(1-Diphenylmethylpiperidin-4-yl)-hexyl]-5-(pyridin-3-yl)-2,4-pentadiensäureamid;
N-[4-(4-Diphenylmethylpiperazin-1-yl)-3-hydroxy-butyl]-3-pyridin-3-yl-acrylamid;
N-[3-(4-Diphenylmethylpiperazin-1-yl)-propoxy]-3-pyridin-3-yl-acrylamid;
N-[4-(4-Diphenylmethylpiperazin-1-yl)-4-oxo-butyl]-3-pyridin-3-yl-acrylamid;
N-[3-(4-Diphenylmethylpiperazin-1-sulfonyl)-propyl]-3-pyridin-3-yl-acrylamid;
N-{2-[2-(4-Diphenylmethylpiperazin-1-yl)-ethoxy]-ethyl}-3-pyridin-3-yl-acrylamid;
N-(4-{4-[Bis-(4-fluorphenyl)-methyl]-piperazin-1-yl}-but-2-inyl)-3-pyridin-3-yl-acrylamid;
N-{4-[4-(4-Carboxyphenyl-phenylmethyl)-piperazin-1-yl]-butyl}-3-pyridin-3-yl-acrylamid;
N-(4-{4-[(4-Aminophenyl)-phenylmethyl]-piperazin-1-yl}-butyl)-3-pyridin-3-yl-acrylamid;
N-{4-[4-(9H-Fluoren-9-yl)-piperazin-1-yl]-butyl}-2-(pyridin-3-yloxy)-acetamid;
N-{5-[4-(9H-Fluoren-9-yl)-piperazin-1-yl]-pentyl}-3-pyridin-3-yl-acrylamid;
N-{6-[4-(9H-Fluoren-9-yl)-piperazin-1-yl]-hexyl}-3-pyridin-3-yl-acrylamid;
3-Pyridin-3-yl-N-{4-[4-(1,2,3,4-tetrahydronaphthalin-1-yl)-piperazin-1-yl]-butyl}-acrylamid;
3-Pyridin-3-yl-N-{4-[4-(5,6,7,8-tetrahydronaphthalin-1-yl)-piperazin-1-yl]-butyl}-acrylamid;
N-{4-[4-(Naphthalin-1-yl)-piperazin-1-yl]-butyl}-3-pyridin-3-yl-acrylamid;
N-[4-(4-Biphenyl-2-yl-piperazin-1-yl)-butyl]-3-pyridin-3-yl-propionamid;
N-[5-(4-Biphenyl-2-yl-piperazin-1-yl)-pentyl]-3-pyridin-3-yl-acrylamid;
N-[6-(4-Biphenyl-2-yl-piperazin-1-yl)-hexyl]-3-pyridin-3-yl-acrylamid;
N-[4-(4-Biphenyl-2-yl-piperazin-1-ly)-butyl]-2-(pyridin-3-yloxy)-acetamid;
N-[4-(4-Biphenyl-2-yl-piperazin-1-yl)-butyl]-5-(pyridin-3-yl)-penta-2,4-diensäureamid;
N-{4-[4-(10,11-Dihydro-5H-dibenzo[a,d]cyclohepten-5-yl)-piperazin-1-yl]-butyl}-3-pyridin-3-yl-propionamid;
N-{5-[4-(10,11-Dihydro-5H-dibenzo[a,d]cyclohepten-5-yl)-piperazin-1-yl]-pentyl}-3-pyridin-3-yl-acrylamid;
N-{6-[4-(10,11-Dihydro-5H-dibenzo[a,d]cyclohepten-5-yl)-piperazin-1-yl]-hexyl}-3-pyridin-3-yl-acrylamid;
N-{4-[4-(10,11-Dihydro-5H-dibenzo[a,d]cyclohepten-5-yl)-piperazin-1-yl]-butyl}-5-(pyridin-3-yl)-penta-2,4-diensäureamid;
N-{4-[4-(6,11-Dihydro-dibenzo[b,e]oxepin-11-yl)-piperazin-1-yl]-butyl-3-pyridin-3-yl-propionamid;
N-{2-[4-(6,11-Dihydro-dibenzo[b,e]thiepin-11-yl)-piperazin-1-yl]-ethyl}-3-pyridin-3-yl-acrylamid;
N-[4-(4-Diphenylacetylpiperazin-1-yl)-butyl]-3-pyridin-3-yl-acrylamid;
N-[4-(4-Benzoylpiperazin-1-yl)-butyl]-3-pyridin-3-yl-acrylamid;
N-{4-[4-(2-Aminobenzoyl)-piperazin-1-yl]-butyl}-3-pyridin-3-yl-acrylamid;
N-{4-[4-(4-Carboxybenzoyl)-piperazin-1-yl]-butyl}-3-pyridin-3-yl-acrylamid;
N-{4-[4-(Biphenyl-2-carbonyl)-piperazin-1-yl]-butyl}-3-pyridin-3-yl-acrylamid;
N-{4-[4-(9-Oxo-9H-fluoren-4-carbonyl)-piperazin-1-yl]-butyl}-3-pyridin-3-yl-acrylamid;
N-{4-[4-(Furan-2-carbonyl)-piperazin-1-yl]-butyl}-3-pyridin-3-yl-acrylamid;
N-{4-[4-(Naphthalin-1-yl-aminocarbonyl)-piperazin-1-yl]-butyl}-3-pyridin-3-yl-propionamid;
N-{4-[4-(Diphenylaminocarbonyl)-piperazin-1-yl]-butyl}-3-pyridin-3-yl-acrylamid;
N-{4-[4-(Naphthalin-2-sulfonyl)-piperazin-1-yl]-butyl}-3-pyridin-3-yl-acrylamid;
N-[4-(4-Diphenylphosphinonyl-piperazin-1-yl)-butyl]-3-pyridin-3-yl-acrylamid;
N-[4-(4-Biphenyl-2-yl-piperazin-1-yl)-butyl]-3-pyridin-3-yl-acrylamid;
N-{4-[4-(9H-Fluoren-9-yl)-piperazin-1-yl]-butyl}-3-pyridin-3-yl-acrylamid;
N-{4-[4-(10,11-Dihydro-5H-dibenzo[a,d]cyclohepten-5-yl)-piperazin-1-yl]-butyl}-3-pyridin-3-yl-acrylamid;
N-[4-(4-Phenylpiperidin-1-yl)-butyl]-3-pyridin-3-yl-acrylamid;
N-{4-[4-(1H-Indol-3-yl)-piperidin-1-yl]-butyl}-3-pyridin-3-yl-acrylamid;
N-{4-[4-(2-Oxo-2,3-dihydrobenzimidazol-1-yl)-piperidin-1-yl]-butyl}-3-pyridin-3-yl-acrylamid;
N-[4-(4-Benzotriazol-1-yl-piperidin-1-yl)-butyl]-3-pyridin-3-yl-acrylamid;
N-{4-[4-(Hydroxy-diphenylmethyl)-piperidin-1-yl]-butyl}-2-(pyridin-3-yloxy)-acetamid;
N-[4-(4,4-Diphenylpiperidin-1-yl)-butyl]-3-pyridin-3-yl-acrylamid;
N-{4-[4-(6,11-Dihydrodibenzo[b,e]thiepin-11-yliden)-piperidin-1-yl]-butyl}-3-pyridin-3-ylpropionamid-Dihydrochlorid/Semiisopropanol;
N-{4-[4-(6,11-Dihydrodibenzo[b,e]thiepin-11-yliden)-piperidin-1-yl]-butyl}-5-pyridin-3-yl-pentanamid;
N-{4-[4-(4,9-Dihydro-thieno[2,3-b]-benzo[e]thiepin-4-yliden)-piperidin-1-yl]-butyl}-3-pyridin-3-yl-propionamid;
N-{4-[4-(4,9-Dihydro-thieno[2,3-b]-benzo[e]thiepin-4-yliden)-piperidin-1-yl]-butyl}-3-pyridin-3-yl-acrylamid;
N-[4-(4-Diphenylphosphinoyloxypiperidin-1-yl)-butyl]-3-pyridin-3-yl-acrylamid;
N-[4-(1,4-Dioxa-8-azaspiro[4,5]dec-8-yl)-butyl]-3-pyridin-3-yl-acrylamid;
N-[4-(2,5-Dioxo-3,4-diphenyl-2,5-dihydropyrrol-1-yl)-butyl]-3-pyridin-3-yl-acrylamid;
N-[4-(2,6-Dioxo-4-phenylpiperidin-1-yl)-butyl]-3-pyridin-3-yl-acrylamid;
N-[4-(1,3-Dioxo-4,5,6,7-tetraphenyl-1,3-dihydroisoindol-2-yl)-butyl]-3-pyridin-3-yl-acrylamid;
N-[4-(3-Benzyl-2,4,5-trioxo-imidazolidin-1-yl)-butyl]-3-pyridin-3-yl-acrylamid;
N-[4-(1,3,10-Trioxo-1,4,5,6,10,10a-hexahydroacenaphtho[1,8a-c]pyrrol-2-yl)-butyl]-3-pyridin-3-yl-acrylamid;
N-[4-(2,5-Dioxo-4,4-diphenylimidazolidin-1-yl)-butyl-3-pyridin-3-yl-acrylamid;
N-[4-(2,5-Dioxo-3-phenyl-2,5-dihydropyrrol-1-yl)-butyl]-3-pyridin-3-yl-acrylamid;
N-[3-(2,5-Dioxo-3,4-diphenyl-2,5-dihydro-pyrrol-1-yl)-propyl]-3-pyridin-3-yl-acrylamid;
N-[4-(3-Pyridin-3-yl-acroylamino)-butyl]-2,3:5,6-dibenzobicyclo[2.2.2]octan-7,8-dicarbonsäureimid;
N-[4-(5-Benzyliden-2,4-dioxothiazolidin-3-yl)-butyl]-3-pyridin-3-yl-acrylamid;
N-[4-(4-Benzyl-2,6-dioxopiperazin-1-yl)-butyl]-3-pyridin-3-yl-acrylamid;
N-[6-(2,5-Dioxo-3,4-diphenyl-2,5-dihydropyrrol-1-yl)-hexyl]-3-pyridin-3-yl-acrylamid;
N-[4-(2,5-Dioxo-3,4-diphenyl-2,5-dihydropyrrol-1-yl)-butyl]-3-pyridin-3-yl-propionamid;
N-[4-(1,3-Dioxo-1,3-dihydro-isoindol-2-yl)-butyl]-3-pyridin-3-yl-acrylamid;
N-[4-(1,3-Dioxo-1H,3H-benzo[de]isochinolin-2-yl)-butyl]-3-(1-oxidopyridin-3-yl)-acrylamid;
N-[6-(1,3-Dioxo-1H,3H-benzo[de]isochinolin-2-yl)-hexyl]-3-pyridin-3-yl-acrylamid;
N-[2-(1,3-Dibxo-1H,3H-benzo[de]isochinolin-2-yl)-ethyl]-3-pyridin-3-yl-acrylamid;
N-[4-(1,3-Dioxo-1H,3H-benzo[de]isochinolin-2-yl)-butyl]-3-pyridin-3-yl-acrylamid;
N-[8,8-Bis-(4-flüorphenyl)-octyl]-3-pyridin-3-yl-acrylamid·Hydrochlorid;
N-[6-(3,3-Diphenylureido)-hexyl]-3-pyridin-3-yl-acrylamid;
N-[4-(1-Phenyl-1,2,4,5-tetrahydrobenzo[d]azepin-3-yl)-butyl]-3-pyridin-3-yl-acrylamid;
N-(8,8-Diphenyloctyl)-3-pyridin-3-yl-acrylamid;
N-(8-Hydroxy-8,8-diphenyloctyl)-3-pyridin-3-yl-acrylamid;
N-[4-(3,3-Diphenylureido)-butyl]-3-pyridin-3-yl-acrylamid;
N-[4-(1H,3H-Benzo[de]isochinolin-2-yl)-butyl]-3-pyridin-3-yl-acrylamid;
N-[6-(10,11-Dihydro-dibenzo[b,f]azepin-5-yl-carbonylamino)-hexyl]-3-pyridin-3-yl-acrylamid;
3-Pyridin-3-yl-N-[6-(tosylamino)-hexyl]-acrylamid; N-[4-(1,1-Dioxo-1-thia-2-aza-acenaphthylen-2-yl)-butyl]-3-pyridin-3-yl-acrylamid;
N-(6-Hydroxy-6,6-diphenylhexyl)-3-pyridin-3-yl-acrylamid;
N-(6,6-Diphenyl-hex-5-enyl)-3-pyridin-3-yl-acrylamid;
N-[4-(4,5-Diphenylimidazol-1-yl)-butyl]-3-pyridin-3-yl-acrylamid;
N-[4-(trans-2-Phenylcyclopropylcarbonylamino)-butyl]-3-pyridin-3-yl-acrylamid;
N-(5-Hydroxy-5,5-diphenyl-pentyl)-3-pyridin-3-yl-acrylamid;
N-(7-Phenylheptyl)-3-pyridin-3-yl-acrylamid;
N-(4-Diphenylacetylamino-butyl)-3-pyridin-3-yl-acrylamid;
N-[4-(Benzhydrylamino)-butyl]-3-pyridin-3-yl-acrylamid und
N-(4-{[2-(Benzhydrylmethylamino)-ethyl]-methylamino}-butyl)-3-pyridin-3-yl-acrylamid.

3. Zusammensetzung gemäss Anspruch 1 oder 2, umfassend ein weiteres cancerostatisches oder immunsuppressives Mittel, das keine Verbindung der Formel (I) ist.

4. Zusammensetzung gemäss einem der Ansprüche 1 bis 3, worin die Verbindung(en) der Formel (I) und die Verbindung(en) der Formeln (II) bis (Vb) getrennt voneinander in der Zusammensetzung enthalten sind.

5. Zusammensetzung gemäss einem der Ansprüche 1 bis 4, worin die Verbindung(en) der Formel (I) und die Verbindung(en) der Formeln (II) bis (Vb) in getrennten Dosisformen vorhanden sind, und die Dosisformen zur gemeinsamen Verabreichung zusammen verpackt sind.

6. Zusammensetzung gemäss einem der Ansprüche 1 bis 5, die Tryptophan als Prodrug enthält.

7. Zusammensetzung gemäss einem der Ansprüche 1 bis 6, die sich befindet:
in einer festen, peroral verabreichbaren Form als Tablette, Kapsel, Dragee, gegebenenfalls als Retard oder magensaftresistente Zubereitung, als flüssige Arzneiform, peroral verabreichbare Lösung, Suspension, Brausetablette, in Form von Tabs oder Sachets, gegebenenfalls in retardierter Form,
in Form einer geeigneten Injektions- oder Infusionszubereitung zusammen mit geeigneten, pharmazeutisch annehmbaren Trägern und Hilfsstoffen, gegebenenfalls in retardierter Form oder als parenterale Depotarzneiform oder Implantat, in Form eines Konzentrats, Pulvers oder Lyophilisats,
in Form eines Inhalationstherapeutikums, in Form eines Sprays, zusammen mit geeigneten, pharmazeutisch annehmbaren Treibmitteln, Trägern und Hilfsstoffen, in Form eines transdermalen therapeutischen Systems zur systemischen Behandlung,
in Form eines gastrointestinalen therapeutischen Systems (GITS) zur systemischen Behandlung,
in Form einer Salbe, Suspension, Emulsion, eines Balsams oder Pflasters oder in Form einer äusserlich applizierbaren Lösung,
in Form einer rektal, genital oder transurethral verabreichbaren Emulsion, einer Lösung, einer liposomalen Lösung, eines Implantats, Suppositoriums oder einer Kapsel,
in Form einer nasal, otologisch oder ophthalmologisch applizierbaren Zusammensetzung oder
in bukkal applizierbarer Form.

8. Zusammensetzung gemäss einem der Ansprüche 1 bis 6 zur Verabreichung mit einem Dosieraerosol oder in Form einer Trockenpulver-Dosierformulierung.

9. Zusammensetzung gemäss einem der Ansprüche 1 bis 8, worin eine Dosiseinheit zur Einzelverabreichung 0,001 bis 1.000, 2.000, 3.000, 4.000 oder 5.000 mg der Verbindung(en) gemäss Formel (I) enthält.

10. Verwendung einer Verbindung mit Vitamin PP-Aktivität oder ein Prodrug davon zur Herstellung einer pharmazeutischen Zusammensetzung zur Verhinderung, Verminderung oder Beseitigung von Nebenwirkungen oder Aufhebung der Nebenwirkungen eines cancerostatischen oder immunsuppressiven Mittels, das prophylaktisch oder therapeutisch einem Patienten verabreicht wird.

11. Verwendung gemäss Anspruch 10, worin die Verbindung mit Vitamin PP-Aktivität oder ein Prodrug davon ausgewählt ist aus der Gruppe bestehend aus Verbindungen der Formeln (II), (IIa), (IIb), (III), (IIIa), (IIIb), (IIIc), (IV), (IVa), (IVb), (V), (Va) und (Vb) : 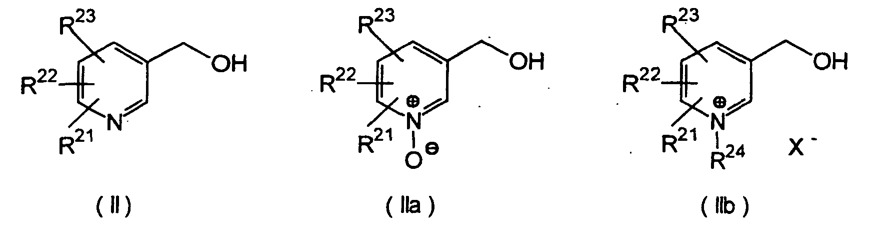 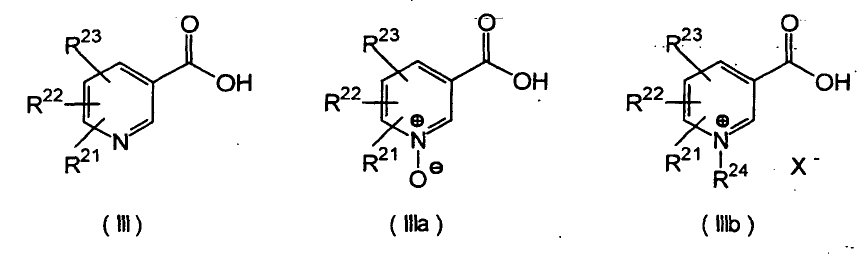 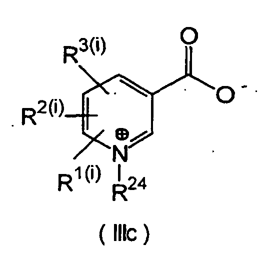 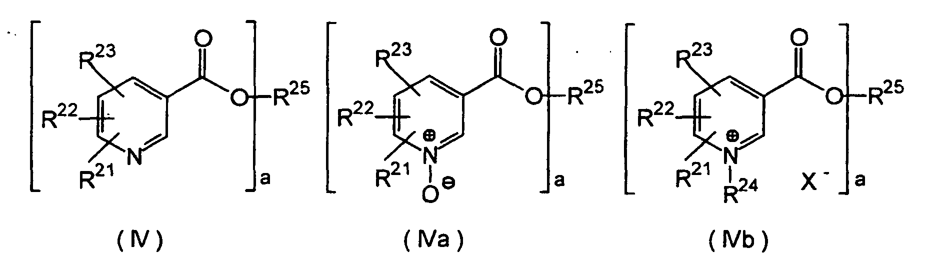 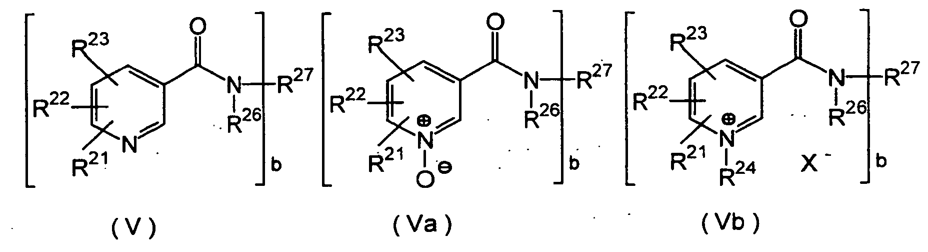 worin:
a eine ganze Zahl von 1 bis 6 ist;
b eine ganze Zahl von 1 bis 2 ist;
X⁻ ausgewählt ist aus der Gruppe bestehend aus Fluorid, Chlorid, Bromid, Iodid, Hydrogensulfat, Methansulfonat, Trifluormethansulfonat, Tosylat, Tetrafluorborat, Dihydrogenphosphat und Acetat;
R²¹ ausgewählt ist aus der Gruppe bestehend aus Wasserstoff, Halogen, Cyano, C₁₋₆-Alkyl, Trifluormethyl, C₁₋₆-Hydroxyalkyl, Hydroxy, C₁₋₆-Alkoxy, C₁₋₇-Alkanoyloxy, C₁₋₆-Alkylthio, C₁₋₆-Aminoalkyl, Amino, C₁₋₆-Alkylamino, C₂₋₁₂-Dialkylamino, Formyl, C₂₋₇-Alkoxycarbonyl, Aminocarbonyl, C₂₋₇-Alkylaminocarbonyl, C₃₋₁₃-Dialkylamino-carbonyl und Carboxy;
R²² ausgewählt ist aus der Gruppe bestehend aus Wasserstoff, Halogen, C₁₋₆-Alkyl, Trifluormethyl, C₁₋₆-Hydroxyalkyl, Hydroxy, C₁₋₆-Alkoxy, C₁₋₇-Alkanoyloxy, C₁₋₆-Aminoalkyl, Amino, C₂₋₇-Alkoxycarbonyl, Aminocarbonyl und Carboxy;
R²³ ausgewählt ist aus der Gruppe bestehend aus Wasserstoff, C₁₋₆-Alkyl und C₁₋₆-Hydroxyalkyl;
R²⁴ ausgewählt ist aus der Gruppe bestehend aus C₁₋₆-Alkyl, C₃₋₆-Alkenyl, C₂₋₆-Hydroxyalkyl, C₂₋₆-Alkoxyalkyl und Benzyl;
R²⁵ derart ist, dass der Alkohol R²⁵(OH)ₐ ausgewählt ist aus monovalenten, linearen und verzweigten C₁₋₁₀-Alkanolen und ω-Dialkylaminoalkanolen, Benzylalkohol, divalenten, linearen und verzweigten C₂₋₁₀-Diolen, mono- oder divalenten C₅₋₇-Cycloalkanolen, C₅₋₇-Cycloalkandiolen, C₅₋₇-Cycloalkanmethanolen, gesättigten C₅₋₇-Heterocyclomethanolen; Glycerin, 2,2-Bis(hydroxymethyl)-1-octanol, Erythritol, Pentaerythritol, Arabitol, Xylitol, Sorbitol, Mannitol, Isobarbitol, Tetra(hydroxymethyl)cyclohexanol und Inositol;
R²⁶ ausgewählt ist aus der Gruppe bestehend aus Wasserstoff, C₁₋₆-Alkyl, C₁₋₆-Hydroxyalkyl, C₃₋₆-Alkoxyalkyl, C₁₋₆-Aminoalkyl, C₄₋₁₂-Dialkylaminoalkyl und Carboxymethyl;
wenn b 1 ist,
dann ist R²⁷ ausgewählt aus der Gruppe bestehend aus Wasserstoff, C₁₋₆-Alkyl, C₁₋₆-Hydroxyalkyl, C₃₋₆-Alkoxyalkyl, C₁₋₆-Aminoalkyl, C₄₋₁₂-Dialkylaminoalkyl und Carboxymethyl;
wenn b 2 ist,
dann ist R²⁷ C₂₋₁₀-Alkylen oder C₅₋₁₀-Alkylen, worin eine Methylengruppe isosterisch durch O, NH oder N-Alkyl ersetzt ist;
und ihre Thioxoanaloge,
und die Säureadditionssalze oder anionischen Salze davon.

12. Verwendung gemäss Anspruch 10 oder 11, worin die Verbindung mit Vitamin PP-Aktivität oder ein Prodrug davon ausgewählt ist aus der Gruppe bestehend aus Nikotinsäure, Nikotinamid und ihren pharmazeutisch annehmbaren Ester- oder Amidderivaten, anionischen, quaternären und Additionssalzen und analogen Thioxoderivaten, ihren Isomeren und Prodrugs davon.

13. Verwendung gemäss einem der Ansprüche 10 bis 12, worin die Verbindung mit Vitamin PP-Aktivität oder ein Prodrug davon ausgewählt ist aus der Gruppe bestehend aus Nikotinsäure, Nikotinamid und Mischungen davon.

14. Verwendung gemäss einem der Ansprüche 10 bis 12, worin die Verbindung mit Vitamin PP-Aktivität oder ein Prodrug davon Tryptophan ist.

15. Verwendung gemäss einem der Ansprüche 10 bis 14, worin das cancerostatisches oder immunsuppressives Mittel ausgewählt ist aus der Gruppe, bestehend aus Verbindungen der Formel (I): 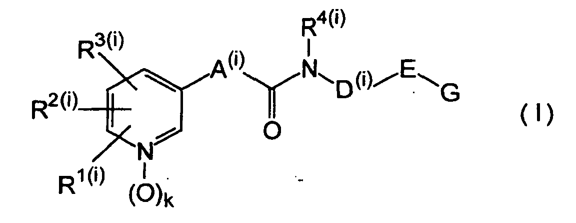 worin:
R¹⁽ⁱ⁾ ausgewählt ist aus Wasserstoff, Halogen, Cyano, C₁₋₆-Alkyl, C₃₋₆-Alkenyl, C₂₋₆-Alkinyl, Trifluormethyl, Hydroxy, C₃₋₈-Cycloalkyl, C₁₋₆-Hydroxyalkyl, C₁₋₆-Alkoxy, C₃₋₆-Alkenyloxy, C₃₋₆-Alkinyloxy, Benzyloxy, C₁₋₇-Alkanoyloxy, C₁₋₇-Alkoxycarbonyloxy, C₁₋₆-Alkylthio, C₃₋₆-Alkenylthio, C₃₋₆-Alkinylthio, C₃₋₈-Cycloalkyloxy, C₃₋₈-Cycloalkylthio, C₂₋₇-Alkoxycarbonyl, Aminocarbonyl, C₂₋₇-Alkylaminocarbonyl, C₃₋₁₃-Dialkylaminocarbonyl, Carboxy, Phenyl; Phenoxy, Phenylthio, Pyridyloxy, Pyridylthio und NR⁵⁽ⁱ⁾R⁶⁽ⁱ⁾, worin
R⁵⁽ⁱ⁾ und R⁶⁽ⁱ⁾ unabhängig voneinander ausgewählt sind aus Wasserstoff, C₁₋₆-Alkyl, C₃₋₆-Alkenyl, C₃₋₆-Alkinyl, Benzyl und Phenyl;
R²⁽ⁱ⁾ ausgewählt ist aus Wasserstoff, Halogen, Cyano, C₁₋₆-Alkyl, Trifluormethyl, Hydroxy, C₁₋₆-Alkoxy, Benzyl und C₁₋₆-Alkanoyloxy; oder
R¹⁽ⁱ⁾ und R²⁽ⁱ⁾, wenn sie benachbart sind, gegebenenfalls eine Brücke bilden, ausgewählt aus -(CH₂)₄-, -(CH=CH)₂- und -CH₂O-CR⁷⁽ⁱ⁾R⁸⁽ⁱ⁾-O-, worin
R⁷⁽ⁱ⁾ und R⁸⁽ⁱ⁾ unabhängig voneinander ausgewählt sind aus Wasserstoff und C₁₋₆-Alkyl;
R³⁽ⁱ⁾ ausgewählt ist aus Wasserstoff, Halogen, C₁₋₆-Alkyl, Trifluormethyl und C₁₋₆-Hydroxyalkyl;
R⁴⁽ⁱ⁾ ausgewählt ist aus Wasserstoff, Hydroxy, C₁₋₆-Alkyl, C₃₋₆-Alkenyl, C₃₋₆-Alkinyl, C₃₋₆-Cycloalkyl, C₁₋₆-Alkoxy und Benzyloxy;
k 0 oder 1 ist,
A⁽ⁱ⁾ ausgewählt ist aus
C₁₋₆-Alkylen, gegebenenfalls ein- bis dreimal substituiert durch C₁₋₆-Alkyl, C₁₋₃-Alkoxy, Hydroxy, Fluor oder Phenyl,
C₂₋₆-Alkylen, worin eine Methyleneinheit isosterisch ersetzt ist durch O, S, NR⁹⁽ⁱ⁾, CO, SO oder SO₂, worin, mit Ausnahme von CO, die isosterische Substitution nicht zur Aminogruppe benachbart ist und R⁹⁽ⁱ⁾ ausgewählt ist aus Wasserstoff, C₁₋₆-Alkyl, C₃₋₆-Alkenyl, C₃₋₆-Alkinyl, C₃₋₆-Acyl und C₁₋₆-Alkansulfonyl,
1,2-Cyclopropylen,
C₂₋₆-Alkenylen, gegebenenfalls ein- bis dreimal substituiert durch C₁₋₆-Alkyl, Hydroxy, C₁₋₃-Alkoxy, Fluor, Cyano oder Phenyl,
C₄₋₆-Alkadienylen, gegebenenfalls ein- oder zweimal substituiert durch C₁₋₃-Alkyl, Fluor, Cyano oder Phenyl,
1,3,5-Hexatrienylen, gegebenenfalls substituiert durch C₁₋₆-Alkyl, Fluor, Cyano oder Phenyl, und
Ethinylen;
D⁽ⁱ⁾ ausgewählt ist aus
C₁₋₁₂-Alkylen, gegebenenfalls ein- oder zweimal substituiert durch C₁₋₆-Alkyl, Hydroxy, C₁₋₆-Alkoxy oder Phenyl,
C₂₋₁₂-Alkenylen oder C₄₋₁₂-Alkadienylen, gegebenenfalls ein- oder zweimal substituiert durch C₁₋₆-Alkyl, Hydroxy, C₁₋₆-Alkoxy oder Phenyl, worin eine Doppelbindung gegebenenfalls benachbart zu Ring E sein kann, für den Fall, dass Ring E über ein C-Atom verknüpft ist,
C₃₋₁₂-Alkinylen oder C₄₋₁₂-Alkeninylen, gegebenenfalls ein- oder zweimal substituiert durch C₁₋₆-Alkyl, Hydroxy, C₁₋₆-Alkoxy oder Phenyl, und
C₁₋₁₂-Alkylen, C₂₋₁₂-Alkenylen oder C₃₋₁₂-Alkinylen, worin ein bis drei Methyleneinheiten, mit Ausnahme der (G)-terminalen Methylengruppe, im Fall, dass E eine Bindung darstellt, isosterisch ersetzt
sind durch O, S, NR¹⁰⁽ⁱ⁾, CO, SO oder SO₂, worin R¹⁰⁽ⁱ⁾ die gleiche Bedeutung wie R⁹⁽ⁱ⁾ hat, aber unabhängig davon ausgewählt ist;
E ausgewählt ist aus E¹⁽ⁱ⁾, E²⁽ⁱ⁾, E³, E⁴, E⁵ und E⁶, worin
E¹⁽ⁱ⁾ 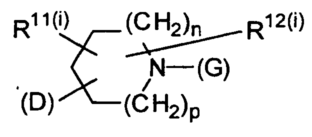 ist,
E²⁽ⁱ⁾ 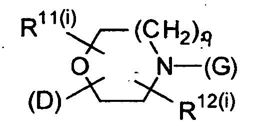 ist,
E³ 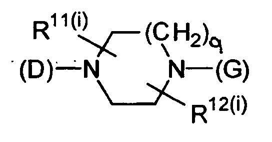 ist,
E⁴ 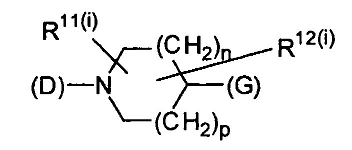 ist,
E⁵ 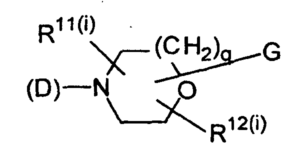 ist, und
E⁶ eine Einfach- oder Doppelbindung darstellt,
worin die heterocyclischen Ringe E¹⁽ⁱ⁾ bis E⁵ gegebenenfalls eine Doppelbindung aufweisen,
n und p unabhängig voneinander 0, 1, 2 oder 3 sind, mit dem Proviso, dass n + p ≤ 4,
q 1, 2 oder 3 ist;
R¹¹⁽ⁱ⁾ ausgewählt ist aus Wasserstoff, C₁₋₆-Alkyl, Hydroxy, Hydroxymethyl, Carboxy und C₂₋₇-Alkoxycarbonyl,
R¹²⁽ⁱ⁾ ausgewählt ist aus Wasserstoff, C₁₋₆-Alkyl und einer zu einem Stickstoffatom benachbarten Oxogruppe, oder
R¹¹⁽ⁱ⁾ und R¹²⁽ⁱ⁾ gegebenenfalls zusammen eine C₁₋₃-Alkylenbrücke unter Bildung eines bicyclischen Ringsystems bilden, und
(a) im Fall, dass E E¹⁽ⁱ⁾, E²⁽ⁱ⁾ oder E³ darstellt, der Substituent G gegebenenfalls ausgewählt ist aus G¹⁽ⁱ⁾, G²⁽ⁱ⁾, G³⁽ⁱ⁾, G⁴⁽ⁱ⁾ und G⁵⁽ⁱ⁾, worin
G¹⁽ⁱ⁾ 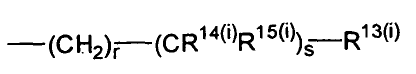 ist, worin
r 0 bis 3 ist und
s 0 oder 1 ist,
R¹³⁽ⁱ⁾ ausgewählt ist aus
Wasserstoff, C₁₋₆-Alkyl, C₃₋₆-Alkenyl, C₃₋₆-Alkinyl und C₃₋₈-Cycloalkyl;
gesättigten oder ungesättigten, 4- bis 7-gliedrigen Heterocyclen, die ein oder zwei Heteroatome, ausgewählt aus N, S und O, enthalten;
Benzyl, Phenyl;
monocyclischen, aromatischen, 5- oder 6-gliedrigen Heterocyclen, die ein bis drei Heteroatome, ausgewählt aus N, S und O, enthalten, und entweder direkt oder über eine Methylengruppe gebunden sind;
kondensierten, bi- und tricyclischen, aromatischen oder teilweise hydrierten, carbocyclischen Ringsystemen mit 8 bis 16, 17 oder 18 Ringatomen und mindestens einem aromatischen Ring, worin die Verknüpfung entweder über einen aromatischen oder einen hydrierten Ring und entweder direkt oder über eine Methylengruppe geschieht;
kondensierten, bi- und tricyclischen, aromatischen oder teilweise hydrierten, heterocyclischen Ringsystem mit 8 bis 16, 17 oder 18 Ringatomen, und mindestens einem aromatischen Ring, worin ein bis drei Ringatome ausgewählt sind aus N, S und O, und die Verknüpfung entweder über einen aromatischen oder einen hydrierten Ring und entweder direkt oder über eine Methylengruppe geschieht;
R¹⁴⁽ⁱ⁾ die gleiche Bedeutung wie R¹³⁽ⁱ⁾ aufweist, aber unabhängig davon ausgewählt ist;
R¹⁵⁽ⁱ⁾ ausgewählt ist aus
Wasserstoff, Hydroxy, Methyl, Benzyl, Phenyl,
monocyclischen, aromatischen, 5- oder 6-gliedrigen Heterocyclen, die ein bis drei Heteroatome, ausgewählt aus N, S und O, enthalten und entweder direkt oder über eine Methylengruppe gebunden sind;
kondensierten, bi- und tricyclischen, aromatischen oder teilweise hydrierten, carbocyclischen Ringsystemen mit 8 bis 16, 17 oder 18 Ringatomen und mindestens einem aromatischen Ring, worin die Verknüpfung entweder über einen aromatischen oder einen hydrierten Ring und entweder direkt oder über eine Methylengruppe geschieht;
kondensierten, bi- und tricyclischen, aromatischen oder teilweise hydrierten, heterocyclischen Ringsystemen mit 8 bis 16, 17 oder 18 Ringatomen und mindestens einem aromatischen Ring, worin ein bis drei Ringatome ausgewählt sind aus N, S und O, und die Verknüpfung entweder über einen aromatischen oder einen hydrierten Ring, und entweder direkt oder über eine Methylengruppe geschieht;
G²⁽ⁱ⁾ ausgewählt ist aus 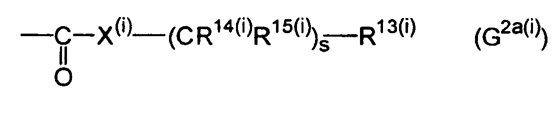 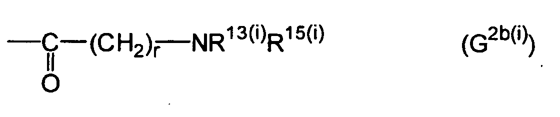 worin r, s und die Substituenten R¹³⁽ⁱ⁾, R¹⁴⁽ⁱ⁾ und R¹⁵⁽ⁱ⁾ die obigen Bedeutungen aufweisen,
oder die Gruppe
**-NR**^{**13(i)**}**R**^{**15(i)**}
ein über das Stickstoffatom gebundener stickstoffhaltiger Heterocyclus ist, wobei der stickstoffhaltige Heterocyclus ausgewählt ist aus
gesättigten und ungesättigten, monocyclischen, 4- bis 8-gliedrigen Heterocyclen, die neben dem essentiellen Stickstoffatom gegebenenfalls ein oder zwei weitere Heteroatome, ausgewählt aus N, S und O, enthalten,
und
gesättigten und ungesättigten, bi- oder tricyclischen, kondensierten oder überbrückten Heterocyclen mit 8 bis 16, 17 oder 18 Ringatomen, die neben dem essentiellen Stickstoffatom gegebenenfalls ein oder zwei weitere Heteroatome, ausgewählt aus N, S und O, enthalten,
X⁽ⁱ⁾ ausgewählt ist aus Methylen, Ethylen, Ethenylen, Propylen und C₃₋₇-Cycloalkylen oder eine Bindung darstellt;
G³⁽ⁱ⁾ 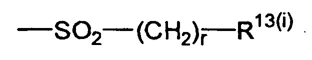 ist; worin r und R¹³⁽ⁱ⁾ die obigen Bedeutungen aufweisen,
G⁴⁽ⁱ⁾ 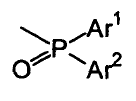 ist, worin Ar¹ und Ar² unabhängig voneinander ausgewählt sind aus Phenyl, Pyridyl und Naphthyl;
G⁵⁽ⁱ⁾
**-COR**^{**16(i)**}
ist, worin
R¹⁶⁽ⁱ⁾ ausgewählt ist aus Trifluormethyl, C₁₋₆-Alkoxy, C₃₋₆-Alkenyloxy und Benzyloxy,
(b) im Fall, dass E E⁴ oder E⁵ ist, dann G gegebenenfalls G¹⁽ⁱ⁾, G²⁽ⁱ⁾, G⁶⁽ⁱ⁾, G⁷ oder G⁸ ist,
worin G¹⁽ⁱ⁾ und G²⁽ⁱ⁾ die obigen Bedeutungen aufweisen und
G⁶⁽ⁱ⁾
**=(C)**_{**u**}**R**^{**13(i)**}**R**^{**15(i)**}
ist, worin R¹³⁽ⁱ⁾ und R¹⁵⁽ⁱ⁾ die obigen Bedeutungen aufweisen, und
u 0 oder 1 ist,
oder, wenn u = 1, dann bilden R¹³⁽ⁱ⁾ und R¹⁵⁽ⁱ⁾ zusammen mit dem Kohlenstoffatom, an das sie gebunden sind, ein Ringsystem, ausgewählt aus
C₃₋₈-Cycloalkyl,
gesättigten, 4- bis 7-gliedrigen Heterocyclen, die gegebenenfalls ein oder zwei Heteroatome, ausgewählt aus N, S und O, enthalten;
kondensierten, bi- und tricyclischen, teilweise hydrierten, carbocyclischen Ringsystemen mit 8 bis 16, 17 oder 18 Ringatomen und mindestens einem aromatischen Ring;
kondensierten, bi- und tricyclischen, teilweise hydrierten, heterocyclischen Ringsystemen mit 8 bis 16, 17 oder 18 Ringatomen und mindestens einem aromatischen Ring, worin ein bis drei Ringatome ausgewählt sind aus N, S und O;
oder, wenn u = 0, dann bilden R¹³⁽ⁱ⁾ und R¹⁵⁽ⁱ⁾ zusammen mit dem Kohlenstoffatom des Rings E, an das sie gebunden sind, ein Ringsystem, ausgewählt aus
C₃₋₈-Cycloalkyl,
gesättigten, 4- bis 7-gliedrigen Heterocyclen, die ein oder zwei Heteroatome, ausgewählt aus N, S und O, enthalten;
kondensierten, bi- und tricyclischen, teilweise hydrierten, carbocyclischen Ringsystemen mit 8 bis 16, 17 oder 18 Ringatomen und mindestens einem aromatischen Ring; und
kondensierten, bi- und tricyclischen, teilweise hydrierten, heterocyclischen Ringsystemen mit 8 bis 16, 17 oder 18 Ringatomen und mindestens einem aromatischen Ring, worin ein bis drei Ringatome ausgewählt sind aus N, S und O;
G⁷ ausgewählt ist aus 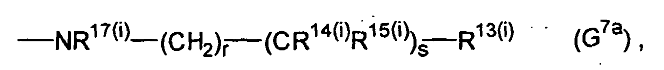 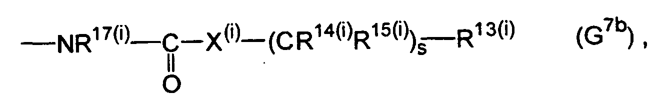 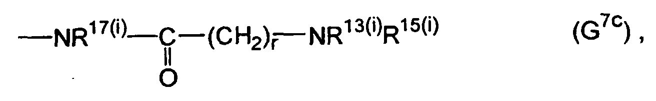 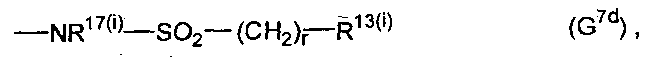 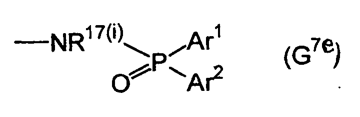 und
**-NR**^{**17(i)**}**-COR**^{**16(i)**} **(G**^{**7f**}**),**
worin r, s, X⁽ⁱ⁾, die Substituenten R¹³⁽ⁱ⁾, R¹⁴⁽ⁱ⁾, R¹⁵⁽ⁱ⁾ und R¹⁶⁽ⁱ⁾ und die Gruppe
**-NR**^{**13(i)**}**R**^{**15(i)**}
die obigen Bedeutungen aufweisen, und
R¹⁷⁽ⁱ⁾ die gleichen Bedeutungen wie R⁵⁽ⁱ⁾ aufweist, aber unabhängig davon ausgewählt ist,
Ar¹ und Ar² unabhängig voneinander ausgewählt sind aus Phenyl, Pyridyl und Naphthyl;
G⁸ ausgewählt ist aus 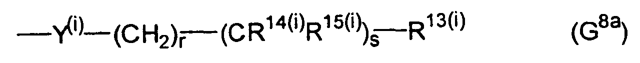 und 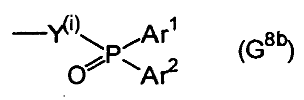 worin r, s und die Substituenten R¹³⁽ⁱ⁾, R¹⁴⁽ⁱ⁾, R¹⁵⁽ⁱ⁾, Ar¹ und Ar² die obigen Bedeutungen aufweisen, und
Y⁽ⁱ⁾ O oder S ist;
(c) im Fall, dass der Substituent E E⁶ ist, dann ist der Substituent G gegebenenfalls ausgewählt aus G^{7d}, G^{7e}, G^{8b}, G⁹, G¹⁰, G¹¹, G¹² und G¹³, worin G^{7d}, G^{7e} und G^{8b} die obigen Bedeutungen aufweisen, und
G⁹ ausgewählt ist aus 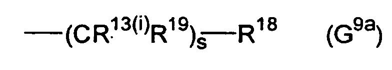 und
**-NR**^{**13(i)**}**R**^{**18**} **(G**^{**9b**}**)**
worin s und R¹³⁽ⁱ⁾ wie oben definiert sind, und
R¹⁸ ausgewählt ist aus
Benzyl, Diphenylmethyl, Phenyl;
monocyclischen, aromatischen, 5- und 6-gliedrigen Heterocyclen, die ein bis drei Heteroatome, ausgewählt aus N, S und O, enthalten können und entweder direkt oder über eine Methylengruppe gebunden sind;
kondensierten, bi- und tricyclischen, aromatischen oder teilweise hydrierten, carbocyclischen Ringsystemen mit 8 bis 16, 17 oder 18 Ringatomen und mindestens einem aromatischen Ring, worin die Verknüpfung entweder über einen aromatischen oder einen hydrierten Ring und entweder direkt oder über eine Methylengruppe geschieht; und
kondensierten, bi- und tricyclischen, aromatischen oder teilweise hydrierten, heterocyclischen Ringsystemen mit 8 bis 16, 17 oder 18 Ringatomen und mindestens einem aromatischen Ring, worin ein bis drei Ringatome ausgewählt sind aus N, S und O, und die Verknüpfung entweder über einen aromatischen oder einen hydrierten Ring und entweder direkt oder über eine Methylengruppe geschieht;
R¹⁹ die gleichen Bedeutungen wie R¹³⁽ⁱ⁾ aufweist, aber unabhängig davon ausgewählt ist, und zusätzlich Hydroxy sein kann;
oder die Gruppe
**-NR**^{**13(i)**}**R**^{**18**}
ist gegebenenfalls ein über das Stickstoffatom gebundener, stickstoffhaltiger Heterocyclus, wobei der stickstoffhaltige Heterocyclus ausgewählt ist aus
kondensierten, bi- und tricyclischen, aromatischen oder teilweise hydrierten, heterocyclischen Ringsystemen mit 8 bis 16, 17 oder 18 Ringatomen und mindestens einem aromatischen Ring, der neben dem essentiellen Stickstoffatom gegebenenfalls ein oder zwei weitere Heteroatome, ausgewählt aus N, S und O, enthält;
G¹⁰
**=CR**^{**13(i)**}**R**^{**18**} **(G**^{**10**}**)**
ist, die an D über eine Doppelbindung gebunden ist, worin R¹³⁽ⁱ⁾ und R¹⁸ die obigen Bedeutungen aufweisen oder worin G¹⁰ gegebenenfalls ein über das Kohlenstoffatom gebundenes Ringsystem ist, ausgewählt aus
kondensierten, bi- und tricyclischen, teilweise hydrierten, carbocyclischen Ringsystemen mit 8 bis 16, 17 oder 18 Ringatomen und mindestens einem aromatischen Ring; und
kondensierten, bi- und tricyclischen, teilweise hydrierten, heterocyclischen Ringsystemen mit 8 bis 16, 17 oder 18 Ringatomen und mindestens einem aromatischen Ring, worin ein bis drei Ringatome gegebenenfalls ausgewählt sind aus N, S und O;
G¹¹ ausgewählt ist aus 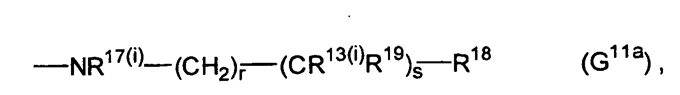 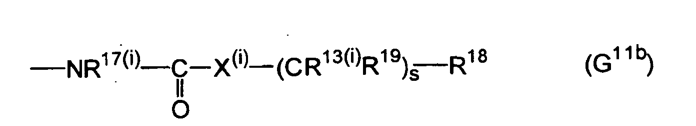 und 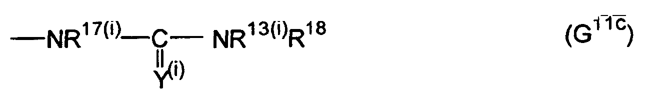 worin r, s, X⁽ⁱ⁾, Y⁽ⁱ⁾, die Substituenten R¹³⁽ⁱ⁾, R¹⁷⁽ⁱ⁾, R¹⁸ und R¹⁹ und die Gruppe -NR¹³⁽ⁱ⁾R¹⁸⁽ⁱ⁾ die obigen Bedeutungen aufweisen;
G¹² 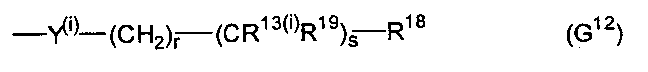 ist, worin r, s, Y⁽ⁱ⁾ und die Substituenten R¹³⁽ⁱ⁾, R¹⁸ und R¹⁹ die obigen Bedeutungen aufweisen;
G¹³ ausgewählt ist aus 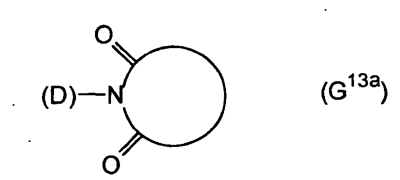 und 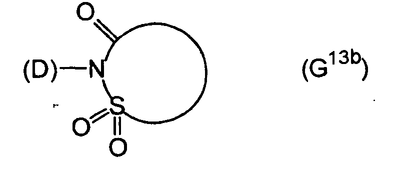 die an D über das Imidstickstoffatom gebunden sind, ausgewählt aus
gesättigten und ungesättigten monocyclischen Imiden mit 5 bis 7 Ringatomen, die, neben dem essentiellen Imidstickstoffatom, gegebenenfalls ein oder zwei weitere Heteroatome, ausgewählt aus N, S und O, enthalten;
gesättigten, ungesättigten und aromatischen, kondensierten, bi-, tri- oder tetracyclischen Imiden mit 8 bis 18 Ringatomen, die, neben dem essentiellen Imidstickstoffatom, gegebenenfalls ein oder zwei weitere Heteroatome, ausgewählt aus N, S und O, enthalten;
gesättigten und ungesättigten, überbrückten bi-, tri-, tetra- oder pentacyclischen Imiden mit 8 bis 22 Ringatomen, die, neben dem essentiellen Imidstickstoffatom, gegebenenfalls ein oder zwei weitere Heteroatome, ausgewählt aus N, S und O, enthalten; und
gesättigten und ungesättigten, spirocyclischen Imiden, gegebenenfalls ein- oder zweifach kondensiert und mit einer Gesamtzahl von 9 bis 23 Ringatomen, die, neben dem essentiellen Imidstickstoffatom, gegebenenfalls ein oder zwei weitere Heteroatome, ausgewählt aus N, S und O, enthalten,
worin diese cyclischen Imide gegebenenfalls durch ein bis fünf der gleichen oder unterschiedlichen Gruppen substituiert sind, unabhängig ausgewählt voneinander aus
Halogen, Cyano, C₁₋₆-Alkyl, C₁₋₆-Alkyliden, Trifluormethyl, C₃₋₈-Cycloalkyl, C₃₋₈-Cycloalkyliden, Phenyl-C₁₋₃-alkyl, Phenyl-C₁₋₃-alkyliden, Diphenyl-C₁₋₃-alkyl, Diphenyl-C₁₋₃-alkyliden, Triphenylmethyl, Phenyl, Hydroxy, C₁₋₆-Hydroxyalkyl, C₁₋₆-Alkoxy, C₁₋₆-Alkoxy, das vollständig oder teilweise durch Fluor substituiert ist, Benzyloxy, Phenoxy, Naphthyloxy, Mercapto, C₁₋₆-Alkylthio, Phenylthio, Naphthylthio, Pyridylthio, C₁₋₆-Alkansulfonyl, Phenylsulfonyl, Naphthylsulfonyl, Pyridylsulfonyl, Sulfo, Carboxy, C₂₋₇-Carboxyalkyl, C₃₋₇-Carboxyalkenyl, C₂₋₇-Alkoxycarbonyl, Benzyloxycarbonyl, Nitro, Amino, C₁₋₆-Aminoalkyl, Mono-C₁₋₆-alkylamino, Di(C₁₋₆-alkyl)amino, Phenylamino, Phenyl-C₁₋₃-alkylamino, Pyridylamino,
gesättigten und ungesättigten, 4- bis 7-gliedrigen Heterocyclen, die ein oder zwei Heteroatome, ausgewählt aus N, S und O, enthalten und entweder direkt oder über eine Methylengruppe oder eine Methingruppe gebunden sind,
monocyclischen, aromatischen, 5- und 6-gliedrigen Heterocyclen, die ein bis drei Heteroatome enthalten, ausgewählt aus N, S und O, und die entweder direkt oder über eine Methylengruppe oder eine Methingruppe gebunden sind,
kondensierten, bicyclischen, aromatischen und teilweise hydrierten, carbocyclischen Ringsystemen mit 8 bis 12 Ringatomen, die entweder direkt oder über eine Methylengruppe oder eine Methingruppe gebunden sind, und
kondensierten, bicyclischen, aromatischen und teilweise hydrierten, heterocyclischen Ringsystemen mit 8 bis 12 Ringatomen, worin ein bis drei Ringatome ausgewählt sind aus N, S und O, und die entweder direkt oder über eine Methylengruppe oder eine Methingruppe gebunden sind,
worin aromatische Ringsysteme in den Substituenten R¹⁽ⁱ⁾, R²⁽ⁱ⁾, R⁴⁽ⁱ⁾, R⁵⁽ⁱ⁾, R⁶⁽ⁱ⁾, R¹³⁽ⁱ⁾, R¹⁴⁽ⁱ⁾, R¹⁵⁽ⁱ⁾, R¹⁶⁽ⁱ⁾, R¹⁷⁽ⁱ⁾, R¹⁸, R¹⁹, Ar¹ und Ar², in den Gruppen A⁽ⁱ⁾ und D⁽ⁱ⁾, in den Ringsystemen =CR¹³⁽ⁱ⁾R¹⁵⁽ⁱ⁾, =CR¹³⁽ⁱ⁾R18, -NR¹³⁽ⁱ⁾R¹⁵⁽ⁱ⁾ und -NR¹³⁽ⁱ⁾R¹⁸⁽ⁱ⁾ sowie Substituenten und/oder Substituenten in den cyclischen Imiden G¹³ gegebenenfalls unabhängig voneinander substituiert sind durch ein bis drei der gleichen oder unterschiedlichen Gruppen, ausgewählt aus
Halogen, Cyano, C₁₋₆-Alkyl, Trifluormethyl, C₃₋₈-Cycloalkyl, Benzyl, Phenyl, Hydroxy, C₁₋₆-Hydroxyalkyl, C₁₋₆-Alkoxy, C₁₋₆-Alkoxy, das vollständig oder teilweise durch Fluor substituiert ist, Benzyloxy, Phenoxy, Mercapto, C₁₋₆-Alkylthio, Phenylthio, Sulfo, Carboxy, C₂₋₇-Carboxyalkyl, C₃₋₇-Carboxyalkenyl, C₂₋₇-Alkoxycarbonyl, Benzyloxycarbonyl, Nitro, Amino, C₁₋₆-Aminoalkyl, Mono-C₁₋₆-alkylamino und Di(C₁₋₆-alkyl)amino, und im Fall von zwei benachbarten Resten am aromatischen Ring, Methylendioxy,
worin Alkyl- und Cycloalkylreste in den Gruppen G gegebenenfalls durch ein oder zwei der gleichen oder verschiedenen Gruppen substituiert sind, ausgewählt aus
Hydroxy, Carboxy, C₂₋₇-Alkoxycarbonyl, Benzyloxycarbonyl, Amino, Mono-C₁₋₆-alkylamino und Di(C₁₋₆-alkyl)amino;
und die Stereoisomere oder racemische oder nichtracemische Mischungen von Stereoisomeren davon,
und die Tautomere davon, wenn G ein heterocyclischer aromatischer Ring oder ein aromatischer Ring, substituiert durch eine Hydroxy-, Mercapto- oder Aminogruppe, ist,
und die pharmakologisch annehmbaren Säureadditionssalze davon.

16. Verwendung gemäss Anspruch 15, worin die Verbindung der Formel (I) ausgewählt ist aus der Gruppe bestehend aus:
N-[2-(1-Benzylpiperidin-4-yl)-ethyl]-3-(pyridin-3-yl)-propionamid;
N-{2-[1-(2-Phenylethyl)-piperidin-4-yl]-ethyl}-3-(pyridin-3-yl)-propionamid;
N-{2-[1-(4-Phenylbutyl)-piperidin-4-yl]-ethyl}-3-(pyridin-3-yl)-propionamid;
N-{2-[1-(4-Hydroxy-4-phenylbutyl)-piperidin-4-yl]-ethyl}-3-(pyridin-3-yl)-propionamid;
N-[2-(1-Diphenylmethylpiperidin-4-yl)-ethyl]-3-(pyridin-3-yl)-propionamid;
N-[3-(1-Diphenylmethylpiperidin-4-yl)-propyl]-3-(pyridin-3-yl)-propionamid;
N-[4-(1-Diphenylmethylpiperidin-4-yl)-butyl]-3-(pyridin-3-yl)-propionamid;
N-[4-(1-Benzylpiperidin-4-yl)-butyl]-3-(pyridin-3-yl)-acrylamid;
N-{4-[1-(2-Phenylethyl)-piperidin-4-yl]-butyl}-3-(pyridin-3-yl)-acrylamid;
N-{4-[1-(4-Biphenylylmethyl)-piperidin-4-yl]-butyl}-3-(pyridin-3-yl)-acrylamid;
N-{4-[1-(1-Naphthylmethyl)-piperidin-4-yl]-butyl}-3-(pyridin-3-yl)-acrylamid;
N-{4-[1-(9-Anthrylmethyl)-piperidin-4-yl]-butyl}-3-(pyridin-3-yl)-acrylamid;
N-{4-[1-(Cyclohexylphenylmethyl)-piperidin-4-yl]-butyl}-3-(pyridin-3-yl)-acrylamid;
N-{4-[1-(10,11-Dihydro-5H-dibenzo[a,d]cyclohepten-5-yl)-piperidin-4-yl]-butyl}-3-(pyridin-3-yl)-acrylamid;
N-[2-(1-Diphenylmethylpiperidin-4-yl)-ethyl]-3-(pyridin-3-yl)-acrylamid;
N-[3-(1-Diphenylmethylpiperidin-4-yl)-propyl]-3-(pyridin-3-yl)-acrylamid;
N-[5-(1-Diphenylmethylpiperidin-4-yl)-pentyl]-3-(pyridin-3-yl)-acrylamid;
N-[6-(1-Diphenylmethylpiperidin-4-yl)-hexyl]-3-(pyridin-3-yl)-acrylamid;
N-[4-(1-Diphenylmethylpiperidin-4-yl)-butyl]-5-(pyridin-3-yl)-2,4-pentadiensäureamid;
N-(4-{1-[Bis-(4-fluorphenyl)-methyl]-piperidin-4-yl}-butyl)-3-(pyridin-3-yl)-acrylamid;
N-(4-{1-[Bis-(2-chlorphenyl)-methyl]-piperidin-4-yl}-butyl)-3-(pyridin-3-yl)-acrylamid;
N-[4-(1-Diphenylmethylpiperidin-4-yl)-butyl]-3-(2-fluorpyridin-3-yl)-acrylamid;
N-[4-(1-Diphenylmethylpiperidin-4-yl)-butyl]-3-(6-fluorpyridin-3-yl)-acrylamid;
N-[4-(1-Diphenylmethylpiperidin-4-yl)-butyl]-3-(pyridin-3-yl)-acrylamid;
N-[4-(1-Diphenylmethylpiperidin-4-yl)-butyl]-3-(pyridin-3-yl)-acrylamid·Dihydrochlorid;
N-[4-(1-Diphenylmethylpiperidin-4-yl)-butyl]-3-(pyridin-3-yl)-acrylamid·Methansulfonat;
N-[4-(1-Acetyl-piperidin-4-yl) -butyl]-3-(pyridin-3-yl)-propionamid;
N-[4-(1-Benzoyl-piperidin-4-yl)-butyl]-3-(pyridin-3-yl)-propionamid;
N-[4-(1-Diphenylacetyl-piperidin-4-yl)-butyl]-3-(pyridin-3-yl)-propionamid;
N-{4-[1-(9-Oxo-9H-fluoren-4-carbonyl)-piperidin-4-yl]-butyl}-3-(pyridin-3-yl)-propionamid;
N-[4-(1-Methylsulfonyl-piperidin-4-yl)-butyl]-3-(pyridin-3-yl)-propionamid;
N-{4-[1-(2-Naphthyl-sulfonyl)-piperidin-4-yl]-butyl}-3-(pyridin-3-yl)-propionamid;
N-[4-(1-Benzyl-piperidin-4-yl)-butyl]-3-(pyridin-3-yl)-propionamid;
N-(4-{1-[Bis-(2-chlorphenyl)-methyl]-piperidin-4-yl}-butyl)-3-(pyridin-3-yl)-propionamid;
N-{4-[1-(Phenylpyridin-3-yl-methyl)-piperidin-4-yl]-butyl}-3-(pyridin-3-yl)-propionamid;
N-{4-[1-(9H-Fluoren-9-yl)-piperidin-4-yl]-butyl}-3-(pyridin-3-yl)-propionamid;
N-{4-[1-(6,11-Dihydrodibenzo[b,e]oxepin-11-yl)-piperidin-4-yl]-butyl}-3-(pyridin-3-yl)-propionamid;
N-{4-[1-(1-Naphthylamino-carbonyl)-piperidin-4-yl]-butyl}-3-(pyridin-3-yl)-propionamid;
N-[4-(1-Diphenylamino-carbonyl-piperidin-4-yl)-butyl]-3-(pyridin-3-yl)-propionamid;
N-{4-[1-(10,11-Dihydro-dibenzo[b,f]azepin-5-yl-carbonyl)-piperidin-4-yl]-butyl}-3-(pyridin-3-yl)-propionamid;
N-[4-(1-Diphenylphosphinoyl-piperidin-4-yl)-butyl]-3-(pyridin-3-yl)-propionamid;
N-[4-(1-Diphenylmethylpiperidin-4-yl)-butyl]-3-(2-fluorpyridin-3-yl)-propionamid;
N-[4-(1-Diphenylmethylpiperidin-4-yl)-butyl]-3-(5-fluorpyridin-3-yl)-propionamid;
N-[4-(1-Diphenylmethylpiperidin-4-yl)-butyl]-2-fluor-3-(pyridin-3-yl)-propionamid;
N-[4-(1-Diphenylmethylpiperidin-4-yl)-butyl]-2,2-difluor-3-(pyridin-3-yl)-propionamid;
N-[5-(1-Diphenylmethylpiperidin-4-yl)-pentyl]-3-(pyridin-3-yl)-propionamid;
N-[6-(1-Diphenylmethylpiperidin-4-yl)-hexyl]-3-(pyridin-3-yl)-propionamid;
N-[2-(1-Diphenylmethylpiperidin-4-yl)-ethyl]-5-(pyridin-3-yl)-pentansäureamid;
N-[4-(1-Diphenylmethylpiperidin-4-yl)-butyl]-5-(pyridin-3-yl)-pentansäureamid; .
N-[4-(1-Diphenylmethylpiperidin-4-yl)-butyl]-N-hydroxy-3-(pyridin-3-yl)-propionamid;
N-[4-(1-Diphenylmethylpiperidin-4-yl)-butyl]-2-hydroxy-3-(pyridin-3-yl)-propionamid;
N-[4-(1-Diphenylmethylpiperidin-4-yl)-butyl]-3-hydroxy-3-(pyridin-3-yl)-propionamid;
N-[4-(1-Diphenylmethylpiperidin-4-yl)-butyl]-3-(pyridin-3-yl)-propionamid;
N-[4-(1-Methylsulfonylpiperidin-4-yl)-butyl]-3-(pyridin-3-yl)-acrylamid;
N-{4-[1-(2-Naphthylsulfonyl)-piperidin-4-yl]-butyl}-3-(pyridin-3-yl)-acrylamid;
N-{4-[1-(2-Naphthylsulfonyl)-piperidin-4-yl]-butyl}-5-(pyridin-3-yl)-2,4-pentadiensäureamid;
N-{4-[1-(1-Naphthylaminocarbonyl)-piperidin-4-yl]-butyl}-3-(pyridin-3-yl)-acrylamid;
N-[4-(1-Diphenylaminocarbonylpiperidin-4-yl)-butyl]-3-(pyridin-3-yl)-acrylamid;
N-[4-(1-Diphenylaminocarbonyl-piperidin-4-yl)-butyl]-5-(pyridin-3-yl)-2,4-pentadiensäureamid;
N-{4-[1-(10,11-Dihydrodibenzo[b,f]azepin-5-yl-carbonyl)-piperidin-4-yl]-butyl}-3-(pyridin-3-yl)-acrylamid;
N-[4-(1-Diphenylphosphinoyl-piperidin-4-yl)-butyl]-3-(pyridin-3-yl)-acrylamid;
N-[4-(1-Acetylpiperidin-4-yl)-butyl]-3-(pyridin-3-yl)-acrylamid;
N-[4-(1-Diphenylacetylpiperidin-4-yl)-butyl]-3-(pyridin-3-yl)-acrylamid;
N-{4-[1-(3,3-Diphenylpropionyl)-piperidin-4-yl]-butyl}-3-(pyridin-3-yl)-acrylamid;
N-[4-(1-Benzoylpiperidin-4-yl)-butyl]-3-(pyridin-3-yl)-acrylamid;
N-[4-(1-Benzoylpiperidin-4-yl)-butyl]-5-(pyridin-3-yl)-2,4-pentadiensäureamid;
N-{4-[1-(9-Oxo-9H-fluoren-4-yl-carbonyl)-piperidin-4-yl]-butyl}-3-(pyridin-3-yl)-acrylamid;
N-{4-[1-(Phenylpyridin-3-yl-methyl)-piperidin-4-yl]-butyl}-3-(pyridin-3-yl)-acrylamid;
N-{4-[1-(Phenylpyridin-4-yl-methyl)-piperidin-4-yl]-butyl}-3-(pyridin-3-yl)-acrylamid;
N-{4-[1-(6,11-Dihydrodibenzo[b,e]oxepin-11-yl)-piperidin-4-yl]-butyl}-3-(pyridin-3-yl)-acrylamid;
N-{4-[1-(6,11-Dihydrodibenzo[b,e]thiepin-11-yl)-piperidin-4-yl]-butyl}-3-(pyridin-3-yl)-acrylamid;
N-[7-(1-Diphenylmethylpiperidin-4-yl)-heptyl]-3-(pyridin-3-yl)-acrylamid;
N-[8-(1-Diphenylmethylpiperidin-4-yl)-octyl]-3-(pyridin-3-yl)-acrylamid;
N-[3-(1-Diphenylmethylpiperidin-4-yloxy)-propyl]-3-(pyridin-3-yl)-acrylamid;
N-[3-(1-Benzylpiperidin-4-yloxy)-propyl]-3-(pyridin-3-yl)-acrylamid;
N-[2-(1-Diphenylmethylpiperidin-4-yl)-ethyl]-5-(pyridin-3-yl)-2,4-pentadiensäureamid;
N-[4-(1-Diphenylmethylpiperidin-4-yl)-butyl]-5-(pyridin-3-yl)-2,4-pentadiensäureamid;
N-[5-(1-Diphenylmethylpiperidin-4-yl)-pentyl]-5-(pyridin-3-yl)-2,4-pentadiensäureamid;
N-[6-(1-Diphenylmethylpiperidin-4-yl)-hexyl]-5-(pyridin-3-yl)-2,4-pentadiensäureamid;
N-[4-(4-Diphenylmethylpiperazin-1-yl)-3-hydroxy-butyl]-3-pyridin-3-yl-acrylamid;
N-[3-(4-Diphenylmethylpiperazin-1-yl)-propoxy]-3-pyridin-3-yl-acrylamid;
N-[4-(4-Diphenylmethylpiperazin-1-yl)-4-oxo-butyl]-3-pyridin-3-yl-acrylamid;
N-[3-(4-Diphenylmethylpiperazin-1-sulfonyl)-propyl]-3-pyridin-3-yl-acrylamid;
N-{2-[2-(4-Diphenylmethylpiperazin-1-yl)-ethoxy]-ethyl}-3-pyridin-3-yl-acrylamid;
N-(4-{4-[Bis-(4-fluorphenyl)-methyl]-piperazin-1-yl}-but-2-inyl)-3-pyridin-3-yl-acrylamid;
N-{4-[4-(4-Carboxyphenyl-phenylmethyl)-piperazin-1-yl]-butyl}-3-pyridin-3-yl-acrylamid;
N-(4-{4-[(4-Aminophenyl)-phenylmethyl]-piperazin-1-yl}-butyl)-3-pyridin-3-yl-acrylamid;
N-{4-[4-(9H-Fluoren-9-yl)-piperazin-1-yl]-butyl}-2-(pyridin-3-yloxy)-acetamid;
N-{5-[4-(9H-Fluoren-9-yl)-piperazin-1-yl]-pentyl}-3-pyridin-3-yl-acrylamid;
N-{6-[4-(9H-Fluoren-9-yl)-piperazin-1-yl]-hexyl}-3-pyridin-3-yl-acrylamid;
3-Pyridin-3-yl-N-{4-[4-(1,2,3,4-tetrahydronaphthalin-1-yl)-piperazin-1-yl]-butyl}-acrylamid;
3-Pyridin-3-yl-N-{4-[4-(5,6,7,8-tetrahydronaphthalin-1-yl)-piperazin-1-yl]-butyl}-acrylamid;
N-{4-[4-(Naphthalin-1-yl)-piperazin-1-yl]-butyl}-3-pyridin-3-yl-acrylamid;
N-[4-(4-Biphenyl-2-yl-piperazin-1-yl)-butyl]-3-pyridin-3-yl-propionamid;
N-[5-(4-Biphenyl-2-yl-piperazin-1-yl)-pentyl]-3-pyridin-3-yl-acrylamid;
N-[6-(4-Biphenyl-2-yl-piperazin-1-yl)-hexyl]-3-pyridin-3-yl-acrylamid;
N-[4-(4-Biphenyl-2-yl-piperazin-1-ly)-butyl]-2-(pyridin-3-yloxy)-acetamid;
N-[4-(4-Biphenyl-2-yl-piperazin-1-yl)-butyl]-5-(pyridin-3-yl)-penta-2,4-diensäureamid;
N-{4-[4-(10,11-Dihydro-5H-dibenzo[a,d]cyclohepten-5-yl)-piperazin-1-yl]-butyl}-3-pyridin-3-yl-propionamid;
N-{5-[4-(10,11-Dihydro-5H-dibenzo[a,d]cyclohepten-5-yl)-piperazin-1-yl]-pentyl}-3-pyridin-3-yl-acrylamid;
N-{6-[4-(10,11-Dihydro-5H-dibenzo[a,d]cyclohepten-5-yl)-piperazin-1-yl]-hexyl}-3-pyridin-3-yl-acrylamid;
N-{4-[4-(10,11-Dihydro-5H-dibenzo[a,d]cyclohepten-5-yl)-piperazin-1-yl]-butyl}-5-(pyridin-3-yl)-penta-2,4-diensäureamid;
N-{4-[4-(6,11-Dihydro-dibenzo[b,e]oxepin-11-yl)-piperazin-1-yl]-butyl-3-pyridin-3-yl-propionamid;
N-{2-[4-(6,11-Dihydro-dibenzo[b,e]thiepin-11-yl)-piperazin-1-yl]-ethyl}-3-pyridin-3-yl-acrylamid;
N-[4-(4-Diphenylacetylpiperazin-1-yl)-butyl]-3-pyridin-3-yl-acrylamid;
N-[4-(4-Benzoylpiperazin-1-yl)-butyl]-3-pyridin-3-yl-acrylamid;
N-{4-[4-(2-Aminobenzoyl)-piperazin-1-yl]-butyl}-3-pyridin-3-yl-acrylamid;
N-{4-[4-(4-Carboxybenzoyl)-piperazin-1-yl]-butyl}-3-pyridin-3-yl-acrylamid;
N-{4-[4-(Biphenyl-2-carbonyl)-piperazin-1-yl]-butyl}-3-pyridin-3-yl-acrylamid;
N-{4-[4-(9-Oxo-9H-fluoren-4-carbonyl)-piperazin-1-yl]-butyl}-3-pyridin-3-yl-acrylamid;
N-{4-[4-(Furan-2-carbonyl)-piperazin-1-yl]-butyl}-3-pyridin-3-yl-acrylamid;
N-{4-[4-(Naphthalin-1-yl-aminocarbonyl)-piperazin-1-yl]-butyl}-3-pyridin-3-yl-propionamid;
N-{4-[4-(Diphenylaminocarbonyl)-piperazin-1-yl]-butyl}-3-pyridin-3-yl-acrylamid;
N-{4-[4-(Naphthalin-2-sulfonyl)-piperazin-1-yl]-butyl}-3-pyridin-3-yl-acrylamid;
N-[4-(4-Diphenylphosphinonyl-piperazin-1-yl)-butyl]-3-pyridin-3-yl-acrylamid;
N-[4-(4-Biphenyl-2-yl-piperazin-1-yl)-butyl]-3-pyridin-3-yl-acrylamid;
N-{4-[4-(9H-Fluoren-9-yl)-piperazin-1-yl]-butyl}-3-pyridin-3-yl-acrylamid;
N-{4-[4-(10,11-Dihydro-5H-dibenzo[a,d]cyclohepten-5-yl)-piperazin-1-yl]-butyl}-3-pyridin-3-yl-acrylamid;
N-[4-(4-Phenylpiperidin-1-yl)-butyl]-3-pyridin-3-yl-acrylamid;
N-{4-[4-(1H-Indol-3-yl)-piperidin-1-yl]-butyl}-3-pyridin-3-yl-acrylamid;
N-{4-[4-(2-Oxo-2,3-dihydrobenzimidazol-1-yl)-piperidin-1-yl]-butyl}-3-pyridin-3-yl-acrylamid;
N-[4-(4-Benzotriazol-1-yl-piperidin-1-yl)-butyl]-3-pyridin-3-yl-acrylamid;
N-{4-[4-(Hydroxy-diphenylmethyl)-piperidin-1-yl]-butyl}-2-(pyridin-3-yloxy)-acetamid;
N-[4-(4,4-Diphenylpiperidin-1-yl)-butyl]-3-pyridin-3-yl-acrylamid;
N-{4-[4-(6,11-Dihydrodibenzo[b,e]thiepin-11-yliden)-piperidin-1-yl]-butyl}-3-pyridin-3-ylpropionamid·Dihydrochlorid/Semiisopropanol;
N-{4-[4-(6,11-Dihydrodibenzo[b,e]thiepin-11-yliden)-piperidin-1-yl]-butyl}-5-pyridin-3-yl-pentanamid;
N-{4-[4-(4,9-Dihydro-thieno[2,3-b]-benzo[e]thiepin-4-yliden)-piperidin-1-yl]-butyl}-3-pyridin-3-yl-propionamid;
N-{4-[4-(4,9-Dihydro-thieno[2,3-b]-benzo[e]thiepin-4-yliden)-piperidin-1-yl]-butyl}-3-pyridin-3-yl-acrylamid;
N-[4-(4-Diphenylphosphinoyloxypiperidin-1-yl)-butyl]-3-pyridin-3-yl-acrylamid;
N-[4-(1,4-Dioxa-8-azaspiro[4,5]dec-8-yl)-butyl]-3-pyridin-3-yl-acrylamid;
N-[4-(2,5-Dioxo-3,4-diphenyl-2,5-dihydropyrrol-1-yl)-butyl]-3-pyridin-3-yl-acrylamid;
N-[4-(2,6-Dioxo-4-phenylpiperidin-1-yl)-butyl]-3-pyridin-3-yl-acrylamid;
N-[4-(1,3-Dioxo-4,5,6,7-tetraphenyl-1,3-dihydroisoindol-2-yl)-butyl]-3-pyridin-3-yl-acrylamid;
N-[4-(3-Benzyl-2,4,5-trioxo-imidazolidin-1-yl)-butyl]-3-pyridin-3-yl-acrylamid;
N-[4-(1,3,10-Trioxo-1,4,5,6,10,10a-hexahydroacenaphtho[1,8a-c]pyrrol-2-yl)-butyl]-3-pyridin-3-yl-acrylamid;
N-[4-(2,5-Dioxo-4,4-diphenylimidazolidin-1-yl)-butyl-3-pyridin-3-yl-acrylamid;
N-[4-(2,5-Dioxo-3-phenyl-2,5-dihydropyrrol-1-yl)-butyl]-3-pyridin-3-yl-acrylamid;
N-[3-(2,5-Dioxo-3,4-diphenyl-2,5-dihydro-pyrrol-1-yl)-propyl]-3-pyridin-3-yl-acrylamid;
N-[4-(3-Pyridin-3-yl-acroylamino)-butyl]-2,3:5,6-dibenzobicyclo[2.2.2]octan-7,8-dicarbonsäureimid;
N-[4-(5-Benzyliden-2,4-dioxothiazolidin-3-yl)-butyl]-3-pyridin-3-yl-acrylamid;
N-[4-(4-Benzyl-2,6-dioxopiperazin-1-yl)-butyl]-3-pyridin-3-yl-acrylamid;
N-[6-(2,5-Dioxo-3,4-diphenyl-2,5-dihydropyrrol-1-yl)-hexyl]-3-pyridin-3-yl-acrylamid;
N-[4-(2,5-Dioxo-3,4-diphenyl-2,5-dihydropyrrol-1-yl)-butyl]-3-pyridin-3-yl-propionamid;
N-[4-(1,3-Dioxo-1,3-dihydro-isoindol-2-yl)-butyl]-3-pyridin-3-yl-acrylamid;
N-[4-(1,3-Dioxo-1H,3H-benzo[de]isochinolin-2-yl)-butyl]-3-(1-oxidopyridin-3-yl)-acrylamid;
N-[6-(1,3-Dioxo-1H,3H-benzo[de]isochinolin-2-yl)-hexyl]-3-pyridin-3-yl-acrylamid;
N-[2-(1,3-Dioxo-1H,3H-benzo[de]isochinolin-2-yl)-ethyl]-3-pyridin-3-yl-acrylamid;
N-[4-(1,3-Dioxo-1H,3H-benzo[de]isochinolin-2-yl)-butyl]-3-pyridin-3-yl-acrylamid;
N-[8,8-Bis-(4-fluorphenyl)-octyl]-3-pyridin-3-yl-acrylamid-Hydrochlorid;
N-[6-(3,3-Diphenylureido)-hexyl]-3-pyridin-3-yl-acrylamid;
N-[4-(1-Phenyl-1,2,4,5-tetrahydrobenzo[d]azepin-3-yl)-butyl]-3-pyridin-3-yl-acrylamid;
N-(8,8-Diphenyloctyl)-3-pyridin-3-yl-acrylamid; N-(8-Hydroxy-8,8-diphenyloctyl)-3-pyridin-3-yl-acrylamid;
N-[4-(3,3-Diphenylureido)-butyl]-3-pyridin-3-yl-acrylamid;
N-[4-(1H,3H-Benzo[de]isochinolin-2-yl)-butyl]-3-pyridin-3-yl-acrylamid;
N-[6-(10,11-Dihydro-dibenzo[b,f]azepin-5-yl-carbonylamino)-hexyl]-3-pyridin-3-yl-acrylamid;
3-Pyridin-3-yl-N-[6-(tosylamino)-hexyl]-acrylamid;
N-[4-(1,1-Dioxo-1-thia-2-aza-acenaphthylen-2-yl)-butyl]-3-pyridin-3-yl-acrylamid;
N-(6-Hydroxy-6,6-diphenylhexyl)-3-pyridin-3-yl-acrylamid;
N-(6,6-Diphenyl-hex-5-enyl)-3-pyridin-3-yl-acrylamid;
N-[4-(4,5-Diphenylimidazol-1-yl)-butyl]-3-pyridin-3-yl-acrylamid;
N-[4-(trans-2-Phenylcyclopropylcarbonylamino)-butyl]-3-pyridin-3-yl-acrylamid;
N-(5-Hydroxy-5,5-diphenyl-pentyl)-3-pyridin-3-yl-acrylamid;
N-(7-Phenylheptyl)-3-pyridin-3-yl-acrylamid;
N-(4-Diphenylacetylamino-butyl)-3-pyridin-3-yl-acrylamid;
N-[4-(Benzhydrylamino)-butyl]-3-pyridin-3-yl-acrylamid und
N-(4-{[2-(Benzhydrylmethylamino)-ethyl]-methylamino}-butyl)-3-pyridin-3-yl-acrylamid.

17. Verwendung gemäss Anspruch 15 oder 16, worin zusätzlich zu dem prophylaktisch oder therapeutisch verabreichten cancerostatischen oder immunsuppressiven Mittel gemäss Formel (I) ein weiteres, davon verschiedenes, cancerostatisches oder immunsuppressives Mittel verabreicht wird.

## Revendications

1. Composition pharmaceutique comprenant :
(a) au moins un agent cancérostatique ou immunosuppresseur choisi dans le groupe constitué par les composés de formule (I) : 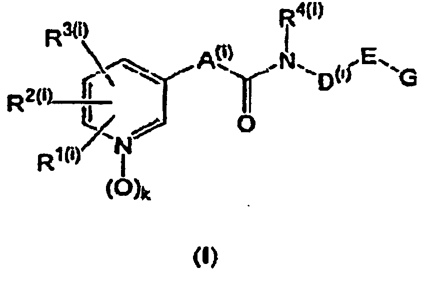 dans laquelle :
R¹⁽ⁱ⁾ est choisi parmi
un hydrogène, un halogène, un cyano, un alkyle en C₁-C₆, un alcényle en C₃-C₆, un alcynyle en C₂-C₆, un trifluorométhyle, un hydroxy, un cycloalkyle en C₃-C₈, un hydroxyalkyle en C₁-C₆, un alcoxy en C₁-C₆, un alcényloxy en C₃-C₆, un alcynyloxy en C₃-C₆, un benzyloxy, un alcanoyloxy en C₁-C₇, un alcoxycarbonyloxy en C₁-C₇, un alkylthio en C₁-C₆, un alcénylthio en C₃-C₆, un alcynylthio en C₃-C₆, un cycloalkyloxy en C₃-C₈, un cycloalkylthio en C₃-C₈, un alcoxycarbonyle en C₂-C₇, un aminocarbonyle, un alkylaminocarbonyle en C₂-C₇, un dialkylaminocarbonyle en C₃-C₁₃, un carboxy, un phényle, un phénoxy, un phénylthio, un pyridyloxy, un pyridylthio et NR⁵⁽ⁱ⁾R⁶⁽ⁱ⁾, où R⁵⁽ⁱ⁾ et R⁶⁽ⁱ⁾ sont choisis indépendamment l'un de l'autre parmi un hydrogène, un alkyle en C₁-C₆, un alcényle en C₃-C₆, un alcynyle en C₃-C₆, un benzyle et un phényle ;
R²⁽ⁱ⁾ est choisi parmi
un hydrogène, un halogène, un cyano, un alkyle en C₁-C₆, un trifluorométhyle, un hydroxy, un alcoxy en C₁-C₆, un benzyle et un alcanoyloxy en C₁-C₆ ; ou
R¹⁽ⁱ⁾ et R²⁽ⁱ⁾ lorsqu'ils sont adjacents forment éventuellement un pont choisi parmi
-(CH₂)₄-, -(CH=CH)₂- et -CH₂O-CR⁷⁽ⁱ⁾R⁸⁽ⁱ⁾-O-, où
R⁷⁽ⁱ⁾ et R⁸⁽ⁱ⁾ sont choisis indépendamment l'un de l'autre parmi un hydrogène et un alkyle en C₁-C₆ ;
R³⁽ⁱ⁾ est choisi parmi
un hydrogène, un halogène, un alkyle en C₁-C₆, un trifluorométhyle et un hydroxyalkyle en C₁-C₆ ;
R⁴⁽ⁱ⁾ est choisi parmi
un hydrogène, un hydroxy, un alkyle en C₁-C₆, un alcényle en C₃-C₆, un alcynyle en C₃-C₆, un cycloalkyle en C₃-C₆, un alcoxy en C₁-C₆ et un benzyloxy ;
k est 0 ou 1,
A⁽ⁱ⁾ est choisi parmi
un alkylène en C₁-C₆, éventuellement substitué une à trois fois par un alkyle en C₁-C₆, un alcoxy en C₁-C₃, hydroxy, un fluor, ou un phényle,
un alkylène en C₂-C₆, où un motif méthylène est remplacé de façon isostérique par O, S, NR⁹⁽ⁱ⁾, CO, SO ou SO₂ où, à l'exception de CO, la substitution isostérique n'est pas adjacente au groupe amide, et R⁹⁽ⁱ⁾ est choisi parmi un hydrogène, un alkyle en C₁-C₆, un alcényle en C₃-C₆, un alcynyle en C₃-C₆, un acyle en C₃-C₆ et un alcanesulfonyle en C₁-C₆,
le 1,2-cyclopropylène,
un alcénylène en C₂-C₆, éventuellement substitué une à trois fois par un alkyle en C₁-C₆, un hydroxy, un alcoxy en C₁-C₃, un fluor, un cyano ou un phényle,
un alcadiénylène en C₄-C₆, éventuellement substitué une fois ou deux fois par un alkyle en C₁-C₃, un fluor, un cyano ou un phényle,
un 1,3,5-hexatriénylène, éventuellement substitué par un alkyle en C₁-C₆, un fluor, un cyano ou un phényle, et
l'éthynylène ;
D⁽ⁱ⁾ est choisi parmi
un alkylène en C₁-C₁₂, éventuellement substitué une fois ou deux fois par un alkyle en C₁-C₆, un hydroxy, un alcoxy en C₁-C₆ ou un phényle,
un alcénylène en C₂-C₁₂ ou alcadiénylène en C₄-C₁₂, éventuellement substitué une fois ou deux fois par un alkyle en C₁-C₆, un hydroxy, un alcoxy en C₁-C₆ ou un phényle, où une double liaison peut éventuellement se présenter au cycle E dans le cas où le cycle E est lié sur un atome de C,
un alcynylène en C₃-C₁₂ ou alcénynylène en C₄-C₁₂, éventuellement substitué une fois ou deux fois par un alkyle en C₁-C₆, un hydroxy, un alcoxy en C₁-C₆ ou un phényle, et
un alkylène en C₁-C₁₂, alcénylène en C₂-C₁₂ ou alcynylène en C₃-C₁₂, où un à trois motifs méthylène, à l'exception du groupe méthylène (G)-terminal, dans le cas où E représente une liaison, sont remplacés de façon isostérique par O, S, NR¹⁰⁽ⁱ⁾, CO, SO ou SO₂, où R¹⁰⁽ⁱ⁾ a la même signification que R⁹⁽ⁱ⁾, mais est choisi indépendamment de celui-ci ;
E est choisi parmi E¹⁽ⁱ⁾, E²⁽ⁱ⁾, E³, E⁴, E⁵ et E⁶, où
E¹⁽ⁱ⁾ est 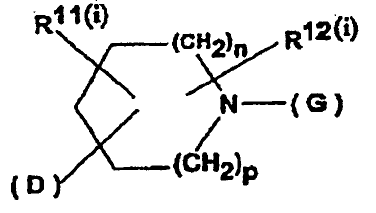
E²⁽ⁱ⁾ est 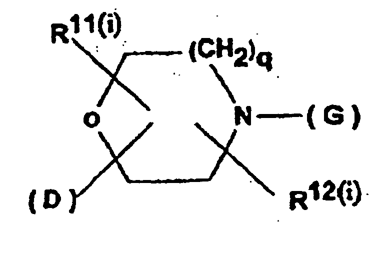
E³ est 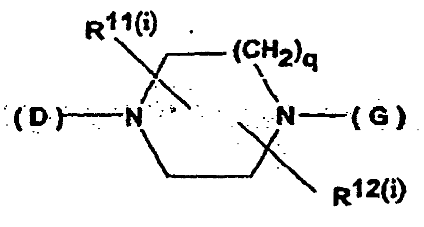
E⁴ est 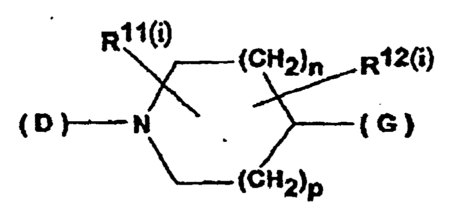
E⁵ est 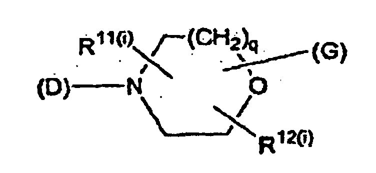 et
E⁶ représente une liaison simple ou double,
où les noyaux hétérocycliques E¹⁽ⁱ⁾ à E⁵ ont éventuellement une double liaison,
n et p sont, indépendamment l'un de l'autre, 0, 1, 2 ou 3 , à condition que n + p ≤ 4,
q est 1, 2 ou 3 ;
R¹¹⁽ⁱ⁾ est choisi parmi
un hydrogène, un alkyle en C₁-C₆, un hydroxy, un hydroxyméthyle, un carboxy et un alcoxycarbonyle en C₂-C₇ ;
R¹²⁽ⁱ⁾ est choisi parmi
un hydrogène, un alkyle en C₁-C₆ et un groupe oxo adjacent à un atome d'azote, ou
R¹¹⁽ⁱ⁾ et R¹²⁽ⁱ⁾, éventuellement conjointement, forment un pont alkylène en C₁-C₃ par formation d'un système à noyau bicyclique, et
(a) dans le cas où E représente E¹⁽ⁱ⁾, E²⁽ⁱ⁾ ou E³, le substituant G est éventuellement choisi parmi G¹⁽ⁱ⁾, G²⁽ⁱ⁾, G³⁽ⁱ⁾, G⁴⁽ⁱ⁾ et G⁵⁽ⁱ⁾, où
G¹⁽ⁱ⁾ est
-(CH₂)ᵣ-(CR¹⁴⁽ⁱ⁾R¹⁵⁽ⁱ⁾)ₛ-R¹³⁽ⁱ⁾
où
r est 0 à 3, et
s est 0 ou 1,
R¹³⁽ⁱ⁾ est choisi parmi
un hydrogène, un alkyle en C₁-C₆, un alcényle en C₃-C₆, un alcynyle en C₃-C₆ et un cycloalkyle en C₃-C₈ ;
des hétérocycles, saturés ou insaturés, de quatre à sept chaînons, qui contiennent un ou deux hétéroatomes choisis parmi N, S et O ;
un benzyle, un phényle ;
des hétérocycles monocycliques aromatiques, de cinq ou six chaînons, qui contiennent un à trois hétéroatomes choisis parmi N, S et O, et qui sont liés soit directement, soit sur un groupe méthylène ;
des systèmes cycliques carbocycliques, partiellement hydrogénés ou aromatiques bi- et tricycliques condensés, avec 8 à 16, 17 ou 18 atomes de cycle et au moins un cycle aromatique, où la liaison se produit soit sur un cycle aromatique soit sur un cycle hydrogéné et soit directement soit sur un groupe méthylène ;
des systèmes cycliques hétérocycliques partiellement hydrogénés ou aromatiques bi- et tricycliques condensés, avec 8 à 16, 17 ou 18 atomes de cycle et au moins un cycle aromatique, où un à trois atomes de cycle sont choisis parmi N, S et O et la liaison se produit soit sur un cycle aromatique soit sur un cycle hydrogéné et soit directement soit sur un groupe méthylène ;
R¹⁴⁽ⁱ⁾ a les mêmes significations que R¹³⁽ⁱ⁾, mais est choisi indépendamment de celui-ci ;
R¹⁵⁽ⁱ⁾ est choisi parmi
un hydrogène, un hydroxy, un méthyle, un benzyle, un phényle,
des hétérocycles monocycliques aromatiques de cinq ou six chaînons qui contiennent un à trois hétéroatomes choisis parmi N, S et O et qui sont liés soit directement soit sur un groupe méthylène ;
des systèmes cycliques carbocycliques, partiellement hydrogénés ou aromatiques bi- et tricycliques condensés, avec 8 à 16, 17 ou 18 atomes de cycle et au moins un cycle aromatique, où la liaison se produit soit sur un cycle aromatique soit sur un cycle hydrogéné et soit directement soit sur un groupe méthylène ;
des systèmes cycliques hétérocycliques partiellement hydrogénés ou aromatiques bi- et tricycliques condensés, avec 8 à 16, 17 ou 18 atomes de cycle et au moins un cycle aromatique, où un à trois atomes de cycle sont choisis parmi N, S et O et la liaison se produit soit sur un cycle aromatique soit sur un cycle hydrogéné et soit directement soit sur un groupe méthylène ;
G²⁽ⁱ⁾ est choisi parmi 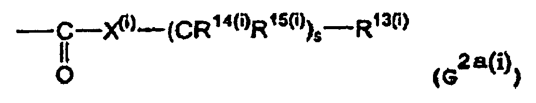 et 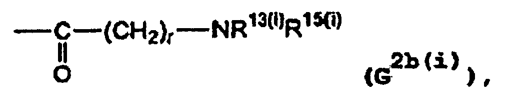 ou r, s et les substituants R¹³⁽ⁱ⁾, R¹⁴⁽ⁱ⁾ et R¹⁵⁽ⁱ⁾ ont les significations ci-dessus,
ou le groupe -NR¹³⁽ⁱ⁾R¹⁵⁽ⁱ⁾ est un hétérocycle contenant de azote lié sur l'atome d'azote, lequel hétérocycle contenant de l'azote est choisi parmi
des hétérocycles monocycliques saturés et insaturés, de quatre à huit chaînons, qui, en dehors de l'atome d'azote essentiel, contiennent éventuellement un ou deux autres hétéroatomes choisis parmi N, S et O,
et
des hétérocycles saturés et insaturés, bi- ou tricycliques, condensés ou pontés, avec 8 à 16, 17 ou 18 atomes de cycle, qui, en dehors de l'atome d'azote essentiel, contiennent éventuellement un ou deux autres hétéroatomes choisis parmi N, S et O,
X⁽ⁱ⁾ est choisi parmi
un méthylène, un éthylène, un éthénylène, un propylène et un cycloalkylène en C₃-C₇, ou représente une liaison ;
G³⁽ⁱ⁾ est
-SO₂-(CH₂)ᵣ-R¹³⁽ⁱ⁾
où r et R¹³⁽ⁱ⁾ ont les significations ci-dessus,
G⁴⁽ⁱ⁾ est 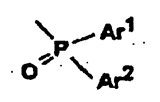 où
Ar¹ et Ar² sont choisis indépendamment l'un de l'autre parmi un phényle, un pyridyle et un naphtyle ;
G⁵⁽ⁱ⁾ est
-COR¹⁶⁽ⁱ⁾
où
R¹⁶⁽ⁱ⁾ est choisi parmi un trifluorométhyle, un alcoxy en C₁-C₆, un alcényloxy en C₃-C₆ et un benzyloxy,
(b) dans le cas où E est E⁴ ou E⁵,
alors G est éventuellement G¹⁽ⁱ⁾, G²⁽ⁱ⁾, G⁶⁽ⁱ⁾, G⁷ ou G⁸,
où G¹⁽ⁱ⁾ et G²⁽ⁱ⁾ ont les significations ci-dessus et
G⁶⁽ⁱ⁾ est
=(C)ᵤR¹³⁽ⁱ⁾R¹⁵⁽ⁱ⁾,
où R¹³⁽ⁱ⁾ et R¹⁵⁽ⁱ⁾ ont les significations ci-dessus et
u est 0 ou 1,
ou lorsque u = 1, alors R¹³⁽ⁱ⁾ et R¹⁵⁽ⁱ⁾ conjointement avec l'atome de carbone sur lequel ils sont attachés forment un système cyclique choisi parmi
un cycloalkyle en C₃-C₈,
des hétérocycles saturés, de quatre à sept chaînons qui contiennent éventuellement un ou deux hétéroatomes, choisis parmi N, S et O ;
des systèmes cycliques carbocycliques bi- et tricycliques condensés partiellement hydrogénés, avec 8 à 16, 17 ou 18 atomes de cycle et au moins un cycle aromatique ;
des systèmes cycliques hétérocycliques bi- et tricycliques condensés partiellement hydrogénés, avec 8 à 16, 17 ou 18 atomes de cycle et au moins un cycle aromatique, où un à trois atomes de cycle sont choisis parmi N, S et O ;
ou lorsque u = 0, alors R¹³⁽ⁱ⁾ et R¹⁵⁽ⁱ⁾ conjointement avec l'atome de carbone du cycle E sur lequel ils sont attachés forment un système cyclique choisi parmi
un cycloalkyle en C₃-C₈,
des hétérocycliques saturés, de quatre à sept chaînons qui contiennent un ou deux hétéroatomes, choisis parmi N, S et O ;
des systèmes cycliques carbocycliques bi- et tricycliques condensés partiellement hydrogénés, avec 8 à 16, 17 ou 18 atomes de cycle et au moins un cycle aromatique ; et
des systèmes cycliques hétérocycliques bi- et tricycliques condensés partiellement hydrogénés, avec 8 à 16, 17 ou 18 atomes de cycle et au moins un cycle aromatique, où un à trois atomes de cycle sont choisis parmi N, S et O ;
G⁷ est choisi parmi
-**NR**^{**17(i)**}**-(CH**_{**2**}**)**_{**r**}**-(CR**^{**14(i)**}**R**^{**15(i)**}**)**_{**s**}**-R**^{**13(i)**} **(G**^{**7a**}**),** 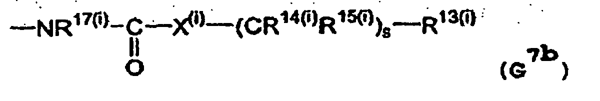 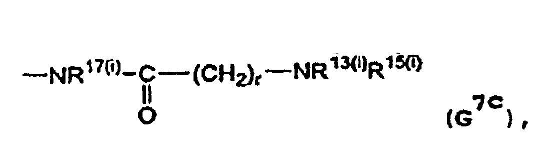
**-NR**^{**17(i)**}**-SO**_{**2**}**-(CH**_{**2**}**)**_{**r-**}**R**^{**13(i)**} **(G**^{**7d**}**),** 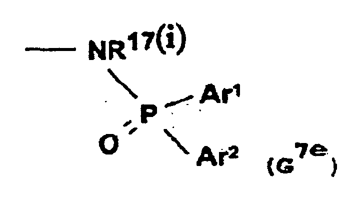
et
-**NR**^{**17(i)**}**-COR**^{**16(i)**} **(G**^{**7f**}**),**
où r, s, X⁽ⁱ⁾, les substituants R¹³⁽ⁱ⁾, R¹⁴⁽ⁱ⁾, R¹⁵⁽ⁱ⁾ et R¹⁶⁽ⁱ⁾, et le groupe
-NR¹³⁽ⁱ⁾R¹⁵⁽ⁱ⁾
ont les significations ci-dessus, et
R¹⁷⁽ⁱ⁾ a les mêmes significations que R⁵⁽ⁱ⁾, mais est choisi indépendamment de celui-ci ;
Ar¹ et Ar² sont choisis indépendamment l'un de l'autre parmi un phényle, un pyridyle et un naphtyle ;
G⁸ est choisi parmi 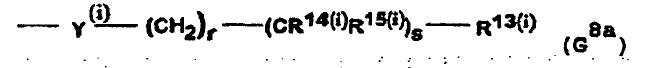 et 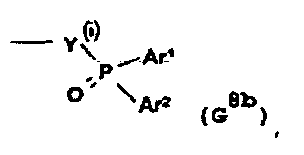 où r, s et les substituants R¹³⁽ⁱ⁾, R¹⁴⁽ⁱ⁾, R¹⁵⁽ⁱ⁾, Ar¹ et Ar², ont les significations ci-dessus, et
Y⁽ⁱ⁾ est O ou S ;
(c) dans le cas où le substituant E est E⁶,
alors le substituant G est éventuellement choisi parmi G^{7d}, G^{7e}, G^{8b}, G⁹, G¹⁰, G¹¹, G¹² et G¹³, où G^{7d}, G^{7e} et G^{8b} ont les significations ci-dessus et
G⁹ est choisi parmi
-(CR¹³⁽ⁱ⁾R¹⁹)ₛ-R¹⁸ (G^{9a})
et
-NR¹³⁽ⁱ⁾R¹⁸ (G^{9b}),
où s et R¹³⁽ⁱ⁾ sont définis comme ci-dessus, et
R¹⁸ est choisi parmi
un benzyle, un diphénylméthyle, un phényle ;
des hétérocycles monocycliques aromatiques de cinq et six chaînons qui peuvent contenir un à trois hétéroatomes choisis parmi N, S et O et qui sont liés soit directement soit sur un groupe méthylène ;
des systèmes cycliques carbocycliques partiellement hydrogénés ou aromatiques bi- et tricycliques condensés, avec 8 à 16, 17 ou 18 atomes de cycle et au moins un cycle aromatique, où la liaison se produit soit sur un cycle aromatique soit sur un cycle hydrogéné et soit directement soit sur un groupe méthylène ; et
des systèmes cycliques hétérocycliques partiellement hydrogénés ou aromatiques bi- et , tricycliques condensés avec 8 à 16, 17 ou 18 atomes de cycle et au moins un cycle aromatique, où un à trois atomes de cycle sont choisis parmi N, S et O et la liaison se produit soit sur un cycle aromatique soit sur un cycle hydrogéné et soit directement soit sur un groupe méthylène ;
R¹⁹ a les mêmes significations que R¹³⁽ⁱ⁾ mais est choisi indépendamment de celui-ci et, de plus, il peut être un groupe hydroxy ;
ou le groupe
-NR¹³⁽ⁱ⁾R¹⁸
est éventuellement un hétérocycle contenant de l'azote lié sur l'atome d'azote, lequel hétérocycle contenant de l'azote est choisi parmi
des systèmes cycliques hétérocycliques partiellement hydrogénés ou aromatiques bi- et tricycliques condensés, avec 8 à 16, 17 ou 18 atomes de cycle et au moins un cycle aromatique, qui en dehors de l'atome d'azote essentiel, contiennent éventuellement un ou deux autres hétéroatomes choisis parmi N, S et O ;
G¹⁰ est
CR¹³⁽ⁱ⁾R¹⁸ (G¹⁰)
lié à D au moyen d'une double liaison, où R¹³⁽ⁱ⁾ et R¹⁸ ont les significations ci-dessus, ou bien où G¹⁰ est éventuellement un système cyclique lié sur l'atome de carbone, choisi parmi
des systèmes cycliques carbocycliques partiellement hydrogénés bi- et tricycliques condensés, avec 8 à 16, 17 ou 18 atomes de cycle et au moins un cycle aromatique ; et
des systèmes cycliques hétérocycliques partiellement hydrogénés bi- et tricycliques condensés, avec 8 à 16, 17 ou 18 atomes de cycle et au moins un cycle aromatique, où un à trois atomes de cycle sont éventuellement choisis parmi N, S et O ;
G¹¹ est choisi parmi
-**NR**^{**17(i)**}**-(CH**_{**2**}**)**_{**r**}**-(CR**^{**13(i)**}**R**^{**19**}**)**_{**s**}-**R**^{**18**} **(G**^{**11a**}**),** 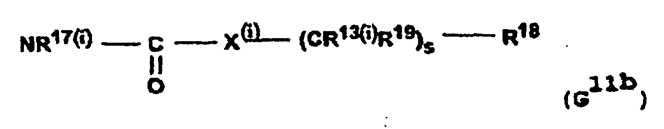
et 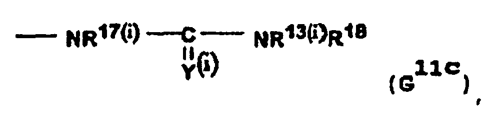 où r, s, X⁽ⁱ⁾, Y⁽ⁱ⁾, les substituants R¹³⁽ⁱ⁾, R¹⁷⁽ⁱ⁾, R¹⁸ et R¹⁹, et le groupe -NR¹³⁽ⁱ⁾R¹⁸, ont les significations ci-dessus ;
G¹² est
-Y⁽ⁱ⁾-(CH₂)ᵣ-(CR¹³⁽ⁱ⁾R¹⁹)ₛ-R¹⁸ (G¹²)
où r, s, Y⁽ⁱ⁾, et les substituants R¹³⁽ⁱ⁾, R¹⁸ et R¹⁹ ont les significations ci-dessus ;
G¹³ est choisi parmi 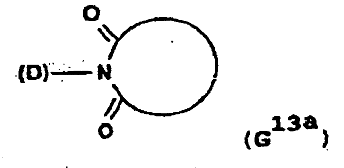 et 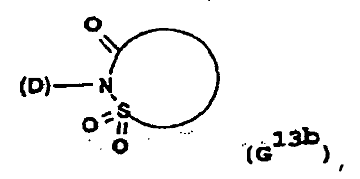 lié à D sur l'atome d'azote d'imide, choisi parmi
des imides monocycliques saturés et insaturés, avec 5 à 7 atomes de cycle, qui, en dehors de l'atome d'azote d'imide essentiel, contiennent éventuellement un ou deux autres hétéroatomes choisis parmi N, S et O ;
des imides bi-, tri- ou tétracycliques saturés, insaturés et aromatiques condensés, avec 8 à 18 atomes de cycle, qui, en dehors de l'atome d'azote d'imide essentiel, contiennent éventuellement un ou deux autres hétéroatomes choisis parmi N, S et O ;
des imides bi-, tri-, tétra- ou pentacycliques, pontés saturés et insaturés avec 8 à 22 atomes de cycle, qui, en dehors de l'atome d'azote d'imide essentiel, contiennent éventuellement un ou deux autres hétéroatomes choisis parmi N, S et O ; et
des imides spirocycliques saturés et insaturés, éventuellement condensés une ou deux fois, et avec un total de 9 à 23 atomes de cycle, qui, en dehors de l'atome d'azote d'imide essentiel, contiennent éventuellement un ou deux autres hétéroatomes choisis parmi N, S et O ;
où ces imides cycliques sont éventuellement substitués par un à cinq des groupes identiques ou différents choisis indépendamment les uns des autres parmi
un halogène, un cyano, un alkyle en C₁-C₆, un alkylidène en C₁-C₆, un trifluorométhyle, un cycloalkyle en C₃-C₈, un cycloalkylidène en C₃-C₈, un phénylalkyle en C₁-C₃, un phénylalkylidène en C₁-C₃, un diphénylalkyle en C₁-C₃, un diphénylalkylidène en C₁-C₃, un triphénylméthyle, un phényle, un hydroxy, un hydroxyalkyle en C₁-C₆, un alcoxy en C₁-C₆, un alcoxy en C₁-C₆ entièrement ou partiellement substitué par le fluor, un benzyloxy, un phénoxy, un naphtyloxy, un mercapto, un alkylthio en C₁-C₆, un phénylthio, un naphtylthio, un pyridylthio, un alcanesulfonyle en C₁-C₆, un phénylsulfonyle, un naphtylsulfonyle, un pyridylsulfonyle, un sulfo, un carboxy, un carboxyalkyle en C₂-C₇, un carboxyalcényle en C₃-C₇, un alcoxycarbonyle en C₂-C₇, un benzyloxycarbonyle, un nitro, un amino, un aminoalkyle en C₁-C₆, un monoalkyl (en C₁-C₆) amino, un dialkyl(en C₁-C₆)amino, un phénylamino, un phénylalkyl(en C₁-C₃)amino, un pyridylamino,
des hétérocycles saturés et insaturés, de quatre à sept chaînons, qui contiennent un ou deux hétéroatomes choisis parmi N, S et O, et qui sont liés soit directement soit sur un groupe méthylène ou un groupe méthine,
des hétérocycles monocycliques aromatiques, de cinq et six chaînons, qui contiennent un à trois hétéroatomes choisis parmi N, S et O, et qui sont liés soit directement soit sur un groupe méthylène ou un groupe méthine,
des systèmes cycliques carbocycliques partiellement hydrogénés ou aromatiques bicycliques condensés, avec 8 à 12 atomes de cycle qui sont liés soit directement soit sur un groupe méthylène ou un groupe méthine ; et
des systèmes cycliques hétérocycliques partiellement hydrogénés ou aromatiques bicycliques condensés, avec 8 à 12 atomes de cycle, où un à trois atomes de cycle sont choisis parmi N, S et O et qui sont liés soit directement soit sur un groupe méthylène ou un groupe méthine ;
où les systèmes cycliques aromatiques dans les substituants R¹⁽ⁱ⁾, R²⁽ⁱ⁾, R⁴⁽ⁱ⁾, R⁵⁽ⁱ⁾, R⁶⁽ⁱ⁾, R¹³⁽ⁱ⁾, R¹⁴⁽ⁱ⁾, R¹⁵⁽ⁱ⁾, R¹⁶⁽ⁱ⁾, R¹⁷⁽ⁱ⁾, R¹⁸, R¹⁹, Ar¹ et Ar², dans les groupes A⁽ⁱ⁾ et D⁽ⁱ⁾, dans les systèmes cycliques =CR¹³⁽ⁱ⁾R¹⁵⁽ⁱ⁾, =CR¹³⁽ⁱ⁾R¹⁸, -NR¹³⁽ⁱ⁾R¹⁵⁽ⁱ⁾ et -NR¹³⁽ⁱ⁾R¹⁸⁽ⁱ⁾, ainsi que les substituants et/ou les substituants dans les imides cycliques G¹³ sont éventuellement indépendamment substitués par un à trois des groupes identiques ou différents, choisis parmi
un halogène, un cyano, un alkyle en C₁-C₆, un trifluorométhyle, un cycloalkyle en C₃-C₈, un benzyle, un phényle, un hydroxy, un hydroxyalkyle en C₁-C₆, un alcoxy en C₁-C₆, un alcoxy en C₁-C₆ entièrement ou partiellement substitué par le fluor, un benzyloxy, un phénoxy, un mercapto, un alkylthio en C₁-C₆, un phénylthio, un sulfo, un carboxy, un carboxyalkyle en C₂-C₇, un carboxyalcényle en C₃-C₇, un alcoxycarbonyle en C₂-C₇, un benzyloxycarbonyle, un nitro, un amino, un aminoalkyle en C₁-C₆, un monoalkyl(en C₁-C₆)amino et un dialkyl (en C₁-C₆)amino et, dans le cas de deux résidus adjacents sur le cycle aromatique, un méthylènedioxy,
où les résidus alkyle et cycloalkyle dans les groupes G sont éventuellement substitués par un ou deux des groupes identiques ou différents, choisis parmi
un hydroxy, un carboxy, un alcoxycarbonyle en C₂-C₇, un benzyloxycarbonyle, un amino, monoalkyl(en C₁-C₆)amino, et un dialkyl(en C₁-C₆)amino ;
et les stéreoisomères ou mélanges racémiques ou non racémiques des stéréoisomères de ceux-ci,
et les tautomères de ceux-ci lorsque G est un cycle hétérocyclique aromatique ou un cycle aromatique substitué par un groupe hydroxy, mercapto ou amino,
et les sels d'addition d'acide pharmacologiquement acceptables de ceux-ci ;
(b) au moins un composé présentant une activité de vitamine PP ou un précurseur de médicament de celui-ci qui est choisi dans le groupe constitué par les composés de formules II, IIa, IIb, III, IIIa, IIIb, IIIc, IV, IVa, IVb, V, Va et Vb : 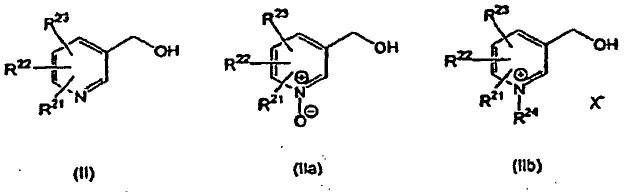 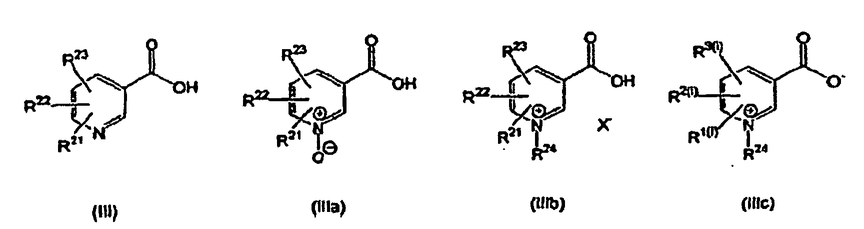 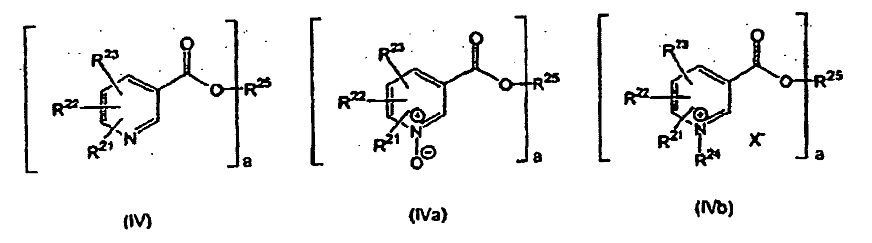 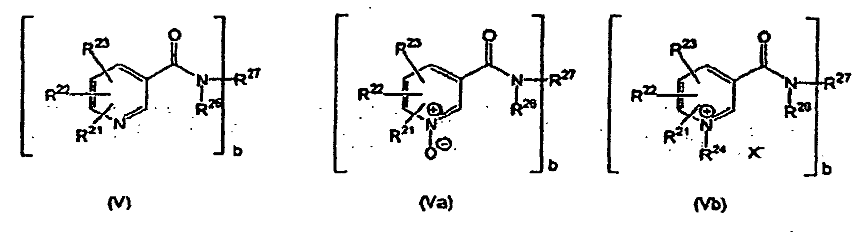 dans lesquelles :
a est un nombre entier de 1 à 6 ;
b est un nombre entier de 1 à 2 ;
X⁻ est choisi dans le groupe constitué par un fluorure, un chlorure, un bromure, un iodure, un hydrogénosulfate, un méthanesulfonate, un trifluorométhanesulfonate, un tosylate, un tétrafluoroborate, un dihydrogénophosphate et un acétate ;
R²¹ est choisi dans le groupe constitué par un hydrogène, un halogène, un cyano, un alkyle en C₁-C₆, un trifluorométhyle, un hydroxyalkyle en C₁-C₆, un hydroxy, un alcoxy en C₁-C₆, un alcanoyloxy en C₁-C₇, un alkylthio en C₁-C₆, un aminoalkyle en C₁-C₆, un amino, un alkylamino en C₁-C₆, un dialkylamino en C₂-C₁₂, un formyle, un alcoxycarbonyle en C₂-C₇, un aminocarbonyle, un alkylaminocarbonyle en C₂-C₇, un dialkylaminocarbonyle en C₃-C₁₃ et un carboxy ;
R²² est choisi dans le groupe constitué par un hydrogène, un halogène, un alkyle en C₁-C₆, un trifluorométhyle, un hydroxyalkyle en C₁-C₆, un hydroxy, un alcoxy en C₁-C₆, un alcanoyloxy en C₁-C₇, un aminoalkyle en C₁-C₆, un amino, un alcoxycarbonyle en C₂-C₇, un aminocarbonyle et un carboxy ;
R²³ est choisi dans le groupe constitué par un hydrogène, un alkyle en C₁-C₆ et un hydroxyalkyle en C₁-C₆ ;
R²⁴ est choisi dans le groupe constitué par un alkyle en C₁-C₆, un alcényle en C₃-C₆, un hydroxyalkyle en C₂-C₆, un alcoxyalkyle en C₂-C₆ et un benzyle ;
R²⁵ est tel que l'alcool R²⁵(OH)ₐ est choisi parmi les les ω-dialkylaminoalcanols et alcanols en C₁-C₁₀, monovalents, linéaires et ramifiés, l'alcool benzylique, les diols en C₂-C₁₀ divalents, linéaires et ramifiés, les cycloalcanols en C₅-C₇ mono ou divalents, les cycloalcanediols en C₅-C₇, les cycloalcaneméthanols en C₅-C₇, les hétérocyclométhanols en C₅-C₇ saturés, la glycérine, le 2,2-bis(hydroxyméthyl)-1-octanol, l'érythritol, le pentaérythritol, l'arabitol, le xylitol, le sorbitol, le mannitol, l'isosorbitol, le tétra(hydrométhyl)cyclohexanol et l'inositol ;
R²⁶ est choisi dans le groupe constitué par un hydrogène, un alkyle en C₁-C₆, un hydroxyalkyle en C₁-C₆, un alcoxyalkyle en C₃-C₆, un aminoalkyle en C₁-C₆, un dialkylaminoalkyle en C₄-C₁₂ et un carboxyméthyle ;
lorsque b est 1,
R²⁷ est choisi dans le groupe constitué par un hydrogène, un alkyle en C₁-C₆, un hydroxyalkyle en C₁-C₆, un alcoxyalkyle en C₃-C₆, un aminoalkyle en C₁-C₆, un dialkylaminoalkyle en C₄-C₁₂ et un carboxyméthyle ;
lorsque b est 2,
R²⁷ est un alkylène en C₂-C₁₀, ou un alkylène en C₅-C₁₀, où un groupe méthylène est remplacé de façon isostérique par O, NH ou N-alkyle ;
et leurs analogues thioxo,
et les sels d'addition d'acide ou sels anioniques de ceux-ci ; et
(c) au moins un support physiologiquement acceptable et au moins un adjuvant toxicologiquement sûr.

2. Composition selon la revendication 1, dans laquelle le ou les composés de formule (I) sont choisis parmi :
le N-[2-(1-benzylpipéridin-4-yl)-éthyl]-3-(pyridin-3-yl)-propionamide ;
le N-{2-[1-(2-phényléthyl)-pipéridin-4-yl]-éthyl}-3-(pyridin-3-yl)-propionamide ;
le N-{2-[1-(4-phénylbutyl)-pipéridin-4-yl]-éthyl}-3-(pyridin-3-yl)-propionamide ;
le N-{2-[1-(4-hydroxy-4-phénylbutyl)-pipéridin-4-yl]-éthyl}-3-(pyridin-3-yl)-propionamide ;
le N-[2-(1-diphénylméthylpipéridin-4-yl)-éthyl]-3-(pyridin-3-yl)-propionamide ;
le N-[3-(1-diphénylméthylpipéridin-4-yl)-propyl]-3-(pyridin-3-yl)-propionamide ;
le N-[4-(1-diphénylméthylpipéridin-4-yl)-propyl]-3-(pyridin-3-yl)-propionamide ;
le N-[4-(1-benzylpipéridin-4-yl)-butyl]-3-(pyridin-3-yl)-acrylamide ;
le N-{4-[1-(2-phényléthyl)-pipéridin-4-yl]-butyl}-3-(pyridin-3-yl)-acrylamide ;
le N-{4-[1-(4-biphénylylméthyl)-pipéridin-4-yl]-butyl}-3-(pyridin-3-yl)-acrylamide ;
le N-{4-[1-(1-naphtylméthyl)-pipéridin-4-yl]-butyl}-3-(pyridin-3-yl)-acrylamide ;
le N-{4-[1-(9-anthrylméthyl)-pipéridin-4-yl]-butyl}-3-(pyridin-3-yl)-acrylamide ;
le N-{4-[1-(cyclohexylphénylméthyl)-pipéridin-4-yl]-butyl}-3-(pyridin-3-yl)-acrylamide ;
le N-{4-[1-(10,11-dihydro-5H-dibenzo[a,d]cycloheptèn-5-yl)-pipéridin-4-yl]-butyl}-3-(pyridin-3-yl)acrylamide ;
le N-[2-(1-diphénylméthylpipéridin-4-yl)-éthyl]-3-(pyridin-3-yl)-acrylamide ;
le N-[3-(1-diphénylméthylpipéridin-4-yl)-propyl]-3-(pyridin-3-yl)-acrylamide ;
le N-[5-(1-diphénylméthylpipéridin-4-yl)-pentyl]-3-(pyridin-3-yl)-acrylamide ;
le N-[6-(1-diphénylméthylpipéridin-4-yl)-hexyl]-3-(pyridin-3-yl)-acrylamide ;
l'amide d'acide N-[4-(1-diphénylméthylpipéridin-4-yl)-butyl]-5-(pyridin-3-yl)-2,4-pentdiénique ;
le N-(4-{1-[bis(4-fluorophényl)-méthyl]-pipéridin-4-yl}-butyl)-3-(pyridin-3-yl)-acrylamide ;
le N- (4-{1-[bis(2-chlorophényl)-méthyl]-pipéridin-4-yl}-butyl)-3-(pyridin-3-yl)-acrylamide ;
le N-[4-(1-diphénylméthylpipéridin-4-yl)-butyl]-3-(2-fluoropyridin-3-yl)-acrylamide ;
le N-[4-(1-diphénylméthylpipéridin-4-yl)-butyl]-3-(6-fluoropyridin-3-yl)-acrylamide ;
le N-[4-(1-diphénylméthylpipéridin-4-yl)-butyl]-3-(pyridin-3-yl)-acrylamide ;
le dichlorhydrate de N-[4-(1-diphénylméthylpipéridin-4-yl)-butyl]-3-(pyridin-3-yl)-acrylamide ;
le méthanesulfonate de N-[4-(1-diphénylméthylpipéridin-4-yl)-butyl]-3-(pyridin-3-yl)-acrylamide ;
le N-[4-(1-acétylpipéridin-4-yl)-butyl]-3-(pyridin-3-yl)-propionamide ;
le N-[4-(1-benzoylpipéridin-4-yl)-butyl]-3-(pyridin-3-yl)-propionamide ;
le N-[4-(1-diphénylacétylpipéridin-4-yl)-butyl]-3-(pyridin-3-yl)-propionamide ;
le N-{4-[1-(9-oxo-9H-fluorén-4-carbonyl)-pipéridin-4-yl]-butyl}-3-(pyridin-3-yl)-propionamide ;
le N-[4-(1-méthylsulfonylpipéridin-4-yl)-butyl]-3-(pyridin-3-yl)-propionamide ;
le N-{4-[1-(2-naphtylsulfonyl)-pipéridin-4-yl]-butyl}-3-(pyridin-3-yl)-propionamide ;
le N-[4-(1-benzylpipéridin-4-yl)-butyl]-3-(pyridin-3-yl)-propionamide ;
le N-(4-{1-[bis(2-chlorophényl)-méthyl]-pipéridin-4-yl}-butyl)-3-(pyridin-3-yl)-propionamide ;
le N-{4-[1-(phénylpyridin-3-yl)-pipéridin-4-yl]-butyl}-3-(pyridin-3-yl)-propionamide ;
le N-{4-[1-(9H-fluorén-9-yl)-pipéridin-4-yl]-butyl}-3-(pyridin-3-yl)-propionamide ;
le N-{4-[1-(6,11-dihydrodibenzo[b,e]oxépin-11-yl)-pipéridin-4-yl]-butyl}-3-(pyridin-3-yl)-propionamide ;
le N-{4-[1-(1-naphtylaminocarbonyl)-pipéridin-4-yl]-butyl}-3-(pyridin-3-yl)-propionamide ;
le N-[4-(1-diphénylaminocarbonyl-pipéridin-4-yl)-butyl]-3-(pyridin-3-yl)-propionamide ;
le N-{4-[1-(10,11-dihydrodibenzo[b,f]azépin-5-yl-carbonyl)-pipéridin-4-yl]-butyl}-3-(pyridin-3-yl)-propionamide ;
le N-[4-(1-diphénylphosphinoyl-pipéridin-4-yl)-butyl]-3-(pyridin-3-yl)-propionamide ;
le N-[4-(1-diphénylméthylpipéridin-4-yl)-butyl]-3-(2-fluoropyridin-3-yl)-propionamide ;
le N-[4-(1-diphénylméthylpipéridin-4-yl)-butyl]-3-(5-fluoropyridin-3-yl)-propionamide ;
le N-[4-(1-diphénylméthylpipéridin-4-yl)-butyl]-2-fluoro-3-(pyridin-3-yl)-propionamide ;
le N-[4-(1-diphénylméthylpipéridin-4-yl)-butyl]-2,2-difluoro-3-(pyridin-3-yl)-propionamide ;
le N-[5-(1-diphénylméthylpipéridin-4-yl)-pentyl]-3-(pyridin-3-yl)-propionamide ;
le N-[6-(1-diphénylméthylpipéridin-4-yl)-hexyl]-3-(pyridin-3-yl)-propionamide ;
l'amide d'acide N-[2-(1-diphénylméthylpipéridin-4-yl)-éthyl]-5-(pyridin-3-yl)-pentanoïque ;
l'amide d'acide N-[4-(1-diphénylméthylpipéridin-4-yl)-butyl]-5-(pyridin-3-yl)-pentanoïque ;
le N-[4-(1-diphénylméthylpipéridin-4-yl)-butyl]-N-hydroxy-3-(pyridin-3-yl)-propionamide ;
le N-[4-(1-diphénylméthylpipéridin-4-yl)-butyl]-2-hydroxy-3-(pyridin-3-yl)-propionamide ;
le N-[4-(1-diphénylméthylpipéridin-4-yl)-butyl]-3-hydroxy-3-(pyridin-3-yl)-propionamide ;
le N-[4-(1-diphénylméthylpipéridin-4-yl)-butyl]-3-(pyridin-3-yl)-propionamide ;
le N-[4-(1-méthylsulfonylpipéridin-4-yl)-butyl]-3-(pyridin-3-yl)-acrylamide ;
le N-{4-[1-(2-naphtylsulfonyl)-pipéridin-4-yl]-butyl}-3-(pyridin-3-yl)-acrylamide ;
l'amide d'acide N-{4-[1-(2-naphtylsulfonyl)-pipéridin-4-yl]-butyl}-5-(pyridin-3-yl)-2,4-pentadiénique ;
le N-{4-[1-(1-naphtylaminocarbonyl)-pipéridin-4-yl]-butyl}-3-(pyridin-3-yl)-acrylamide ;
le N-[4-(1-diphénylaminocarbonylpipéridin-4-yl)-butyl]-3-(pyridin-3-yl)-acrylamide ;
l'amide d'acide N-[4-(1-diphénylaminocarbonylpipéridin-4-yl)-butyl]-5-(pyridin-3-yl)-2,4-pentadiénique ;
le N-{4-[1-(10,11-dihydrodibenzo[b,f]azépin-5-yl-carbonyl)-pipéridin-4-yl]-butyl}-3-(pyridin-3-yl)-acrylamide ;
le N-[4-(1-diphénylphosphinoylpipéridin-4-yl)-butyl]-3-(pyridin-3-yl)-acrylamide ;
le N-[4-(1-acétylpipéridin-4-yl)-butyl]-3-(pyridin-3-yl)-acrylamide ;
le N-[4-(1-diphénylacétylpipéridin-4-yl)-butyl]-3-(pyridin-3-yl)-acrylamide ;
le N-{4-[1-(3,3-diphénylpropionyl)-pipéridin-4-yl]-butyl}-3-(pyridin-3-yl)-acrylamide ;
le N-[4-(1-benzoylpipéridin-4-yl)-butyl]-3-(pyridin-3-yl)-acrylamide ;
l'amide d'acide N-[4-(1-benzoylpipéridin-4-yl)-butyl]-5-(pyridin-3-yl)-2,4-pentadiénique ;
le N-{4-[1-(9-oxo-9H-fluorén-4-carbonyl)-pipéridin-4-yl]-butyl}-3-(pyridin-3-yl)-acrylamide ;
le N-{4-[1-(phénylpyridin-3-ylméthyl)-pipéridin-4-yl]-butyl}-3-(pyridin-3-yl)-acrylamide ;
le N-{4-[1-(phénylpyridin-4-ylméthyl)-pipéridin-4-yl]-butyl}-3-(pyridin-3-yl)-acrylamide ;
le N-{4-[1-(6,11-dihydrodibenzo[b,e]oxépin-11-yl)-pipéridin-4-yl]-butyl}-3-(pyridin-3-yl)-acrylamide ;
le N-{4-[1-(6,11-dihydrodibenzo[b,e]thiépin-11-yl)-pipéridin-4-yl]-butyl}-3-(pyridin-3-yl)-acrylamide ;
le N-[7-(1-diphénylméthylpipéridin-4-yl)-heptyl]-3-(pyridin-3-yl)-acrylamide ;
le N-[8-(1-diphénylméthylgipéridin-4-yl)-octyl]-3-(pyridin-3-yl)-acrylamide ;
le N-[3-(1-diphénylméthylpipéridin-4-yloxy)-propyl]-3-(pyridin-3-yl)-acrylamide ;
le N-[3-(1-benzylpipéridin-4-yloxy)-propyl]-3-(pyridin-3-yl)-acrylamide ;
l'amide d'acide N-[2-(1-diphénylméthylpipéridin-4-yl)-éthyl]-5-(pyridin-3-yl)-2,4-pentadiénique ;
l'amide d'acide N-[4-(1-diphénylméthylpipéridin-4-yl)-butyl]-5-(pyridin-3-yl)-2,4-pentadiénique ;
l'amide d'acide N-[5-(1-diphénylméthylpipéridin-4-yl)-pentyl]-5-(pyridin-3-yl)-2,4-pentadiénique ;
l'amide d'acide N-[6-(1-diphénylméthylpipéridin-4-yl)-hexyl]-5-(pyridin-3-yl)-2,4-pentadiénique ;
le N-[4-(4-diphénylméthylpipéridin-1-yl)-3-hydroxy-butyl]-3-(pyridin-3-yl)-acrylamide ;
le N-[3-(4-diphénylméthylpipéridin-1-yl)-propoxy]-3-(pyridin-3-yl)-acrylamide ;
le N-[4-(4-diphénylméthylpipéridin-1-yl)-4-oxo-butyl]-3-(pyridin-3-yl)-acrylamide ;
le N-[3-(4-diphénylméthylpipéridin-1-sulfonyl)-propyl]-3-(pyridin-3-yl)-acrylamide ;
le N-{2-[2-(4-diphénylméthylpipéridin-1-yl)-éthoxy]-éthyl}-3-(pyridin-3-yl)-acrylamide ;
le N-(4-{4-[bis(4-fluorophényl)-méthyl]-pipéridin-1-yl}-but-2-inyl)-3-pyridin-3-yl-acrylamide ;
le N-{4-[4-(4-carboxyphénylphénylméthyl)-pipéridin-1-yl)-butyl}-3-pyridin-3-yl-acrylamide ;
le N-(4-{4-[(4-aminophényl)-phénylméthyl]-pipéridin-1-yl}-butyl)-3-pyridin-3-yl-acrylamide ;
le N-{4-[4-(9H-fluorén-9-yl)-pipéridin-1-yl]-butyl}-2-pyridin-3-yloxy)-acrylamide ;
le N-{5-[4-(9H-fluorén-9-yl)-pipéridin-1-yl]-pentyl}-2-pyridin-3-yloxy)-acrylamide ;
le N-{6-[4-(9H-fluorén-9-yl)-pipéridin-1-yl]-hexyl}-2-pyridin-3-yloxy)-acrylamide ;
le 3-pyridin-3-yl-N-{4-[4-(1,2,3,4-tétrahydronaphtalèn-1-yl)-pipéridin-1-yl]-butyl}-acrylamide ;
le 3-pyridin-3-yl-N-{4-[4-(5,6,7,8-tétrahydronaphtalèn-1-yl)-pipéridin-1-yl]-butyl}-acrylamide ;
le N-{4-[4-(naphtalèn-1-yl)-pipéridin-1-yl)-butyl}-3-pyridin-3-yl-acrylamide ;
le N-[4-(4-biphényl-2-yl-pipérazin-1-yl)-butyl]-3-pyridin-3-yl-propionamide ;
le N-[5-(4-biphényl-2-yl-pipérazin-1-yl)-pentyl]-3-pyridin-3-yl-acrylamide ;
le N-[6-(4-biphényl-2-yl-pipérazin-1-yl)-hexyl]-3-pyridin-3-yl-acrylamide ;
le N-[4-(4-biphényl-2-yl-pipérazin-1-yl)-butyl]-2-(pyridin-3-yloxy)-acétamide ;
l'amide d'acide N-[4-(4-biphényl-2-yl-pipérazin-1-yl)-butyl]-5-(pyridin-3-yl)-penta-2,4-diénique ;
le N-{4-[4-(10,11-dihydro-5H-dibenzo[a,d]cycloheptén-5-yl)-pipéridin-1-yl]-butyl}-3-pyridin-3-ylpropionamide ;
le N-{5-[4-(10,11-dihydro-5H-dibenzo[a,d]cycloheptén-5-yl)-pipéridin-1-yl]-pentyl}-3-pyridin-3-yl-acrylamide ;
le N-{6-[4-(10,11-dihydro-5H-dibenzo[a,d]cycloheptén-5-yl)-pipéridin-1-yl]-hexyl}-3-pyridin-3-yl-acrylamide ;
l'amide d'acide N-{4-[4-(10,11-dihydro-5H-dibenzo[a,d]-cycloheptén-5-yl)-pipéridin-1-yl]-butyl}-5-(pyridin-3-yl)-penta-2,4-diénique ;
le N-{4-[4-(6,11-dihydrodibenzo[b,e]oxépin-11-yl)-pipéridin-1-yl]-butyl}-3-pyridin-3-yl-propionamide ;
le N-{2-[4-(6,11-dihydrodibenzo[b,e]thiépin-11-yl)-pipéridin-1-yl]-éthyl}-3-pyridin-3-yl-acrylamide ;
le N-[4-(4-diphénylacétylpipérazin-1-yl)-butyl]-3-pyridin-3-yl-acrylamide ;
le N-[4-(4-benzoylpipérazin-1-yl)-butyl]-3-pyridin-3-yl-acrylamide ;
le N-{4-[4-(2-aminobenzoyl)-pipéridin-1-yl]-butyl}-3-pyridin-3-yl-acrylamide ;
le N-{4-[4-(4-carboxybenzoyl)-pipéridin-1-yl]-butyl}-3-pyridin-3-yl-acrylamide ;
le N-{4-[4-(biphényl-2-carbonyl)-pipéridin-1-yl]-butyl}-3-pyridin-3-yl-acrylamide ;
le N-{4-[4-(9-oxo-9H-fluorén-4-carbonyl)-pipéridin-1-yl]-butyl}-3-pyridin-3-yl-acrylamide ;
le N-{4-[4-(furan-2-carbonyl)-pipéridin-1-yl]-butyl}-3-pyridin-3-yl-acrylamide ;
le N-{4-[4-(naphtalèn-1-ylaminocarbonyl)-pipéridin-1-yl]-butyl}-3-pyridin-3-yl-propionamide ;
le N-{4-[4-(diphénylaminocarbonyl)-pipéridin-1-yl]-butyl}-3-pyridin-3-yl-acrylamide ;
le N-{4-[4-(naphtalèn-2-sulfonyl)-pipéridin-1-yl]-butyl}-3-pyridin-3-yl-acrylamide ;
le N-[4-(4-diphénylphosphinoyl-pipérazin-1-yl)-butyl]-3-pyridin-3-yl-acrylamide ;
le N-[4-(4-biphényl-2-yl-pipérazin-1-yl)-butyl]-3-pyridin-3-yl-acrylamide ;
le N-{4-[4-(9H-fluorén-9-yl)-pipéridin-1-yl]-butyl}-3-pyridin-3-yl-acrylamide ;
le N-{4-[4-(10,11-dihydro-5H-dibenzo[a,d]cycloheptén-5-yl)-pipéridin-1-yl]-butyl}-3-pyridin-3-yl-acrylamide ;
le N-[4-(4-phénylpipérazin-1-yl)-butyl]-3-pyridin-3-yl-acrylamide ;
le N-{4-[4-(1H-indol-3-yl)-pipéridin-1-yl]-butyl}-3-pyridin-3-yl-acrylamide ;
le N-{4-[4-(2-oxo-2,3-dihydrobenzimidazol-1-yl)-pipéridin-1-yl]-butyl}-3-pyridin-3-yl-acrylamide ;
le N-[4-(4-benzotriazol-1-yl-pipérazin-1-yl)-butyl]-3-pyridin-3-yl-acrylamide ;
le N-{4-[4-(hydroxydiphénylméthyl)-pipéridin-1-yl]-butyl}-2-(pyridin-3-yloxy)-acétamide ;
le N-[4-(4,4-diphénylpipérazin-1-yl)-butyl]-3-pyridin-3-yl-acrylamide ;
le semi-isopropanol de dichlorhydrate de N-{4-[4-(6,11-dihydrodibenzo[b,e]thiépin-11-ylidèn)-pipéridin-1-yl]-butyl}-3-pyridin-3-yl-propionamide ;
le N-{4-[4-(6,11-dihydrodibenzo[b,e]thiépin-11-ylidèn)-pipéridin-1-yl]-butyl}-5-pyridin-3-yl-pentanamide ;
le N-{4-[4-(4,9-dihydro-thiéno[2,3-b]-benzo[b,e]-thiépin-4-ylidèn)-pipéridin-1-yl]-butyl}-3-pyridin-3-yl-propionamide ;
le N-{4-[4-(4,9-dihydro-thiéno[2,3-b]-benzo[b,e]-thiépin-4-ylidèn)-pipéridin-1-yl]-butyl}-3-pyridin-3-yl-acrylamide ;
le N-[4-(4-diphénylphosphinoyloxypipérazin-1-yl)-butyl]-3-pyridin-3-yl-acrylamide ;
le N-[4-(1,4-dioxa-8-azaspiro[4,5]déc-8-yl)-butyl]-3-pyridin-3-yl-acrylamide ;
le N-[4-(2,5-dioxo-3,4-diphényl-2,5-dihydropyrrol-1-yl)-butyl]-3-pyridin-3-yl-acrylamide ;
le N-[4-(2,6-dioxo-4-phényl-pipéridin-1-yl)-butyl]-3-pyridin-3-yl-acrylamide ;
le N-[4-(1,3-dioxo-4,5,6,7-tétraphényl-1,3-dihydroisoindol-2-yl)-butyl]-3-pyridin-3-ylacrylamide ;
le N-[4-(3-benzyl-2,4,5-trioxo-imidazolidin-1-yl)-butyl]-3-pyridin-3-yl-acrylamide ;
le N-[4-(1,3,10-trioxo-1,4,5,6,10,10a-hexahydro-acénaphto[1,8a-c]pyrrol-2-yl)-butyl]-3-pyridin-3-ylacrylamide ;
le N-[4-(2,5-dioxo-4,4-diphénylimidazolidin-1-yl)-butyl]-3-pyridin-3-yl-acrylamide ;
le N-[4-(2,5-dioxo-3-phényl-2,5-dihydropyrrol-1-yl)-butyl]-3-pyridin-3-yl-acrylamide ;
le N-[3-(2,5-dioxo-3,4-diphényl-2,5-dihydropyrrol-1-yl)-propyl]-3-pyridin-3-yl-acrylamide ;
N-[4-(3-pyridin-3-yl-acroylamino)-butyl]-2,3:5,6-dibenzobicyclo[2.2.2]octan-7,8-dicarboximide ;
le N-[4-(5-benzylidèn-2,4-dioxothiazolidin-3-yl)-butyl]-3-pyridin-3-yl-acrylamide ;
le N-[4-(4-benzyl-2,6-dioxopipérazin-1-yl)-butyl]-3-pyridin-3-yl-acrylamide ;
le N-[6-(2,5-dioxo-3,4-diphényl-2,5-dihydropyrrol-1-yl)-hexyl]-3-pyridin-3-yl-acrylamide ;
le N-[4-(2,5-dioxo-3,4-diphényl-2,5-dihydropyrrol-1-yl)-butyl]-3-pyridin-3-yl-propionamide ;
le N-[4-(1,3-dioxo-1,3-dihydroisoindol-2-yl)-butyl]-3-pyridin-3-yl-acrylamide ;
le N-[4-(1,3-dioxo-1H,3H-benzo[de]isoquinolin-2-yl)-butyl]-3-(1-oxidopyridin-3-yl-acrylamide ;
le N-[6-(1,3-dioxo-1H,3H-benzo[de]isoquinolin-2-yl)-hexyl]-3-pyridin-3-yl-acrylamide ;
le N-[2-(1,3-dioxo-1H,3H-benzo[de]isoquinolin-2-yl)-éthyl]-3-pyridin-3-yl-acrylamide ;
le N-[4-(1,3-dioxo-1H,3H-benzo[de]isoquinolin-2-yl)-butyl]-3-pyridin-3-yl-acrylamide ;
le chlorhydrate de N-[8,8-bis(4-fluorophényl)octyl]-3-pyridin-3-yl-acrylamide ;
le N-[6-(3,3-diphényluréido)hexyl]-3-pyridin-3-yl-acrylamide ;
le N-[4-(1-phényl-1,2,3,4-tétrahydrobenzo[d]azépin-3-yl)-butyl]-3-pyridin-3-yl-acrylamide ;
le N-(8,8-diphényloctyl)-3-pyridin-3-yl-acrylamide ;
le N-(8-hydroxy-8,8-diphényloctyl)-3-pyridin-3-yl-acrylamide ;
le N-[4-(3,3-diphényluréido)butyl]-3-pyridin-3-yl-acrylamide ;
le N-[4-[1-(1H,3H-benzo[de]isoquinolin-2-yl)-butyl]-3-pyridin-3-yl-acrylamide ;
le N-[6-(10,11-dihydrodibenzo[b,f]azépin-5-yl-carbonylamino)-hexyl]-3-pyridin-3-yl-acrylamide ;
le 3-pyridin-3-yl-N-[6-(tosylamino)-hexyl]-acrylamide ;
le N-[4-(1,1-dioxo-1-thia-2-aza-acénaphtylèn-2-yl)-butyl]-3-pyridin-3-yl-acrylamide ;
le N-(6-hydroxy-6,6-diphénylhexyl)-3-pyridin-3-yl-acrylamide ;
le N-(6,6-diphényl-hex-5-ényl)-3-pyridin-3-yl-acrylamide ;
le N-[4-(4,5-diphénylimidazol-1-yl)-butyl]-3-pyridin-3-yl-acrylamide ;
le N-[4-(trans-2-phénylcyclopropylcarbonylamino)-butyl]-3-pyridin-3-yl-acrylamide ;
le N-(5-hydroxy-5,5-diphénylgentyl)-3-pyridin-3-yl-acrylamide ;
le N-(7-phénylheptyl)-3-pyridin-3-yl-acrylamide ;
le N-(4-diphénylacétylamino-butyl]-3-pyridin-3-yl-acrylamide ;
le N-[4-(benzhydrylamino)-butyl]-3-pyridin-3-yl-acrylamide ; et
le N-(4-{[2-(benzhydrylméthylamino)-éthyl]méthylamino}-butyl)-3-pyridin-3-yl-acrylamide.

3. Composition selon la revendication 1 ou 2 comprenant un autre agent cancérostatique ou immunosuppresseur qui n'est pas un composé de formule (I).

4. Composition selon l'une quelconque des revendications 1 à 3, dans laquelle le ou les composés de formule (I) et le ou les composés de formules (II) à (Vb) sont contenus séparément au sein de la composition.

5. Composition selon l'une quelconque des revendications 1 à 4, dans laquelle le ou les composés de formule (I) et le ou les composés de formules (II) à (Vb) sont présents sous des formes galéniques séparées et les formes galéniques sont emballées ensemble pour une administration conjointe.

6. Composition selon l'une quelconque des revendications 1 à 5, qui contient du tryptophane en tant que précurseur de médicament.

7. Composition selon l'une quelconque des revendications 1 à 6, qui est :
sous une forme solide administrable par voie perorale sous la forme d'un comprimé, d'une capsule, d'un comprimé enrobé, éventuellement sous forme de préparation à action modifiée ou résistante au fluide gastrique ou en tant que solution, suspension, comprimé effervescent, administrable sous une forme liquide par voie perorale, sous la forme de comprimés ou de sachets, éventuellement sous une forme à action modifiée,
sous la forme d'une préparation injectable ou perfusable appropriée conjointement avec des supports et adjuvants pharmaceutiquement acceptables appropriés, éventuellement sous une forme à action modifiée ou sous une forme médicinale de dépôt parentéral ou d'implant ou sous la forme d'un concentré, d'une poudre ou d'un produit lyophilisé,
sous la forme d'un agent thérapeutique d'inhalation, sous la forme d'un pulvérisateur avec des propulseurs, supports et adjuvants pharmaceutiquement acceptables appropriés,
sous la forme d'un système thérapeutique transdermique pour un traitement systémique,
sous la forme d'un système thérapeutique gastro-intestinal (GITS) pour un traitement systémique,
sous la forme d'un onguent, d'une suspension, d'une émulsion, d'un baume ou d'un emplâtre ou sous la forme d'une solution applicable par voie externe,
sous la forme d'émulsions administrables par voie rectale, génitale, ou transuréthrale, d'une solution, d'une solution liposomale, d'un implant, d'un suppositoire ou d'une capsule,
sous la forme d'une composition capable d'être appliquée de façon nasale, otologique ou ophtalmologique, ou
sous une forme applicable buccalement.

8. Composition selon l'une quelconque des revendications 1 à 6 pour une administration au moyen d'un aérosol à dosage contrôlé ou sous la forme d'une formulation à dose de poudre sèche.

9. Composition selon l'une quelconque des revendications 1 à 8, dans laquelle une unité posologique pour une administration individuelle contient 0,001 à 1000, 2000, 3000, 4000 ou 5000 mg du ou des composés de formule (I).

10. Utilisation d'un composé présentant une activité de vitamine PP ou un précurseur de médicament de celui-ci, pour la fabrication d'une composition pharmaceutique pour prévenir, réduire ou éliminer les effets secondaires ou pour neutraliser les effets secondaires d'un agent cancérostatique ou immunosuppresseur administré à titre prophylactique ou thérapeutique à un patient.

11. Utilisation selon la revendication 10, dans laquelle le composé présentant une activité de vitamine PP ou un précurseur de médicament de celui-ci est choisi dans le groupe constitué par les composés de formules II, IIa, IIb, III, IIIa, IIIb, IIIc, IV, IVa, IVb, V, Va et Vb : 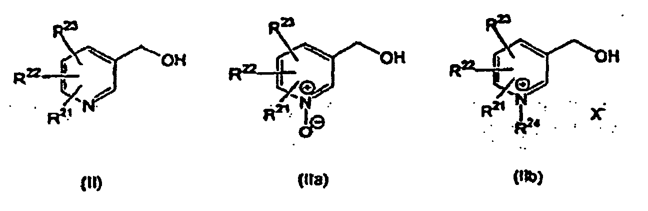 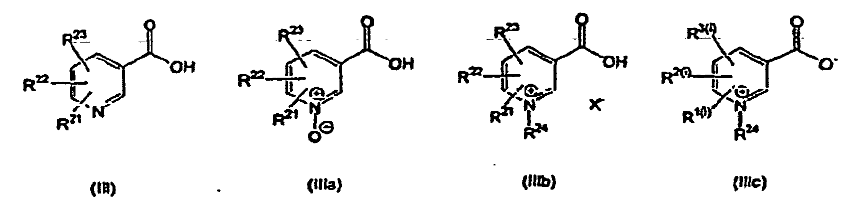 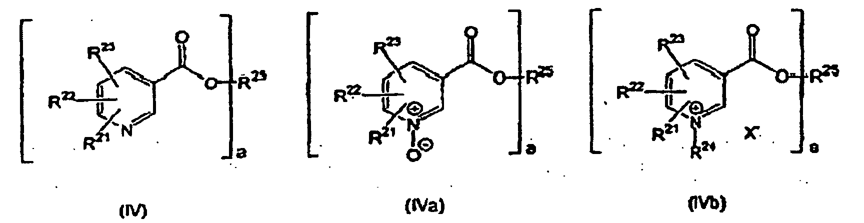 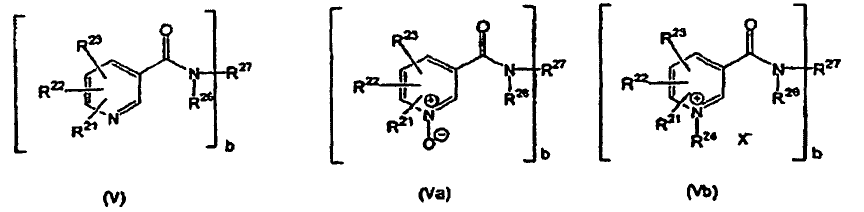 dans lesquelles :
a est un nombre entier de 1 à 6 ;
b est un nombre entier de 1 à 2 ;
X⁻ est choisi dans le groupe constitué par un fluorure, un chlorure, un bromure, un iodure, un hydrogénosulfate, un méthanesulfonate, un trifluorométhanesulfonate, un tosylate, un tétrafluoroborate, un dihydrogénophosphate et un acétate ;
R²¹ est choisi dans le groupe constitué par un hydrogène, un halogène, un cyano, un alkyle en C₁-C₆, un trifluorométhyle, un hydroxyalkyle en C₁-C₆, un hydroxy, un alcoxy en C₁-C₆, un alcanoyloxy en C₁-C₇, un alkylthio en C₁-C₆, un aminoalkyle en C₁-C₆, un amino, un alkylamino en C₁-C₆, un dialkylamino en C₂-C₁₂, un formyle, un alcoxycarbonyle en C₂-C₇, un aminocarbonyle, un alkylaminocarbonyle en C₂-C₇, un dialkylaminocarbonyle en C₃-C₁₃ et un carboxy ;
R²² est choisi dans le groupe constitué par un hydrogène, un halogène, un alkyle en C₁-C₆, un trifluorométhyle, un hydroxyalkyle en C₁-C₆, un hydroxy, un alcoxy en C₁-C₆, un alcanoyloxy en C₁-C₇, un aminoalkyle en C₁-C₆, un amino, un alcoxycarbonyle en C₂-C₇, un aminocarbonyle et un carboxy ;
R²³ est choisi dans le groupe constitué par un hydrogène, un alkyle en C₁-C₆ et un hydroxyalkyle en C₁-C₆ ;
R²⁴ est choisi dans le groupe constitué par un alkyle en C₁-C₆, un alcényle en C₃-C₆, un hydroxyalkyle en C₂-C₆, un alcoxyalkyle en C₂-C₆ et un benzyle ;
R²⁵ est tel que l'alcool R²⁵(OH)ₐ est choisi parmi les ω-dialkylarninoalcanols et alcanols en C₁-C₁₀, monovalents, linéaires et ramifiés, l'alcool benzylique, les diols en C₂-C₁₀ divalents, linéaires et ramifiés, les cycloalcanols en C₅-C₇ mono ou divalents, les cycloalcanediols en C₅-C₇, les cycloalcaneméthanols en C₅-C₇, les hétérocyclométhanols en C₅-C₇ saturés, la glycérine, le 2,2-bis(hydroxyméthyl)-1-octanol, l'érythritol, le pentaérythritol, l'arabitol, le xylitol, le sorbitol, le mannitol, l'isosorbitol, le tétra(hydrométhyl)cyclohexanol et l'inositol ;
R²⁶ est choisi dans le groupe constitué par un hydrogène, un alkyle en C₁-C₆, un hydroxyalkyle en C₁-C₆, un alcoxyalkyle en C₃-C₆, un aminoalkyle en C₁-C₆, un dialkylaminoalkyle en C₄-C₁₂ et un carboxyméthyle ;
lorsque b 1,
R²⁷ est choisi dans le groupe constitué par un hydrogène, un alkyle en C₁-C₆, un hydroxyalkyle en C₁-C₆, un alcoxyalkyle en C₃-C₆, un aminoalkyle en C₁-C₆, un dialkylaminoalkyle en C₄-C₁₂ et un carboxyméthyle ;
lorsque b est 2,
R²⁷ est un alkylène en C₂-C₁₀, ou un alkylène en C₅-C₁₀, où un groupe méthylène est remplacé de façon isostérique par O, NH ou N-alkyle ;
et leurs analogues thioxo,
et les sels d'addition d'acide ou sels anioniques de ceux-ci.

12. Utilisation selon la revendication 10 ou 11, dans laquelle le composé présentant une activité de vitamine PP ou un précurseur de médicament de celui-ci est choisi dans le groupe constitué par l'acide nicotinique, le nicotinamide et leurs dérivés ester et amide, sels anioniques, quaternaires et d'addition et dérivés analogues thioxo pharmaceutiquement acceptables, leurs isomères et les précurseurs de médicament de ceux-ci.

13. Utilisation selon l'une quelconque des revendications 10 à 12, dans laquelle le composé présentant une activité de vitamine PP ou un précurseur de médicament de celui-ci est choisi dans le groupe constitué par l'acide nicotinique, le nicotinamide et les mélanges de ceux-ci.

14. Utilisation selon l'une quelconque des revendications 10 à 12, dans laquelle le composé présentant une activité de vitamine PP ou un précurseur de médicament de celui-ci est le tryptophane.

15. Utilisation selon l'une quelconque des revendications 10 à 14, dans laquelle l'agent cancérostatique ou immunosuppresseur est choisi dans le groupe constitué par les composés de formule (I) : 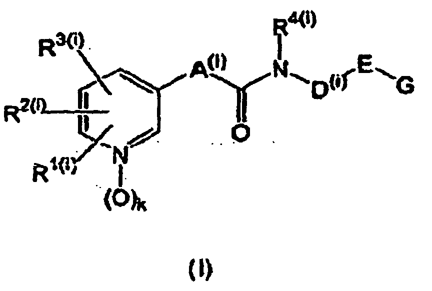 dans laquelle :
R¹⁽ⁱ⁾ est choisi parmi
un hydrogène, un halogène, un cyano, un alkyle en C₁-C₆, un alcényle en C₃-C₆, un alcynyle en C₂-C₆, un trifluorométhyle, un hydroxy, un cycloalkyle en C₃-C₈, un hydroxyalkyle en C₁-C₆, un alcoxy en C₁-C₆, un alcényloxy en C₃-C₆, un alcynyloxy en C₃-C₆, un benzyloxy, un alcanoyloxy en C₁-C₇, un alcoxycarbonyloxy en C₁-C₇, un alkylthio en C₁-C₆, un alcénylthio en C₃-C₆, un alcynylthio en C₃-C₆, un cycloalkyloxy en C₃-C₈, un cycloalkylthio en C₃-C₈, un alcoxycarbonyle en C₂-C₇, un aminocarbonyle, un alkylaminocarbonyle en C₂-C₇, un dialkylaminocarbonyle en C₃-C₁₃, un carboxy, un phényle, un phénoxy, un phénylthio, un pyridyloxy, un pyridylthio et NR⁵⁽ⁱ⁾R⁶⁽ⁱ⁾, où R⁵⁽ⁱ⁾ et R⁶⁽ⁱ⁾ sont choisis indépendamment l'un de l'autre parmi un hydrogène, un alkyle en C₁-C₆, un alcényle en C₃-C₆, un alcynyle en C₃-C₆, un benzyle et un phényle ;
R²⁽ⁱ⁾ est choisi parmi
un hydrogène, un halogène, un cyano, un alkyle en C₁-C₆, un trifluorométhyle, un hydroxy, un alcoxy en C₁-C₆, un benzyle et un alcanoyloxy en C₁-C₆ ; ou
R¹⁽ⁱ⁾ et R²⁽ⁱ⁾ lorsqu'ils sont adjacents forment éventuellement un pont choisi parmi
-(CH₂)₄-, -(CH=CH)₂- et -CH₂O-CR⁷⁽ⁱ⁾R⁸⁽ⁱ⁾-O- où
R⁷⁽ⁱ⁾ et R⁸⁽ⁱ⁾ sont choisis indépendamment l'un de l'autre parmi un hydrogène et un alkyle en C₁-C₆ ;
R³⁽ⁱ⁾ est choisi parmi
un hydrogène, un halogène, un alkyle en C₁-C₆, un trifluorométhyle et un hydroxyalkyle en C₁-C₆ ;
R⁴⁽ⁱ⁾ est choisi parmi
un hydrogène, un hydroxy, un alkyle en C₁-C₆, un alcényle en C₃-C₆, un alcynyle en C₃-C₆, un cycloalkyle en C₃-C₆, un alcoxy en C₁-C₆ et un benzyloxy ;
k est 0 ou 1,
A⁽ⁱ⁾ est choisi parmi
un alkylène en C₁-C₆, éventuellement substitué une à trois fois par un alkyle en C₁-C₆, un alcoxy en C₁-C₃, un hydroxy, un fluor, ou un phényle,
un alkylène en C₂-C₆, où un motif méthylène est remplacé de façon isostérique par O, S, NR⁹⁽ⁱ⁾, CO, SO ou SO₂ où, à l'exception de CO, la substitution isostérique n'est pas adjacente au groupe amide, et R⁹⁽ⁱ⁾ est choisi parmi un hydrogène, un alkyle en C₁-C₆, un alcényle en C₃-C₆, un alcynyle en C₃-C₆, un acyle en C₃-C₆ et un alcanesulfonyle en C₁-C₆,
le 1,2-cyclopropylène,
un alcénylène en C₂-C₆, éventuellement substitué une à trois fois par un alkyle en C₁-C₆, un hydroxy, un alcoxy en C₁-C₃, un fluor, un cyano ou un phényle,
un alcadiénylène en C₄-C₆, éventuellement substitué une fois ou deux fois par un alkyle en C₁-C₃, un fluor, un cyano ou un phényle,
un 1,3,5-hexatriénylène, éventuellement substitué par un alkyle en C₁-C₆, un fluor, un cyano ou un phényle, et
l'éthynylène ;
D⁽ⁱ⁾ est choisi parmi
un alkylène en C₁-C₁₂, éventuellement substitué une fois ou deux fois par un alkyle en C₁-C₆, un hydroxy, un alcoxy en C₁-C₆ ou un phényle,
un alcénylène en C₂-C₁₂ ou alcadiénylène en C₄-C₁₂, éventuellement substitué une fois ou deux fois par un alkyle en C₁-C₆, un hydroxy, un alcoxy en C₁-C₆ ou un phényle, où une double liaison peut éventuellement se présenter au cycle E dans le cas où le cycle E est lié sur un atome de C,
un alcynylène en C₃-C₁₂ ou alcénynylène en C₄-C₁₂, éventuellement substitué une fois ou deux fois par un alkyle en C₁-C₆, un hydroxy, un alcoxy en C₁-C₆ ou un phényle, et
un alkylène en C₁-C₁₂, alcénylène en C₂-C₁₂ ou alcynylène en C₃-C₁₂ où un à trois motifs méthylène, à l'exception du groupe méthylène (G) - terminal dans le cas où E représente une liaison, sont remplacés de façon isostérique par O, S, NR¹⁰⁽ⁱ⁾, CO, SO ou SO₂ où, R¹⁰⁽ⁱ⁾ a la même signification que R⁹⁽ⁱ⁾, mais est choisi indépendamment de celui-ci ;
E est choisi parmi E¹⁽ⁱ⁾, E²⁽ⁱ⁾, E³, E⁴, E⁵ et E⁶, où
E¹⁽ⁱ⁾ est 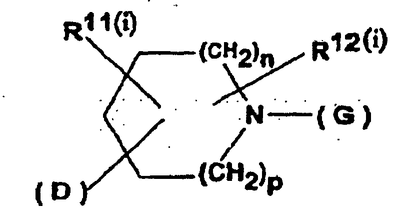
E²⁽ⁱ⁾ est 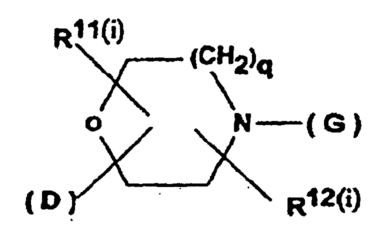
E³ est 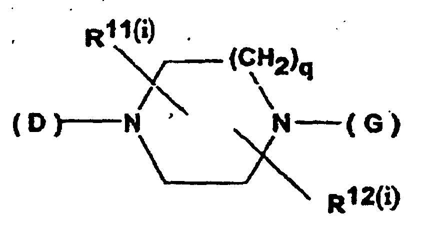
E⁴ est 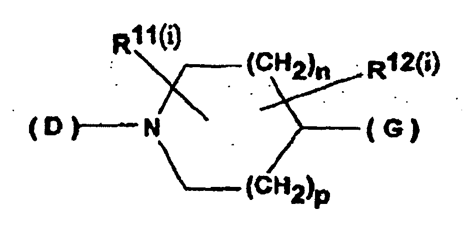
E⁵ est 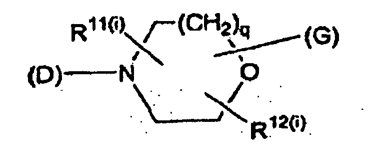 et
E⁶ représente une liaison simple ou double,
où les noyaux hétérocycliques E¹⁽ⁱ⁾ à E⁵ ont éventuellement une double liaison,
n et p sont, indépendamment l'un de l'autre, 0, 1, 2 ou 3, à condition que n + p ≤ 4,
q est 1, 2 ou 3 ;
R¹¹⁽ⁱ⁾ est choisi parmi
un hydrogène, un alkyle en C₁-C₆, un hydroxy, un hydroxyméthyle, un carboxy et un alcoxycarbonyle en C₂-C₇ ;
R¹²⁽ⁱ⁾ est choisi parmi
un hydrogène, un alkyle en C₁-C₆ et un groupe oxo adjacent à un atome d'azote, ou
R¹¹⁽ⁱ⁾ et R¹²⁽ⁱ⁾, éventuellement conjointement, forment un pont alkylène en C₁-C₃ par formation d'un système à noyau bicyclique, et
(a) dans le cas où E représente E¹⁽ⁱ⁾, E²⁽ⁱ⁾ ou E³, le substituant G est éventuellement choisi parmi G¹⁽ⁱ⁾, G²⁽ⁱ⁾, G³⁽ⁱ⁾, G⁴⁽ⁱ⁾ et G⁵⁽ⁱ⁾, où
G¹⁽ⁱ⁾ est
-(CH₂)ᵣ- (CR¹⁴⁽ⁱ⁾R¹⁵⁽ⁱ⁾)_{*s*}-R¹³⁽ⁱ⁾
où
r est 0 à 3, et
s est 0 ou 1,
R¹³⁽ⁱ⁾ est choisi parmi
un hydrogène, un alkyle en C₁-C₆, un alcényle en C₃-C₆, un alcynyle en C₃-C₆ et un cycloalkyle en C₃-C₈ ;
des hétérocycles, saturés ou insaturés, de quatre à sept chaînons, qui contiennent un ou deux hétéroatomes choisis parmi N, S et O ;
un benzyle, un phényle ;
des hétérocycles monocycliques aromatiques de cinq ou six chaînons, qui contiennent un à trois hétéroatomes choisis parmi N, S et O, et qui sont liés soit directement soit sur un groupe méthylène ;
des systèmes cycliques carbocycliques, partiellement hydrogénés ou aromatiques bi- et tricycliques condensés, avec 8 à 16, 17 ou 18 atomes de cycle et au moins un cycle aromatique, où la liaison se produit soit sur un cycle aromatique soit sur un cycle hydrogéné et soit directement soit sur un groupe méthylène ;
des systèmes cycliques hétérocycliques partiellement hydrogénés ou aromatiques bi- et tricycliques condensés, avec 8 à 16, 17 ou 18 atomes de cycle et au moins un cycle aromatique, où un à trois atomes de cycle sont choisis parmi N, S et O et la liaison se produit soit sur un cycle aromatique soit sur un cycle hydrogéné et soit directement soit sur un groupe méthylène ;
R¹⁴⁽ⁱ⁾ a la même signification que R¹³⁽ⁱ⁾, mais est choisi indépendamment de celui-ci ;
R¹⁵⁽ⁱ⁾ est choisi parmi
un hydrogène, un hydroxy, un méthyle, un benzyle, un phényle,
des hétérocycles monocycliques aromatiques de cinq ou six chaînons qui contiennent un à trois hétéroatomes choisis parmi N, S et O et qui sont liés soit directement soit sur un groupe méthylène ;
des systèmes cycliques carbocycliques, partiellement hydrogénés ou aromatiques bi- et tricycliques condensés, avec 8 à 16, 17 ou 18 atomes de cycle et au moins un cycle aromatique, où la liaison se produit soit sur un cycle aromatique soit sur un cycle hydrogéné et soit directement soit sur un groupe méthylène ;
des systèmes cycliques hétérocycliques partiellement hydrogénés ou aromatiques bi- et tricycliques condensés, avec 8 à 16, 17 ou 18 atomes de cycle et au moins un cycle aromatique, où un à trois atomes de cycle sont choisis parmi N, S et O et la liaison se produit soit sur un cycle aromatique soit sur un cycle hydrogéné et soit directement soit sur un groupe méthylène ;
G²⁽ⁱ⁾ est choisi parmi 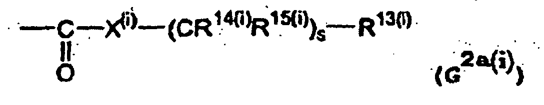 et 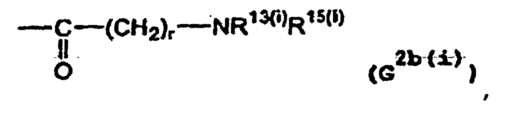 où r, s et les substituants R¹³⁽ⁱ⁾, R¹⁴⁽ⁱ⁾ et R¹⁵⁽ⁱ⁾ ont les significations ci-dessus,
ou le groupe -NR¹³⁽ⁱ⁾R¹⁵⁽ⁱ⁾ est un hétérocycle contenant de l'azote lié sur l'atome d'azote, lequel hétérocycle contenant de l'azote est choisi parmi
des hétérocycles saturés et insaturés, monocycliques, de quatre à huit chaînons, qui en dehors de l'atome d'azote essentiel, contiennent éventuellement un ou deux autres hétéroatomes choisis parmi N, S et O,
et
des hétérocycles saturés et insaturés, bi- ou tricycliques, condensés ou pontés, avec 8 à 16, 17 ou 18 atomes de cycle, qui, en dehors de l'atome d'azote essentiel, contiennent éventuellement un ou deux autres hétéroatomes choisis parmi N, S et O,
X⁽ⁱ⁾ est choisi parmi
un méthylène, un éthylène, un éthénylène, un propylène, un cycloalkylène en C₃-C₇, ou représente une liaison ;
G³⁽ⁱ⁾ est
-SO₂-(CH₂)ᵣ-R¹³⁽ⁱ⁾
où r et R¹³⁽ⁱ⁾ ont les significations ci-dessus,
G⁴⁽ⁱ⁾ est 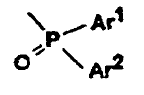 où
Ar¹ et Ar² sont choisis indépendamment l'un de l'autre parmi un phényle, un pyridyle et un naphtyle ;
G⁵⁽ⁱ⁾ est
-COR¹⁶⁽ⁱ⁾
où
R¹⁶⁽ⁱ⁾ est choisi parmi
un trifluorométhyle, un alcoxy en C₁-C₆, un alcényloxy en C₃-C₆ et un benzyloxy,
(c) dans le cas où E est E⁴ ou E⁵,
alors G est éventuellement G¹⁽ⁱ⁾, G²⁽ⁱ⁾, G⁶⁽ⁱ⁾, G⁷ ou G⁸,
où G¹⁽ⁱ⁾ et G²⁽ⁱ⁾ ont les significations ci-dessus et
G⁶⁽ⁱ⁾ est
= (C)ᵤR¹³⁽ⁱ⁾R¹⁵⁽ⁱ⁾,
où R¹³⁽ⁱ⁾ et R¹⁵⁽ⁱ⁾ ont les significations ci-dessus et
u est 0 ou 1,
ou lorsque u = 1, alors R¹³⁽ⁱ⁾ et R¹⁵⁽ⁱ⁾ conjointement avec l'atome de carbone sur lequel ils sont attachés forment un système cyclique choisi parmi
un cycloalkyle en C₃-C₈,
des hétérocycles saturés, de quatre à sept chaînons qui contiennent éventuellement un ou deux hétéroatomes, choisis parmi N, S et O ;
des systèmes cycliques carbocycliques bi- et tricycliques condensés partiellement hydrogénés, avec 8 à 16, 17 ou 18 atomes de cycle et au moins un cycle aromatique ;
des systèmes cycliques hétérocycliques bi- et tricycliques condensés partiellement hydrogénés, avec 8 à 16, 17 ou 18 atomes de cycle et au moins un cycle aromatique, où un à trois atomes de cycle sont choisis parmi N, S et O ;
ou lorsque u = 0, alors R¹³⁽ⁱ⁾ et R¹⁵⁽ⁱ⁾ conjointement avec l'atome de carbone du cycle E sur lequel ils sont attachés forment un système cyclique choisi parmi
un groupe cycloalkyle en C₃-C₈,
des hétérocycles saturés, de quatre à sept chaînons qui contiennent un ou deux hétéroatomes, choisis parmi N, S et O ;
des systèmes cycliques carbocycliques, bi- et tricycliques condensés partiellement hydrogénés, avec 8 à 16, 17 ou 18 atomes de cycle et au moins un cycle aromatique ;
des systèmes cycliques hétérocycliques, bi- et tricycliques condensés partiellement hydrogénés avec 8 à 16, 17 ou 18 atomes de cycle et au moins un cycle aromatique, où un à trois atomes de cycle sont choisis parmi N, S et O ;
G⁷ est choisi parmi
-**NR**^{**17(i)**}**-(CH**_{**2**}**)**_{**r**}**-(CR**^{**14(i)**}**R**^{**15(i)**}**)**_{**s**}**-R**^{**13(i)**} **(G**^{**7a**}**),** 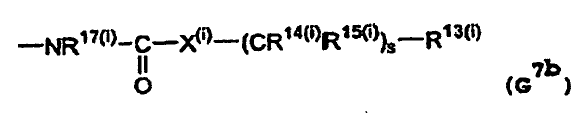 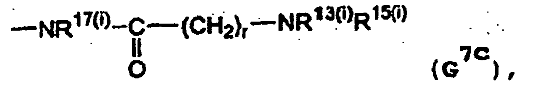
**-NR**^{**17(i)**}**-SO**_{**2**}**-(CH**_{**2**}**)**_{**r**}**-R**^{**13(i)**} **(G**^{**7d**}**),** 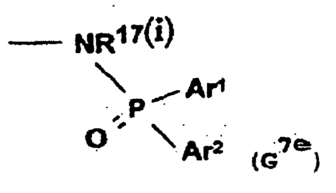
et
-**NR**^{**17(i)**}**-COR**^{**16(i)**} **(G**^{**7f**}**),**
où r, s, X⁽ⁱ⁾, les substituants R¹³⁽ⁱ⁾, R¹⁴⁽ⁱ⁾, R¹⁵⁽ⁱ⁾ et R¹⁶⁽ⁱ⁾, et le groupe
-NR¹³⁽ⁱ⁾R¹⁵⁽ⁱ⁾,
ont les significations ci-dessus, et
R¹⁷⁽ⁱ⁾ a les mêmes significations que R⁵⁽ⁱ⁾, mais est choisi indépendamment de celui-ci ;
Ar¹ et Ar² sont choisis indépendamment l'un de l'autre parmi un phényle, un pyridyle et un naphtyle ;
G⁸ est choisi parmi 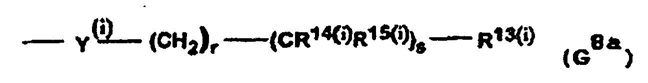 et 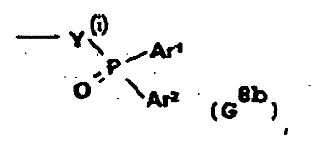 où r, s et les substituants R¹³⁽ⁱ⁾, R¹⁴⁽ⁱ⁾, R¹⁵⁽ⁱ⁾, Ar¹ et Ar², ont les significations ci-dessus, et
Y⁽ⁱ⁾ est O ou S ;
(c) dans le cas où le substituant E est E⁶,
alors le substituant G est éventuellement choisi parmi G^{7d}, G^{7e}, G^{8b}, G⁹, G¹⁰, G¹¹, G¹² et G¹³, où G^{7d}, G^{7e} et G^{8b} ont les significations ci-dessus et
G⁹ est choisi parmi
-(CR¹³⁽ⁱ⁾R¹⁹)ₛ-R¹⁸ (G^{9a})
et
-NR¹³⁽ⁱ⁾R¹⁸ (G^{9b}),
où s et R¹³⁽ⁱ⁾ sont tels que définis ci-dessus, et
R¹⁸ est choisi parmi
un benzyle, un diphénylméthyle, un phényle ;
des hétérocycles monocycliques aromatiques de cinq et six chaînons qui peuvent contenir un à trois hétéroatomes choisis parmi N, S et O et qui sont liés soit directement soit sur un groupe méthylène ;
des systèmes cycliques carbocycliques bi- et tricycliques partiellement hydrogénés ou aromatiques condensés, avec 8 à 16, 17 ou 18 atomes de cycle et au moins un cycle aromatique, où la liaison se produit soit sur un cycle aromatique soit sur un cycle hydrogéné et soit directement soit sur un groupe méthylène ; et
des systèmes cycliques hétérocycliques bi- et tricycliques partiellement hydrogénés ou aromatiques condensés, avec 8 à 16, 17 ou 18 atomes de cycle et au moins un cycle aromatique, où un à trois atomes de cycle sont choisis parmi N, S et O et la liaison se produit soit sur un cycle aromatique soit sur un cycle hydrogéné et soit directement soit sur un groupe méthylène ;
R¹⁹ a les mêmes significations que R¹³⁽ⁱ⁾, mais est choisi indépendamment de celui-ci et, de plus, il peut être un groupe hydroxy ;
ou le groupe
-NR¹³⁽ⁱ⁾R¹⁸
est éventuellement un hétérocycle contenant de l'azote lié sur l'atome d'azote, lequel hétérocycle contenant de l'azote est choisi parmi
des systèmes cycliques hétérocycliques bi- et tricycliques partiellement hydrogénés ou aromatiques condensés, avec 8 à 16, 17 ou 18 atomes de cycle et au moins un cycle aromatique, qui, en dehors de l'atome d'azote essentiel, contiennent éventuellement un ou deux autres hétéroatomes choisis parmi N, S et O ;
G¹⁰ est
=CR¹³⁽ⁱ⁾R¹⁸ (G¹⁰)
lié à D au moyen d'une double liaison, où R¹³⁽ⁱ⁾ et R¹⁸ ont les significations ci-dessus, ou bien où G¹⁰ est éventuellement un système cyclique lié sur l'atome de carbone choisi parmi
des systèmes cycliques carbocycliques bi- et tricycliques partiellement hydrogénés condensés, avec 8 à 16, 17 ou 18 atomes de cycle et au moins un cycle aromatique ; et
des systèmes cycliques hétérocycliques bi- et tricycliques partiellement hydrogénés condensés, avec 8 à 16, 17 ou 18 atomes de cycle et au moins un cycle aromatique, où un à trois atomes de cycle sont éventuellement choisis parmi N, S et O ;
G¹¹ est choisi parmi
-**NR**^{**17(i)**}**-(CH**_{**2**}**)**_{**r**}**-(CR**^{**13(i)**}**R**^{**19**}**)**_{**s**}**-R**^{**18**} **(G**^{**11a**}**),** 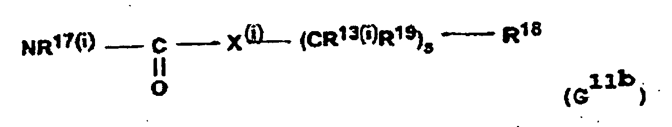
et 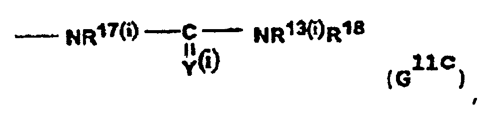 où r, s, X⁽ⁱ⁾, Y⁽ⁱ⁾, les substituants R¹³⁽ⁱ⁾, R¹⁷⁽ⁱ⁾, R¹⁸ et R¹⁹, et le groupe -NR¹³⁽ⁱ⁾R¹⁸, ont les significations ci-dessus ;
G¹² est
-Y⁽ⁱ⁾-(CH₂)ᵣ-(CR¹³⁽ⁱ⁾R¹⁹)ₛ-R¹⁸ (G¹²)
où r, s, Y⁽ⁱ⁾, les substituants R¹³⁽ⁱ⁾, R¹⁸ et R¹⁹ ont les significations ci-dessus ;
G¹³ est choisi parmi 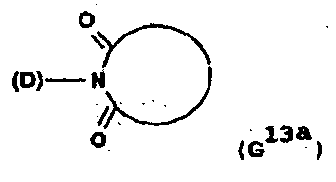 et 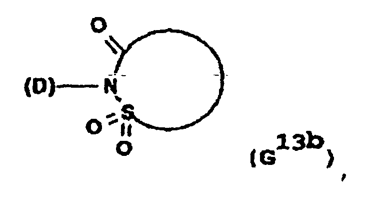 lié à D sur l'atome d'azote d'imide, choisi parmi
des imides monocycliques saturés et insaturés, avec 5 à 7 atomes de cycle, qui, en dehors de l'atome d'azote d'imide essentiel, contiennent éventuellement un ou deux autres hétéroatomes choisis parmi N, S et O ;
des imides bi-, tri- ou tétracycliques saturés, insaturés et aromatiques condensés, avec 8 à 18 atomes de cycle, qui, en dehors de l'atome d'azote d'imide essentiel, contiennent éventuellement un ou deux autres hétéroatomes choisis parmi N, S et O ;
des imides bi-, tri-, tétra- ou pentacycliques saturés et insaturés, pontés, avec 8 à 22 atomes de cycle, qui, en dehors de l'atome d'azote d'imide essentiel, contiennent éventuellement un ou deux autres hétéroatomes choisis parmi N, S et O ; et
des imides spirocycliques saturés et insaturés, éventuellement condensés un ou deux fois, et avec un total de 9 à 23 atomes de cycle, qui, en dehors de l'atome d'azote d'imide essentiel, contiennent éventuellement un ou deux autres hétéroatomes choisis parmi N, S et O ;
où ces imides cycliques sont éventuellement substitués par un à cinq des groupes identiques ou différents choisis indépendamment les uns des autres parmi
un halogène, un cyano, un alkyle en C₁-C₆, un alkylidène en C₁-C₆, un trifluorométhyle, un cycloalkyle en C₃-C₈, un cycloalkylidène en C₃-C₈, un phénylalkyle en C₁-C₃, un phénylalkylidène en C₁-C₃, un diphénylalkyle en C₁-C₃, un diphénylalkylidène en C₁-C₃, un triphénylméthyle, un phényle, un hydroxy, un hydroxyalkyle en C₁-C₆, un alcoxy en C₁-C₆, un alcoxy en C₁-C₆ entièrement ou partiellement substitué par le fluor, un benzyloxy, un phénoxy, un naphtyloxy, un mercapto, un alkylthio en C₁-C₆, un phénylthio, un naphtylthio, un pyridylthio, un alcanesulfonyle en C₁-C₆, un phénylsulfonyle, un naphtylsulfonyle, un pyridylsulfonyle, un sulfo, un carboxy, un carboxyalkyle en C₂-C₇, un carboxyalcényle en C₃-C₇, un alcoxycarbonyle en C₂-C₇, un benzyloxycarbnyle, un nitro, un amino, un aminoalkyle en C₁-C₆, un monoalkyl(en C₁-C₆)amino, dialkyl(en C₁-C₆)amino, un phénylamino, un phénylalkyl(en C₁-C₃)amino, un pyridylamino,
des hétérocycles saturés et insaturés, de quatre à sept chaînons, qui contiennent un ou deux hétéroatomes choisis parmi N, S et O, et qui sont liés soit directement soit sur un groupe méthylène ou un groupe méthine ;
des hétérocycles monocycliques aromatiques, de cinq et six chaînons, qui contiennent un à trois hétéroatomes choisis parmi N, S et O, et qui sont liés soit directement soit sur un groupe méthylène ou un groupe méthine ;
des systèmes cycliques carbocycliques, bicycliques, partiellement hydrogénés et aromatiques condensés, avec 8 à 12 atomes de cycle qui sont liés soit directement soit sur un groupe méthylène ou un groupe méthine ; et
des systèmes cycliques hétérocycliques, bicycliques, partiellement hydrogénés et aromatiques condensés, avec 8 à 12 atomes de cycle, où un à trois atomes de cycle sont choisis parmi N, S et O et qui sont liés soit directement soit ou sur un groupe méthylène ou un groupe méthine ;
où les systèmes cycliques aromatiques dans les substituants R¹⁽ⁱ⁾, R²⁽ⁱ⁾, R⁴⁽ⁱ⁾, R⁵⁽ⁱ⁾, R⁶⁽ⁱ⁾, R¹³⁽ⁱ⁾, R¹⁴⁽ⁱ⁾, R¹⁵⁽ⁱ⁾, R¹⁶⁽ⁱ⁾, R¹⁷⁽ⁱ⁾, R¹⁸, R¹⁹, Ar¹ et Ar², dans les groupes A⁽ⁱ⁾ et D⁽ⁱ⁾, dans les systèmes cycliques =CR¹³⁽ⁱ⁾R¹⁵⁽ⁱ⁾, =CR¹³⁽ⁱ⁾R¹⁸, -NR¹³⁽ⁱ⁾R¹⁵⁽ⁱ⁾ et -NR¹³⁽ⁱ⁾R¹⁸⁽ⁱ⁾, ainsi que les substituants et/ou les substituants dans les imides cycliques G¹³ sont éventuellement indépendamment substitués par un à trois des groupes identiques ou différents, choisis parmi
un halogène, un cyano, un alkyle en C₁-C₆, un trifluorométhyle, un cycloalkyle en C₃-C₈, un benzyle, un phényle, un hydroxy, un hydroxyalkyle en C₁-C₆, un alcoxy en C₁-C₆, un alcoxy en C₁-C₆ entièrement ou partiellement substitué par le fluor, un benzyloxy, un phénoxy, un mercapto, un alkylthio en C₁-C₆, un phénylthio, un sulfo, un carboxy, un carboxyalkyle en C₂-C₇, un carboxyalcényle en C₃-C₇, un alcoxycarbonyle en C₂-C₇, un benzyloxycarbonyle, un nitro, un amino, un aminoalkyle en C₁-C₆, un monoalkyl (en C₁-C₆)amino et un dialkyl(en C₁-C₆)amino et, dans le cas de deux résidus adjacents sur le cycle aromatique, un méthylènedioxy,
où les résidus alkyle et cycloalkyle dans les groupes G sont éventuellement substitués par un ou deux des groupes identiques ou différents, choisis parmi
un hydroxy, un carboxy, un alcoxycarbonyle en C₂-C₇, un benzyloxycarbonyle, un amino, un monoalkyl (en C₁-C₆)amino, et un dialkyl(en C₁-C₆)amino ;
et les stéréoisomères ou mélanges racémiques ou non racémiques des stéréoisomères de ceux-ci,
et les tautomères de ceux-ci lorsque G est un cycle aromatique hétérocyclique ou un cycle aromatique substitué par un hydroxy, un mercapto ou un amino,
et les sels d'addition d'acide pharmacologiquement acceptables de ceux-ci ;

16. Utilisation selon la revendication 15, dans laquelle le composé de formule (I) est choisi dans le groupe constitué par :
le N-[2-(1-benzylpipéridin-4-yl)-éthyl]-3-(pyridin-3-yl)-propionamide ;
le N-{2-[1-(2-phényléthyl)-pipéridin-4-yl]-éthyl}-3-(pyridin-3-yl)-propionamide ;
le N-{2-[1-(4-phénylbutyl)-pipéridin-4-yl]-éthyl}-3-(pyridin-3-yl)-propionamide ;
le N-{2-[1-(4-hydroxy-4-phénylbutyl)-pipéridin-4-yl]-éthyl}-3-(pyridin-3-yl)-propionamide ;
le N-[2-(1-diphénylméthylpipéridin-4-yl)-éthyl]-3-(pyridin-3-yl)-propionamide ;
le N-[3-(1-diphénylméthylpipéridin-4-yl)-propyl]-3-(pyridin-3-yl)-propionamide ;
le N-[4-(1-diphénylméthylpipéridin-4-yl)-propyl]-3-(pyridin-3-yl)-propionamide ;
le N-[4-(1-benzylpipéridin-4-yl)-butyl]-3-(pyridin-3-yl)-acrylamide ;
le N-{4-[1-(2-phényléthyl)-pipéridin-4-yl]-butyl}-3-(pyridin-3-yl)-acrylamide ;
le N-{4-[1-(4-biphényléthyl)-pipéridin-4-yl]-butyl}-3-(pyridin-3-yl)-acrylamide ;
le N-{4-[1-(1-naphtyléthyl)-pipéridin-4-yl]-butyl}-3-(pyridin-3-yl)-acrylamide ;
le N-{4-[1-(9-anthryléthyl)-pipéridin-4-yl]-butyl}-3-(pyridin-3-yl)-acrylamide ;
le N-{4-[1-(cyclohexylphényléthyl)-pipéridin-4-yl]-butyl}-3-(pyridin-3-yl)-acrylamide ;
le N-{4-[1-(10,11-dihydro-5H-dibenzo[a,d]cycloheptèn-5-yl)-pipéridin-4-yl]-butyl}-3-(pyridin-3-yl)acrylamide ;
le N-[2-(1-diphénylméthylpipéridin-4-yl)-éthyl]-3-(pyridin-3-yl)-acrylamide ;
le N-[3-(1-diphénylméthylpipéridin-4-yl)-propyl]-3-(pyridin-3-yl)-acrylamide ;
le N- [5-(1-diphénylméthylpipéridin-4-yl)-pentyl]-3-(pyridin-3-yl)-acrylamide ;
le N-[6-(1-diphénylméthylpipéridin-4-yl)-hexyl]-3-(pyridin-3-yl)-acrylamide ;
l'amide d'acide N-[4-(1-diphénylméthylpipéridin-4-yl)-butyl]-5-(pyridin-3-yl)-2,4-pentdiénique ;
le N-(4-{1-[bis(4-fluorophényl)-méthyl]-pipéridin-4-yl}-butyl)-3-(pyridin-3-yl)-acrylamide ;
le N-(4-{1-[bis(2-chlorophényl)-méthyl]-pipéridin-4-yl}-butyl)-3-(pyridin-3-yl)-acrylamide ;
le N-[4-(1-diphénylméthylpipéridin-4-yl)-butyl]-3-(2-fluoropyridin-3-yl)-acrylamide ;
le N-[4-(1-diphénylméthylpipéridin-4-yl)-butyl]-3-(6-fluoropyridin-3-yl)-acrylamide ;
le N-[4-(1-diphénylméthylpipéridin-4-yl)-butyl]-3-(pyridin-3-yl)-acrylamide ;
le dichlorhydrate de N-[4-(1-diphénylméthylpipéridin-4-yl)-butyl]-3-(pyridin-3-yl)-acrylamide ;
le méthanesulfonate de N-[4-(1-diphénylméthylpipéridin-4-yl)-butyl]-3-(pyridin-3-yl)-acrylamide ;
le N-[4-(1-acétylpipéridin-4-yl)-butyl]-3-(pyridin-3-yl)-propionamide ;
le N-[4-(1-benzoylpipéridin-4-yl)-butyl]-3-(pyridin-3-yl)-propionamide ;
le N-[4-(1-diphénylacétylpipéridin-4-yl)-butyl]-3-(pyridin-3-yl)-propionamide ;
le N-{4-[1-(9-oxo-9H-fluorén-4-carbonyl)-pipéridin-4-yl]-butyl}-3-(pyridin-3-yl)-propionamide ;
le N-[4-(1-méthylsulfonylpipéridin-4-yl)-butyl]-3-(pyridin-3-yl)-propionamide ;
le N-{4-[1-(2-naphtylsulfonyl)-pipéridin-4-yl]-butyl}-3-(pyridin-3-yl)-propionamide ;
le N-[4-(1-benzylpipéridin-4-yl)-butyl]-3-(pyridin-3-yl)-propionamide ;
le N-(4-{1-[bis(2-chlorophényl)-méthyl]-pipéridin-4-yl}-butyl)-3-(pyridin-3-yl)-propionamide ;
le N-{4-[1-(phénylpyridin-3-yl)-pipéridin-4-yl]-butyl}-3-(pyridin-3-yl)-propionamide ;
le N-{4-[1-(9H-fluorén-9-yl)-pipéridin-4-yl]-butyl}-3-(pyridin-3-yl)-propionamide ;
le N-{4-[1-(6,11-dihydrodibenzo[b,e]-oxépin-11-yl)-pipéridin-4-yl]-butyl}-3-(pyridin-3-yl)-propionamide ;
le N-{4-[1-(1-naphtylaminocarbonyl)-pipéridin-4-yl]-butyl}-3-(pyridin-3-yl)-propionamide ;
le N-[4-(1-diphénylaminocarbonyl-pipéridin-4-yl)-butyl]-3-(pyridin-3-yl)-propionamide ;
le N-{4-[1-(10,11-dihydrodibenzo[b,f]azépin-5-yl-carbonyl)-pipéridin-4-yl]-butyl}-3-(pyridin-3-yl)-propionamide ;
le N-[4-(1-diphénylphosphinoyl-pipéridin-4-yl)-butyl]-3-(pyridin-3-yl)-propionamide ;
le N-[4-(1-diphénylméthylpipéridin-4-yl)-butyl]-3-(2-fluoropyridin-3-yl)-propionamide ;
le N-[4-(1-diphénylméthylpipéridin-4-yl)-butyl]-3-(5-fluoropyridin-3-yl)-propionamide ;
le N-[4-(1-diphénylméthylpipéridin-4-yl)-butyl]-2-fluoro-3-(pyridin-3-yl)-propionamide ;
le N-[4-(1-diphénylméthylpipéridin-4-yl)-butyl]-2,2-difluoro-3-(pyridin-3-yl)-propionamide ;
le N-[5-(1-diphénylméthylpipéridin-4-yl)-gentyl]-3-(pyridin-3-yl)-propionamide ;
le N-[5-(1-diphénylméthylpipéridin-4-yl)-hexyl]-3-(pyridin-3-yl)-propionamide ;
l'amide d'acide N-[2-(1-diphénylméthylpipéridin-4-yl)-éthyl]-3-(pyridin-3-yl)-pentanoïque ;
l'amide d'acide N-[4-(1-diphénylmé-thylpipéridin-4-yl)-butyl]-3-(pyridin-3-yl)-pentanoique ;
le N-[4-(1-diphénylméthylpipéridin-4-yl)-butyl]-N-hydroxy-3-(pyridin-3-yl)-propionamide ;
le N-[4-(1-diphénylméthylpipéridin-4-yl)-butyl]-2-hydroxy-3-(pyridin-3-yl)-propionamide ;
le N-[4-(1-diphénylméthylpipéridin-4-yl)-butyl]-3-hydroxy-3-(pyridin-3-yl)-propionamide ;
le N-[4-(1-diphénylméthylpipéridin-4-yl)-butyl]-3-(pyridin-3-yl)-propionamide ;
le N-[4-(1-méthylsulfonylpipéridin-4-yl)-butyl]-3-(pyridin-3-yl)-acrylamide ;
le N-{4-[1-(2-naphtylsulfonyl)-pipéridin-4-yl]-butyl}-3-(pyridin-3-yl)-acrylamide ;
l'amide d'acide N-{4-[1-(2-naphtylsulfonyl)-pipéridin-4-yl]-butyl}-5-(pyridin-3-yl)-2,4-pentadiénique ;
le N-{4-[1-(1-naphtylaminocarbonyl)-pipéridin-4-yl]-butyl}-3-(pyridin-3-yl)-acrylamide ;
le N-[4-(1-diphénylaminocarbonylpipéridin-4-yl)-butyl]-3-(pyridin-3-yl)-acrylamide ;
l'amide d'acide N-[4-(1-diphénylaminocarbonylpipéridin-4-yl)-butyl]-5-(pyridin-3-yl)-2,4-pentadiénique ;
le N-{4-[1-(10,11-dihydrodibenzo[b,f]azépin-5-yl-carbonyl)-pipéridin-4-yl]-butyl}-3-(pyridin-3-yl)-acrylamide ;
le N-[4-(1-diphénylphosphinoylpipéridin-4-yl)-butyl]-3-(pyridin-3-yl)-acrylamide ;
le N-[4-(1-acétylpipéridin-4-yl)-butyl]-3-(pyridin-3-yl)-acrylamide ;
le N-[4-(1-diphénylacétylpipéridin-4-yl)-butyl]-3-(pyridin-3-yl)-acrylamide ;
le N-{4-[1-(3,3-diphénylpropionyl)-pipéridin-4-yl]-butyl}-3-(pyridin-3-yl)-acrylamide ;
le N-[4-(1-benzoylpipéridin-4-yl)-butyl]-3-(pyridin-3-yl)-acrylamide ;
l'amide d'acide N-[4-(1-benzoylpipéridin-4-yl)-butyl]-5-(pyridin-3-yl)-2,4-pentadiénique ;
le N-{4-[1-(9-oxo-9H-fluorén-4-carbonyl)-pipéridin-4-yl]-butyl}-3-(pyridin-3-yl)-acrylamide ;
le N-{4-[1-(phénylpyridin-3-ylméthyl)-pipéridin-4-yl]-butyl}-3-(pyridin-3-yl)-acrylamide ;
le N-{4-[1-(phénylpyridin-4-ylméthyl)-pipéridin-4-yl]-butyl}-3-(pyridin-3-yl)-acrylamide ;
le N-{4-[1-(6,11-dihydrodibenzo[b,e]oxépin-11-yl)-pipéridin-4-yl]-butyl}-3-(pyridin-3-yl)-acrylamide ;
le N-{4-[1-(6,11-dihydrodibenzo[b,e]thiépin-11-yl)-pipéridin-4-yl]-butyl}-3-(pyridin-3-yl)-acrylamide ;
le N-[7-(1-diphénylméthylpipéridin-4-yl)-heptyl]-3-(pyridin-3-yl)-acrylamide ;
le N-[8-(1-diphénylméthylpipéridin-4-yl)-octyl]-3-(pyridin-3-yl)-acrylamide ;
le N-[3-(1-diphénylméthylpipéridin-4-yloxy)-propyl]-3-(pyridin-3-yl)-acrylamide ;
le N-[3-(1-benzylpipéridin-4-yloxy)-propyl]-3-(pyridin-3-yl)-acrylamide ;
l'amide d'acide N-[2-(1-diphénylméthylpipéridin-4-yl)-éthyl]-5-(pyridin-3-yl)-2,4-pentadiénique ;
l'amide d'acide N-[4-(1-diphénylméthylpipéridin-4-yl)-butyl]-5-(pyridin-3-yl)-2,4-pentadiénique ;
l'amide d'acide N-[5-(1-diphénylméthylpipéridin-4-yl)-pentyl]-5-(pyridin-3-yl)-2,4-pentadiénique ;
l'amide d'acide N-[6-(1-diphénylméthylpipéridin-4-yl)-hexyl]-5-(pyridin-3-yl)-2,4-pentadiénique ;
le N-[4-(4-diphénylméthylpipéridin-1-yl)-3-hydroxy-butyl]-3-(pyridin-3-yl)-acrylamide ;
le N-[3-(4-diphénylméthylpipéridin-1-yl)-propoxy]-3-(pyridin-3-yl)-acrylamide ;
le N-[4-(4-diphénylméthylpipéridin-1-yl)-4-oxo-butyl]-3-(pyridin-3-yl)-acrylamide ;
le N-[3-(4-diphénylméthylpipéridin-1-sulfonyl)-propyl]-3-(pyridin-3-yl)-acrylamide ;
le N-{2-[2-(4-diphénylméthylpipéridin-1-yl)-éthoxy]-éthyl}-3-(pyridin-3-yl)-acrylamide ;
le N-(4-{4-[bis(4-fluorophényl)-méthyl]-pipéridin-1-yl}-but-2-inyl)-3-pyridin-3-yl-acrylamide ;
le N-{4-[4-(4-carboxyphénylphénylméthyl)-pipéridin-1-yl)-butyl}-3-pyridin-3-yl-acrylamide ;
le N-(4-{4-[(4-aminophényl)-phénylméthyl]-pipéridin-1-yl}-butyl)-3-pyridin-3-yl-acrylamide ;
le N-{4-[4-(9H-fluorén-9-yl)-pipéridin-1-yl]-butyl}-2-pyridin-3-yloxy)-acrylamide ;
le N-{5-[4-(9H-fluorén-9-yl)-pipéridin-1-yl]-pentyl}-2-pyridin-3-yloxy)-acrylamide ;
le N-{6-[4-(9H-fluorén-9-yl)-pipéridin-1-yl]-hexyl}-2-pyridin-3-yloxy)-acrylamide ;
le 3-pyridin-3-yl-N-{4-[4-(1,2,3,4-tétrahydronaphtalèn-1-yl)-pipéridin-1-yl]-butyl}-acrylamide ;
le 3-pyridin-3-yl-N-{4-[4-(5,6,7,8-tétrahydronaphtalèn-1-yl)-pipéridin-1-yl]-butyl}-acrylamide ;
le N-{4-[4-(naphtalèn-1-yl)-pipéridin-1-yl)-butyl}-3-pyridin-3-yl-acrylamide ;
le N-[4-(4-biphényl-2-yl-pipérazin-1-yl)-butyl]-3-pyridin-3-yl-propionamide ;
le N- [5-(4-biphényl-2-yl-pipérazin-1-yl)-pentyl]-3-pyridin-3-yl-acrylamide ;
le N-[6-(4-biphényl-2-yl-pipérazin-1-yl)-hexyl]-3-pyridin-3-yl-acrylamide ;
le N-[4-(4-biphényl-2-yl-pipérazin-1-yl)-butyl]-2-(pyridin-3-yloxy)-acétamide ;
l'amide d'acide N-[4-(4-biphényl-2-yl-pipérazin-1-yl)-butyl]-5-(pyridin-3-yl)-penta-2,4-diénique ;
le N-{4-[4-(10,11-dihydro-5H-dibenzo[a,d]cycloheptén-5-yl)-pipéridin-1-yl]-butyl}-3-pyridin-3-ylpropionamide ;
le N-{5-[4-(10,11-dihydro-5H-dibenzo[a,d]cycloheptén-5-yl)-pipéridin-1-yl]-pentyl}-3-pyridin-3-yl-acrylamide ;
le N-{6-[4-(10,11-dihydro-5H-dibenzo[a,d]cycloheptén-5-yl)-pipéridin-1-yl]-hexyl}-3-pyridin-3-yl-acrylamide ;
l'amide d'acide N-{4-[4-(10,11-dihydro-5H-dibenzo[a,d]-cycloheptén-5-yl)-pipéridin-1-yl]-butyl}-5-(pyridin-3-yl)-penta-2,4-diénique ;
le N-{4-[4-(6,11-dihydrodibenzo[b,e]oxépin-11-yl)-pipéridin-l-yl]-butyl}-3-pyridin-3-yl-propionamide ;
le N-{2-[4-(6,11-dihydrodibenzo[b,e]thiépin-11-yl)-pipéridin-1-yl]-éthyl}-3-pyridin-3-yl-acrylamide ;
le N-[4-(4-diphénylacétylpipérazin-1-yl)-butyl]-3-pyridin-3-yl-acrylamide ;
le N-[4-(4-benzoylpipérazin-1-yl)-butyl]-3-pyridin-3-yl-acrylamide ;
le N-{4-[4-(2-aminobenzoyl)-pipéridin-1-yl]-butyl}-3-pyridin-3-yl-acrylamide ;
le N-{4-[4-(4-carboxybenzoyl)-pipéridin-1-yl]-butyl}-3-pyridin-3-yl-acrylamide ;
le N-{4-[4-(biphényl-2-carbonyl)-pipéridin-1-yl]-butyl}-3-pyridin-3-yl-acrylamide ;
le N-{4-[4-(9-oxo-9H-fluorén-4-carbonyl)-pipéridin-1-yl]-butyl}-3-pyridin-3-yl-acrylamide ;
le N-{4-[4-(furan-2-carbonyl)-pipéridin-1-yl]-butyl}-3-pyridin-3-yl-acrylamide ;
le N-{4-[4-(naphtalèn-1-ylaminocarbonyl)-pipéridin-1-yl]-butyl}-3-pyridin-3-yl-propionamide ;
le N-{4-[4-(diphénylaminocarbonyl)-pipéridin-1-yl]-butyl}-3-pyridin-3-yl-acrylamide ;
le N-{4-[4-(naphtalèn-2-sulfonyl)-pipéridin-1-yl]-butyl}-3-pyridin-3-yl-acrylamide ;
le N-[4-(4-diphénylphosphinoyl-pipérazin-1-yl)-butyl]-3-pyridin-3-yl-acrylamide ;
le N-[4-(4-biphényl-2-yl-pipérazin-1-yl)-butyl]-3-pyridin-3-yl-acrylamide ;
le N-{4-[4-(9H-fluorén-9-yl)-pipéridin-1-yl]-butyl}-3-pyridin-3-yl-acrylamide ;
le N-{4-[4-(10,11-dihydro-5H-dibenzo[a,d]cycloheptén-5-yl)-pipéridin-1-yl]-butyl}-3-pyridin-3-yl-acrylamide ;
le N-[4-(4-phénylpipérazin-1-yl)-butyl]-3-pyridin-3-yl-acrylamide ;
le N-{4-[4-(1H-indol-3-yl)-pipéridin-1-yl]-butyl}-3-pyridin-3-yl-acrylamide ;
le N-{4-[4-(2-oxo-2,3-dihydrobenzimidazol-1-yl)-pipéridin-1-yl]-butyl}-3-pyridin-3-yl-acrylamide ;
le N-[-4-(4-benzotriazol-1-yl-pipérazin-1-yl)-butyl]-3-pyridin-3-yl-acrylamide ;
le N-{4-[4-(hydroxydiphénylméthyl)-pipéridin-1-yl]-butyl}-2-(pyridin-3-yloxy)-acétamide ;
le N-[4-(4,4-diphénylpipérazin-1-yl)-butyl]-3-pyridin-3-yl-acrylamide ;
le semi-isopropanol de dichlorhydrate de N-{4-[4-(6,11-dihydrodibenzo[b,e]thiépin-11-ylidèn)-pipéridin-1-yl]-butyl}-3-pyridin-3-yl-propionamide ;
le N-{4-[4-(6,11-dihydrodibenzo[b,e]thiépin-11-ylidèn)-pipéridin-1-yl]-butyl}-5-pyridin-3-yl-pentanamide ;
le N-{4-[4-(4,9-dihydro-thiéno[2,3-b]-benzo[b,e]-thiépin-4-ylidèn)-pipéridin-1-yl]-butyl}-3-pyridin-3-yl-propionamide ;
le N-{4-[4-(4,9-dihydro-thiéno[2,3-b]-benzo[b,e]-thiépin-4-ylidèn)-pipéridin-1-yl]-butyl}-3-pyridin-3-yl-acrylamide ;
le N-[4-(4-diphénylphosphinoyloxypipérazin-1-yl)-butyl]-3-pyridin-3-yl-acrylamide ;
le N-[4-(1,4-dioxa-8-azaspiro[4,5]déc-8-yl)-butyl]-3-pyridin-3-yl-acrylamide ;
le N-[4-(2,5-dioxo-3,4-diphényl-2,5-dihydropyrrol-1-yl)-butyl]-3-pyridin-3-yl-acrylamide ;
le N-[4-(2,6-dioxo-4-phényl-pipéridin-1-yl)-butyl]-3-pyridin-3-yl-acrylamide ;
le N-[4-(1,3-dioxo-4,5,6,7-tétraphényl-1,3-dihydroisoindol-2-yl)-butyl]-3-pyridin-3-ylacrylamide ;
le N-[4-(3-benzyl-2,4,5-trioxo-imidazolidin-1-yl)-butyl]-3-pyridin-3-yl-acrylamide ;
le N-[4-(1,3,10-trioxo-1,4,5,6,10,10a-hexahydroacénaphto[1,8a-c]pyrrol-2-yl)-butyl]-3-pyridin-3-ylacrylamide ;
le N-[4-(2,5-dioxo-4,4-diphénylimidazolidin-1-yl)-butyl]-3-pyridin-3-yl-acrylamide ;
le N-[4-(2,5-dioxo-3-phényl-2,5-dihydropyrrol-1-yl)-butyl]-3-pyridin-3-yl-acrylamide ;
le N-[3-(2,5-dioxo-3,4-diphényl-2,5-dihydropyrrol-1-yl)-propyl]-3-pyridin-3-yl-acrylamide ;
N-[4-(3-pyridin-3-yl-acroylamino)-butyl]-2,3:5,6-dibenzobicyclo[2.2.2]octan-7,8-dicarboximide ;
le N-[4-(5-benzylidèn-2,4-dioxothiazolidin-3-yl)-butyl]-3-pyridin-3-yl-acrylamide ;
le N-[4-(4-benzyl-2,6-dioxopipérazin-1-yl)-butyl]-3-pyridin-3-yl-acrylamide ;
le N-[6-(2,5-dioxo-3,4-diphényl-2,5-dihydropyrrol-1-yl)-hexyl]-3-pyridin-3-yl-acrylamide ;
le N-[4-(2,5-dioxo-3,4-diphényl-2,5-dihydropyrrol-1-yl)-butyl]-3-pyridin-3-yl-propionamide ;
le N-[4-(1,3-dioxo-1,3-dihydroisoindol-2-yl)-butyl]-3-pyridin-3-yl-acrylamide ;
le N-[4-(1,3-dioxo-1H,3H-benzo[de]isoquinolin-2-yl)-butyl]-3-(1-oxidopyridin-3-yl-acrylamide ;
le N-[6-(1,3-dioxo-1H,3H-benzo[de]isoquinolin-2-yl)-hexyl]-3-pyridin-3-yl-acrylamide ;
le N-[2-(1,3-dioxo-1H,3H-benzo[de]isoquinolin-2-yl)-éthyl]-3-pyridin-3-yl-acrylamide ;
le N-[4-(1,3-dioxo-1H,3H-benzo[de]isoquinolin-2-yl)-butyl]-3-pyridin-3-yl-acrylamide ;
le chlorhydrate de N- [8,8-bis(4-fluorophényl)octyl]-3-pyridin-3-yl-acrylamide ;
le N-[6-(3,3-diphényluréido)hexyl]-3-pyridin-3-yl-acrylamide ;
le N-[4-(1-phényl-1,2,3,4-tétrahydrobenzo[d]azépin-3-yl)-butyl]-3-pyridin-3-yl-acrylamide ;
le N-(8,8-diphényloctyl)-3-pyridin-3-yl-acrylamide ;
le N-(8-hydroxy-8,8-diphényloctyl)-3-pyridin-3-yl-acrylamide ;
le N-[4-(3,3-diphényluréido)butyl]-3-pyridin-3-yl-acrylamide ;
le N-[4-[1-(1H,3H-benzo[de]isoquinolin-2-yl)-butyl]-3-pyridin-3-yl-acrylamide ;
le N-[6-(10,11-dihydrodibenzo[b,f]azépin-5-yl-carbonylamino)-hexyl]-3-pyridin-3-yl-acrylamide ;
le 3-pyridin-3-yl-N-[6-(tosylamino)-hexyl]-acrylamide ;
le N-[4-(1,1-dioxo-1-thia-2-aza-acénaphtylèn-2-yl)-butyl]-3-pyridin-3-yl-acrylamide ;
le N-(6-hydroxy-6,6-diphénylhexyl)-3-pyridin-3-yl-acrylamide ;
le N-(6,6-diphényl-hex-5-ényl)-3-pyridin-3-yl-acrylamide ;
le N-[4-(4,5-diphénylimidazol-1-yl)-butyl]-3-pyridin-3-yl-acrylamide ;
le N-[4-(trans-2-phénylcyclopropylcarbonylamino)-butyl]-3-pyridin-3-yl-acrylamide ;
le N-(5-hydroxy-5,5-diphénylpentyl)-3-pyridin-3-yl-acrylamide ;
le N-(7-phénylheptyl)-3-pyridin-3-yl-acrylamide ;
le N-(4-diphénylacétylamino-butyl]-3-pyridin-3-yl-acrylamide ;
le N-[4-(benzhydrylamino)-butyl]-3-pyridin-3-yl-acrylamide ; et
le N-(4-{[2-(benzhydrylméthylamino)-éthyl]méthylamino}-butyl)-3-pyridin-3-yl-acrylamide.

17. Utilisation selon la revendication 15 ou 16 dans laquelle, en plus de l'agent cancérostatique ou immunosuppresseur administré à titre prophylactique ou thérapeutique de formule (I), un autre agent cancérostatique ou immunosuppresseur différent de celui-ci est administré.
